(19) Europäisches Patentamt / European Patent Office / Office européen des brevets

(11) **EP 4 566 673 A1**

(12) **EUROPEAN PATENT APPLICATION**
published in accordance with Art. 153(4) EPC

(43) Date of publication:
**11.06.2025  Bulletin 2025/24**

(21) Application number: **23849519.6**

(22) Date of filing: **04.08.2023**

(51) International Patent Classification (IPC):
*A61P 35/00* (2006.01)    *C07K 16/00* (2006.01)
*C07K 16/28* (2006.01)    *C12N 15/13* (2006.01)
*C07K 16/40* (2006.01)

(52) Cooperative Patent Classification (CPC):
**A61P 35/00; C07K 16/00; C07K 16/28; C07K 16/40**

(86) International application number:
**PCT/CN2023/111149**

(87) International publication number:
**WO 2024/027815 (08.02.2024 Gazette 2024/06)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority: 05.08.2022  CN 202210937340
16.05.2023  CN 202310549681

(71) Applicants:
• **Jiangsu Hengrui Pharmaceuticals Co., Ltd.
Lianyungang, Jiangsu 222047 (CN)**
• **Shanghai Hengrui Pharmaceutical Co., Ltd.
Shanghai 200245 (CN)**

(72) Inventors:
• **YE, Xin
Shanghai 200245 (CN)**

• **CHEN, Yuxiao
Shanghai 200245 (CN)**
• **AMINA, Abula
Shanghai 200245 (CN)**
• **YING, Hua
Shanghai 200245 (CN)**
• **TAO, Weikang
Shanghai 200245 (CN)**

(74) Representative: **Dragotti & Associati S.R.L.
Via Nino Bixio, 7
20129 Milano (IT)**

Remarks:
The complete document including Reference Table(s) and the Sequence Listing(s) can be downloaded from the EPO website

(54) **ANTIGEN-BINDING MOLECULE SPECIFICALLY BINDING TO GUCY2C AND CD3 AND PHARMACEUTICAL USE THEREOF**

(57)    Provided are an antigen-binding molecule specifically binding to GUCY2C and CD3 and pharmaceutical use thereof. The antigen-binding molecule can be used for treating tumor.

EP 4 566 673 A1

**Description**

**TECHNICAL FIELD**

The present disclosure relates to the field of biotechnology, and more specifically, the present disclosure relates to an antigen-binding molecule that specifically binds to GUCY2C and CD3 and medical use thereof.

**BACKGROUND**

The statements herein merely provide background information related to the present disclosure and may not necessarily constitute the prior art.

GUCY2C (Guanylyl Cyclase C) belongs to the family of transmembrane guanylate cyclases. It consists of an extracellular domain, a single transmembrane domain, and an intracellular catalytic domain and is anchored to the cell membrane as a homodimer or trimer. Its main function is to catalyze GTP to form cGMP so as to maintain normal physiological functions such as intestinal homeostasis (Michaela Kuhn, Physiological Reviews: (2016), 96(2):751-804). In normal tissues, GUCY2C is expressed only in intestinal epithelial cells, with no or very low expression in other tissues. Meanwhile, GUCY2C is expressed in gastrointestinal malignancies, including in no less than 90% of colorectal cancer cases at various stages and no less than 50% of gastric or gastroesophageal junction cancer cases (Ruth Birbe MD., Human Pathology, (2005), 36(2):170-179; Hadi Danaee, PLoS One, (2017), 12(12): e0189953). In addition, because the expression polarity at the cellular level and the enrichment degree of blood vessels in normal intestinal tissues are significantly different from those in intestinal cancer, the probability of GUCY2C in the intestinal cancers contacting the blood vessels is higher than that in the normal tissues (Divya Mathur, Clinical Cancer Research, (2020),26(9): 2188-2202). Therefore, GUCY2C is a specific and highly potential target for gastrointestinal cancer, especially intestinal cancer.

The bispecific antibody targeting CD3 and TAA is a novel immunotherapy, and it can bind to T cells and tumor cells simultaneously and simulate the interaction between MHC and TCR, leading to the formation of a lytic synapse in T cells, which then release perforin and granzymes to specifically kill tumor cells. Activated T cells can release cytokines, initiate other immune cells, and expand immune responses against tumors, ultimately leading to T cell proliferation and a cascade for killing tumor cells.

**SUMMARY**

The present disclosure provides an antigen-binding molecule that specifically binds to GUCY2C and CD3 and an antibody that specifically binds to GUCY2C.

In one aspect, the present disclosure provides an antigen-binding molecule that specifically binds to GUCY2C and CD3. The antigen-binding molecule comprises at least one antigen-binding moiety that specifically binds to GUCY2C and at least one antigen-binding moiety that specifically binds to CD3, wherein the antigen-binding moiety that specifically binds to GUCY2C comprises a heavy chain variable region GUCY2C-VH and a light chain variable region GUCY2C-VL, and the antigen-binding moiety that specifically binds to CD3 comprises a heavy chain variable region CD3-VH and a light chain variable region CD3-VL.

In some embodiments, provided is the antigen-binding molecule according to the above, wherein:

(i) GUCY2C-HCDR1, GUCY2C-HCDR2, and GUCY2C-HCDR3 in the GUCY2C-VH comprise the amino acid sequences of GUCY2C-HCDR1, GUCY2C-HCDR2, and GUCY2C-HCDR3 in SEQ ID NO: 25, respectively, and GUCY2C-LCDR1, GUCY2C-LCDR2, and GUCY2C-LCDR3 in the GUCY2C-VL comprise the amino acid sequences of GUCY2C-LCDR1, GUCY2C-LCDR2, and GUCY2C-LCDR3 in SEQ ID NO: 54, 26, 50, 51, 52, or 53, respectively; or

(ii) GUCY2C-HCDR1, GUCY2C-HCDR2, and GUCY2C-HCDR3 in the GUCY2C-VH comprise the amino acid sequences of GUCY2C-HCDR1, GUCY2C-HCDR2, and GUCY2C-HCDR3 in SEQ ID NO: 37 or 15, respectively, and GUCY2C-LCDR1, GUCY2C-LCDR2, and GUCY2C-LCDR3 in the GUCY2C-VL comprise the amino acid sequences of GUCY2C-LCDR1, GUCY2C-LCDR2, and GUCY2C-LCDR3 in SEQ ID NO: 16, 34, 35, or 36, respectively; or

(iii) GUCY2C-HCDR1, GUCY2C-HCDR2, and GUCY2C-HCDR3 in the GUCY2C-VH comprise the amino acid sequences of GUCY2C-HCDR1, GUCY2C-HCDR2, and GUCY2C-HCDR3 in SEQ ID NO: 42 or 17, respectively, and GUCY2C-LCDR1, GUCY2C-LCDR2, and GUCY2C-LCDR3 in the GUCY2C-VL comprise the amino acid sequences of GUCY2C-LCDR1, GUCY2C-LCDR2, and GUCY2C-LCDR3 in SEQ ID NO: 41 or 18, respectively; or

iv-1) GUCY2C-HCDR1, GUCY2C-HCDR2, and GUCY2C-HCDR3 in the GUCY2C-VH comprise the amino acid sequences of GUCY2C-HCDR1, GUCY2C-HCDR2, and GUCY2C-HCDR3 in SEQ ID NO: 273, respectively, and GUCY2C-LCDR1, GUCY2C-LCDR2, and GUCY2C-LCDR3 in the GUCY2C-VL comprise the amino acid sequences of GUCY2C-LCDR1, GUCY2C-LCDR2, and GUCY2C-LCDR3 in SEQ ID NO: 274, respectively; or

iv-2) GUCY2C-HCDR1, GUCY2C-HCDR2, and GUCY2C-HCDR3 in the GUCY2C-VH comprise the amino acid sequences of GUCY2C-HCDR1, GUCY2C-HCDR2, and GUCY2C-HCDR3 in SEQ ID NO: 275, respectively, and GUCY2C-LCDR1, GUCY2C-LCDR2, and GUCY2C-LCDR3 in the GUCY2C-VL comprise the amino acid sequences of GUCY2C-LCDR1, GUCY2C-LCDR2, and GUCY2C-LCDR3 in SEQ ID NO: 276, respectively; or

iv-3) GUCY2C-HCDR1, GUCY2C-HCDR2, and GUCY2C-HCDR3 in the GUCY2C-VH comprise the amino acid sequences of GUCY2C-HCDR1, GUCY2C-HCDR2, and GUCY2C-HCDR3 in SEQ ID NO: 277, respectively, and GUCY2C-LCDR1, GUCY2C-LCDR2, and GUCY2C-LCDR3 in the GUCY2C-VL comprise the amino acid sequences of GUCY2C-LCDR1, GUCY2C-LCDR2, and GUCY2C-LCDR3 in SEQ ID NO: 278, respectively; or

iv-4) GUCY2C-HCDR1, GUCY2C-HCDR2, and GUCY2C-HCDR3 in the GUCY2C-VH comprise the amino acid sequences of GUCY2C-HCDR1, GUCY2C-HCDR2, and GUCY2C-HCDR3 in SEQ ID NO: 279, respectively, and GUCY2C-LCDR1, GUCY2C-LCDR2, and GUCY2C-LCDR3 in the GUCY2C-VL comprise the amino acid sequences of GUCY2C-LCDR1, GUCY2C-LCDR2, and GUCY2C-LCDR3 in SEQ ID NO: 280, respectively; or

iv-5) GUCY2C-HCDR1, GUCY2C-HCDR2, and GUCY2C-HCDR3 in the GUCY2C-VH comprise the amino acid sequences of GUCY2C-HCDR1, GUCY2C-HCDR2, and GUCY2C-HCDR3 in SEQ ID NO: 330, respectively, and GUCY2C-LCDR1, GUCY2C-LCDR2, and GUCY2C-LCDR3 in the GUCY2C-VL comprise the amino acid sequences of GUCY2C-LCDR1, GUCY2C-LCDR2, and GUCY2C-LCDR3 in SEQ ID NO: 331, respectively; or

iv-6) GUCY2C-HCDR1, GUCY2C-HCDR2, and GUCY2C-HCDR3 in the GUCY2C-VH comprise the amino acid sequences of GUCY2C-HCDR1, GUCY2C-HCDR2, and GUCY2C-HCDR3 in SEQ ID NO: 332, respectively, and GUCY2C-LCDR1, GUCY2C-LCDR2, and GUCY2C-LCDR3 in the GUCY2C-VL comprise the amino acid sequences of GUCY2C-LCDR1, GUCY2C-LCDR2, and GUCY2C-LCDR3 in SEQ ID NO: 333, respectively; or

iv-7) GUCY2C-HCDR1, GUCY2C-HCDR2, and GUCY2C-HCDR3 in the GUCY2C-VH comprise the amino acid sequences of GUCY2C-HCDR1, GUCY2C-HCDR2, and GUCY2C-HCDR3 in SEQ ID NO: 334, respectively, and GUCY2C-LCDR1, GUCY2C-LCDR2, and GUCY2C-LCDR3 in the GUCY2C-VL comprise the amino acid sequences of GUCY2C-LCDR1, GUCY2C-LCDR2, and GUCY2C-LCDR3 in SEQ ID NO: 335, respectively; or

iv-8) GUCY2C-HCDR1, GUCY2C-HCDR2, and GUCY2C-HCDR3 in the GUCY2C-VH comprise the amino acid sequences of GUCY2C-HCDR1, GUCY2C-HCDR2, and GUCY2C-HCDR3 in SEQ ID NO: 336, respectively, and GUCY2C-LCDR1, GUCY2C-LCDR2, and GUCY2C-LCDR3 in the GUCY2C-VL comprise the amino acid sequences of GUCY2C-LCDR1, GUCY2C-LCDR2, and GUCY2C-LCDR3 in SEQ ID NO: 337, respectively; or

iv-9) GUCY2C-HCDR1, GUCY2C-HCDR2, and GUCY2C-HCDR3 in the GUCY2C-VH comprise the amino acid sequences of GUCY2C-HCDR1, GUCY2C-HCDR2, and GUCY2C-HCDR3 in SEQ ID NO: 338, respectively, and GUCY2C-LCDR1, GUCY2C-LCDR2, and GUCY2C-LCDR3 in the GUCY2C-VL comprise the amino acid sequences of GUCY2C-LCDR1, GUCY2C-LCDR2, and GUCY2C-LCDR3 in SEQ ID NO: 339, respectively; or

iv-10) GUCY2C-HCDR1, GUCY2C-HCDR2, and GUCY2C-HCDR3 in the GUCY2C-VH comprise the amino acid sequences of GUCY2C-HCDR1, GUCY2C-HCDR2, and GUCY2C-HCDR3 in SEQ ID NO: 340, respectively, and GUCY2C-LCDR1, GUCY2C-LCDR2, and GUCY2C-LCDR3 in the GUCY2C-VL comprise the amino acid sequences of GUCY2C-LCDR1, GUCY2C-LCDR2, and GUCY2C-LCDR3 in SEQ ID NO: 341, respectively; or

iv-11) GUCY2C-HCDR1, GUCY2C-HCDR2, and GUCY2C-HCDR3 in the GUCY2C-VH comprise the amino acid sequences of GUCY2C-HCDR1, GUCY2C-HCDR2, and GUCY2C-HCDR3 in SEQ ID NO: 342, respectively, and GUCY2C-LCDR1, GUCY2C-LCDR2, and GUCY2C-LCDR3 in the GUCY2C-VL comprise the amino acid sequences of GUCY2C-LCDR1, GUCY2C-LCDR2, and GUCY2C-LCDR3 in SEQ ID NO: 343, respectively; or

iv-12) GUCY2C-HCDR1, GUCY2C-HCDR2, and GUCY2C-HCDR3 in the GUCY2C-VH comprise the amino acid sequences of GUCY2C-HCDR1, GUCY2C-HCDR2, and GUCY2C-HCDR3 in SEQ ID NO: 344, respectively, and GUCY2C-LCDR1, GUCY2C-LCDR2, and GUCY2C-LCDR3 in the GUCY2C-VL comprise the amino acid sequences of GUCY2C-LCDR1, GUCY2C-LCDR2, and GUCY2C-LCDR3 in SEQ ID NO: 345, respectively; or

iv-13) GUCY2C-HCDR1, GUCY2C-HCDR2, and GUCY2C-HCDR3 in the GUCY2C-VH comprise the amino acid sequences of GUCY2C-HCDR1, GUCY2C-HCDR2, and GUCY2C-HCDR3 in SEQ ID NO: 346, respectively, and GUCY2C-LCDR1, GUCY2C-LCDR2, and GUCY2C-LCDR3 in the GUCY2C-VL comprise the amino acid sequences of GUCY2C-LCDR1, GUCY2C-LCDR2, and GUCY2C-LCDR3 in SEQ ID NO: 347, respectively; or

iv-14) GUCY2C-HCDR1, GUCY2C-HCDR2, and GUCY2C-HCDR3 in the GUCY2C-VH comprise the amino acid sequences of GUCY2C-HCDR1, GUCY2C-HCDR2, and GUCY2C-HCDR3 in SEQ ID NO: 348, respectively, and GUCY2C-LCDR1, GUCY2C-LCDR2, and GUCY2C-LCDR3 in the GUCY2C-VL comprise the amino acid sequences of GUCY2C-LCDR1, GUCY2C-LCDR2, and GUCY2C-LCDR3 in SEQ ID NO: 349, respectively; or

iv-15) GUCY2C-HCDR1, GUCY2C-HCDR2, and GUCY2C-HCDR3 in the GUCY2C-VH comprise the amino acid sequences of GUCY2C-HCDR1, GUCY2C-HCDR2, and GUCY2C-HCDR3 in SEQ ID NO: 350, respectively, and GUCY2C-LCDR1, GUCY2C-LCDR2, and GUCY2C-LCDR3 in the GUCY2C-VL comprise the amino acid sequences of GUCY2C-LCDR1, GUCY2C-LCDR2, and GUCY2C-LCDR3 in SEQ ID NO: 351, respectively; or

iv-16) GUCY2C-HCDR1, GUCY2C-HCDR2, and GUCY2C-HCDR3 in the GUCY2C-VH comprise the amino acid sequences of GUCY2C-HCDR1, GUCY2C-HCDR2, and GUCY2C-HCDR3 in SEQ ID NO: 352, respectively, and GUCY2C-LCDR1, GUCY2C-LCDR2, and GUCY2C-LCDR3 in the GUCY2C-VL comprise the amino acid sequences of GUCY2C-LCDR1, GUCY2C-LCDR2, and GUCY2C-LCDR3 in SEQ ID NO: 353, respectively.

In some embodiments, provided is the antigen-binding molecule according to the above, wherein:

(i) GUCY2C-HCDR1, GUCY2C-HCDR2, and GUCY2C-HCDR3 in the GUCY2C-VH comprise the amino acid sequences of GUCY2C-HCDR1, GUCY2C-HCDR2, and GUCY2C-HCDR3 in SEQ ID NO: 25, respectively, and GUCY2C-LCDR1, GUCY2C-LCDR2, and GUCY2C-LCDR3 in the GUCY2C-VL comprise the amino acid sequences of GUCY2C-LCDR1, GUCY2C-LCDR2, and GUCY2C-LCDR3 in SEQ ID NO: 54, respectively, or
(ii) GUCY2C-HCDR1, GUCY2C-HCDR2, and GUCY2C-HCDR3 in the GUCY2C-VH comprise the amino acid sequences of GUCY2C-HCDR1, GUCY2C-HCDR2, and GUCY2C-HCDR3 in SEQ ID NO: 37, respectively, and GUCY2C-LCDR1, GUCY2C-LCDR2, and GUCY2C-LCDR3 in the GUCY2C-VL comprise the amino acid sequences of GUCY2C-LCDR1, GUCY2C-LCDR2, and GUCY2C-LCDR3 in SEQ ID NO: 16, respectively, or
(iii) GUCY2C-HCDR1, GUCY2C-HCDR2, and GUCY2C-HCDR3 in the GUCY2C-VH comprise the amino acid sequences of GUCY2C-HCDR1, GUCY2C-HCDR2, and GUCY2C-HCDR3 in SEQ ID NO: 42, respectively, and GUCY2C-LCDR1, GUCY2C-LCDR2, and GUCY2C-LCDR3 in the GUCY2C-VL comprise the amino acid sequences of GUCY2C-LCDR1, GUCY2C-LCDR2, and GUCY2C-LCDR3 in SEQ ID NO: 41, respectively.

In some embodiments, the GUCY2C-HCDR1, the GUCY2C-HCDR2, the GUCY2C-HCDR3, the GUCY2C-LCDR1, the GUCY2C-LCDR2, and the GUCY2C-LCDR3 are defined according to the Kabat, IMGT, Chothia, AbM, or Contact numbering scheme. In some embodiments, provided is the antigen-binding molecule according to any one of the above, wherein:

i) the GUCY2C-VH comprises: a GUCY2C-HCDR1 comprising the amino acid sequence of SEQ ID NO: 19, a GUCY2C-HCDR2 comprising the amino acid sequence of SEQ ID NO: 20, and a GUCY2C-HCDR3 comprising the amino acid sequence of SEQ ID NO: 21; and the GUCY2C-VL comprises: a GUCY2C-LCDR1 comprising the amino acid sequence of SEQ ID NO: 47, 22, 43, 44, 45, or 46, a GUCY2C-LCDR2 comprising the amino acid sequence of SEQ ID NO: 23, and a GUCY2C-LCDR3 comprising the amino acid sequence of SEQ ID NO: 24; or
ii) the GUCY2C-VH comprises: a GUCY2C-HCDR1 comprising the amino acid sequence of SEQ ID NO: 4, a GUCY2C-HCDR2 comprising the amino acid sequence of SEQ ID NO: 5, and a GUCY2C-HCDR3 comprising the amino acid sequence of SEQ ID NO: 27 or 6; and the GUCY2C-VL comprises: a GUCY2C-LCDR1 comprising the amino acid sequence of SEQ ID NO: 7, a GUCY2C-LCDR2 comprising the amino acid sequence of SEQ ID NO: 8, 28, 29, or 30, and a GUCY2C-LCDR3 comprising the amino acid sequence of SEQ ID NO: 9; or
iii) the GUCY2C-VH comprises: a GUCY2C-HCDR1 comprising the amino acid sequence of SEQ ID NO: 10, a GUCY2C-HCDR2 comprising the amino acid sequence of SEQ ID NO: 11, and a GUCY2C-HCDR3 comprising the amino acid sequence of SEQ ID NO: 40 or 12; and the GUCY2C-VL comprises: a GUCY2C-LCDR1 comprising the amino acid sequence of SEQ ID NO: 13, a GUCY2C-LCDR2 comprising the amino acid sequence of SEQ ID NO: 39 or 8, and a GUCY2C-LCDR3 comprising the amino acid sequence of SEQ ID NO: 14; or
iv-1) the GUCY2C-VH comprises: a GUCY2C-HCDR1 comprising the amino acid sequence of SEQ ID NO: 250, a GUCY2C-HCDR2 comprising the amino acid sequence of SEQ ID NO: 251, and a GUCY2C-HCDR3 comprising the amino acid sequence of SEQ ID NO: 252; and the GUCY2C-VL comprises: a GUCY2C-LCDR1 comprising the amino acid sequence of SEQ ID NO: 253, a GUCY2C-LCDR2 comprising the amino acid sequence of SEQ ID NO: 254, and a GUCY2C-LCDR3 comprising the amino acid sequence of SEQ ID NO: 255; or
iv-2) the GUCY2C-VH comprises: a GUCY2C-HCDR1 comprising the amino acid sequence of SEQ ID NO: 256, a GUCY2C-HCDR2 comprising the amino acid sequence of SEQ ID NO: 257, and a GUCY2C-HCDR3 comprising the amino acid sequence of SEQ ID NO: 258; and the GUCY2C-VL comprises: a GUCY2C-LCDR1 comprising the amino acid sequence of SEQ ID NO: 259, a GUCY2C-LCDR2 comprising the amino acid sequence of SEQ ID NO: 8, and a GUCY2C-LCDR3 comprising the amino acid sequence of SEQ ID NO: 260; or
iv-3) the GUCY2C-VH comprises: a GUCY2C-HCDR1 comprising the amino acid sequence of SEQ ID NO: 261, a GUCY2C-HCDR2 comprising the amino acid sequence of SEQ ID NO: 262, and a GUCY2C-HCDR3 comprising the amino acid sequence of SEQ ID NO: 263; and the GUCY2C-VL comprises: a GUCY2C-LCDR1 comprising the amino acid sequence of SEQ ID NO: 264, a GUCY2C-LCDR2 comprising the amino acid sequence of SEQ ID NO: 265, and a GUCY2C-LCDR3 comprising the amino acid sequence of SEQ ID NO: 266; or
iv-4) the GUCY2C-VH comprises: a GUCY2C-HCDR1 comprising the amino acid sequence of SEQ ID NO: 267, a GUCY2C-HCDR2 comprising the amino acid sequence of SEQ ID NO: 268, and a GUCY2C-HCDR3 comprising the amino acid sequence of SEQ ID NO: 269; and the GUCY2C-VL comprises: a GUCY2C-LCDR1 comprising the amino acid sequence of SEQ ID NO: 270, a GUCY2C-LCDR2 comprising the amino acid sequence of SEQ ID NO: 271, and a GUCY2C-LCDR3 comprising the amino acid sequence of SEQ ID NO: 272; or

iv-5) the GUCY2C-VH comprises: a GUCY2C-HCDR1 comprising the amino acid sequence of SEQ ID NO: 281, a GUCY2C-HCDR2 comprising the amino acid sequence of SEQ ID NO: 282, and a GUCY2C-HCDR3 comprising the amino acid sequence of SEQ ID NO: 283; and the GUCY2C-VL comprises: a GUCY2C-LCDR1 comprising the amino acid sequence of SEQ ID NO: 284, a GUCY2C-LCDR2 comprising the amino acid sequence of SEQ ID NO: 8, and a GUCY2C-LCDR3 comprising the amino acid sequence of SEQ ID NO: 285; or

iv-6) the GUCY2C-VH comprises: a GUCY2C-HCDR1 comprising the amino acid sequence of SEQ ID NO: 286, a GUCY2C-HCDR2 comprising the amino acid sequence of SEQ ID NO: 287, and a GUCY2C-HCDR3 comprising the amino acid sequence of SEQ ID NO: 288; and the GUCY2C-VL comprises: a GUCY2C-LCDR1 comprising the amino acid sequence of SEQ ID NO: 289, a GUCY2C-LCDR2 comprising the amino acid sequence of SEQ ID NO: 265, and a GUCY2C-LCDR3 comprising the amino acid sequence of SEQ ID NO: 290; or

iv-7) the GUCY2C-VH comprises: a GUCY2C-HCDR1 comprising the amino acid sequence of SEQ ID NO: 291, a GUCY2C-HCDR2 comprising the amino acid sequence of SEQ ID NO: 292, and a GUCY2C-HCDR3 comprising the amino acid sequence of SEQ ID NO: 293; and the GUCY2C-VL comprises: a GUCY2C-LCDR1 comprising the amino acid sequence of SEQ ID NO: 294, a GUCY2C-LCDR2 comprising the amino acid sequence of SEQ ID NO: 295, and a GUCY2C-LCDR3 comprising the amino acid sequence of SEQ ID NO: 296; or

iv-8) the GUCY2C-VH comprises: a GUCY2C-HCDR1 comprising the amino acid sequence of SEQ ID NO: 261, a GUCY2C-HCDR2 comprising the amino acid sequence of SEQ ID NO: 297, and a GUCY2C-HCDR3 comprising the amino acid sequence of SEQ ID NO: 298; and the GUCY2C-VL comprises: a GUCY2C-LCDR1 comprising the amino acid sequence of SEQ ID NO: 264, a GUCY2C-LCDR2 comprising the amino acid sequence of SEQ ID NO: 299, and a GUCY2C-LCDR3 comprising the amino acid sequence of SEQ ID NO: 300; or

iv-9) the GUCY2C-VH comprises: a GUCY2C-HCDR1 comprising the amino acid sequence of SEQ ID NO: 261, a GUCY2C-HCDR2 comprising the amino acid sequence of SEQ ID NO: 301, and a GUCY2C-HCDR3 comprising the amino acid sequence of SEQ ID NO: 302; and the GUCY2C-VL comprises: a GUCY2C-LCDR1 comprising the amino acid sequence of SEQ ID NO: 303, a GUCY2C-LCDR2 comprising the amino acid sequence of SEQ ID NO: 299, and a GUCY2C-LCDR3 comprising the amino acid sequence of SEQ ID NO: 304; or

iv-10) the GUCY2C-VH comprises: a GUCY2C-HCDR1 comprising the amino acid sequence of SEQ ID NO: 305, a GUCY2C-HCDR2 comprising the amino acid sequence of SEQ ID NO: 306, and a GUCY2C-HCDR3 comprising the amino acid sequence of SEQ ID NO: 21; and the GUCY2C-VL comprises: a GUCY2C-LCDR1 comprising the amino acid sequence of SEQ ID NO: 22, a GUCY2C-LCDR2 comprising the amino acid sequence of SEQ ID NO: 23, and a GUCY2C-LCDR3 comprising the amino acid sequence of SEQ ID NO: 24; or

iv-11) the GUCY2C-VH comprises: a GUCY2C-HCDR1 comprising the amino acid sequence of SEQ ID NO: 128, a GUCY2C-HCDR2 comprising the amino acid sequence of SEQ ID NO: 307, and a GUCY2C-HCDR3 comprising the amino acid sequence of SEQ ID NO: 308; and the GUCY2C-VL comprises: a GUCY2C-LCDR1 comprising the amino acid sequence of SEQ ID NO: 309, a GUCY2C-LCDR2 comprising the amino acid sequence of SEQ ID NO: 310, and a GUCY2C-LCDR3 comprising the amino acid sequence of SEQ ID NO: 311; or

iv-12) the GUCY2C-VH comprises: a GUCY2C-HCDR1 comprising the amino acid sequence of SEQ ID NO: 312, a GUCY2C-HCDR2 comprising the amino acid sequence of SEQ ID NO: 313, and a GUCY2C-HCDR3 comprising the amino acid sequence of SEQ ID NO: 314; and the GUCY2C-VL comprises: a GUCY2C-LCDR1 comprising the amino acid sequence of SEQ ID NO: 22, a GUCY2C-LCDR2 comprising the amino acid sequence of SEQ ID NO: 23, and a GUCY2C-LCDR3 comprising the amino acid sequence of SEQ ID NO: 315; or

iv-13) the GUCY2C-VH comprises: a GUCY2C-HCDR1 comprising the amino acid sequence of SEQ ID NO: 286, a GUCY2C-HCDR2 comprising the amino acid sequence of SEQ ID NO: 287, and a GUCY2C-HCDR3 comprising the amino acid sequence of SEQ ID NO: 316; and the GUCY2C-VL comprises: a GUCY2C-LCDR1 comprising the amino acid sequence of SEQ ID NO: 317, a GUCY2C-LCDR2 comprising the amino acid sequence of SEQ ID NO: 265, and a GUCY2C-LCDR3 comprising the amino acid sequence of SEQ ID NO: 300; or

iv-14) the GUCY2C-VH comprises: a GUCY2C-HCDR1 comprising the amino acid sequence of SEQ ID NO: 318, a GUCY2C-HCDR2 comprising the amino acid sequence of SEQ ID NO: 319, and a GUCY2C-HCDR3 comprising the amino acid sequence of SEQ ID NO: 320; and the GUCY2C-VL comprises: a GUCY2C-LCDR1 comprising the amino acid sequence of SEQ ID NO: 321, a GUCY2C-LCDR2 comprising the amino acid sequence of SEQ ID NO: 322, and a GUCY2C-LCDR3 comprising the amino acid sequence of SEQ ID NO: 323; or

iv-15) the GUCY2C-VH comprises: a GUCY2C-HCDR1 comprising the amino acid sequence of SEQ ID NO: 324, a GUCY2C-HCDR2 comprising the amino acid sequence of SEQ ID NO: 325, and a GUCY2C-HCDR3 comprising the amino acid sequence of SEQ ID NO: 326; and the GUCY2C-VL comprises: a GUCY2C-LCDR1 comprising the amino acid sequence of SEQ ID NO: 22, a GUCY2C-LCDR2 comprising the amino acid sequence of SEQ ID NO: 23, and a GUCY2C-LCDR3 comprising the amino acid sequence of SEQ ID NO: 327; or

iv-16) the GUCY2C-VH comprises: a GUCY2C-HCDR1 comprising the amino acid sequence of SEQ ID NO: 261, a GUCY2C-HCDR2 comprising the amino acid sequence of SEQ ID NO: 328, and a GUCY2C-HCDR3 comprising the amino acid sequence of SEQ ID NO: 302; and the GUCY2C-VL comprises: a GUCY2C-LCDR1 comprising the amino acid sequence of SEQ ID NO: 264, a GUCY2C-LCDR2 comprising the amino acid sequence of SEQ ID NO: 329, and a GUCY2C-LCDR3 comprising the amino acid sequence of SEQ ID NO: 300.

In some embodiments, provided is the antigen-binding molecule according to any one of the above, wherein:

i) the GUCY2C-VH comprises: a GUCY2C-HCDR1 comprising the amino acid sequence of SEQ ID NO: 19, a GUCY2C-HCDR2 comprising the amino acid sequence of SEQ ID NO: 20, and a GUCY2C-HCDR3 comprising the amino acid sequence of SEQ ID NO: 21; and the GUCY2C-VL comprises: a GUCY2C-LCDR1 comprising the amino acid sequence of SEQ ID NO: 47, a GUCY2C-LCDR2 comprising the amino acid sequence of SEQ ID NO: 23, and a GUCY2C-LCDR3 comprising the amino acid sequence of SEQ ID NO: 24; or

ii) the GUCY2C-VH comprises: a GUCY2C-HCDR1 comprising the amino acid sequence of SEQ ID NO: 4, a GUCY2C-HCDR2 comprising the amino acid sequence of SEQ ID NO: 5, and a GUCY2C-HCDR3 comprising the amino acid sequence of SEQ ID NO: 27; and the GUCY2C-VL comprises: a GUCY2C-LCDR1 comprising the amino acid sequence of SEQ ID NO: 7, a GUCY2C-LCDR2 comprising the amino acid sequence of SEQ ID NO: 8, and a GUCY2C-LCDR3 comprising the amino acid sequence of SEQ ID NO: 9; or

iii) the GUCY2C-VH comprises: a GUCY2C-HCDR1 comprising the amino acid sequence of SEQ ID NO: 10, a GUCY2C-HCDR2 comprising the amino acid sequence of SEQ ID NO: 11, and a GUCY2C-HCDR3 comprising the amino acid sequence of SEQ ID NO: 40; and the GUCY2C-VL comprises: a GUCY2C-LCDR1 comprising the amino acid sequence of SEQ ID NO: 13, a GUCY2C-LCDR2 comprising the amino acid sequence of SEQ ID NO: 39, and a GUCY2C-LCDR3 comprising the amino acid sequence of SEQ ID NO: 14.

In some embodiments, provided is the antigen-binding molecule according to any one of the above, wherein:

i) the GUCY2C-VH comprises: a GUCY2C-HCDR1 comprising the amino acid sequence of SEQ ID NO: 19, a GUCY2C-HCDR2 comprising the amino acid sequence of SEQ ID NO: 20, and a GUCY2C-HCDR3 comprising the amino acid sequence of SEQ ID NO: 21; and the GUCY2C-VL comprises: a GUCY2C-LCDR1 comprising the amino acid sequence of SEQ ID NO: 22, a GUCY2C-LCDR2 comprising the amino acid sequence of SEQ ID NO: 23, and a GUCY2C-LCDR3 comprising the amino acid sequence of SEQ ID NO: 24; or

ii) the GUCY2C-VH comprises: a GUCY2C-HCDR1 comprising the amino acid sequence of SEQ ID NO: 4, a GUCY2C-HCDR2 comprising the amino acid sequence of SEQ ID NO: 5, and a GUCY2C-HCDR3 comprising the amino acid sequence of SEQ ID NO: 6; and the GUCY2C-VL comprises: a GUCY2C-LCDR1 comprising the amino acid sequence of SEQ ID NO: 7, a GUCY2C-LCDR2 comprising the amino acid sequence of SEQ ID NO: 8, and a GUCY2C-LCDR3 comprising the amino acid sequence of SEQ ID NO: 9; or

iii) the GUCY2C-VH comprises: a GUCY2C-HCDR1 comprising the amino acid sequence of SEQ ID NO: 10, a GUCY2C-HCDR2 comprising the amino acid sequence of SEQ ID NO: 11, and a GUCY2C-HCDR3 comprising the amino acid sequence of SEQ ID NO: 12; and the GUCY2C-VL comprises: a GUCY2C-LCDR1 comprising the amino acid sequence of SEQ ID NO: 13, a GUCY2C-LCDR2 comprising the amino acid sequence of SEQ ID NO: 8, and a GUCY2C-LCDR3 comprising the amino acid sequence of SEQ ID NO: 14.

In some embodiments, provided is the antigen-binding molecule according to any one of the above, wherein:

i) the GUCY2C-VH comprises: a GUCY2C-HCDR1 comprising the amino acid sequence of SEQ ID NO: 19, a GUCY2C-HCDR2 comprising the amino acid sequence of SEQ ID NO: 20, and a GUCY2C-HCDR3 comprising the amino acid sequence of SEQ ID NO: 21; and the GUCY2C-VL comprises: a GUCY2C-LCDR1 comprising the amino acid sequence of SEQ ID NO: 43, a GUCY2C-LCDR2 comprising the amino acid sequence of SEQ ID NO: 23, and a GUCY2C-LCDR3 comprising the amino acid sequence of SEQ ID NO: 24; or

the GUCY2C-VH comprises: a GUCY2C-HCDR1 comprising the amino acid sequence of SEQ ID NO: 19, a GUCY2C-HCDR2 comprising the amino acid sequence of SEQ ID NO: 20, and a GUCY2C-HCDR3 comprising the amino acid sequence of SEQ ID NO: 21; and the GUCY2C-VL comprises: a GUCY2C-LCDR1 comprising the amino acid sequence of SEQ ID NO: 44, a GUCY2C-LCDR2 comprising the amino acid sequence of SEQ ID NO: 23, and a GUCY2C-LCDR3 comprising the amino acid sequence of SEQ ID NO: 24; or

the GUCY2C-VH comprises: a GUCY2C-HCDR1 comprising the amino acid sequence of SEQ ID NO: 19, a GUCY2C-HCDR2 comprising the amino acid sequence of SEQ ID NO: 20, and a GUCY2C-HCDR3 comprising the amino acid sequence of SEQ ID NO: 21; and the GUCY2C-VL comprises: a GUCY2C-LCDR1 comprising the amino acid sequence of SEQ ID NO: 45, a GUCY2C-LCDR2 comprising the amino acid sequence of SEQ ID NO: 23, and a GUCY2C-LCDR3 comprising the amino acid sequence of SEQ ID NO: 24; or

ii) the GUCY2C-VH comprises: a GUCY2C-HCDR1 comprising the amino acid sequence of SEQ ID NO: 4, a GUCY2C-HCDR2 comprising the amino acid sequence of SEQ ID NO: 5, and a GUCY2C-HCDR3 comprising the amino acid sequence of SEQ ID NO: 6; and the GUCY2C-VL comprises: a GUCY2C-LCDR1 comprising the amino acid sequence of SEQ ID NO: 7, a GUCY2C-LCDR2 comprising the amino acid sequence of SEQ ID NO: 28, and a GUCY2C-LCDR3 comprising the amino acid sequence of SEQ ID NO: 9; or

the GUCY2C-VH comprises: a GUCY2C-HCDR1 comprising the amino acid sequence of SEQ ID NO: 4, a GUCY2C-HCDR2 comprising the amino acid sequence of SEQ ID NO: 5, and a GUCY2C-HCDR3 comprising the amino acid sequence of SEQ ID NO: 6; and the GUCY2C-VL comprises: a GUCY2C-LCDR1 comprising the amino acid sequence of SEQ ID NO: 7, a GUCY2C-LCDR2 comprising the amino acid sequence of SEQ ID NO: 30, and a GUCY2C-LCDR3 comprising the amino acid sequence of SEQ ID NO: 9.

In some embodiments, provided is the antigen-binding molecule according to any one of the above, wherein the GUCY2C-HCDR1, the GUCY2C-HCDR2, the GUCY2C-HCDR3, the GUCY2C-LCDR1, the GUCY2C-LCDR2, and the GUCY2C-LCDR3 are defined according to the Kabat numbering scheme.

In some embodiments, provided is the antigen-binding molecule according to any one of the above, wherein the GUCY2C-VH and the GUCY2C-VL are humanized and each comprise the FR region of a human antibody.

In some embodiments, provided is the antigen-binding molecule according to any one of the above, wherein:

(i) the GUCY2C-VH comprises: a GUCY2C-HCDR1 comprising the amino acid sequence of SEQ ID NO: 19, a GUCY2C-HCDR2 comprising the amino acid sequence of SEQ ID NO: 20, and a GUCY2C-HCDR3 comprising the amino acid sequence of SEQ ID NO: 21, and the FR region of the GUCY2C-VH comprises one or more amino acid substitutions selected from the group consisting of 1E, 39L, 43K, 69L, 71V, and 73K; and the GUCY2C-VL comprises: a GUCY2C-LCDR1 comprising the amino acid sequence of SEQ ID NO: 47, 22, 43, 44, 45, or 46, a GUCY2C-LCDR2 comprising the amino acid sequence of SEQ ID NO: 23, and a GUCY2C-LCDR3 comprising the amino acid sequence of SEQ ID NO: 24, and the FR region of the GUCY2C-VL comprises one or more amino acid substitutions selected from the group consisting of 2V, 3V, 68R, and 74K; or

(ii) the GUCY2C-VH comprises: a GUCY2C-HCDR1 comprising the amino acid sequence of SEQ ID NO: 4, a GUCY2C-HCDR2 comprising the amino acid sequence of SEQ ID NO: 5, and a GUCY2C-HCDR3 comprising the amino acid sequence of SEQ ID NO: 27 or 6, and the FR region of the GUCY2C-VH comprises one or more amino acid substitutions selected from the group consisting of 1E, 38K, 66K, and 67A; and the GUCY2C-VL comprises: a GUCY2C-LCDR1 comprising the amino acid sequence of SEQ ID NO: 7, a GUCY2C-LCDR2 comprising the amino acid sequence of SEQ ID NO: 8, 28, 29, or 30, and a GUCY2C-LCDR3 comprising the amino acid sequence of SEQ ID NO: 9, and the FR region of the GUCY2C-VL comprises one or more amino acid substitutions selected from the group consisting of 15P, 16G, 42S, 43S, 45K, 46S, 47W, 49Y, 58V, 71Y, and 76S; or

(iii) the GUCY2C-VH comprises: a GUCY2C-HCDR1 comprising the amino acid sequence of SEQ ID NO: 10, a GUCY2C-HCDR2 comprising the amino acid sequence of SEQ ID NO: 11, and a GUCY2C-HCDR3 comprising the amino acid sequence of SEQ ID NO: 40 or 12, and the FR region of the GUCY2C-VH comprises one or more amino acid substitutions selected from the group consisting of 1E, 2F, 28S, 69L, 71V, 73Q, and 75S; and the GUCY2C-VL comprises: a GUCY2C-LCDR1 comprising the amino acid sequence of SEQ ID NO: 13, a GUCY2C-LCDR2 comprising the amino acid sequence of SEQ ID NO: 39 or 8, and a GUCY2C-LCDR3 comprising the amino acid sequence of SEQ ID NO: 14, and the FR region of the GUCY2C-VL comprises one or more amino acid substitutions selected from the group consisting of 42S, 43S, 46P, 47W, 58V, and 71Y.

In some embodiments, provided is the antigen-binding molecule according to any one of the above, wherein:

(i) the GUCY2C-VH comprises: a GUCY2C-HCDR1 comprising the amino acid sequence of SEQ ID NO: 19, a GUCY2C-HCDR2 comprising the amino acid sequence of SEQ ID NO: 20, and a GUCY2C-HCDR3 comprising the amino acid sequence of SEQ ID NO: 21, and the FR region of the GUCY2C-VH comprises one or more amino acid substitutions selected from the group consisting of 1E, 39L, 43K, 69L, 71V, and 73K; and the GUCY2C-VL comprises: a GUCY2C-LCDR1 comprising the amino acid sequence of SEQ ID NO: 47, a GUCY2C-LCDR2 comprising the amino acid sequence of SEQ ID NO: 23, and a GUCY2C-LCDR3 comprising the amino acid sequence of SEQ ID NO: 24, and the FR region of the GUCY2C-VL comprises one or more amino acid substitutions selected from the group consisting of 2V, 3V, 68R, and 74K; or

(ii) the GUCY2C-VH comprises: a GUCY2C-HCDR1 comprising the amino acid sequence of SEQ ID NO: 4, a GUCY2C-HCDR2 comprising the amino acid sequence of SEQ ID NO: 5, and a GUCY2C-HCDR3 comprising the amino acid sequence of SEQ ID NO: 27, and the FR region of the GUCY2C-VH comprises one or more amino acid substitutions selected from the group consisting of 1E, 38K, 66K, and 67A; and the GUCY2C-VL comprises: a GUCY2C-LCDR1 comprising the amino acid sequence of SEQ ID NO: 7, a GUCY2C-LCDR2 comprising the amino acid sequence of SEQ ID NO: 8, and a GUCY2C-LCDR3 comprising the amino acid sequence of SEQ ID NO: 9, and the FR region of the GUCY2C-VL comprises one or more amino acid substitutions selected from the group consisting of 15P, 16G, 42S, 43S, 45K, 46S, 47W, 49Y, 58V, 71Y, and 76S; or

(iii) the GUCY2C-VH comprises: a GUCY2C-HCDR1 comprising the amino acid sequence of SEQ ID NO: 10, a GUCY2C-HCDR2 comprising the amino acid sequence of SEQ ID NO: 11, and a GUCY2C-HCDR3 comprising the amino acid sequence of SEQ ID NO: 40, and the FR region of the GUCY2C-VH comprises one or more amino acid substitutions selected from the group consisting of 1E, 2F, 28S, 69L, 71V, 73Q, and 75S; and the GUCY2C-VL comprises: a GUCY2C-LCDR1 comprising the amino acid sequence of SEQ ID NO: 13, a GUCY2C-LCDR2 comprising the amino acid sequence of SEQ ID NO: 39, and a GUCY2C-LCDR3 comprising the amino acid sequence of SEQ ID NO: 14, and the FR region of the GUCY2C-VL comprises one or more amino acid substitutions selected from the group consisting of 42S, 43S, 46P, 47W, 58V, and 71Y.

In some embodiments, provided is the antigen-binding molecule according to any one of the above, wherein: the CD3-VH comprises: a CD3-HCDR1 comprising the amino acid sequence of SEQ ID NO: 71, a CD3-HCDR2 comprising the amino acid sequence of SEQ ID NO: 72, and a CD3-HCDR3 comprising the amino acid sequence of SEQ ID NO: 73 or 88; and the CD3-VL comprises: a CD3-LCDR1 comprising the amino acid sequence of SEQ ID NO: 74, a CD3-LCDR2 comprising the amino acid sequence of SEQ ID NO: 75, and a CD3-LCDR3 comprising the amino acid sequence of SEQ ID NO: 76.

In some embodiments, provided is the antigen-binding molecule according to any one of the above, wherein: the CD3-VH comprises: a CD3-HCDR1 comprising the amino acid sequence of SEQ ID NO: 71, a CD3-HCDR2 comprising the amino acid sequence of SEQ ID NO: 72, and a CD3-HCDR3 comprising the amino acid sequence of SEQ ID NO: 73; and the CD3-VL comprises: a CD3-LCDR1 comprising the amino acid sequence of SEQ ID NO: 74, a CD3-LCDR2 comprising the amino acid sequence of SEQ ID NO: 75, and a CD3-LCDR3 comprising the amino acid sequence of SEQ ID NO: 76.

In some embodiments, provided is the antigen-binding molecule according to any one of the above, wherein: the CD3-VH comprises: a CD3-HCDR1 comprising the amino acid sequence of SEQ ID NO: 71, a CD3-HCDR2 comprising the amino acid sequence of SEQ ID NO: 72, and a CD3-HCDR3 comprising the amino acid sequence of SEQ ID NO: 88; and the CD3-VL comprises: a CD3-LCDR1 comprising the amino acid sequence of SEQ ID NO: 74, a CD3-LCDR2 comprising the amino acid sequence of SEQ ID NO: 75, and a CD3-LCDR3 comprising the amino acid sequence of SEQ ID NO: 76.

In some embodiments, provided is the antigen-binding molecule according to any one of the above, wherein:

i) the GUCY2C-VH comprises the amino acid sequence of SEQ ID NO: 56, 25, or 57, and the GUCY2C-VL comprises the amino acid sequence of SEQ ID NO: 54, 55, 26, 48, 49, 50, 51, 52, or 53; or

ii) the GUCY2C-VH comprises the amino acid sequence of SEQ ID NO: 37, 15, or 38, and the GUCY2C-VL comprises the amino acid sequence of SEQ ID NO: 33, 16, 31, 32, 34, 35, or 36; or

iii) the GUCY2C-VH comprises the amino acid sequence of SEQ ID NO: 42 or 17, and the GUCY2C-VL comprises the amino acid sequence of SEQ ID NO: 41 or 18; or

iv-1) the GUCY2C-VH comprises the amino acid sequence of SEQ ID NO: 273, and the GUCY2C-VL comprises the amino acid sequence of SEQ ID NO: 274; or

iv-2) the GUCY2C-VH comprises the amino acid sequence of SEQ ID NO: 275, and the GUCY2C-VL comprises the amino acid sequence of SEQ ID NO: 276; or

iv-3) the GUCY2C-VH comprises the amino acid sequence of SEQ ID NO: 277, and the GUCY2C-VL comprises the amino acid sequence of SEQ ID NO: 278; or

iv-4) the GUCY2C-VH comprises the amino acid sequence of SEQ ID NO: 279, and the GUCY2C-VL comprises the amino acid sequence of SEQ ID NO: 280; or

iv-5) the GUCY2C-VH comprises the amino acid sequence of SEQ ID NO: 330, and the GUCY2C-VL comprises the amino acid sequence of SEQ ID NO: 331; or

iv-6) the GUCY2C-VH comprises the amino acid sequence of SEQ ID NO: 332, and the GUCY2C-VL comprises the amino acid sequence of SEQ ID NO: 333; or

iv-7) the GUCY2C-VH comprises the amino acid sequence of SEQ ID NO: 334, and the GUCY2C-VL comprises the amino acid sequence of SEQ ID NO: 335; or

iv-8) the GUCY2C-VH comprises the amino acid sequence of SEQ ID NO: 336, and the GUCY2C-VL comprises the amino acid sequence of SEQ ID NO: 337; or

iv-9) the GUCY2C-VH comprises the amino acid sequence of SEQ ID NO: 338, and the GUCY2C-VL comprises the amino acid sequence of SEQ ID NO: 339; or

iv-10) the GUCY2C-VH comprises the amino acid sequence of SEQ ID NO: 340, and the GUCY2C-VL comprises the amino acid sequence of SEQ ID NO: 341; or

iv-11) the GUCY2C-VH comprises the amino acid sequence of SEQ ID NO: 342, and the GUCY2C-VL comprises the amino acid sequence of SEQ ID NO: 343; or

iv-12) the GUCY2C-VH comprises the amino acid sequence of SEQ ID NO: 344, and the GUCY2C-VL comprises the amino acid sequence of SEQ ID NO: 345; or

iv-13) the GUCY2C-VH comprises the amino acid sequence of SEQ ID NO: 346, and the GUCY2C-VL comprises the amino acid sequence of SEQ ID NO: 347; or

iv-14) the GUCY2C-VH comprises the amino acid sequence of SEQ ID NO: 348, and the GUCY2C-VL comprises the amino acid sequence of SEQ ID NO: 349; or

iv-15) the GUCY2C-VH comprises the amino acid sequence of SEQ ID NO: 350, and the GUCY2C-VL comprises the amino acid sequence of SEQ ID NO: 351; or

iv-16) the GUCY2C-VH comprises the amino acid sequence of SEQ ID NO: 352, and the GUCY2C-VL comprises the amino acid sequence of SEQ ID NO: 353.

In some embodiments, provided is the antigen-binding molecule according to any one of the above, wherein:

i) the GUCY2C-VH comprises the amino acid sequence of SEQ ID NO: 56, and the GUCY2C-VL comprises the amino acid sequence of SEQ ID NO: 54, 55, 48, 49, 50, 51, or 52, or

the GUCY2C-VH comprises the amino acid sequence of SEQ ID NO: 25, and the GUCY2C-VL comprises the amino acid sequence of SEQ ID NO: 26, or

ii) the GUCY2C-VH comprises the amino acid sequence of SEQ ID NO: 37, and the GUCY2C-VL comprises the amino acid sequence of SEQ ID NO: 33, or

the GUCY2C-VH comprises the amino acid sequence of SEQ ID NO: 38, and the GUCY2C-VL comprises the amino acid sequence of SEQ ID NO: 33, 32, 34, or 36, or the GUCY2C-VH comprises the amino acid sequence of SEQ ID NO: 15, and the GUCY2C-VL comprises the amino acid sequence of SEQ ID NO: 16, or

iii) the GUCY2C-VH comprises the amino acid sequence of SEQ ID NO: 42, and the GUCY2C-VL comprises the amino acid sequence of SEQ ID NO: 41, or

the GUCY2C-VH comprises the amino acid sequence of SEQ ID NO: 17, and the GUCY2C-VL comprises the amino acid sequence of SEQ ID NO: 18.

In some embodiments, provided is the antigen-binding molecule according to any one of the above, wherein:

i) the GUCY2C-VH comprises the amino acid sequence of SEQ ID NO: 56, and the GUCY2C-VL comprises the amino acid sequence of SEQ ID NO: 54, or

the GUCY2C-VH comprises the amino acid sequence of SEQ ID NO: 56, and the GUCY2C-VL comprises the amino acid sequence of SEQ ID NO: 55; or

ii) the GUCY2C-VH comprises the amino acid sequence of SEQ ID NO: 37, and the GUCY2C-VL comprises the amino acid sequence of SEQ ID NO: 33; or

iii) the GUCY2C-VH comprises the amino acid sequence of SEQ ID NO: 42, and the GUCY2C-VL comprises the amino acid sequence of SEQ ID NO: 41.

In some embodiments, provided is the antigen-binding molecule according to any one of the above, wherein the amino acid sequence of the GUCY2C-VH is set forth in SEQ ID NO: 56, and the amino acid sequence of the GUCY2C-VL is set forth in SEQ ID NO: 54.

In some embodiments, provided is the antigen-binding molecule according to any one of the above, wherein the amino acid sequence of the GUCY2C-VH is set forth in SEQ ID NO: 56, and the amino acid sequence of the GUCY2C-VL is set forth in SEQ ID NO: 55.

In some embodiments, provided is the antigen-binding molecule according to any one of the above, wherein the amino acid sequence of the GUCY2C-VH is set forth in SEQ ID NO: 37, and the amino acid sequence of the GUCY2C-VL is set forth in SEQ ID NO: 33.

In some embodiments, provided is the antigen-binding molecule according to any one of the above, wherein the amino acid sequence of the GUCY2C-VH is set forth in SEQ ID NO: 42, and the amino acid sequence of the GUCY2C-VL is set forth in SEQ ID NO: 41.

In some embodiments, provided is the antigen-binding molecule according to any one of the above, wherein: the CD3-VH comprises the amino acid sequence of SEQ ID NO: 77 or 89, and the CD3-VL comprises the amino acid sequence of SEQ ID NO: 78.

In some embodiments, provided is the antigen-binding molecule according to any one of the above, wherein: the amino acid sequence of the CD3-VH is set forth in SEQ ID NO: 77, and the amino acid sequence of the CD3-VL is set forth in SEQ ID NO: 78.

In some embodiments, provided is the antigen-binding molecule according to any one of the above, wherein: the amino acid sequence of the CD3-VH is set forth in SEQ ID NO: 89, and the amino acid sequence of the CD3-VL is set forth in SEQ ID NO: 78.

In some embodiments, provided is the antigen-binding molecule according to any one of the above, wherein the antigen-binding moiety that specifically binds to GUCY2C or the antigen-binding moiety that specifically binds to CD3 comprises a Titin chain and an Obscurin chain capable of forming a dimer.

In some embodiments, provided is the antigen-binding molecule according to any one of the above, wherein the antigen-binding moiety that specifically binds to GUCY2C comprises a Titin chain and an Obscurin chain capable of forming a dimer.

In some embodiments, provided is the antigen-binding molecule according to any one of the above, wherein the antigen-binding moiety that specifically binds to CD3 comprises a Titin chain and an Obscurin chain capable of forming a dimer.

In some embodiments, provided is the antigen-binding molecule according to any one of the above, wherein the Titin chain comprises an amino acid sequence selected from the group consisting of SEQ ID NO: 182 to SEQ ID NO: 200, and the Obscurin chain comprises an amino acid sequence selected from the group consisting of SEQ ID NO: 201 to SEQ ID NO: 241.

In some embodiments, provided is the antigen-binding molecule according to any one of the above, wherein the Titin chain comprises the amino acid sequence of SEQ ID NO: 198.

In some embodiments, provided is the antigen-binding molecule according to any one of the above, wherein the Obscurin chain comprises the amino acid sequence of SEQ ID NO: 236.

In some embodiments, provided is the antigen-binding molecule according to any one of the above, wherein the antigen-binding molecule comprises an Fc region.

In some embodiments, provided is the antigen-binding molecule according to any one of the above, wherein the antigen-binding molecule comprises an IgG Fc region.

In some embodiments, provided is the antigen-binding molecule according to any one of the above, wherein the antigen-binding molecule comprises an IgG1 Fc region.

In some embodiments, provided is the antigen-binding molecule according to any one of the above, wherein the antigen-binding molecule comprises an Fc region, and the Fc region comprises one or more amino acid substitutions capable of reducing the binding of the Fc region to an Fcγ receptor.

In some embodiments, provided is the antigen-binding molecule according to any one of the above, wherein the antigen-binding molecule comprises an Fc region; the Fc region is a human IgG1 Fc region, and the amino acids at positions 234 and 235 are A, as numbered according to the EU index.

In some embodiments, provided is the antigen-binding molecule according to any one of the above, wherein the antigen-binding molecule comprises an Fc region; the Fc region comprises a first subunit Fc1 and a second subunit Fc2 capable of associating with each other, and the Fc1 and the Fc2 each independently have one or more amino acid substitutions that reduce homodimerization of the Fc region.

In some embodiments, provided is the antigen-binding molecule according to any one of the above, wherein the antigen-binding molecule comprises an Fc region; the Fc region comprises a first subunit Fc1 and a second subunit Fc2 capable of associating with each other, the Fc1 has a knob structure according to the knob-into-hole technique, and the Fc2 has a hole structure according to the knob-into-hole technique.

In some embodiments, provided is the antigen-binding molecule according to any one of the above, wherein the antigen-binding molecule comprises an Fc region; the Fc region comprises a first subunit Fc1 and a second subunit Fc2 capable of associating with each other, the Fc1 has a knob structure according to the knob-into-hole technique, and the Fc2 has a hole structure according to the knob-into-hole technique; for the Fc1, the amino acid at position 366 is W, and for the Fc2, the amino acid at position 366 is S, the amino acid at position 368 is A, and the amino acid at position 407 is V, as numbered according to the EU index.

In some embodiments, provided is the antigen-binding molecule according to any one of the above, wherein the antigen-binding molecule comprises an Fc region; the Fc region comprises a first subunit Fc1 and a second subunit Fc2 capable of associating with each other, the Fc1 comprises the amino acid sequence of SEQ ID NO: 79 or 85, and the Fc2 comprises the amino acid sequence of SEQ ID NO: 80 or 86.

In some embodiments, provided is the antigen-binding molecule according to any one of the above, wherein the antigen-binding molecule comprises an Fc region; the Fc region comprises a first subunit Fc1 and a second subunit Fc2 capable of associating with each other, the Fc1 has a knob structure according to the knob-into-hole technique, and the Fc2 has a hole structure according to the knob-into-hole technique; for the Fc1, the amino acid at position 354 is C and the amino acid at position 366 is W, and for the Fc2, the amino acid at position 349 is C, the amino acid at position 366 is S, the amino acid at position 368 is A, and the amino acid at position 407 is V, as numbered according to the EU index.

In some embodiments, provided is the antigen-binding molecule according to any one of the above, wherein the antigen-binding molecule comprises an Fc region; the Fc region comprises a first subunit Fc1 and a second subunit Fc2 capable of associating with each other, the Fc1 comprises the amino acid sequence of SEQ ID NO: 85, and the Fc2 comprises the amino acid sequence of SEQ ID NO: 86.

In some embodiments, provided is the antigen-binding molecule according to any one of the above, wherein the antigen-binding molecule comprises an Fc region; the Fc region comprises a first subunit Fc1 and a second subunit Fc2 capable of associating with each other, the Fc1 has a knob structure according to the knob-into-hole technique, and the Fc2 has a hole structure according to the knob-into-hole technique; for the Fc1, the amino acid at position 237 is A, the amino acid at position 349 is C, and the amino acid at position 366 is W, and for the Fc2, the amino acid at position 237 is A, the amino acid at position 354 is C, the amino acid at position 366 is S, the amino acid at position 368 is A, and the amino acid at position 407 is V, as numbered according to the EU index.

In some embodiments, provided is the antigen-binding molecule according to any one of the above, wherein the antigen-binding molecule comprises an Fc region; the Fc region comprises a first subunit Fc1 and a second subunit Fc2 capable of associating with each other, the Fc1 comprises the amino acid sequence of SEQ ID NO: 79, and the Fc2 comprises the amino acid sequence of SEQ ID NO: 80.

In some embodiments, provided is the antigen-binding molecule according to any one of the above, wherein the antigen-binding molecule comprises one antigen-binding moiety that specifically binds to GUCY2C and one antigen-binding moiety that specifically binds to CD3.

In some embodiments, provided is the antigen-binding molecule according to any one of the above, wherein the antigen-binding molecule comprises one antigen-binding moiety that specifically binds to GUCY2C and one antigen-binding moiety that specifically binds to CD3, and the antigen-binding moieties that specifically bind to GUCY2C and CD3 are both scFvs.

In some embodiments, provided is the antigen-binding molecule according to any one of the above, which comprises

one first chain having a structure represented by formula (a) and one second chain having a structure represented by formula (b):

(a) [GUCY2C-VL]-[linker 1]-[CD3-VH]-[linker 2]-[Fc1], and
(b) [CD3-VL]-[linker 3]-[GUCY2C-VH]-[linker 2]-[Fc2],

wherein the linker 1, the linker 2, and the linker 3 are identical or different peptide linkers; or the linker 1, the linker 2, and the linker 3 are absent;
the structures represented by formulas (a) and (b) are arranged from N-terminus to C-terminus.

In some embodiments, provided is the antigen-binding molecule according to any one of the above, wherein:

i) the GUCY2C-VH comprises the amino acid sequence of SEQ ID NO: 56, and the GUCY2C-VL comprises the amino acid sequence of SEQ ID NO: 54; and the CD3-VH comprises the amino acid sequence of SEQ ID NO: 77, and the CD3-VL comprises the amino acid sequence of SEQ ID NO: 78; or
the GUCY2C-VH comprises the amino acid sequence of SEQ ID NO: 56, and the GUCY2C-VL comprises the amino acid sequence of SEQ ID NO: 55; and the CD3-VH comprises the amino acid sequence of SEQ ID NO: 77, and the CD3-VL comprises the amino acid sequence of SEQ ID NO: 78; or
ii) the GUCY2C-VH comprises the amino acid sequence of SEQ ID NO: 15, and the GUCY2C-VL comprises the amino acid sequence of SEQ ID NO: 16; and the CD3-VH comprises the amino acid sequence of SEQ ID NO: 77, and the CD3-VL comprises the amino acid sequence of SEQ ID NO: 78; or
iii) the GUCY2C-VH comprises the amino acid sequence of SEQ ID NO: 17, and the GUCY2C-VL comprises the amino acid sequence of SEQ ID NO: 18; and the CD3-VH comprises the amino acid sequence of SEQ ID NO: 77, and the CD3-VL comprises the amino acid sequence of SEQ ID NO: 78.

In some embodiments, provided is the antigen-binding molecule according to any one of the above, wherein: the amino acid sequence of the GUCY2C-VH is set forth in SEQ ID NO: 56, and the amino acid sequence of the GUCY2C-VL is set forth in SEQ ID NO: 54; and the amino acid sequence of the CD3-VH is set forth in SEQ ID NO: 77, and the amino acid sequence of the CD3-VL is set forth in SEQ ID NO: 78.

In some embodiments, provided is the antigen-binding molecule according to any one of the above, wherein: the amino acid sequence of the GUCY2C-VH is set forth in SEQ ID NO: 56, and the amino acid sequence of the GUCY2C-VL is set forth in SEQ ID NO: 55; and the amino acid sequence of the CD3-VH is set forth in SEQ ID NO: 77, and the amino acid sequence of the CD3-VL is set forth in SEQ ID NO: 78.

In some embodiments, provided is the antigen-binding molecule according to any one of the above, wherein: the amino acid sequence of the GUCY2C-VH is set forth in SEQ ID NO: 15, and the amino acid sequence of the GUCY2C-VL is set forth in SEQ ID NO: 16; and the amino acid sequence of the CD3-VH is set forth in SEQ ID NO: 77, and the amino acid sequence of the CD3-VL is set forth in SEQ ID NO: 78.

In some embodiments, provided is the antigen-binding molecule according to any one of the above, wherein the linker 1 and the linker 2 are both peptide linkers known in the art, so long as the antigen-binding molecule is capable of exhibiting the desired antigen binding activity. For example, the peptide linkers may be flexible peptides having 1-50 or 3-20 amino acid residues. In some embodiments, the peptide linkers each independently have the structure of $L_1$-(GGGGS)n-$L_2$, wherein $L_1$ is a bond, A, G (SEQ ID NO: 82), GS, GGG (SEQ ID NO: 96), GGS (SEQ ID NO: 245), GGGS (SEQ ID NO: 246), or GGGG (SEQ ID NO: 247), n is 0, 1, 2, 3, 4, 5, 6, 7, 8, 9, or 10, $L_2$ is a bond, G (SEQ ID NO: 82), GG, GGG (SEQ ID NO: 96), or GGGG (SEQ ID NO: 247), and the peptide linkers are not bonds. In some embodiments, the amino acid sequence of the linker 1 is set forth in SEQ ID NO: 81, the amino acid sequence of the linker 2 is set forth in SEQ ID NO: 82, and the amino acid sequence of the linker 3 is set forth in SEQ ID NO: 83.

In some embodiments, provided is the antigen-binding molecule according to any one of the above, wherein the amino acid sequence of the Fc1 is set forth in SEQ ID NO: 79, and the amino acid sequence of the Fc2 is set forth in SEQ ID NO: 80.

In some embodiments, provided is the antigen-binding molecule according to any one of the above, wherein the antigen-binding molecule comprises:

i) one first chain comprising the amino acid sequence of SEQ ID NO: 99 and one second chain comprising the amino acid sequence of SEQ ID NO: 100; or

one first chain comprising the amino acid sequence of SEQ ID NO: 101 and one second chain comprising the amino acid sequence of SEQ ID NO: 100; or

ii) one first chain comprising the amino acid sequence of SEQ ID NO: 102 and one second chain comprising the amino acid sequence of SEQ ID NO: 103; or

iii) one first chain comprising the amino acid sequence of SEQ ID NO: 104 and one second chain comprising the amino acid sequence of SEQ ID NO: 105.

In some embodiments, provided is the antigen-binding molecule according to any one of the above, wherein the antigen-binding molecule comprises: one first chain having the amino acid sequence set forth in SEQ ID NO: 99 and one second chain having the amino acid sequence set forth in SEQ ID NO: 100.

In some embodiments, provided is the antigen-binding molecule according to any one of the above, wherein the antigen-binding molecule comprises: one first chain having the amino acid sequence set forth in SEQ ID NO: 101 and one second chain having the amino acid sequence set forth in SEQ ID NO: 100.

In some embodiments, provided is the antigen-binding molecule according to any one of the above, wherein the antigen-binding molecule comprises: one first chain having the amino acid sequence set forth in SEQ ID NO: 102 and one second chain having the amino acid sequence set forth in SEQ ID NO: 103.

In some embodiments, provided is the antigen-binding molecule according to any one of the above, wherein the antigen-binding molecule comprises one antigen-binding moiety that specifically binds to CD3 and one antigen-binding moiety that specifically binds to GUCY2C; the antigen-binding moiety that specifically binds to CD3 is a Fab, and the antigen-binding moiety that specifically binds to GUCY2C is a replaced Fab comprising a Titin chain and an Obscurin chain capable of forming a dimer.

In some embodiments, provided is the antigen-binding molecule according to any one of the above, wherein the antigen-binding molecule comprises one first chain having a structure represented by formula (c), one second chain having a structure represented by formula (d), one third chain having a structure represented by formula (e), and one fourth chain having a structure represented by formula (f):

(c) [CD3-VH]-[CH1]-[Fc1],
(d) [CD3-VL]-[CL],
(e) [GUCY2C-VH]-[linker 4]-[Obscurin chain]-[Fc2], and
(f) [GUCY2C-VL]-[linker 4]-[Titin chain],
wherein the linker 4 is a peptide linker; or the linker 4 is absent;
the structures represented by formulas (c), (d), (e), and (f) are arranged from the N-terminus to the C-terminus.

In some embodiments, provided is the antigen-binding molecule according to any one of the above, wherein:

i) the CD3-VH comprises the amino acid sequence of SEQ ID NO: 77, and the CD3-VL comprises the amino acid sequence of SEQ ID NO: 78; and the GUCY2C-VH comprises the amino acid sequence of SEQ ID NO: 37, and the GUCY2C-VL comprises the amino acid sequence of SEQ ID NO: 33; or

the CD3-VH comprises the amino acid sequence of SEQ ID NO: 89, and the CD3-VL comprises the amino acid sequence of SEQ ID NO: 78; and the GUCY2C-VH comprises the amino acid sequence of SEQ ID NO: 37, and the GUCY2C-VL comprises the amino acid sequence of SEQ ID NO: 33; or

the CD3-VH comprises the amino acid sequence of SEQ ID NO: 77, and the CD3-VL comprises the amino acid sequence of SEQ ID NO: 78; and the GUCY2C-VH comprises the amino acid sequence of SEQ ID NO: 15, and the GUCY2C-VL comprises the amino acid sequence of SEQ ID NO: 16; or

the CD3-VH comprises the amino acid sequence of SEQ ID NO: 77, and the CD3-VL comprises the amino acid sequence of SEQ ID NO: 78; and the GUCY2C-VH comprises the amino acid sequence of SEQ ID NO: 37, and the GUCY2C-VL comprises the amino acid sequence of SEQ ID NO: 31; or

the CD3-VH comprises the amino acid sequence of SEQ ID NO: 77, and the CD3-VL comprises the amino acid sequence of SEQ ID NO: 78; and the GUCY2C-VH comprises the amino acid sequence of SEQ ID NO: 37, and the GUCY2C-VL comprises the amino acid sequence of SEQ ID NO: 32; or

the CD3-VH comprises the amino acid sequence of SEQ ID NO: 77, and the CD3-VL comprises the amino acid sequence of SEQ ID NO: 78; and the GUCY2C-VH comprises the amino acid sequence of SEQ ID NO: 37, and the GUCY2C-VL comprises the amino acid sequence of SEQ ID NO: 34; or

the CD3-VH comprises the amino acid sequence of SEQ ID NO: 77, and the CD3-VL comprises the amino acid sequence of SEQ ID NO: 78; and the GUCY2C-VH comprises the amino acid sequence of SEQ ID NO: 37, and the GUCY2C-VL comprises the amino acid sequence of SEQ ID NO: 35; or

the CD3-VH comprises the amino acid sequence of SEQ ID NO: 77, and the CD3-VL comprises the amino acid sequence of SEQ ID NO: 78; and the GUCY2C-VH comprises the amino acid sequence of SEQ ID NO: 37, and the GUCY2C-VL comprises the amino acid sequence of SEQ ID NO: 36; or

ii) the CD3-VH comprises the amino acid sequence of SEQ ID NO: 77, and the CD3-VL comprises the amino acid sequence of SEQ ID NO: 78; and the GUCY2C-VH comprises the amino acid sequence of SEQ ID NO: 56, and the GUCY2C-VL comprises the amino acid sequence of SEQ ID NO: 54; or

iii) the CD3-VH comprises the amino acid sequence of SEQ ID NO: 77, and the CD3-VL comprises the amino acid sequence of SEQ ID NO: 78; and the GUCY2C-VH comprises the amino acid sequence of SEQ ID NO: 42, and the GUCY2C-VL comprises the amino acid sequence of SEQ ID NO: 41; or

the CD3-VH comprises the amino acid sequence of SEQ ID NO: 77, and the CD3-VL comprises the amino acid sequence of SEQ ID NO: 78; and the GUCY2C-VH comprises the amino acid sequence of SEQ ID NO: 17, and the GUCY2C-VL comprises the amino acid sequence of SEQ ID NO: 18.

In some embodiments, provided is the antigen-binding molecule according to any one of the above, wherein: the amino acid sequence of the CD3-VH is set forth in SEQ ID NO: 77, and the amino acid sequence of the CD3-VL is set forth in SEQ ID NO: 78; and the amino acid sequence of the GUCY2C-VH is set forth in SEQ ID NO: 37, and the amino acid sequence of the GUCY2C-VL is set forth in SEQ ID NO: 33.

In some embodiments, provided is the antigen-binding molecule according to any one of the above, wherein: the amino acid sequence of the CD3-VH is set forth in SEQ ID NO: 89, and the amino acid sequence of the CD3-VL is set forth in SEQ ID NO: 78; and the amino acid sequence of the GUCY2C-VH is set forth in SEQ ID NO: 37, and the amino acid sequence of the GUCY2C-VL is set forth in SEQ ID NO: 33.

In some embodiments, provided is the antigen-binding molecule according to any one of the above, wherein: the amino acid sequence of the CD3-VH is set forth in SEQ ID NO: 77, and the amino acid sequence of the CD3-VL is set forth in SEQ ID NO: 78; and the amino acid sequence of the GUCY2C-VH is set forth in SEQ ID NO: 42, and the amino acid sequence of the GUCY2C-VL is set forth in SEQ ID NO: 41.

In some embodiments, provided is the antigen-binding molecule according to any one of the above, wherein the Titin chain and the Obscurin chain are any Titin chain and Obscurin chain in Table 3-1 and Table 3-2 of the present disclosure that can form a dimer. In some embodiments, provided is the antigen-binding molecule according to any one of the above, wherein the Titin chain comprises the amino acid sequence of SEQ ID NO: 198. In some embodiments, provided is the antigen-binding molecule according to any one of the above, wherein the Obscurin chain comprises the amino acid sequence of SEQ ID NO: 236.

In some embodiments, provided is the antigen-binding molecule according to any one of the above, wherein the linkers 4 are all peptide linkers known in the art, so long as the antigen-binding molecule is capable of exhibiting the desired antigen binding activity. For example, the peptide linkers may be flexible peptides comprising 1-50 or 3-20 amino acid residues. In some embodiments, the peptide linker is 3-15 amino acid residues in length. In some embodiments, the peptide linkers each independently have a structure of (GGGGS)n, wherein n is 1, 2, or 3. In some embodiments, the sequence of the linker 4 is GGGGS (SEQ ID NO: 90).

In some embodiments, provided is the antigen-binding molecule according to any one of the above, wherein the CH1 is the CH1 sequence of an IgG. In some embodiments, the CH1 is the CH1 of an IgG1. In some embodiments, the CH1 comprises the amino acid sequence of SEQ ID NO: 84.

In some embodiments, provided is the antigen-binding molecule according to any one of the above, wherein the CL is the light chain constant region of an antibody. In some embodiments, provided is the antigen-binding molecule according to any one of the above, wherein the CL is a kappa or lamada light chain constant region. In some embodiments, the CL comprises the amino acid sequence of SEQ ID NO: 87.

In some embodiments, provided is the antigen-binding molecule according to any one of the above, wherein the amino acid sequence of the Fc1 is set forth in SEQ ID NO: 85, and the amino acid sequence of the Fc2 is set forth in SEQ ID NO: 86.

In some embodiments, provided is the antigen-binding molecule according to any one of the above, wherein the antigen-binding molecule comprises:

i) one first chain comprising the amino acid sequence of SEQ ID NO: 91, one second chain comprising the amino acid sequence of SEQ ID NO: 92, one third chain comprising the amino acid sequence of SEQ ID NO: 110, and one fourth chain comprising the amino acid sequence of SEQ ID NO: 113; or

one first chain comprising the amino acid sequence of SEQ ID NO: 242, one second chain comprising the amino acid sequence of SEQ ID NO: 92, one third chain comprising the amino acid sequence of SEQ ID NO: 110, and one fourth chain comprising the amino acid sequence of SEQ ID NO: 113; or
one first chain comprising the amino acid sequence of SEQ ID NO: 91, one second chain comprising the amino acid sequence of SEQ ID NO: 92, one third chain comprising the amino acid sequence of SEQ ID NO: 108, and one fourth chain comprising the amino acid sequence of SEQ ID NO: 109; or
one first chain comprising the amino acid sequence of SEQ ID NO: 91, one second chain comprising the amino acid sequence of SEQ ID NO: 92, one third chain comprising the amino acid sequence of SEQ ID NO: 110, and one fourth chain comprising the amino acid sequence of SEQ ID NO: 111; or
one first chain comprising the amino acid sequence of SEQ ID NO: 91, one second chain comprising the amino acid sequence of SEQ ID NO: 92, one third chain comprising the amino acid sequence of SEQ ID NO: 110, and one fourth chain comprising the amino acid sequence of SEQ ID NO: 112; or
one first chain comprising the amino acid sequence of SEQ ID NO: 91, one second chain comprising the amino acid sequence of SEQ ID NO: 92, one third chain comprising the amino acid sequence of SEQ ID NO: 110, and one fourth chain comprising the amino acid sequence of SEQ ID NO: 114; or
one first chain comprising the amino acid sequence of SEQ ID NO: 91, one second chain comprising the amino acid sequence of SEQ ID NO: 92, one third chain comprising the amino acid sequence of SEQ ID NO: 110, and one fourth chain comprising the amino acid sequence of SEQ ID NO: 115; or
one first chain comprising the amino acid sequence of SEQ ID NO: 91, one second chain comprising the amino acid sequence of SEQ ID NO: 92, one third chain comprising the amino acid sequence of SEQ ID NO: 110, and one fourth chain comprising the amino acid sequence of SEQ ID NO: 116; or

ii) one first chain comprising the amino acid sequence of SEQ ID NO: 91, one second chain comprising the amino acid sequence of SEQ ID NO: 92, one third chain comprising the amino acid sequence of SEQ ID NO: 106, and one fourth chain comprising the amino acid sequence of SEQ ID NO: 107; or
iii) one first chain comprising the amino acid sequence of SEQ ID NO: 91, one second chain comprising the amino acid sequence of SEQ ID NO: 92, one third chain comprising the amino acid sequence of SEQ ID NO: 119, and one fourth chain comprising the amino acid sequence of SEQ ID NO: 120; or

one first chain comprising the amino acid sequence of SEQ ID NO: 91, one second chain comprising the amino acid sequence of SEQ ID NO: 92, one third chain comprising the amino acid sequence of SEQ ID NO: 117, and one fourth chain comprising the amino acid sequence of SEQ ID NO: 118.

In some embodiments, provided is the antigen-binding molecule according to any one of the above, wherein the antigen-binding molecule comprises: one first chain having the amino acid sequence set forth in SEQ ID NO: 91, one second chain having the amino acid sequence set forth in SEQ ID NO: 92, one third chain having the amino acid sequence set forth in SEQ ID NO: 110, and one fourth chain having the amino acid sequence set forth in SEQ ID NO: 113.

In some embodiments, provided is the antigen-binding molecule according to any one of the above, wherein the antigen-binding molecule comprises: one first chain having the amino acid sequence set forth in SEQ ID NO: 242, one second chain having the amino acid sequence set forth in SEQ ID NO: 92, one third chain having the amino acid sequence set forth in SEQ ID NO: 110, and one fourth chain having the amino acid sequence set forth in SEQ ID NO: 113.

In some embodiments, provided is the antigen-binding molecule according to any one of the above, wherein the antigen-binding molecule comprises: one first chain having the amino acid sequence set forth in SEQ ID NO: 91, one second chain having the amino acid sequence set forth in SEQ ID NO: 92, one third chain having the amino acid sequence set forth in SEQ ID NO: 119, and one fourth chain having the amino acid sequence set forth in SEQ ID NO: 120.

In some embodiments, provided is the antigen-binding molecule according to any one of the above, wherein the antigen-binding molecule comprises one antigen-binding moiety that specifically binds to GUCY2C and one antigen-binding moiety that specifically binds to CD3; the antigen-binding moiety that specifically binds to GUCY2C is a replaced Fab comprising a Titin chain and an Obscurin chain capable of forming a dimer, and the antigen-binding moiety that specifically binds to CD3 is a Fab. In some embodiments, provided is the antigen-binding molecule according to any one of the above, wherein the antigen-binding molecule comprises one first chain having a structure represented by formula (g), one second chain having a structure represented by formula (f), one third chain having a structure represented by formula (h), and one fourth chain having a structure represented by formula (d):

(g) [GUCY2C-VH]-[linker 4]-[Obscurin chain]-[Fc1],
(f) [GUCY2C-VL]-[linker 4]-[Titin chain],
(h) [CD3-VH]-[CH1]-[Fc2], and
(d) [CD3-VL]-[CL],

wherein the linker 4 is a peptide linker; or the linker 4 is absent;
the structures represented by formulas (g), (f), (h), and (d) are arranged from N-terminus to C-terminus.

In some embodiments, provided is the antigen-binding molecule according to any one of the above, wherein:

i) the CD3-VH comprises the amino acid sequence of SEQ ID NO: 77, and the CD3-VL comprises the amino acid sequence of SEQ ID NO: 78; and the GUCY2C-VH comprises the amino acid sequence of SEQ ID NO: 56, and the GUCY2C-VL comprises the amino acid sequence of SEQ ID NO: 54; or
ii) the CD3-VH comprises the amino acid sequence of SEQ ID NO: 77, and the CD3-VL comprises the amino acid sequence of SEQ ID NO: 78; and the GUCY2C-VH comprises the amino acid sequence of SEQ ID NO: 37, and the GUCY2C-VL comprises the amino acid sequence of SEQ ID NO: 31; or

the CD3-VH comprises the amino acid sequence of SEQ ID NO: 77, and the CD3-VL comprises the amino acid sequence of SEQ ID NO: 78; and the GUCY2C-VH comprises the amino acid sequence of SEQ ID NO: 37, and the GUCY2C-VL comprises the amino acid sequence of SEQ ID NO: 32; or
the CD3-VH comprises the amino acid sequence of SEQ ID NO: 77, and the CD3-VL comprises the amino acid sequence of SEQ ID NO: 78; and the GUCY2C-VH comprises the amino acid sequence of SEQ ID NO: 37, and the GUCY2C-VL comprises the amino acid sequence of SEQ ID NO: 33; or
the CD3-VH comprises the amino acid sequence of SEQ ID NO: 77, and the CD3-VL comprises the amino acid sequence of SEQ ID NO: 78; and the GUCY2C-VH comprises the amino acid sequence of SEQ ID NO: 37, and the GUCY2C-VL comprises the amino acid sequence of SEQ ID NO: 34; or
the CD3-VH comprises the amino acid sequence of SEQ ID NO: 77, and the CD3-VL comprises the amino acid sequence of SEQ ID NO: 78; and the GUCY2C-VH comprises the amino acid sequence of SEQ ID NO: 37, and the GUCY2C-VL comprises the amino acid sequence of SEQ ID NO: 35; or
the CD3-VH comprises the amino acid sequence of SEQ ID NO: 77, and the CD3-VL comprises the amino acid sequence of SEQ ID NO: 78; and the GUCY2C-VH comprises the amino acid sequence of SEQ ID NO: 37, and the GUCY2C-VL comprises the amino acid sequence of SEQ ID NO: 36; or

iii) the CD3-VH comprises the amino acid sequence of SEQ ID NO: 77, and the CD3-VL comprises the amino acid sequence of SEQ ID NO: 78; and the GUCY2C-VH comprises the amino acid sequence of SEQ ID NO: 42, and the GUCY2C-VL comprises the amino acid sequence of SEQ ID NO: 41.

In some embodiments, provided is the antigen-binding molecule according to any one of the above, wherein: the amino acid sequence of the CD3-VH is set forth in SEQ ID NO: 77, and the amino acid sequence of the CD3-VL is set forth in SEQ ID NO: 78; and the amino acid sequence of the GUCY2C-VH is set forth in SEQ ID NO: 56, and the amino acid sequence of the GUCY2C-VL is set forth in SEQ ID NO: 54.

In some embodiments, provided is the antigen-binding molecule according to any one of the above, wherein the Titin chain and the Obscurin chain are any Titin chain and Obscurin chain in Table 3-1 and Table 3-2 of the present disclosure that can form a dimer. In some embodiments, provided is the antigen-binding molecule according to any one of the above, wherein the Titin chain comprises the amino acid sequence of SEQ ID NO: 198. In some embodiments, provided is the antigen-binding molecule according to any one of the above, wherein the Obscurin chain comprises the amino acid sequence of SEQ ID NO: 236.

In some embodiments, provided is the antigen-binding molecule according to any one of the above, wherein the linkers 4 are all peptide linkers known in the art, so long as the antigen-binding molecule is capable of exhibiting the desired antigen binding activity. For example, the peptide linkers may be flexible peptides comprising 1-50 or 3-20 amino acid residues. In some embodiments, the peptide linker is 3-15 amino acid residues in length. In some embodiments, the peptide linkers each independently have a structure of (GGGGS)n, wherein n is 1, 2, or 3. In some embodiments, the sequence of the linker 4 is GGGGS (SEQ ID NO: 90).

In some embodiments, provided is the antigen-binding molecule according to any one of the above, wherein the CH1 is the CH1 sequence of an IgG. In some embodiments, the CH1 is the CH1 of an IgG1. In some embodiments, the CH1 comprises the amino acid sequence of SEQ ID NO: 84.

In some embodiments, provided is the antigen-binding molecule according to any one of the above, wherein the CL is the light chain constant region of an antibody. In some embodiments, provided is the antigen-binding molecule according to any one of the above, wherein the CL is a kappa or lamada light chain constant region. In some embodiments, the CL comprises the amino acid sequence of SEQ ID NO: 87.

In some embodiments, provided is the antigen-binding molecule according to any one of the above, wherein the amino acid sequence of the Fc1 is set forth in SEQ ID NO: 85, and the amino acid sequence of the Fc2 is set forth in SEQ ID NO: 86.

In some embodiments, provided is the antigen-binding molecule according to any one of the above, wherein the antigen-binding molecule comprises:

> i) one first chain comprising the amino acid sequence of SEQ ID NO: 121, one second chain comprising the amino acid sequence of SEQ ID NO: 107, one third chain comprising the amino acid sequence of SEQ ID NO: 93, and one fourth chain comprising the amino acid sequence of SEQ ID NO: 92; or
> ii) one first chain comprising the amino acid sequence of SEQ ID NO: 122, one second chain comprising the amino acid sequence of SEQ ID NO: 111, one third chain comprising the amino acid sequence of SEQ ID NO: 93, and one fourth chain comprising the amino acid sequence of SEQ ID NO: 92; or

> > one first chain comprising the amino acid sequence of SEQ ID NO: 122, one second chain comprising the amino acid sequence of SEQ ID NO: 112, one third chain comprising the amino acid sequence of SEQ ID NO: 93, and one fourth chain comprising the amino acid sequence of SEQ ID NO: 92; or
> > one first chain comprising the amino acid sequence of SEQ ID NO: 122, one second chain comprising the amino acid sequence of SEQ ID NO: 113, one third chain comprising the amino acid sequence of SEQ ID NO: 93, and one fourth chain comprising the amino acid sequence of SEQ ID NO: 92; or
> > one first chain comprising the amino acid sequence of SEQ ID NO: 122, one second chain comprising the amino acid sequence of SEQ ID NO: 114, one third chain comprising the amino acid sequence of SEQ ID NO: 93, and one fourth chain comprising the amino acid sequence of SEQ ID NO: 92; or
> > one first chain comprising the amino acid sequence of SEQ ID NO: 122, one second chain comprising the amino acid sequence of SEQ ID NO: 115, one third chain comprising the amino acid sequence of SEQ ID NO: 93, and one fourth chain comprising the amino acid sequence of SEQ ID NO: 92; or
> > one first chain comprising the amino acid sequence of SEQ ID NO: 122, one second chain comprising the amino acid sequence of SEQ ID NO: 116, one third chain comprising the amino acid sequence of SEQ ID NO: 93, and one fourth chain comprising the amino acid sequence of SEQ ID NO: 92; or

> iii) one first chain comprising the amino acid sequence of SEQ ID NO: 123, one second chain comprising the amino acid sequence of SEQ ID NO: 120, one third chain comprising the amino acid sequence of SEQ ID NO: 93, and one fourth chain comprising the amino acid sequence of SEQ ID NO: 92.

In some embodiments, provided is the antigen-binding molecule according to any one of the above, wherein the antigen-binding molecule comprises: one first chain having the amino acid sequence set forth in SEQ ID NO: 121, one second chain having the amino acid sequence set forth in SEQ ID NO: 107, one third chain having the amino acid sequence set forth in SEQ ID NO: 93, and one fourth chain having the amino acid sequence set forth in SEQ ID NO: 92.

In some embodiments, provided is the antigen-binding molecule according to any one of the above, wherein the antigen-binding molecule comprises two antigen-binding moieties that specifically bind to GUCY2C and two antigen-binding moieties that specifically bind to CD3, and the antigen-binding moieties that specifically bind to GUCY2C and CD3 are all scFvs.

In some embodiments, provided is the antigen-binding molecule according to any one of the above, wherein the antigen-binding molecule comprises two first chains having a structure represented by formula (i) and two second chains having a structure represented by formula (j):

> (i) [GUCY2C-VH]-[CH1]-[CD3-VH]-[linker 5]-[CD3-VL]-[linker 6]-[one subunit of Fc region], and
> (j) [GUCY2C-VL]-[CL],

wherein the linker 5 and the linker 6 are peptide linkers; or the linker 5 and the linker 6 are absent; the structures represented by formulas (i) and (j) are arranged from N-terminus to C-terminus.

In some embodiments, provided is the antigen-binding molecule according to any one of the above, wherein:

the CD3-VH comprises the amino acid sequence of SEQ ID NO: 77, and the CD3-VL comprises the amino acid sequence of SEQ ID NO: 78; and the GUCY2C-VH comprises the amino acid sequence of SEQ ID NO: 56, and the GUCY2C-VL comprises the amino acid sequence of SEQ ID NO: 54; or
the CD3-VH comprises the amino acid sequence of SEQ ID NO: 77, and the CD3-VL comprises the amino acid sequence of SEQ ID NO: 78; and the GUCY2C-VH comprises the amino acid sequence of SEQ ID NO: 56, and the GUCY2C-VL comprises the amino acid sequence of SEQ ID NO: 55.

In some embodiments, provided is the antigen-binding molecule according to any one of the above, wherein: the amino acid sequence of the CD3-VH is set forth in SEQ ID NO: 77, and the amino acid sequence of the CD3-VL is set forth in SEQ ID NO: 78; and the amino acid sequence of the GUCY2C-VH is set forth in SEQ ID NO: 56, and the amino acid sequence of the GUCY2C-VL is set forth in SEQ ID NO: 54.

In some embodiments, provided is the antigen-binding molecule according to any one of the above, wherein the linker 5 and the linker 6 are both peptide linkers known in the art, so long as the antigen-binding molecule is capable of exhibiting the desired antigen binding activity. For example, the peptide linkers may be flexible peptides comprising 1-50 or 3-20 amino acid residues. In some embodiments, the peptide linkers each independently have the structure of $L_1$-(GGGGS)n-$L_2$, wherein $L_1$ is a bond, A, G, GS, GGG (SEQ ID NO: 96), GGS (SEQ ID NO: 245), GGGS (SEQ ID NO: 246), or GGGG (SEQ ID NO: 247), n is 0, 1, 2, 3, 4, 5, 6, 7, 8, 9, or 10, $L_2$ is a bond, G, GG, GGG (SEQ ID NO: 96), or GGGG (SEQ ID NO: 247), and the peptide linkers are not bonds. In some embodiments, the peptide linker is 3-15 amino acid residues in length. In some embodiments, the peptide linkers each independently have a structure of (GGGGS)n, wherein n is 1, 2, or 3. In some embodiments, the amino acid sequence of the linker 5 is set forth in SEQ ID NO: 95, and the amino acid sequence of the linker 6 is set forth in SEQ ID NO: 96.

In some embodiments, provided is the antigen-binding molecule according to any one of the above, wherein the CH1 is the CH1 sequence of an IgG. In some embodiments, the CH1 is the CH1 of an IgG1. In some embodiments, the CH1 comprises the amino acid sequence of SEQ ID NO: 84.

In some embodiments, provided is the antigen-binding molecule according to any one of the above, wherein the CL is the light chain constant region of an antibody. In some embodiments, provided is the antigen-binding molecule according to any one of the above, wherein the CL is a kappa or lamada light chain constant region. In some embodiments, the CL comprises the amino acid sequence of SEQ ID NO: 59.

In some embodiments, provided is the antigen-binding molecule according to any one of the above, wherein the amino acid sequence of one subunit of the Fc region is set forth in SEQ ID NO: 94.

In some embodiments, provided is the antigen-binding molecule according to any one of the above, wherein the antigen-binding molecule comprises:

two first chains comprising the amino acid sequence of SEQ ID NO: 124 and two second chains comprising the amino acid sequence of SEQ ID NO: 65; or
two first chains comprising the amino acid sequence of SEQ ID NO: 124 and two second chains comprising the amino acid sequence of SEQ ID NO: 66.

In some embodiments, provided is the antigen-binding molecule according to any one of the above, wherein the antigen-binding molecule comprises: two first chains having the amino acid sequence set forth in SEQ ID NO: 124 and two second chains having the amino acid sequence set forth in SEQ ID NO: 65.

In some embodiments, provided is the antigen-binding molecule according to any one of the above, wherein the antigen-binding molecule comprises two antigen-binding moieties that specifically bind to GUCY2C and two antigen-binding moieties that specifically bind to CD3; the antigen-binding moieties that specifically bind to GUCY2C are Fabs, and the antigen-binding moieties that specifically bind to CD3 are replaced Fabs comprising a Titin chain and an Obscurin chain capable of forming a dimer.

In some embodiments, provided is the antigen-binding molecule according to any one of the above, wherein the antigen-binding molecule comprises two first chains having a structure represented by formula (k), two second chains having a structure represented by formula (j), and two third chains having a structure represented by formula (1):

(k) [GUCY2C-VH]-[CH1]-[linker 7]-[CD3-VH]-[linker 4]-[Obscurin chain]-[one subunit of Fc region],
(j) [GUCY2C-VL]-[CL], and
(1) [CD3-VL]-[linker 4]-[Titin chain],

wherein the linker 4 and the linker 7 are peptide linkers; or the linker 4 and the linker 7 are absent;
the structures represented by formulas (k), (j), and (1) are arranged from N-terminus to C-terminus.

In some embodiments, provided is the antigen-binding molecule according to any one of the above, wherein:

the CD3-VH comprises the amino acid sequence of SEQ ID NO: 77, and the CD3-VL comprises the amino acid sequence of SEQ ID NO: 78; and the GUCY2C-VH comprises the amino acid sequence of SEQ ID NO: 56, and the GUCY2C-VL comprises the amino acid sequence of SEQ ID NO: 54; or
the CD3-VH comprises the amino acid sequence of SEQ ID NO: 77, and the CD3-VL comprises the amino acid sequence of SEQ ID NO: 78; and the GUCY2C-VH comprises the amino acid sequence of SEQ ID NO: 56, and the GUCY2C-VL comprises the amino acid sequence of SEQ ID NO: 55.

In some embodiments, provided is the antigen-binding molecule according to any one of the above, wherein: the amino acid sequence of the CD3-VH is set forth in SEQ ID NO: 77, and the amino acid sequence of the CD3-VL is set forth in SEQ ID NO: 78; and the amino acid sequence of the GUCY2C-VH is set forth in SEQ ID NO: 56, and the amino acid sequence of the GUCY2C-VL is set forth in SEQ ID NO: 54.

In some embodiments, provided is the antigen-binding molecule according to any one of the above, wherein the linker 4 and the linker 7 are both peptide linkers known in the art, so long as the antigen-binding molecule is capable of exhibiting the desired antigen binding activity. In some embodiments, the peptide linker is 3-15 amino acid residues in length. In some embodiments, the peptide linkers each independently have a structure of (GGGGS)n, wherein n is 1, 2, or 3. In some embodiments, the amino acid sequence of the linker 4 is set forth in SEQ ID NO: 90, and the amino acid sequence of the linker 7 is set forth in SEQ ID NO: 97.

In some embodiments, provided is the antigen-binding molecule according to any one of the above, wherein the Titin chain and the Obscurin chain are any Titin chain and Obscurin chain in Table 3-1 and Table 3-2 of the present disclosure that can form a dimer. In some embodiments, provided is the antigen-binding molecule according to any one of the above, wherein the Titin chain comprises the amino acid sequence of SEQ ID NO: 198. In some embodiments, provided is the antigen-binding molecule according to any one of the above, wherein the Obscurin chain comprises the amino acid sequence of SEQ ID NO: 236.

In some embodiments, provided is the antigen-binding molecule according to any one of the above, wherein the CH1 is the CH1 sequence of an IgG. In some embodiments, the CH1 is the CH1 of an IgG1. In some embodiments, the CH1 comprises the amino acid sequence of SEQ ID NO: 84.

In some embodiments, provided is the antigen-binding molecule according to any one of the above, wherein the CL is the light chain constant region of an antibody. In some embodiments, provided is the antigen-binding molecule according to any one of the above, wherein the CL is a kappa or lamada light chain constant region. In some embodiments, the CL comprises the amino acid sequence of SEQ ID NO: 59.

In some embodiments, the amino acid sequence of one subunit of the Fc region is set forth in SEQ ID NO: 86.

In some embodiments, provided is the antigen-binding molecule according to any one of the above, wherein the antigen-binding molecule comprises:

two first chains comprising the amino acid sequence of SEQ ID NO: 125, two second chains comprising the amino acid sequence of SEQ ID NO: 65, and two third chains comprising the amino acid sequence of SEQ ID NO: 98; or
two first chains comprising the amino acid sequence of SEQ ID NO: 125, two second chains comprising the amino acid sequence of SEQ ID NO: 66, and two third chains comprising the amino acid sequence of SEQ ID NO: 98.

In some embodiments, provided is the antigen-binding molecule according to any one of the above, wherein the antigen-binding molecule comprises: two first chains having the amino acid sequence set forth in SEQ ID NO: 125, two second chains having the amino acid sequence set forth in SEQ ID NO: 65, and two third chains having the amino acid sequence set forth in SEQ ID NO: 98.

In another aspect, the present disclosure also provides an anti-GUCY2C antibody capable of specifically binding to GUCY2C. The antibody comprises a heavy chain variable region GUCY2C-VH and a light chain variable region GUCY2C-VL, wherein:

(i) GUCY2C-HCDR1, GUCY2C-HCDR2, and GUCY2C-HCDR3 in the GUCY2C-VH comprise the amino acid sequences of GUCY2C-HCDR1, GUCY2C-HCDR2, and GUCY2C-HCDR3 in SEQ ID NO: 25, respectively, and GUCY2C-LCDR1, GUCY2C-LCDR2, and GUCY2C-LCDR3 in the GUCY2C-VL comprise the amino acid sequences of GUCY2C-LCDR1, GUCY2C-LCDR2, and GUCY2C-LCDR3 in SEQ ID NO: 54, 26, 50, 51, 52, or 53, respectively; or
(ii) GUCY2C-HCDR1, GUCY2C-HCDR2, and GUCY2C-HCDR3 in the GUCY2C-VH comprise the amino acid sequences of GUCY2C-HCDR1, GUCY2C-HCDR2, and GUCY2C-HCDR3 in SEQ ID NO: 37 or 15, respectively, and GUCY2C-LCDR1, GUCY2C-LCDR2, and GUCY2C-LCDR3 in the GUCY2C-VL comprise the amino acid sequences of GUCY2C-LCDR1, GUCY2C-LCDR2, and GUCY2C-LCDR3 in SEQ ID NO: 16, 34, 35, or 36, respectively; or

(iii) GUCY2C-HCDR1, GUCY2C-HCDR2, and GUCY2C-HCDR3 in the GUCY2C-VH comprise the amino acid sequences of GUCY2C-HCDR1, GUCY2C-HCDR2, and GUCY2C-HCDR3 in SEQ ID NO: 42 or 17, respectively, and GUCY2C-LCDR1, GUCY2C-LCDR2, and GUCY2C-LCDR3 in the GUCY2C-VL comprise the amino acid sequences of GUCY2C-LCDR1, GUCY2C-LCDR2, and GUCY2C-LCDR3 in SEQ ID NO: 41 or 18, respectively; or

iv-1) GUCY2C-HCDR1, GUCY2C-HCDR2, and GUCY2C-HCDR3 in the GUCY2C-VH comprise the amino acid sequences of GUCY2C-HCDR1, GUCY2C-HCDR2, and GUCY2C-HCDR3 in SEQ ID NO: 273, respectively, and GUCY2C-LCDR1, GUCY2C-LCDR2, and GUCY2C-LCDR3 in the GUCY2C-VL comprise the amino acid sequences of GUCY2C-LCDR1, GUCY2C-LCDR2, and GUCY2C-LCDR3 in SEQ ID NO: 274, respectively; or

iv-2) GUCY2C-HCDR1, GUCY2C-HCDR2, and GUCY2C-HCDR3 in the GUCY2C-VH comprise the amino acid sequences of GUCY2C-HCDR1, GUCY2C-HCDR2, and GUCY2C-HCDR3 in SEQ ID NO: 275, respectively, and GUCY2C-LCDR1, GUCY2C-LCDR2, and GUCY2C-LCDR3 in the GUCY2C-VL comprise the amino acid sequences of GUCY2C-LCDR1, GUCY2C-LCDR2, and GUCY2C-LCDR3 in SEQ ID NO: 276, respectively; or

iv-3) GUCY2C-HCDR1, GUCY2C-HCDR2, and GUCY2C-HCDR3 in the GUCY2C-VH comprise the amino acid sequences of GUCY2C-HCDR1, GUCY2C-HCDR2, and GUCY2C-HCDR3 in SEQ ID NO: 277, respectively, and GUCY2C-LCDR1, GUCY2C-LCDR2, and GUCY2C-LCDR3 in the GUCY2C-VL comprise the amino acid sequences of GUCY2C-LCDR1, GUCY2C-LCDR2, and GUCY2C-LCDR3 in SEQ ID NO: 278, respectively; or

iv-4) GUCY2C-HCDR1, GUCY2C-HCDR2, and GUCY2C-HCDR3 in the GUCY2C-VH comprise the amino acid sequences of GUCY2C-HCDR1, GUCY2C-HCDR2, and GUCY2C-HCDR3 in SEQ ID NO: 279, respectively, and GUCY2C-LCDR1, GUCY2C-LCDR2, and GUCY2C-LCDR3 in the GUCY2C-VL comprise the amino acid sequences of GUCY2C-LCDR1, GUCY2C-LCDR2, and GUCY2C-LCDR3 in SEQ ID NO: 280, respectively; or

iv-5) GUCY2C-HCDR1, GUCY2C-HCDR2, and GUCY2C-HCDR3 in the GUCY2C-VH comprise the amino acid sequences of GUCY2C-HCDR1, GUCY2C-HCDR2, and GUCY2C-HCDR3 in SEQ ID NO: 330, respectively, and GUCY2C-LCDR1, GUCY2C-LCDR2, and GUCY2C-LCDR3 in the GUCY2C-VL comprise the amino acid sequences of GUCY2C-LCDR1, GUCY2C-LCDR2, and GUCY2C-LCDR3 in SEQ ID NO: 331, respectively; or

iv-6) GUCY2C-HCDR1, GUCY2C-HCDR2, and GUCY2C-HCDR3 in the GUCY2C-VH comprise the amino acid sequences of GUCY2C-HCDR1, GUCY2C-HCDR2, and GUCY2C-HCDR3 in SEQ ID NO: 332, respectively, and GUCY2C-LCDR1, GUCY2C-LCDR2, and GUCY2C-LCDR3 in the GUCY2C-VL comprise the amino acid sequences of GUCY2C-LCDR1, GUCY2C-LCDR2, and GUCY2C-LCDR3 in SEQ ID NO: 333, respectively; or

iv-7) GUCY2C-HCDR1, GUCY2C-HCDR2, and GUCY2C-HCDR3 in the GUCY2C-VH comprise the amino acid sequences of GUCY2C-HCDR1, GUCY2C-HCDR2, and GUCY2C-HCDR3 in SEQ ID NO: 334, respectively, and GUCY2C-LCDR1, GUCY2C-LCDR2, and GUCY2C-LCDR3 in the GUCY2C-VL comprise the amino acid sequences of GUCY2C-LCDR1, GUCY2C-LCDR2, and GUCY2C-LCDR3 in SEQ ID NO: 335, respectively; or

iv-8) GUCY2C-HCDR1, GUCY2C-HCDR2, and GUCY2C-HCDR3 in the GUCY2C-VH comprise the amino acid sequences of GUCY2C-HCDR1, GUCY2C-HCDR2, and GUCY2C-HCDR3 in SEQ ID NO: 336, respectively, and GUCY2C-LCDR1, GUCY2C-LCDR2, and GUCY2C-LCDR3 in the GUCY2C-VL comprise the amino acid sequences of GUCY2C-LCDR1, GUCY2C-LCDR2, and GUCY2C-LCDR3 in SEQ ID NO: 337, respectively; or

iv-9) GUCY2C-HCDR1, GUCY2C-HCDR2, and GUCY2C-HCDR3 in the GUCY2C-VH comprise the amino acid sequences of GUCY2C-HCDR1, GUCY2C-HCDR2, and GUCY2C-HCDR3 in SEQ ID NO: 338, respectively, and GUCY2C-LCDR1, GUCY2C-LCDR2, and GUCY2C-LCDR3 in the GUCY2C-VL comprise the amino acid sequences of GUCY2C-LCDR1, GUCY2C-LCDR2, and GUCY2C-LCDR3 in SEQ ID NO: 339, respectively; or

iv-10) GUCY2C-HCDR1, GUCY2C-HCDR2, and GUCY2C-HCDR3 in the GUCY2C-VH comprise the amino acid sequences of GUCY2C-HCDR1, GUCY2C-HCDR2, and GUCY2C-HCDR3 in SEQ ID NO: 340, respectively, and GUCY2C-LCDR1, GUCY2C-LCDR2, and GUCY2C-LCDR3 in the GUCY2C-VL comprise the amino acid sequences of GUCY2C-LCDR1, GUCY2C-LCDR2, and GUCY2C-LCDR3 in SEQ ID NO: 341, respectively; or

iv-11) GUCY2C-HCDR1, GUCY2C-HCDR2, and GUCY2C-HCDR3 in the GUCY2C-VH comprise the amino acid sequences of GUCY2C-HCDR1, GUCY2C-HCDR2, and GUCY2C-HCDR3 in SEQ ID NO: 342, respectively, and GUCY2C-LCDR1, GUCY2C-LCDR2, and GUCY2C-LCDR3 in the GUCY2C-VL comprise the amino acid sequences of GUCY2C-LCDR1, GUCY2C-LCDR2, and GUCY2C-LCDR3 in SEQ ID NO: 343, respectively; or

iv-12) GUCY2C-HCDR1, GUCY2C-HCDR2, and GUCY2C-HCDR3 in the GUCY2C-VH comprise the amino acid sequences of GUCY2C-HCDR1, GUCY2C-HCDR2, and GUCY2C-HCDR3 in SEQ ID NO: 344, respectively, and GUCY2C-LCDR1, GUCY2C-LCDR2, and GUCY2C-LCDR3 in the GUCY2C-VL comprise the amino acid sequences of GUCY2C-LCDR1, GUCY2C-LCDR2, and GUCY2C-LCDR3 in SEQ ID NO: 345, respectively; or

iv-13) GUCY2C-HCDR1, GUCY2C-HCDR2, and GUCY2C-HCDR3 in the GUCY2C-VH comprise the amino acid sequences of GUCY2C-HCDR1, GUCY2C-HCDR2, and GUCY2C-HCDR3 in SEQ ID NO: 346, respectively, and GUCY2C-LCDR1, GUCY2C-LCDR2, and GUCY2C-LCDR3 in the GUCY2C-VL comprise the amino acid sequences of GUCY2C-LCDR1, GUCY2C-LCDR2, and GUCY2C-LCDR3 in SEQ ID NO: 347, respectively; or

iv-14) GUCY2C-HCDR1, GUCY2C-HCDR2, and GUCY2C-HCDR3 in the GUCY2C-VH comprise the amino acid sequences of GUCY2C-HCDR1, GUCY2C-HCDR2, and GUCY2C-HCDR3 in SEQ ID NO: 348, respectively, and GUCY2C-LCDR1, GUCY2C-LCDR2, and GUCY2C-LCDR3 in the GUCY2C-VL comprise the amino acid sequences of GUCY2C-LCDR1, GUCY2C-LCDR2, and GUCY2C-LCDR3 in SEQ ID NO: 349, respectively; or

iv-15) GUCY2C-HCDR1, GUCY2C-HCDR2, and GUCY2C-HCDR3 in the GUCY2C-VH comprise the amino acid sequences of GUCY2C-HCDR1, GUCY2C-HCDR2, and GUCY2C-HCDR3 in SEQ ID NO: 350, respectively, and GUCY2C-LCDR1, GUCY2C-LCDR2, and GUCY2C-LCDR3 in the GUCY2C-VL comprise the amino acid sequences of GUCY2C-LCDR1, GUCY2C-LCDR2, and GUCY2C-LCDR3 in SEQ ID NO: 351, respectively; or

iv-16) GUCY2C-HCDR1, GUCY2C-HCDR2, and GUCY2C-HCDR3 in the GUCY2C-VH comprise the amino acid sequences of GUCY2C-HCDR1, GUCY2C-HCDR2, and GUCY2C-HCDR3 in SEQ ID NO: 352, respectively, and GUCY2C-LCDR1, GUCY2C-LCDR2, and GUCY2C-LCDR3 in the GUCY2C-VL comprise the amino acid sequences of GUCY2C-LCDR1, GUCY2C-LCDR2, and GUCY2C-LCDR3 in SEQ ID NO: 353, respectively.

In some embodiments, provided is the anti-GUCY2C antibody according to the above, wherein:

(i) GUCY2C-HCDR1, GUCY2C-HCDR2, and GUCY2C-HCDR3 in the GUCY2C-VH comprise the amino acid sequences of GUCY2C-HCDR1, GUCY2C-HCDR2, and GUCY2C-HCDR3 in SEQ ID NO: 25, respectively, and GUCY2C-LCDR1, GUCY2C-LCDR2, and GUCY2C-LCDR3 in the GUCY2C-VL comprise the amino acid sequences of GUCY2C-LCDR1, GUCY2C-LCDR2, and GUCY2C-LCDR3 in SEQ ID NO: 54, respectively, or

(ii) GUCY2C-HCDR1, GUCY2C-HCDR2, and GUCY2C-HCDR3 in the GUCY2C-VH comprise the amino acid sequences of GUCY2C-HCDR1, GUCY2C-HCDR2, and GUCY2C-HCDR3 in SEQ ID NO: 37, respectively, and GUCY2C-LCDR1, GUCY2C-LCDR2, and GUCY2C-LCDR3 in the GUCY2C-VL comprise the amino acid sequences of GUCY2C-LCDR1, GUCY2C-LCDR2, and GUCY2C-LCDR3 in SEQ ID NO: 16, respectively, or

(iii) GUCY2C-HCDR1, GUCY2C-HCDR2, and GUCY2C-HCDR3 in the GUCY2C-VH comprise the amino acid sequences of GUCY2C-HCDR1, GUCY2C-HCDR2, and GUCY2C-HCDR3 in SEQ ID NO: 42, respectively, and GUCY2C-LCDR1, GUCY2C-LCDR2, and GUCY2C-LCDR3 in the GUCY2C-VL comprise the amino acid sequences of GUCY2C-LCDR1, GUCY2C-LCDR2, and GUCY2C-LCDR3 in SEQ ID NO: 41, respectively.

In some embodiments, the GUCY2C-HCDR1, the GUCY2C-HCDR2, the GUCY2C-HCDR3, the GUCY2C-LCDR1, the GUCY2C-LCDR2, and the GUCY2C-LCDR3 are defined according to the Kabat, IMGT, Chothia, AbM, or Contact numbering scheme.

In some embodiments, provided is the anti-GUCY2C antibody according to any one of the above, wherein:

i) the GUCY2C-VH comprises: a GUCY2C-HCDR1 comprising the amino acid sequence of SEQ ID NO: 19, a GUCY2C-HCDR2 comprising the amino acid sequence of SEQ ID NO: 20, and a GUCY2C-HCDR3 comprising the amino acid sequence of SEQ ID NO: 21; and the GUCY2C-VL comprises: a GUCY2C-LCDR1 comprising the amino acid sequence of SEQ ID NO: 47, 22, 43, 44, 45, or 46, a GUCY2C-LCDR2 comprising the amino acid sequence of SEQ ID NO: 23, and a GUCY2C-LCDR3 comprising the amino acid sequence of SEQ ID NO: 24; or

ii) the GUCY2C-VH comprises: a GUCY2C-HCDR1 comprising the amino acid sequence of SEQ ID NO: 4, a GUCY2C-HCDR2 comprising the amino acid sequence of SEQ ID NO: 5, and a GUCY2C-HCDR3 comprising the amino acid sequence of SEQ ID NO: 27 or 6; and the GUCY2C-VL comprises: a GUCY2C-LCDR1 comprising the amino acid sequence of SEQ ID NO: 7, a GUCY2C-LCDR2 comprising the amino acid sequence of SEQ ID NO: 8, 28, 29, or 30, and a GUCY2C-LCDR3 comprising the amino acid sequence of SEQ ID NO: 9; or

iii) the GUCY2C-VH comprises: a GUCY2C-HCDR1 comprising the amino acid sequence of SEQ ID NO: 10, a GUCY2C-HCDR2 comprising the amino acid sequence of SEQ ID NO: 11, and a GUCY2C-HCDR3 comprising the amino acid sequence of SEQ ID NO: 40 or 12; and the GUCY2C-VL comprises: a GUCY2C-LCDR1 comprising the amino acid sequence of SEQ ID NO: 13, a GUCY2C-LCDR2 comprising the amino acid sequence of SEQ ID NO: 39 or 8, and a GUCY2C-LCDR3 comprising the amino acid sequence of SEQ ID NO: 14; or

iv-1) the GUCY2C-VH comprises: a GUCY2C-HCDR1 comprising the amino acid sequence of SEQ ID NO: 250, a GUCY2C-HCDR2 comprising the amino acid sequence of SEQ ID NO: 251, and a GUCY2C-HCDR3 comprising the amino acid sequence of SEQ ID NO: 252; and the GUCY2C-VL comprises: a GUCY2C-LCDR1 comprising the amino acid sequence of SEQ ID NO: 253, a GUCY2C-LCDR2 comprising the amino acid sequence of SEQ ID NO: 254, and a GUCY2C-LCDR3 comprising the amino acid sequence of SEQ ID NO: 255; or

iv-2) the GUCY2C-VH comprises: a GUCY2C-HCDR1 comprising the amino acid sequence of SEQ ID NO: 256, a GUCY2C-HCDR2 comprising the amino acid sequence of SEQ ID NO: 257, and a GUCY2C-HCDR3 comprising the amino acid sequence of SEQ ID NO: 258; and the GUCY2C-VL comprises: a GUCY2C-LCDR1 comprising the amino acid sequence of SEQ ID NO: 259, a GUCY2C-LCDR2 comprising the amino acid sequence of SEQ ID NO: 8, and a GUCY2C-LCDR3 comprising the amino acid sequence of SEQ ID NO: 260; or

iv-3) the GUCY2C-VH comprises: a GUCY2C-HCDR1 comprising the amino acid sequence of SEQ ID NO: 261, a GUCY2C-HCDR2 comprising the amino acid sequence of SEQ ID NO: 262, and a GUCY2C-HCDR3 comprising the amino acid sequence of SEQ ID NO: 263; and the GUCY2C-VL comprises: a GUCY2C-LCDR1 comprising the amino acid sequence of SEQ ID NO: 264, a GUCY2C-LCDR2 comprising the amino acid sequence of SEQ ID NO: 265, and a GUCY2C-LCDR3 comprising the amino acid sequence of SEQ ID NO: 266; or

iv-4) the GUCY2C-VH comprises: a GUCY2C-HCDR1 comprising the amino acid sequence of SEQ ID NO: 267, a GUCY2C-HCDR2 comprising the amino acid sequence of SEQ ID NO: 268, and a GUCY2C-HCDR3 comprising the amino acid sequence of SEQ ID NO: 269; and the GUCY2C-VL comprises: a GUCY2C-LCDR1 comprising the amino acid sequence of SEQ ID NO: 270, a GUCY2C-LCDR2 comprising the amino acid sequence of SEQ ID NO: 271, and a GUCY2C-LCDR3 comprising the amino acid sequence of SEQ ID NO: 272; or

iv-5) the GUCY2C-VH comprises: a GUCY2C-HCDR1 comprising the amino acid sequence of SEQ ID NO: 281, a GUCY2C-HCDR2 comprising the amino acid sequence of SEQ ID NO: 282, and a GUCY2C-HCDR3 comprising the amino acid sequence of SEQ ID NO: 283; and the GUCY2C-VL comprises: a GUCY2C-LCDR1 comprising the amino acid sequence of SEQ ID NO: 284, a GUCY2C-LCDR2 comprising the amino acid sequence of SEQ ID NO: 8, and a GUCY2C-LCDR3 comprising the amino acid sequence of SEQ ID NO: 285; or

iv-6) the GUCY2C-VH comprises: a GUCY2C-HCDR1 comprising the amino acid sequence of SEQ ID NO: 286, a GUCY2C-HCDR2 comprising the amino acid sequence of SEQ ID NO: 287, and a GUCY2C-HCDR3 comprising the amino acid sequence of SEQ ID NO: 288; and the GUCY2C-VL comprises: a GUCY2C-LCDR1 comprising the amino acid sequence of SEQ ID NO: 289, a GUCY2C-LCDR2 comprising the amino acid sequence of SEQ ID NO: 265, and a GUCY2C-LCDR3 comprising the amino acid sequence of SEQ ID NO: 290; or

iv-7) the GUCY2C-VH comprises: a GUCY2C-HCDR1 comprising the amino acid sequence of SEQ ID NO: 291, a GUCY2C-HCDR2 comprising the amino acid sequence of SEQ ID NO: 292, and a GUCY2C-HCDR3 comprising the amino acid sequence of SEQ ID NO: 293; and the GUCY2C-VL comprises: a GUCY2C-LCDR1 comprising the amino acid sequence of SEQ ID NO: 294, a GUCY2C-LCDR2 comprising the amino acid sequence of SEQ ID NO: 295, and a GUCY2C-LCDR3 comprising the amino acid sequence of SEQ ID NO: 296; or

iv-8) the GUCY2C-VH comprises: a GUCY2C-HCDR1 comprising the amino acid sequence of SEQ ID NO: 261, a GUCY2C-HCDR2 comprising the amino acid sequence of SEQ ID NO: 297, and a GUCY2C-HCDR3 comprising the amino acid sequence of SEQ ID NO: 298; and the GUCY2C-VL comprises: a GUCY2C-LCDR1 comprising the amino acid sequence of SEQ ID NO: 264, a GUCY2C-LCDR2 comprising the amino acid sequence of SEQ ID NO: 299, and a GUCY2C-LCDR3 comprising the amino acid sequence of SEQ ID NO: 300; or

iv-9) the GUCY2C-VH comprises: a GUCY2C-HCDR1 comprising the amino acid sequence of SEQ ID NO: 261, a GUCY2C-HCDR2 comprising the amino acid sequence of SEQ ID NO: 301, and a GUCY2C-HCDR3 comprising the amino acid sequence of SEQ ID NO: 302; and the GUCY2C-VL comprises: a GUCY2C-LCDR1 comprising the amino acid sequence of SEQ ID NO: 303, a GUCY2C-LCDR2 comprising the amino acid sequence of SEQ ID NO: 299, and a GUCY2C-LCDR3 comprising the amino acid sequence of SEQ ID NO: 304; or

iv-10) the GUCY2C-VH comprises: a GUCY2C-HCDR1 comprising the amino acid sequence of SEQ ID NO: 305, a GUCY2C-HCDR2 comprising the amino acid sequence of SEQ ID NO: 306, and a GUCY2C-HCDR3 comprising the amino acid sequence of SEQ ID NO: 21; and the GUCY2C-VL comprises: a GUCY2C-LCDR1 comprising the amino acid sequence of SEQ ID NO: 22, a GUCY2C-LCDR2 comprising the amino acid sequence of SEQ ID NO: 23, and a GUCY2C-LCDR3 comprising the amino acid sequence of SEQ ID NO: 24; or

iv-11) the GUCY2C-VH comprises: a GUCY2C-HCDR1 comprising the amino acid sequence of SEQ ID NO: 128, a GUCY2C-HCDR2 comprising the amino acid sequence of SEQ ID NO: 307, and a GUCY2C-HCDR3 comprising the amino acid sequence of SEQ ID NO: 308; and the GUCY2C-VL comprises: a GUCY2C-LCDR1 comprising the amino acid sequence of SEQ ID NO: 309, a GUCY2C-LCDR2 comprising the amino acid sequence of SEQ ID NO: 310, and a GUCY2C-LCDR3 comprising the amino acid sequence of SEQ ID NO: 311; or

iv-12) the GUCY2C-VH comprises: a GUCY2C-HCDR1 comprising the amino acid sequence of SEQ ID NO: 312, a GUCY2C-HCDR2 comprising the amino acid sequence of SEQ ID NO: 313, and a GUCY2C-HCDR3 comprising the amino acid sequence of SEQ ID NO: 314; and the GUCY2C-VL comprises: a GUCY2C-LCDR1 comprising the amino acid sequence of SEQ ID NO: 22, a GUCY2C-LCDR2 comprising the amino acid sequence of SEQ ID NO: 23, and a GUCY2C-LCDR3 comprising the amino acid sequence of SEQ ID NO: 315; or

iv-13) the GUCY2C-VH comprises: a GUCY2C-HCDR1 comprising the amino acid sequence of SEQ ID NO: 286, a GUCY2C-HCDR2 comprising the amino acid sequence of SEQ ID NO: 287, and a GUCY2C-HCDR3 comprising the amino acid sequence of SEQ ID NO: 316; and the GUCY2C-VL comprises: a GUCY2C-LCDR1 comprising the amino acid sequence of SEQ ID NO: 317, a GUCY2C-LCDR2 comprising the amino acid sequence of SEQ ID NO: 265, and a GUCY2C-LCDR3 comprising the amino acid sequence of SEQ ID NO: 300; or

iv-14) the GUCY2C-VH comprises: a GUCY2C-HCDR1 comprising the amino acid sequence of SEQ ID NO: 318, a GUCY2C-HCDR2 comprising the amino acid sequence of SEQ ID NO: 319, and a GUCY2C-HCDR3 comprising the amino acid sequence of SEQ ID NO: 320; and the GUCY2C-VL comprises: a GUCY2C-LCDR1 comprising the amino acid sequence of SEQ ID NO: 321, a GUCY2C-LCDR2 comprising the amino acid sequence of SEQ ID NO: 322, and a GUCY2C-LCDR3 comprising the amino acid sequence of SEQ ID NO: 323; or

iv-15) the GUCY2C-VH comprises: a GUCY2C-HCDR1 comprising the amino acid sequence of SEQ ID NO: 324, a GUCY2C-HCDR2 comprising the amino acid sequence of SEQ ID NO: 325, and a GUCY2C-HCDR3 comprising the amino acid sequence of SEQ ID NO: 326; and the GUCY2C-VL comprises: a GUCY2C-LCDR1 comprising the amino acid sequence of SEQ ID NO: 22, a GUCY2C-LCDR2 comprising the amino acid sequence of SEQ ID NO: 23, and a GUCY2C-LCDR3 comprising the amino acid sequence of SEQ ID NO: 327; or

iv-16) the GUCY2C-VH comprises: a GUCY2C-HCDR1 comprising the amino acid sequence of SEQ ID NO: 261, a GUCY2C-HCDR2 comprising the amino acid sequence of SEQ ID NO: 328, and a GUCY2C-HCDR3 comprising the amino acid sequence of SEQ ID NO: 302; and the GUCY2C-VL comprises: a GUCY2C-LCDR1 comprising the amino acid sequence of SEQ ID NO: 264, a GUCY2C-LCDR2 comprising the amino acid sequence of SEQ ID NO: 329, and a GUCY2C-LCDR3 comprising the amino acid sequence of SEQ ID NO: 300.

In some embodiments, provided is the anti-GUCY2C antibody according to any one of the above, wherein:

i) the GUCY2C-VH comprises: a GUCY2C-HCDR1 comprising the amino acid sequence of SEQ ID NO: 19, a GUCY2C-HCDR2 comprising the amino acid sequence of SEQ ID NO: 20, and a GUCY2C-HCDR3 comprising the amino acid sequence of SEQ ID NO: 21; and the GUCY2C-VL comprises: a GUCY2C-LCDR1 comprising the amino acid sequence of SEQ ID NO: 47, a GUCY2C-LCDR2 comprising the amino acid sequence of SEQ ID NO: 23, and a GUCY2C-LCDR3 comprising the amino acid sequence of SEQ ID NO: 24; or

ii) the GUCY2C-VH comprises: a GUCY2C-HCDR1 comprising the amino acid sequence of SEQ ID NO: 4, a GUCY2C-HCDR2 comprising the amino acid sequence of SEQ ID NO: 5, and a GUCY2C-HCDR3 comprising the amino acid sequence of SEQ ID NO: 27; and the GUCY2C-VL comprises: a GUCY2C-LCDR1 comprising the amino acid sequence of SEQ ID NO: 7, a GUCY2C-LCDR2 comprising the amino acid sequence of SEQ ID NO: 8, and a GUCY2C-LCDR3 comprising the amino acid sequence of SEQ ID NO: 9; or

iii) the GUCY2C-VH comprises: a GUCY2C-HCDR1 comprising the amino acid sequence of SEQ ID NO: 10, a GUCY2C-HCDR2 comprising the amino acid sequence of SEQ ID NO: 11, and a GUCY2C-HCDR3 comprising the amino acid sequence of SEQ ID NO: 40; and the GUCY2C-VL comprises: a GUCY2C-LCDR1 comprising the amino acid sequence of SEQ ID NO: 13, a GUCY2C-LCDR2 comprising the amino acid sequence of SEQ ID NO: 39, and a GUCY2C-LCDR3 comprising the amino acid sequence of SEQ ID NO: 14.

In some embodiments, provided is the anti-GUCY2C antibody according to any one of the above, wherein:

i) the GUCY2C-VH comprises: a GUCY2C-HCDR1 comprising the amino acid sequence of SEQ ID NO: 19, a GUCY2C-HCDR2 comprising the amino acid sequence of SEQ ID NO: 20, and a GUCY2C-HCDR3 comprising the amino acid sequence of SEQ ID NO: 21; and the GUCY2C-VL comprises: a GUCY2C-LCDR1 comprising the amino acid sequence of SEQ ID NO: 22, a GUCY2C-LCDR2 comprising the amino acid sequence of SEQ ID NO: 23, and a GUCY2C-LCDR3 comprising the amino acid sequence of SEQ ID NO: 24; or

ii) the GUCY2C-VH comprises: a GUCY2C-HCDR1 comprising the amino acid sequence of SEQ ID NO: 4, a GUCY2C-HCDR2 comprising the amino acid sequence of SEQ ID NO: 5, and a GUCY2C-HCDR3 comprising the amino acid sequence of SEQ ID NO: 6; and the GUCY2C-VL comprises: a GUCY2C-LCDR1 comprising the amino acid sequence of SEQ ID NO: 7, a GUCY2C-LCDR2 comprising the amino acid sequence of SEQ ID NO: 8, and a GUCY2C-LCDR3 comprising the amino acid sequence of SEQ ID NO: 9; or

iii) the GUCY2C-VH comprises: a GUCY2C-HCDR1 comprising the amino acid sequence of SEQ ID NO: 10, a GUCY2C-HCDR2 comprising the amino acid sequence of SEQ ID NO: 11, and a GUCY2C-HCDR3 comprising the amino acid sequence of SEQ ID NO: 12; and the GUCY2C-VL comprises: a GUCY2C-LCDR1 comprising the amino acid sequence of SEQ ID NO: 13, a GUCY2C-LCDR2 comprising the amino acid sequence of SEQ ID NO: 8, and a GUCY2C-LCDR3 comprising the amino acid sequence of SEQ ID NO: 14.

In some embodiments, provided is the anti-GUCY2C antibody according to any one of the above, wherein:

i) the GUCY2C-VH comprises: a GUCY2C-HCDR1 comprising the amino acid sequence of SEQ ID NO: 19, a GUCY2C-HCDR2 comprising the amino acid sequence of SEQ ID NO: 20, and a GUCY2C-HCDR3 comprising the amino acid sequence of SEQ ID NO: 21; and the GUCY2C-VL comprises: a GUCY2C-LCDR1 comprising the amino acid sequence of SEQ ID NO: 43, a GUCY2C-LCDR2 comprising the amino acid sequence of SEQ ID NO: 23, and a GUCY2C-LCDR3 comprising the amino acid sequence of SEQ ID NO: 24; or

the GUCY2C-VH comprises: a GUCY2C-HCDR1 comprising the amino acid sequence of SEQ ID NO: 19, a GUCY2C-HCDR2 comprising the amino acid sequence of SEQ ID NO: 20, and a GUCY2C-HCDR3 comprising the amino acid sequence of SEQ ID NO: 21; and the GUCY2C-VL comprises: a GUCY2C-LCDR1 comprising the amino acid sequence of SEQ ID NO: 44, a GUCY2C-LCDR2 comprising the amino acid sequence of SEQ ID NO: 23, and a GUCY2C-LCDR3 comprising the amino acid sequence of SEQ ID NO: 24; or

the GUCY2C-VH comprises: a GUCY2C-HCDR1 comprising the amino acid sequence of SEQ ID NO: 19, a GUCY2C-HCDR2 comprising the amino acid sequence of SEQ ID NO: 20, and a GUCY2C-HCDR3 comprising the amino acid sequence of SEQ ID NO: 21; and the GUCY2C-VL comprises: a GUCY2C-LCDR1 comprising the amino acid sequence of SEQ ID NO: 45, a GUCY2C-LCDR2 comprising the amino acid sequence of SEQ ID NO: 23, and a GUCY2C-LCDR3 comprising the amino acid sequence of SEQ ID NO: 24; or

ii) the GUCY2C-VH comprises: a GUCY2C-HCDR1 comprising the amino acid sequence of SEQ ID NO: 4, a GUCY2C-HCDR2 comprising the amino acid sequence of SEQ ID NO: 5, and a GUCY2C-HCDR3 comprising the amino acid sequence of SEQ ID NO: 6; and the GUCY2C-VL comprises: a GUCY2C-LCDR1 comprising the amino acid sequence of SEQ ID NO: 7, a GUCY2C-LCDR2 comprising the amino acid sequence of SEQ ID NO: 28, and a GUCY2C-LCDR3 comprising the amino acid sequence of SEQ ID NO: 9; or

the GUCY2C-VH comprises: a GUCY2C-HCDR1 comprising the amino acid sequence of SEQ ID NO: 4, a GUCY2C-HCDR2 comprising the amino acid sequence of SEQ ID NO: 5, and a GUCY2C-HCDR3 comprising the amino acid sequence of SEQ ID NO: 6; and the GUCY2C-VL comprises: a GUCY2C-LCDR1 comprising the amino acid sequence of SEQ ID NO: 7, a GUCY2C-LCDR2 comprising the amino acid sequence of SEQ ID NO: 30, and a GUCY2C-LCDR3 comprising the amino acid sequence of SEQ ID NO: 9.

In some embodiments, provided is the anti-GUCY2C antibody according to any one of the above, wherein the GUCY2C-HCDR1, the GUCY2C-HCDR2, the GUCY2C-HCDR3, the GUCY2C-LCDR1, the GUCY2C-LCDR2, and the GUCY2C-LCDR3 are defined according to the Kabat numbering scheme.

In some embodiments, provided is the anti-GUCY2C antibody according to any one of the above, wherein the anti-GUCY2C antibody is a murine antibody, a chimeric antibody, or a humanized antibody, preferably a humanized antibody.

In some embodiments, provided is the anti-GUCY2C antibody according to any one of the above, wherein the anti-GUCY2C antibody comprises a heavy chain constant region and a light chain constant region; preferably, the heavy chain constant region is a human IgG1 constant region, and the light chain constant region is selected from the group consisting of constant regions of human antibody κ and λ chains; more preferably, the heavy chain constant region comprises the amino acid sequence of SEQ ID NO: 58, and the light chain constant region comprises the amino acid sequence of SEQ ID NO: 59.

In some embodiments, provided is the anti-GUCY2C antibody according to any one of the above, wherein the GUCY2C-VH and the GUCY2C-VL are humanized and each comprise the FR region of a human antibody.

In some embodiments, provided is the anti-GUCY2C antibody according to any one of the above, wherein:

(i) the GUCY2C-VH comprises: a GUCY2C-HCDR1 comprising the amino acid sequence of SEQ ID NO: 19, a GUCY2C-HCDR2 comprising the amino acid sequence of SEQ ID NO: 20, and a GUCY2C-HCDR3 comprising the amino acid sequence of SEQ ID NO: 21, and the FR region of the GUCY2C-VH comprises one or more amino acid substitutions selected from the group consisting of 1E, 39L, 43K, 69L, 71V, and 73K; and the GUCY2C-VL comprises: a GUCY2C-LCDR1 comprising the amino acid sequence of SEQ ID NO: 47, 22, 43, 44, 45, or 46, a GUCY2C-LCDR2 comprising the amino acid sequence of SEQ ID NO: 23, and a GUCY2C-LCDR3 comprising the amino acid sequence of SEQ ID NO: 24, and the FR region of the GUCY2C-VL comprises one or more amino acid substitutions selected from the group consisting of 2V, 3V, 68R, and 74K; or

(ii) the GUCY2C-VH comprises: a GUCY2C-HCDR1 comprising the amino acid sequence of SEQ ID NO: 4, a GUCY2C-HCDR2 comprising the amino acid sequence of SEQ ID NO: 5, and a GUCY2C-HCDR3 comprising the amino acid sequence of SEQ ID NO: 27 or 6, and the FR region of the GUCY2C-VH comprises one or more amino acid substitutions selected from the group consisting of 1E, 38K, 66K, and 67A; and the GUCY2C-VL comprises: a GUCY2C-LCDR1 comprising the amino acid sequence of SEQ ID NO: 7, a GUCY2C-LCDR2 comprising the amino acid sequence of SEQ ID NO: 8, 28, 29, or 30, and a GUCY2C-LCDR3 comprising the amino acid sequence of SEQ ID NO: 9, and the FR region of the GUCY2C-VL comprises one or more amino acid substitutions selected from the group consisting of 15P, 16G, 42S, 43S, 45K, 46S, 47W, 49Y, 58V, 71Y, and 76S; or

(iii) the GUCY2C-VH comprises: a GUCY2C-HCDR1 comprising the amino acid sequence of SEQ ID NO: 10, a GUCY2C-HCDR2 comprising the amino acid sequence of SEQ ID NO: 11, and a GUCY2C-HCDR3 comprising the amino acid sequence of SEQ ID NO: 40 or 12, and the FR region of the GUCY2C-VH comprises one or more amino acid substitutions selected from the group consisting of 1E, 2F, 28S, 69L, 71V, 73Q, and 75S; and the GUCY2C-VL comprises: a GUCY2C-LCDR1 comprising the amino acid sequence of SEQ ID NO: 13, a GUCY2C-LCDR2 comprising the amino acid sequence of SEQ ID NO: 39 or 8, and a GUCY2C-LCDR3 comprising the amino acid sequence of SEQ ID NO: 14, and the FR region of the GUCY2C-VL comprises one or more amino acid substitutions selected from the group consisting of 42S, 43S, 46P, 47W, 58V, and 71Y.

In some embodiments, provided is the anti-GUCY2C antibody according to any one of the above, wherein:

(i) the GUCY2C-VH comprises: a GUCY2C-HCDR1 comprising the amino acid sequence of SEQ ID NO: 19, a GUCY2C-HCDR2 comprising the amino acid sequence of SEQ ID NO: 20, and a GUCY2C-HCDR3 comprising the amino acid sequence of SEQ ID NO: 21, and the FR region of the GUCY2C-VH comprises one or more amino acid substitutions selected from the group consisting of 1E, 39L, 43K, 69L, 71V, and 73K; and the GUCY2C-VL comprises: a GUCY2C-LCDR1 comprising the amino acid sequence of SEQ ID NO: 47, a GUCY2C-LCDR2 comprising the amino acid sequence of SEQ ID NO: 23, and a GUCY2C-LCDR3 comprising the amino acid sequence of SEQ ID NO: 24, and the FR region of the GUCY2C-VL comprises one or more amino acid substitutions selected from the group consisting of 2V, 3V, 68R, and 74K; or
(ii) the GUCY2C-VH comprises: a GUCY2C-HCDR1 comprising the amino acid sequence of SEQ ID NO: 4, a GUCY2C-HCDR2 comprising the amino acid sequence of SEQ ID NO: 5, and a GUCY2C-HCDR3 comprising the amino acid sequence of SEQ ID NO: 27, and the FR region of the GUCY2C-VH comprises one or more amino acid substitutions selected from the group consisting of 1E, 38K, 66K, and 67A; and the GUCY2C-VL comprises: a GUCY2C-LCDR1 comprising the amino acid sequence of SEQ ID NO: 7, a GUCY2C-LCDR2 comprising the amino acid sequence of SEQ ID NO: 8, and a GUCY2C-LCDR3 comprising the amino acid sequence of SEQ ID NO: 9, and the FR region of the GUCY2C-VL comprises one or more amino acid substitutions selected from the group consisting of 15P, 16G, 42S, 43S, 45K, 46S, 47W, 49Y, 58V, 71Y, and 76S; or
(iii) the GUCY2C-VH comprises: a GUCY2C-HCDR1 comprising the amino acid sequence of SEQ ID NO: 10, a GUCY2C-HCDR2 comprising the amino acid sequence of SEQ ID NO: 11, and a GUCY2C-HCDR3 comprising the amino acid sequence of SEQ ID NO: 40, and the FR region of the GUCY2C-VH comprises one or more amino acid substitutions selected from the group consisting of 1E, 2F, 28S, 69L, 71V, 73Q, and 75S; and the GUCY2C-VL comprises: a GUCY2C-LCDR1 comprising the amino acid sequence of SEQ ID NO: 13, a GUCY2C-LCDR2 comprising the amino acid sequence of SEQ ID NO: 39, and a GUCY2C-LCDR3 comprising the amino acid sequence of SEQ ID NO: 14, and the FR region of the GUCY2C-VL comprises one or more amino acid substitutions selected from the group consisting of 42S, 43S, 46P, 47W, 58V, and 71Y.

In some embodiments, provided is the anti-GUCY2C antibody according to any one of the above, wherein:

i) the GUCY2C-VH comprises the amino acid sequence of SEQ ID NO: 56, 25, or 57, and the GUCY2C-VL comprises the amino acid sequence of SEQ ID NO: 54, 55, 26, 48, 49, 50, 51, 52, or 53; or
ii) the GUCY2C-VH comprises the amino acid sequence of SEQ ID NO: 37, 15, or 38, and the GUCY2C-VL comprises the amino acid sequence of SEQ ID NO: 33, 16, 31, 32, 34, 35, or 36; or
iii) the GUCY2C-VH comprises the amino acid sequence of SEQ ID NO: 42 or 17, and the GUCY2C-VL comprises the amino acid sequence of SEQ ID NO: 41 or 18; or
iv-1) the GUCY2C-VH comprises the amino acid sequence of SEQ ID NO: 273, and the GUCY2C-VL comprises the amino acid sequence of SEQ ID NO: 274; or
iv-2) the GUCY2C-VH comprises the amino acid sequence of SEQ ID NO: 275, and the GUCY2C-VL comprises the amino acid sequence of SEQ ID NO: 276; or
iv-3) the GUCY2C-VH comprises the amino acid sequence of SEQ ID NO: 277, and the GUCY2C-VL comprises the amino acid sequence of SEQ ID NO: 278; or
iv-4) the GUCY2C-VH comprises the amino acid sequence of SEQ ID NO: 279, and the GUCY2C-VL comprises the amino acid sequence of SEQ ID NO: 280; or
iv-5) the GUCY2C-VH comprises the amino acid sequence of SEQ ID NO: 330, and the GUCY2C-VL comprises the amino acid sequence of SEQ ID NO: 331; or
iv-6) the GUCY2C-VH comprises the amino acid sequence of SEQ ID NO: 332, and the GUCY2C-VL comprises the amino acid sequence of SEQ ID NO: 333; or
iv-7) the GUCY2C-VH comprises the amino acid sequence of SEQ ID NO: 334, and the GUCY2C-VL comprises the amino acid sequence of SEQ ID NO: 335; or
iv-8) the GUCY2C-VH comprises the amino acid sequence of SEQ ID NO: 336, and the GUCY2C-VL comprises the amino acid sequence of SEQ ID NO: 337; or
iv-9) the GUCY2C-VH comprises the amino acid sequence of SEQ ID NO: 338, and the GUCY2C-VL comprises the amino acid sequence of SEQ ID NO: 339; or

iv-10) the GUCY2C-VH comprises the amino acid sequence of SEQ ID NO: 340, and the GUCY2C-VL comprises the amino acid sequence of SEQ ID NO: 341; or

iv-11) the GUCY2C-VH comprises the amino acid sequence of SEQ ID NO: 342, and the GUCY2C-VL comprises the amino acid sequence of SEQ ID NO: 343; or

iv-12) the GUCY2C-VH comprises the amino acid sequence of SEQ ID NO: 344, and the GUCY2C-VL comprises the amino acid sequence of SEQ ID NO: 345; or

iv-13) the GUCY2C-VH comprises the amino acid sequence of SEQ ID NO: 346, and the GUCY2C-VL comprises the amino acid sequence of SEQ ID NO: 347; or

iv-14) the GUCY2C-VH comprises the amino acid sequence of SEQ ID NO: 348, and the GUCY2C-VL comprises the amino acid sequence of SEQ ID NO: 349; or

iv-15) the GUCY2C-VH comprises the amino acid sequence of SEQ ID NO: 350, and the GUCY2C-VL comprises the amino acid sequence of SEQ ID NO: 351; or

iv-16) the GUCY2C-VH comprises the amino acid sequence of SEQ ID NO: 352, and the GUCY2C-VL comprises the amino acid sequence of SEQ ID NO: 353.

In some embodiments, provided is the antigen-binding molecule according to any one of the above, wherein:

i) the GUCY2C-VH comprises the amino acid sequence of SEQ ID NO: 56, and the GUCY2C-VL comprises the amino acid sequence of SEQ ID NO: 54, 55, 48, 49, 50, 51, or 52, or

the GUCY2C-VH comprises the amino acid sequence of SEQ ID NO: 25, and the GUCY2C-VL comprises the amino acid sequence of SEQ ID NO: 26, or

ii) the GUCY2C-VH comprises the amino acid sequence of SEQ ID NO: 37, and the GUCY2C-VL comprises the amino acid sequence of SEQ ID NO: 33, or

the GUCY2C-VH comprises the amino acid sequence of SEQ ID NO: 38, and the GUCY2C-VL comprises the amino acid sequence of SEQ ID NO: 33, 32, 34, or 36, or the GUCY2C-VH comprises the amino acid sequence of SEQ ID NO: 15, and the GUCY2C-VL comprises the amino acid sequence of SEQ ID NO: 16, or

iii) the GUCY2C-VH comprises the amino acid sequence of SEQ ID NO: 42, and the GUCY2C-VL comprises the amino acid sequence of SEQ ID NO: 41, or

the GUCY2C-VH comprises the amino acid sequence of SEQ ID NO: 17, and the GUCY2C-VL comprises the amino acid sequence of SEQ ID NO: 18.

In some embodiments, provided is the antigen-binding molecule according to any one of the above, wherein:

i) the GUCY2C-VH comprises the amino acid sequence of SEQ ID NO: 56, and the GUCY2C-VL comprises the amino acid sequence of SEQ ID NO: 54, or

the GUCY2C-VH comprises the amino acid sequence of SEQ ID NO: 56, and the GUCY2C-VL comprises the amino acid sequence of SEQ ID NO: 55; or

ii) the GUCY2C-VH comprises the amino acid sequence of SEQ ID NO: 37, and the GUCY2C-VL comprises the amino acid sequence of SEQ ID NO: 33; or

iii) the GUCY2C-VH comprises the amino acid sequence of SEQ ID NO: 42, and the GUCY2C-VL comprises the amino acid sequence of SEQ ID NO: 41.

In some embodiments, provided is the antigen-binding molecule according to any one of the above, wherein the amino acid sequence of the GUCY2C-VH is set forth in SEQ ID NO: 56, and the amino acid sequence of the GUCY2C-VL is set forth in SEQ ID NO: 54.

In some embodiments, provided is the antigen-binding molecule according to any one of the above, wherein the amino acid sequence of the GUCY2C-VH is set forth in SEQ ID NO: 56, and the amino acid sequence of the GUCY2C-VL is set forth in SEQ ID NO: 55.

In some embodiments, provided is the antigen-binding molecule according to any one of the above, wherein the amino acid sequence of the GUCY2C-VH is set forth in SEQ ID NO: 37, and the amino acid sequence of the GUCY2C-VL is set forth in SEQ ID NO: 33.

In some embodiments, provided is the antigen-binding molecule according to any one of the above, wherein the amino acid sequence of the GUCY2C-VH is set forth in SEQ ID NO: 42, and the amino acid sequence of the GUCY2C-VL is set forth in SEQ ID NO: 41.

In some embodiments, provided is the anti-GUCY2C antibody according to any one of the above, wherein the anti-GUCY2C antibody comprises a heavy chain and a light chain, wherein:

the heavy chain comprises an amino acid sequence having at least 85% sequence identity to SEQ ID NO: 64, and the light chain comprises an amino acid sequence having at least 85% sequence identity to SEQ ID NO: 65 or 66; or the heavy chain comprises an amino acid sequence having at least 85% sequence identity to SEQ ID NO: 60, and the light chain comprises an amino acid sequence having at least 85% sequence identity to SEQ ID NO: 61; or the heavy chain comprises an amino acid sequence having at least 85% sequence identity to SEQ ID NO: 62, and the light chain comprises an amino acid sequence having at least 85% sequence identity to SEQ ID NO: 63.

In some embodiments, provided is the anti-GUCY2C antibody according to any one of the above, wherein the anti-GUCY2C antibody comprises a heavy chain and a light chain, wherein:

the heavy chain comprises the amino acid sequence of SEQ ID NO: 64, and the light chain comprises the amino acid sequence of SEQ ID NO: 65 or 66; or
the heavy chain comprises the amino acid sequence of SEQ ID NO: 60, and the light chain comprises the amino acid sequence of SEQ ID NO: 61; or
the heavy chain comprises the amino acid sequence of SEQ ID NO: 62, and the light chain comprises the amino acid sequence of SEQ ID NO: 63.

In some embodiments, provided is the anti-GUCY2C antibody according to any one of the above, wherein the anti-GUCY2C antibody comprises a heavy chain and a light chain; the amino acid sequence of the heavy chain is set forth in SEQ ID NO: 64, and the amino acid sequence of the light chain is set forth in SEQ ID NO: 65.

In some embodiments, provided is the anti-GUCY2C antibody according to any one of the above, wherein the anti-GUCY2C antibody comprises a heavy chain and a light chain; the amino acid sequence of the heavy chain is set forth in SEQ ID NO: 64, and the amino acid sequence of the light chain is set forth in SEQ ID NO: 66.

In some embodiments, provided is the anti-GUCY2C antibody according to any one of the above, wherein the anti-GUCY2C antibody comprises a heavy chain and a light chain; the amino acid sequence of the heavy chain is set forth in SEQ ID NO: 60, and the amino acid sequence of the light chain is set forth in SEQ ID NO: 61.

In some embodiments, provided is the anti-GUCY2C antibody according to any one of the above, wherein the anti-GUCY2C antibody comprises a heavy chain and a light chain; the amino acid sequence of the heavy chain is set forth in SEQ ID NO: 62, and the amino acid sequence of the light chain is set forth in SEQ ID NO: 63.

In some embodiments, provided is the anti-GUCY2C antibody according to any one of the above, wherein the anti-GUCY2C antibody is an antibody fragment, and the antibody fragment is Fab, Fab', F(ab')2, Fd, Fv, scFv, dsFv, or dAb.

**In** some embodiments, provided is the anti-GUCY2C antibody according to any one of the above, wherein the anti-GUCY2C antibody is a bispecific antibody.

In some embodiments, provided is the anti-GUCY2C antibody according to any one of the above, wherein the anti-GUCY2C antibody is a bispecific antibody that specifically binds to GUCY2C and CD3.

**In** another aspect, the present disclosure provides a pharmaceutical composition comprising: a therapeutically effective amount of the antigen-binding molecule according to any one of the above or the anti-GUCY2C antibody according to any one of the above, and one or more pharmaceutically acceptable carriers, diluents, buffers, or excipients.

In another aspect, the present disclosure also provides an isolated nucleic acid encoding the antigen-binding molecule according to any one of the above or the anti-GUCY2C antibody according to any one of the above.

In another aspect, the present disclosure also provides a vector comprising the isolated nucleic acid according to the above.

In another aspect, the present disclosure also provides a host cell comprising the isolated nucleic acid according to the above.

In another aspect, the present disclosure also provides a method for treating a disease, wherein the method comprises administering to a subject a therapeutically effective amount of the antigen-binding molecule according to any one of the above or the anti-GUCY2C antibody according to any one of the above or the composition according to any one of the above.

In another aspect, the present disclosure also provides use of the antigen-binding molecule according to any one of the above or the antibody according to any one of the above or the composition thereof in the preparation of a medicament for treating a disease.

In another aspect, the present disclosure also provides the antigen-binding molecule according to any one of the above or the anti-GUCY2C antibody according to any one of the above or the composition according to any one of the above for use as a medicament. In some embodiments, the medicament is used for treating a disease.

In some embodiments, the disease according to any one of the above is a proliferative disease or a tumor. In some embodiments, the disease according to any one of the above is a tumor, which is gastrointestinal cancer (gastric cancer (gastric adenocarcinoma) and intestinal cancer (small intestine cancer and colorectal cancer (colon cancer and rectal cancer))), esophageal cancer (i.e., esophagus cancer), anal cancer, liver cancer (hepatocellular carcinoma), gallbladder cancer, bile duct cancer, pancreatic cancer, prostate cancer, renal cancer (renal cell carcinoma), urothelial cancer, breast cancer, bladder cancer, thymus cancer, melanoma, glioma (glioblastoma), sarcoma (osteosarcoma), ovarian cancer, thyroid cancer, cervical squamous cell cancer, endometrial cancer, lung cancer (non-small cell lung cancer (lung squamous cell cancer and lung adenocarcinoma) and small cell lung cancer), skin cancer, head and neck cancer (head and neck squamous cell cancer), brain cancer, glioblastoma multiforme, gastroesophageal cancer (gastroesophageal adenocarcinoma), adenocarcinomas metastatic to the liver, multiple myeloma, lymphoma (Hodgkin's lymphoma, non-Hodgkin's lymphoma, or B-cell lymphoma), or leukemia (such as acute myeloid leukemia (AML), acute lymphoblastic leukemia (ALL), and chronic lymphocytic leukemia (CLL)).

In some embodiments, the cells of the tumor according to the above or vascular endothelial cells close to the tumor express GUCY2C.

In some embodiments, the tumor according to the above is a solid tumor.

In some embodiments, the tumor according to the above is a solid tumor, which is gastrointestinal cancer (gastric cancer (gastric adenocarcinoma) and intestinal cancer (small intestine cancer and colorectal cancer (colon cancer and rectal cancer))), esophageal cancer (i.e., esophagus cancer), anal cancer, liver cancer (hepatocellular carcinoma), gallbladder cancer, bile duct cancer, pancreatic cancer, prostate cancer, renal cancer (renal cell carcinoma), urothelial cancer, breast cancer, bladder cancer, thymus cancer, melanoma, glioma (glioblastoma), sarcoma (osteosarcoma), ovarian cancer, thyroid cancer, cervical squamous cell cancer, adenocarcinoma of endometrium, lung cancer (non-small cell lung cancer (lung squamous cell cancer and lung adenocarcinoma), and small cell lung cancer), skin cancer, head and neck cancer (head and neck squamous cell cancer), brain cancer, glioblastoma multiforme, gastroesophageal cancer (gastroesophageal adenocarcinoma), or adenocarcinomas metastatic to the liver.

In some embodiments, the tumor according to the above is gastrointestinal cancer; preferably gastric cancer or intestinal cancer, and more preferably gastric adenocarcinoma, small intestine cancer, or colorectal cancer.

In some embodiments, the tumor according to the above is a non-solid tumor.

In some embodiments, the tumor according to the above is a non-solid tumor, which is multiple myeloma, lymphoma (Hodgkin's lymphoma, non-Hodgkin's lymphoma, or B-cell lymphoma), or leukemia (acute myeloid leukemia (AML), acute lymphoblastic leukemia (ALL), and chronic lymphocytic leukemia (CLL)).

In some embodiments, the aforementioned disease is a disease associated with GUCY2C; in some embodiments, the aforementioned disease is a disease expressing GUCY2C.

In some embodiments, provided is the method for treating a disease according to the above, which further comprises using a second therapeutic agent. In some embodiments, the second therapeutic agent includes an anti-tumor agent, radiation therapy, an antibody-drug conjugate, a bispecific antibody, a bispecific antibody conjugated to an anti-tumor agent, an immune checkpoint inhibitor, or a combination thereof.

In some embodiments, provided is the method for treating a disease according to the above, wherein the second therapeutic agent is administered simultaneously, sequentially, or separately with the antigen-binding molecule according to any one of the embodiments in the present disclosure.

In some embodiments, provided is the method for treating a disease according to the above, wherein the second therapeutic agent is a bispecific antibody that specifically binds to CD28 and EGFR.

In some embodiments, provided is the method for treating a disease according to the above, wherein the second therapeutic agent is a bispecific antibody that specifically binds to CD28 and EGFR, which comprises at least one antigen-binding moiety that specifically binds to CD28 and at least one antigen-binding moiety that specifically binds to EGFR.

In some embodiments, provided is the method for treating a disease according to the above, wherein the second therapeutic agent is a bispecific antibody that specifically binds to CD28 and EGFR, which comprises at least one antigen-binding moiety that specifically binds to CD28 and at least one antigen-binding moiety that specifically binds to EGFR; the antigen-binding moiety that specifically binds to CD28 comprises a heavy chain variable region CD28-VH and a light chain variable region CD28-VL, and the antigen-binding moiety that specifically binds to EGFR comprises a heavy chain variable region EGFR-VH and a light chain variable region EGFR-VL.

In some embodiments, provided is the method for treating a disease according to the above, wherein:

  i) the CD28-VH comprises: a CD28-HCDR1 comprising the amino acid sequence of SEQ ID NO: 146, a CD28-HCDR2 comprising the amino acid sequence of SEQ ID NO: 147, and a CD28-HCDR3 comprising the amino acid sequence of SEQ ID NO: 148; and the CD28-VL comprises: a CD28-LCDR1 comprising the amino acid sequence of SEQ ID NO: 149, a CD28-LCDR2 comprising the amino acid sequence of SEQ ID NO: 150, and a CD28-LCDR3 comprising the amino acid sequence of SEQ ID NO: 151; or

  ii) the CD28-VH comprises: a CD28-HCDR1 comprising the amino acid sequence of SEQ ID NO: 160, a CD28-HCDR2 comprising the amino acid sequence of SEQ ID NO: 161, and a CD28-HCDR3 comprising the amino acid sequence of SEQ ID NO: 162; and the CD28-VL comprises: a CD28-LCDR1 comprising the amino acid sequence of SEQ ID NO: 149, a CD28-LCDR2 comprising the amino acid sequence of SEQ ID NO: 150, and a CD28-LCDR3 comprising the amino acid sequence of SEQ ID NO: 163; or

  iii) the CD28-VH comprises: a CD28-HCDR1 comprising the amino acid sequence of SEQ ID NO: 168, a CD28-HCDR2 comprising the amino acid sequence of SEQ ID NO: 169, and a CD28-HCDR3 comprising the amino acid sequence of SEQ ID NO: 170; and the CD28-VL comprises: a CD28-LCDR1 comprising the amino acid sequence of SEQ ID NO: 171, a CD28-LCDR2 comprising the amino acid sequence of SEQ ID NO: 172, and a CD28-LCDR3 comprising the amino acid sequence of SEQ ID NO: 173.

In some embodiments, provided is the method for treating a disease according to the above, wherein:

i) the CD28-VH comprises the amino acid sequence of SEQ ID NO: 154, and the CD28-VL comprises the amino acid sequence of SEQ ID NO: 155; or
ii) the CD28-VH comprises the amino acid sequence of SEQ ID NO: 164, and the CD28-VL comprises the amino acid sequence of SEQ ID NO: 165; or
iii) the CD28-VH comprises the amino acid sequence of SEQ ID NO: 174, and the CD28-VL comprises the amino acid sequence of SEQ ID NO: 175.

In some embodiments, provided is the method for treating a disease according to the above, wherein the antigen-binding moiety that specifically binds to EGFR comprises a heavy chain variable region EGFR-VH and a light chain variable region EGFR-VL, wherein:
the EGFR-VH comprises: an EGFR-HCDR1 comprising the amino acid sequence of SEQ ID NO: 140, an EGFR-HCDR2 comprising the amino acid sequence of SEQ ID NO: 141, and an EGFR-HCDR3 comprising the amino acid sequence of SEQ ID NO: 142; and the EGFR-VL comprises: an EGFR-LCDR1 comprising the amino acid sequence of SEQ ID NO: 143, an EGFR-LCDR2 comprising the amino acid sequence of SEQ ID NO: 144, and an EGFR-LCDR3 comprising the amino acid sequence of SEQ ID NO: 145.

In some embodiments, provided is the method for treating a disease according to the above, wherein the EGFR-VH comprises the amino acid sequence of SEQ ID NO: 152, and the EGFR-VL comprises the amino acid sequence of SEQ ID NO: 153.

In some embodiments, provided is the method for treating a disease according to the above, wherein the bispecific antibody that specifically binds to CD28 and EGFR comprises an Fc region. In some embodiments, the Fc region is an IgG Fc region. In some embodiments, the Fc region is an IgG1 Fc region. In some embodiments, the Fc region comprises one or more amino acid substitutions capable of reducing the binding of the Fc region to an Fcγ receptor. In some embodiments, the Fc region is a human IgG1 Fc region, and the amino acids at positions 234 and 235 are A, as numbered according to the EU index.

In some embodiments, provided is the method for treating a disease according to the above, wherein the bispecific antibody that specifically binds to CD28 and EGFR comprises an Fc region comprising a first subunit Fc1 and a second subunit Fc2 capable of associating with each other, and the Fc1 and the Fc2 each independently have one or more amino acid substitutions that reduce homodimerization of the Fc region. In some embodiments, the Fc1 has a knob structure according to the knob-into-hole technique, and the Fc2 has a hole structure according to the knob-into-hole technique. In some embodiments, for the Fc1, the amino acid at position 366 is W, and for the Fc2, the amino acid at position 366 is S, the amino acid at position 368 is A, and the amino acid at position 407 is V, as numbered according to the EU index. In some embodiments, for the Fc1, the amino acid at position 354 is C and the amino acid at position 366 is W, and for the Fc2, the amino acid at position 349 is C, the amino acid at position 366 is S, the amino acid at position 368 is A, and the amino acid at position 407 is V, as numbered according to the EU index. In some embodiments, the Fc1 comprises the amino acid sequence of SEQ ID NO: 248, and the Fc2 comprises the amino acid sequence of SEQ ID NO: 249.

In some embodiments, provided is the antigen-binding molecule according to any one of the above, wherein the Titin chain and the Obscurin chain are any Titin chain and Obscurin chain in Table 3-1 and Table 3-2 of the present disclosure that can form a dimer. In some embodiments, provided is the antigen-binding molecule according to any one of the above, wherein the Titin chain comprises the amino acid sequence of SEQ ID NO: 198. In some embodiments, provided is the antigen-binding molecule according to any one of the above, wherein the Obscurin chain comprises the amino acid sequence of SEQ ID NO: 236.

In some embodiments, provided is the antigen-binding molecule according to any one of the above, wherein the linkers 8 are all peptide linkers known in the art, so long as the antigen-binding molecule is capable of exhibiting the desired antigen binding activity. For example, the peptide linkers may be flexible peptides comprising 1-50 or 3-20 amino acid residues. In some embodiments, the peptide linker is 3-15 amino acid residues in length. In some embodiments, the peptide linkers each independently have a structure of (GGGGS)n, wherein n is 1, 2, or 3. In some embodiments, the sequence of the linker 8 is GGGGS (SEQ ID NO: 90).

In some embodiments, provided is the antigen-binding molecule according to any one of the above, wherein the CH1 is the CH1 sequence of an IgG. In some embodiments, the CH1 is the CH1 of an IgG1. In some embodiments, the CH1 comprises the amino acid sequence of SEQ ID NO: 84.

In some embodiments, provided is the antigen-binding molecule according to any one of the above, wherein the CL is the light chain constant region of an antibody. In some embodiments, provided is the antigen-binding molecule according to any one of the above, wherein the CL is a kappa or lamada light chain constant region. In some embodiments, the CL comprises the amino acid sequence of SEQ ID NO: 59.

In some embodiments, provided is the method for treating a disease according to the above, wherein the bispecific antibody that specifically binds to CD28 and EGFR comprises one antigen-binding moiety that specifically binds to CD28 and one antigen-binding moiety that specifically binds to EGFR, the antigen-binding moiety that specifically binds to CD28 is a replaced Fab comprising a Titin chain and an Obscurin chain capable of forming a dimer, and the antigen-binding moiety that specifically binds to EGFR is a Fab.

In some embodiments, provided is the method for treating a disease according to the above, wherein the bispecific antibody that specifically binds to CD28 and EGFR comprises one first chain having a structure represented by formula (m), a second chain having a structure represented by formula (n), a third chain having a structure represented by formula (o), and a fourth chain having a structure represented by formula (p):

(m) [CD28-VH]-[linker 8]-[Titin chain]-[Fc1],
(n) [CD28-VL]-[linker 8]-[Obscurin chain],
(o) [EGFR-VH]-[CH1]-[Fc2], and
(p) [EGFR-VL]-[CL],
wherein the linker 8 is a peptide linker; or the linker 8 is absent;
the structures represented by formulas (m), (n), (o), and (p) are arranged from N-terminus to C-terminus.

In some embodiments, provided is the method for treating a disease according to the above, wherein the bispecific antibody that specifically binds to CD28 and EGFR comprises: one first chain comprising the amino acid sequence of SEQ ID NO: 156, one second chain comprising the amino acid sequence of SEQ ID NO: 157, one third chain comprising the amino acid sequence of SEQ ID NO: 158, and one fourth chain comprising the amino acid sequence of SEQ ID NO: 159; or
the bispecific antibody that specifically binds to CD28 and EGFR comprises: one first chain comprising the amino acid sequence of SEQ ID NO: 166, one second chain comprising the amino acid sequence of SEQ ID NO: 167, one third chain comprising the amino acid sequence of SEQ ID NO: 158, and one fourth chain comprising the amino acid sequence of SEQ ID NO: 159.

In some embodiments, provided is the method for treating a disease according to the above, wherein the bispecific antibody that specifically binds to CD28 and EGFR comprises: one first chain having the amino acid sequence set forth in SEQ ID NO: 156, one second chain having the amino acid sequence set forth in SEQ ID NO: 157, one third chain having the amino acid sequence set forth in SEQ ID NO: 158, and one fourth chain having the amino acid sequence set forth in SEQ ID NO: 159.

In some embodiments, provided is the method for treating a disease according to the above, wherein the bispecific antibody that specifically binds to CD28 and EGFR comprises: one first chain having the amino acid sequence set forth in SEQ ID NO: 166, one second chain having the amino acid sequence set forth in SEQ ID NO: 167, one third chain having the amino acid sequence set forth in SEQ ID NO: 158, and one fourth chain having the amino acid sequence set forth in SEQ ID NO: 159.

In some embodiments, provided is the method for treating a disease according to the above, wherein the bispecific antibody that specifically binds to CD28 and EGFR comprises one antigen-binding moiety that specifically binds to CD28 and one antigen-binding moiety that specifically binds to EGFR, the antigen-binding moiety that specifically binds to CD28 is a Fab, and the antigen-binding moiety that specifically binds to EGFR is a replaced Fab comprising a Titin chain and an Obscurin chain capable of forming a dimer.

In some embodiments, provided is the method for treating a disease according to the above, wherein the bispecific antibody that specifically binds to CD28 and EGFR comprises one first chain having a structure represented by formula (q), a second chain having a structure represented by formula (r), a third chain having a structure represented by formula (s), and a fourth chain having a structure represented by formula (t):

(q) [CD28-VH]-[CH1]-[Fc1],
(r) [CD28-VL]-[CL],
(s) [EGFR-VH]-[linker 8]-[Obscurin chain]-[Fc2], and
(t) [EGFR-VL]-[linker 8]-[Titin chain],
wherein the linker 8 is a peptide linker; or the linker 8 is absent;
the structures represented by formulas (q), (r), (s), and (t) are arranged from N-terminus to C-terminus.

In some embodiments, provided is the method for treating a disease according to the above, wherein the bispecific antibody that specifically binds to CD28 and EGFR comprises: one first chain comprising the amino acid sequence of SEQ ID NO: 176, one second chain comprising the amino acid sequence of SEQ ID NO: 177, one third chain comprising the amino acid sequence of SEQ ID NO: 178, and one fourth chain comprising the amino acid sequence of SEQ ID NO: 179.

In some embodiments, provided is the method for treating a disease according to the above, wherein the bispecific antibody that specifically binds to CD28 and EGFR comprises: one first chain having the amino acid sequence set forth in SEQ ID NO: 176, one second chain having the amino acid sequence set forth in SEQ ID NO: 177, one third chain having the amino acid sequence set forth in SEQ ID NO: 178, and one fourth chain having the amino acid sequence set forth in SEQ ID NO: 179.

The antigen-binding molecule provided by the present disclosure has the following characteristics: good therapeutic activity, safety, pharmacokinetic properties, and druggability (e.g., stability).

## BRIEF DESCRIPTION OF THE DRAWINGS

FIG. 1A: a structural schematic diagram of Format 1 of GUCY2C-CD3 bispecific antibody; FIG. 1B: a structural schematic diagram of Format 2 of GUCY2C-CD3 bispecific antibody, wherein Ob represents Obscurin; FIG. 1C: a structural schematic diagram of Format 3 of GUCY2C-CD3 bispecific antibody, wherein Ob represents Obscurin; FIG. 1D: a structural schematic diagram of Format 4 of GUCY2C-CD3 bispecific antibody; FIG. 1E: a structural schematic diagram of Format 5 of GUCY2C-CD3 bispecific antibody, wherein Ob represents Obscurin.

FIGs. 2A-2C: the ability of GUCY2C monoclonal antibodies to bind to HCT116/hGUCY2C cells; FIG. 2D: the ability of GUCY2C monoclonal antibodies to bind to HT-55 cells; FIG. 2E: the ability of GUCY2C monoclonal antibodies to bind to T84 cells.

FIG. 3A: the ability of GUCY2C-CD3 bispecific antibodies to bind to HCT116/hGUCY2C cells; FIG. 3B: the ability of GUCY2C-CD3 bispecific antibodies to bind to HT55 cells; FIG. 3C: the ability of GUCY2C-CD3 bispecific antibodies to bind to Jurkat cells.

FIG. 4A: the ability of antibodies to activate Jurkat/NFAT cells tested using HCT116/hGUCY2C as target cells; FIG. 4B: the ability of antibodies to activate Jurkat/NFAT cells tested using LS1034 as target cells.

FIG. 5A: the results for IFN-$\gamma$ release under the combined action of the GUCY2C-CD3 bispecific antibody and LS1034 cells; FIGs. 5B and 5C: the results for IFN-$\gamma$ release under the combined action of the GUCY2C-CD3 bispecific antibody and LS174T cells.

FIG. 6A: the results for IL-6 release under the combined action of the GUCY2C-CD3 bispecific antibody and LS1034 cells; FIGs. 6B and 6C: the results for IL-6 release under the combined action of the GUCY2C-CD3 bispecific antibody and LS174T cells.

## DETAILED DESCRIPTION

### Terminology

The terminology used herein is for the purpose of describing embodiments only and is not intended to be limiting. Unless defined otherwise, all technical and scientific terms used herein have the same meaning as commonly understood by one of ordinary skill in the art to which the present disclosure pertains.

Unless the context clearly requires otherwise, throughout the description and the claims, the words "comprise", "have", "include", and the like are to be understood in an inclusive sense as opposed to an exclusive or exhaustive sense; that is to say, in the sense of "including, but not limited to". Unless otherwise stated, "comprise" includes "consist of". For example, for a GUCY2C-HCDR1 comprising the amino acid sequence of SEQ ID NO: 1, it specifically encompasses a GUCY2C-HCDR1 whose amino acid sequence is set forth in SEQ ID NO: 1.

The three-letter and single-letter codes used in the present disclosure for amino acids are as described in J. Biol. Chem, 243, p3558 (1968).

The term "and/or", e.g., "X and/or Y", should be understood to mean "X and Y" or "X or Y" and should be used to provide explicit support for both meanings or either meaning.

The term "CD28" (cluster of differentiation 28, Tp44) refers to any CD28 protein from any vertebrate source, including mammals such as primates (e.g., humans), non-human primates (e.g., cynomolgus monkeys), and rodents (e.g., mice and rats). CD28 is expressed on T cells and provides costimulatory signals required for T cell activation and survival. In addition to the T cell receptor (TCR), stimulation of T cells by CD28 may also provide an effective signal for the production of various interleukins. CD28 is a receptor of the CD80 (B7.1) and CD86 (B7.2) proteins and is the only B7 receptor constitutively expressed on naive T cells. The amino acid sequence of human CD28 is shown in UniProt (www.uniprot.org) under the accession number P10747.

The term "amino acid" refers to naturally occurring and synthetic amino acids, as well as amino acid analogs and amino acid mimics that function in a manner similar to the naturally occurring amino acids. Naturally occurring amino acids are those encoded by genetic codes and those amino acids later modified, e.g., hydroxyproline, $\gamma$-carboxyglutamic acid, and O-phosphoserine. Amino acid analogs refer to compounds that have a substantially identical chemical structure (i.e., an $\alpha$ carbon that binds to hydrogen, carboxyl, amino, and an R group) to naturally occurring amino acids, e.g., homoserine, norleucine, methionine sulfoxide, and methioninemethyl sulfonium. Such analogs have a modified R group (e.g., norleucine) or a modified peptide skeleton, but retain a substantially identical chemical structure to naturally occurring amino acids. Amino acid mimics refer to chemical compounds that have a different structure from amino acids, but function in a manner similar to naturally occurring amino acids.

The term "amino acid mutation" includes amino acid substitutions, deletions, insertions, and modifications. Any combination of substitutions, deletions, insertions, and modifications can be made to obtain a final construct, as long as the final construct possesses the desired properties, such as reduced binding to the Fc receptor. Amino acid sequence deletions and insertions include deletions and insertions at the amino terminus and/or the carboxyl terminus of a polypeptide chain. Specific amino acid mutations may be amino acid substitutions. In one embodiment, the amino acid mutation is a non-conservative amino acid substitution, i.e., replacement of one amino acid with another amino acid having different structural and/or chemical properties. Amino acid substitutions include replacement with non-naturally occurring amino acids or with derivatives of the 20 native amino acids (e.g., 4-hydroxyproline, 3-methylhistidine, ornithine, homoserine, and 5-hydroxylysine). Amino acid mutations can be generated using genetic or chemical methods well known in the art. Genetic methods may include site-directed mutagenesis, PCR, gene synthesis, and the like. It is contemplated that methods for altering amino acid side chain groups other than genetic engineering, such as chemical modification, may also be used. Various names may be used herein to indicate the same amino acid mutation. Herein, the expression of "position + amino acid residue" may be used to denote an amino acid residue at a specific position. For example, 366W indicates that the amino acid residue at position 366 is W. T366W indicates that the amino acid residue at position 366 is mutated from the original T to W.

The term "antigen-binding molecule" is used in the broadest sense and encompasses a variety of molecules that specifically bind to an antigen, including but not limited to antibodies, other polypeptides having antigen binding activity, and antibody fusion proteins in which the two are fused, as long as they exhibit desired antigen binding activity. The antigen-binding molecule herein comprises a variable region (VH) and a variable region (VL) which together comprise an antigen-binding domain. Illustratively, the antigen-binding molecules herein are bispecific antigen-binding molecules (e.g., bispecific antibodies).

The term "antibody" is used in the broadest sense and encompasses a variety of antibody structures, including but not limited to monoclonal antibodies, polyclonal antibodies; monospecific antibodies, multispecific antibodies (e.g., bispecific antibodies), and full-length antibodies, and antibody fragments (or antigen-binding fragments, or antigen-binding moieties), as long as they exhibit the desired antigen binding activity. For example, a native IgG antibody is a heterotetrameric glycoprotein of about 150,000 Daltons composed of two identical light chains and two identical heavy chains linked by a disulfide bond. From the N-terminus to the C-terminus, each heavy chain has one variable region (VH), also known as variable heavy domain or heavy chain variable region, followed by three constant domains (CH1, CH2, and CH3). Similarly, from the N-terminus to the C-terminus, each light chain has one variable region (VL), also known as variable light domain or light chain variable domain, followed by one constant light domain (light chain constant region, CL).

The term "bispecific antibody" refers to an antibody (including an antibody or an antigen-binding fragment thereof, such as a single-chain antibody) capable of specifically binding to two different antigens or at least two different epitopes of the same antigen. Bispecific antibodies of various structures have been disclosed in the prior art; the bispecific antibodies can be classified into IgG-like bispecific antibodies and antibody-fragment-type bispecific antibodies according to the integrity of IgG molecules; the bispecific antibodies can be classified into bivalent, trivalent, tetravalent or higher-valent bispecific antibodies according to the number of the antigen-binding regions; the bispecific antibodies can be classified into symmetric bispecific antibodies and asymmetric bispecific antibodies according to the presence of symmetry in their structures. Among them, the bispecific antibodies based on antibody fragments, such as Fab fragments lacking Fc fragments, formed by binding 2 or more Fab fragments in one molecule, have low immunogenicity, small molecular weight and high tumor tissue permeability, and typical antibody structures of this type are, e.g., F(ab)$_2$, scFv-Fab, and (scFv)$_2$-Fab; IgG-like bispecific antibodies (e.g., having Fc fragments) have relatively large molecular weight, and the Fc fragments facilitate the purification of the antibodies and increase their solubility and stability, and the Fc fragments may further bind to the receptor FcRn and increase the serum half-life of the antibodies, and typical structural models of bispecific antibodies are, e.g., KiH, CrossMAb, Triomab quadroma, Fc$\Delta$Adp, ART-Ig, BiMAb, Biclonics, BEAT, DuoBody, Azymetric, XmAb, 2:1 TCBs, 1Fab-IgG TDB, FynomAb, two-in-one/DAF, scFv-Fab-IgG, DART-Fc, LP- DART, CODV-Fab-TL, HLE-BiTE, F(ab)2-CrossMAb, IgG-(scFv)2, Bs4Ab, DVD-Ig, Tetravalent-DART-Fc, (scFv)4-Fc, CODV-Ig, mAb2, and F(ab)4-CrossMAb (see Aran F. Labrijn et al., Nature Reviews Drug Discovery, volume 18, pages 585-608 (2019); Chen S1 et al., J Immunol Res., Feb. 11, 2019; 2019:4516041).

The term "variable region" or "variable domain" refers to a domain in an antigen-binding molecule that binds to an antigen. Herein, in the antigen-binding moiety that specifically binds to GUCY2C a heavy chain variable region is denoted by GUCY2C-VH, and a light chain variable region is denoted by GUCY2C-VL; in the antigen-binding moiety that specifically binds to CD3, a heavy chain variable region is denoted by CD3-VH, and a light chain variable region is denoted by CD3-VL. VH and VL each comprise four conserved framework regions (FRs) and three complementarity determining regions (CDRs). The term "complementarity determining region" or "CDR" refers to a region in the variable domain that primarily contributes to antigen binding; "framework" or "FR" refers to variable domain residues other than CDR residues. A VH comprises 3 CDRs: HCDR1, HCDR2, and HCDR3; a VL comprises 3 CDRs: LCDR1, LCDR2, and LCDR3. Herein, the 3 CDRs in GUCY2C-VH are denoted by GUCY2C-HCDR1, GUCY2C-HCDR2, and GUCY2C-HCDR3, respectively; the 3 CDRs in GUCY2C-VL are denoted by GUCY2C-LCDR1, GUCY2C-LCDR2, and GUCY2C-LCDR3, respectively; the 3 CDRs in CD3-VH are denoted by CD3-HCDR1, CD3-HCDR2, and CD3-HCDR3, respectively; the 3 CDRs in CD3-VL are denoted by CD3-LCDR1, CD3-LCDR2, and CD3-LCDR3, respectively. Each VH and VL is, when viewed from N-terminus to C-terminus: FR1, CDR1, FR2, CDR2, FR3, CDR3, FR4. A single VH or VL may be sufficient to provide antigen binding specificity.

The amino acid sequence boundaries of the CDRs can be determined by a variety of well-known schemes, for example, the "Kabat" numbering scheme (see Kabat et al. (1991), Sequences of Proteins of Immunological Interest, 5th ed., Public Health Service, National Institutes of Health, Bethesda, MD), the "Chothia" numbering scheme, the "ABM" numbering scheme, the "Contact" numbering scheme (see Martin, ACR. Protein Sequence and Structure Analysis of Antibody Variable Domains[J]. 2001), and the ImMunoGenTics (IMGT) numbering scheme (Lefranc, M.P. et al., Dev. Comp. Immunol., 27, 55-77 (2003); Front Immunol., Oct. 16, 2018; 9: 2278), and the like. The corresponding relationships between the various numbering schemes are well known to those skilled in the art. The numbering schemes of the present disclosure are shown in Table 1 below.

Table 1. Relationships between CDR numbering schemes

| CDR | IMGT | Kabat | AbM | Chothia | Contact |
|---|---|---|---|---|---|
| HCDR1 | 27-38 | 31-35 | 26-35 | 26-32 | 30-35 |
| HCDR2 | 56-65 | 50-65 | 50-58 | 52-56 | 47-58 |
| HCDR3 | 105-117 | 95-102 | 95-102 | 95-102 | 93-101 |
| LCDR1 | 27-38 | 24-34 | 24-34 | 24-34 | 30-36 |
| LCDR2 | 56-65 | 50-56 | 50-56 | 50-56 | 46-55 |
| LCDR3 | 105-117 | 89-97 | 89-97 | 89-97 | 89-96 |

Unless otherwise stated, the "Kabat" numbering scheme is applied to the sequences of the variable regions and CDRs in examples of the present disclosure.

The term "antibody fragment" refers to a molecule different from an intact antibody, which comprises a moiety of an intact antibody that retains the antigen binding ability of the intact antibody. Examples of antibody fragments include, but are not limited to, Fv, Fab, Fab', Fab'-SH, F(ab')$_2$, single-domain antibodies, single-chain Fab (scFab), diabodies, linear antibodies, single-chain antibody molecules (e.g., scFv), and multispecific antibodies (e.g., bispecific antibodies) formed from antibody fragments. The term "Fc region" or "fragment crystallizable region" is used to define the C-terminal region of the heavy chain of an antibody, including native Fc regions and engineered Fc regions. In some embodiments, the Fc region comprises two subunits, which may be identical or different. In some embodiments, the Fc region of the human IgG heavy chain is defined as extending from the amino acid residue at position Cys226 or from Pro230 to its carboxyl terminus. Suitable native sequence Fc regions for the antibodies described herein include human IgG1, IgG2 (IgG2A, IgG2B), IgG3, and IgG4. Unless otherwise stated, the numbering scheme for the Fc region is the EU index. The term "Titin chain" refers to a peptide fragment of 78-118 amino acids in length comprising a Titin Ig-like 152 domain in a Titin protein or a functional variant thereof, wherein the Titin chain is capable of binding to an Obscurin Ig-like 1 domain or an Obscurin-like Ig-like 1 domain to form a dimerized complex. The term "Obscurin chain" refers to a peptide fragment of 87-117 amino acids in length comprising an Obscurin Ig-like 1 domain in an Obscurin protein or a functional variant thereof, or a peptide fragment of 78-118 amino acids in length comprising an Obscurin-like Ig-like 1 domain in an Obscurin-like 1 protein or a functional variant thereof, wherein the Obscurin chain is capable of binding to a Titin Ig-like 152 domain to form a dimerized complex. The Titin chain and the Obscurin chain of the present disclosure can be used to replace the CH1 and CL in a Fab to form a replaced Fab (Fab-S), and the replacement does not affect the binding of the antigen-binding molecule to an antigen.

The term "chimeric" antibody refers to an antibody in which a portion of the heavy and/or light chain is derived from a particular source or species and the remainder of the heavy and/or light chain is derived from a different source or species.

The term "humanized" antibody refers to an antibody that retains the reactivity of a non-human antibody while having low immunogenicity in humans. For example, this can be achieved by retaining the non-human CDRs and replacing the remainder of the antibody with its human counterparts (i.e., the constant regions and the framework region portion of the variable regions).

The term "affinity" refers to the overall strength of the non-covalent interaction between a single binding site of a molecule (e.g., an antibody) and its binding ligand (e.g., an antigen). Unless otherwise indicated, as used herein, "binding affinity" refers to an internal binding affinity that reflects a 1:1 interaction between members of a binding pair (e.g., an antibody and an antigen). The affinity of a molecule X for its ligand Y can be generally expressed by the equilibrium dissociation constant (KD). Affinity can be determined by conventional methods known in the art, including those described herein. The term "kassoc" or "ka" refers to the association rate of a particular antibody-antigen interaction, while the term "kdis" or "kd" as used herein refers to the dissociation rate of a particular antibody-antigen interaction. As used herein, the term "KD" refers to the equilibrium dissociation constant, which is the ratio of kd to ka (i.e., kd/ka) and expressed as molar concentration (M). The KD value of an antibody can be measured using methods known in the art, such as surface plasmon resonance, ELISA, or solution equilibrium titration (SET).

The term "monoclonal antibody" refers to a population of substantially homogeneous antibodies, that is, the amino acid sequences of the antibody molecules comprised in the population are identical, except for a small number of natural mutations that may exist. In contrast, polyclonal antibody formulations generally comprise a plurality of different antibodies having different amino acid sequences in their variable domains, which are generally specific for different epitopes. "Monoclonal" refers to the characteristics of an antibody obtained from a population of substantially homogeneous antibodies and should not be construed as requiring the production of the antibody by any particular method. In some embodiments, the antibody provided by the present disclosure is a monoclonal antibody.

The term "antigen" refers to a molecule or a portion of a molecule that is capable of being bound by a selective binding agent of an antigen-binding protein (e.g., an antibody). An antigen may have one or more epitopes capable of interacting with different antigen-binding proteins (e.g., antibodies).

The term "epitope" refers to an area or region on an antigen that is capable of specifically binding to an antibody or an antigen-binding fragment thereof. Epitopes can be formed by contiguous amino acids (linear epitopes) or comprise non-contiguous amino acids (conformational epitopes), e.g., non-contiguous amino acids that are in spatial proximity to each other due to folding of an antigen (i.e., by tertiary folding of an antigen of a protein nature). The difference between the conformational epitope and the linear epitope is that in the presence of denaturing solvents, the binding of the antibody to the conformational epitope is lost. An epitope comprises at least 3, at least 4, at least 5, at least 6, at least 7, or 8-10 amino acids in a unique spatial conformation. Screening for antibodies that bind to particular epitopes (i.e., those that bind to identical epitopes) can be performed using routine methods in the art, such as but not limited to, alanine scanning, peptide blotting (see Meth. Mol. Biol. 248 (2004) 443-463), peptide cleavage analysis, epitope excision, epitope extraction, chemical modification of the antigen (see Prot. Sci. 9 (2000) 487-496), and cross-blocking (see "Antibodies", Harlow and Lane (Cold Spring Harbor Press, Cold Spring Harb., NY)).

The term "capable of specifically binding", "specifically binding", or "binding" means that an antibody is capable of binding to a certain antigen or epitope with higher affinity than to other antigens or epitopes. Generally, an antibody binds to an antigen or epitope with an equilibrium dissociation constant (KD) of about $1 \times 10^{-7}$ M or less (e.g., about $1 \times 10^{-8}$ M or less). In some embodiments, the KD for the binding of an antibody to an antigen is 10% or less (e.g., 1%) of the KD for the binding of the antibody to a non-specific antigen (e.g., BSA or casein). KD may be determined using known methods, for example, by a FACS or surface plasmon resonance assay. However, an antibody that specifically binds to an antigen or an epitope within the antigen may have cross-reactivity to other related antigens, e.g., to corresponding antigens from other species (homologous), such as humans or monkeys, e.g., *Macaca fascicularis* (cynomolgus, cyno), *Pan troglodytes* (chimpanzee, chimp), or *Callithrix jacchus* (commonmarmoset, marmoset).

The term "not bind" means that the antibody is not capable of binding to a certain antigen or an epitope within the antigen in the manner of the specific binding described above, for example, when the antibody binds to the antigen or the epitope within the antigen with an equilibrium dissociation constant (KD) of about $1 \times 10^{-6}$ M or greater. The term "antigen-binding moiety" refers to a polypeptide molecule specifically binding to a target antigen. A specific antigen-binding moiety includes an antigen-binding domain of an antibody, e.g., comprises a heavy chain variable region and a light chain variable region. The term "antigen-binding moiety that specifically binds to GUCY2C" refers to a moiety that is capable of binding to GUCY2C with sufficient affinity, such that a molecule containing the moiety can be used as a diagnostic agent and/or therapeutic agent targeting GUCY2C. For example, the antigen-binding moiety that specifically binds to GUCY2C has the following equilibrium dissociation constant (KD): < about 10 nM, as measured by a surface plasmon resonance assay. Antigen-binding moieties include antibody fragments as defined herein, e.g., a Fab, a replaced Fab, or an scFv.

The term "linker" refers to a linker unit that links two polypeptide fragments. Herein, the linkers present in the same structural formula may be identical or different. The linker may be a peptide linker comprising one or more amino acids, typically about 1-30, 2-24, or 3-15 amino acids. The linkers used herein may be identical or different. When "-" appears in a structural formula, it means that the units on both sides are directly linked by a covalent bond.

"Tm" is the temperature of dissolution and denaturation (endogenous fluorescence). When the protein is denatured (by heating or by a denaturant), the tertiary structure is opened and the aromatic amino acid microenvironment changes, resulting in a change in the emission fluorescence spectrum. In the present disclosure, Tm1 refers to the temperature at which the fluorescence value changes to half of the maximum value.

"Tonset" is the onset temperature of denaturation. It refers to the temperature at which the protein begins to denature, i.e., the temperature at which the fluorescence value begins to change.

"Tagg" is the onset temperature of aggregation. The temperature at which the sample begins to aggregate is monitored by detecting aggregates at two wavelengths of 266 nm and 473 nm by static light scattering. Tagg 266 refers to the onset temperature of aggregation monitored at 266 nm.

The term "nucleic acid" is used interchangeably herein with the term "polynucleotide" and refers to deoxyribonucleotide or ribonucleotide and a polymer thereof in either single-stranded or double-stranded form. The term encompasses nucleic acids comprising known nucleotide analogs or modified backbone residues or linkages, which are synthetic, naturally occurring, and non-naturally occurring, have similar binding properties to the reference nucleic acid, and are metabolized in a manner similar to the reference nucleotide. Examples of such analogs include, but are not limited to, phosphorothioate, phosphoramidate, methylphosphonate, chiral-methylphosphonate, 2-O-methyl ribonucleotide, and peptide-nucleic acid (PNA).

"Isolated nucleic acid" refers to a nucleic acid molecule that has been separated from components of its natural environment. The isolated nucleic acid includes a nucleic acid molecule comprised in a cell that generally comprises the nucleic acid molecule, but the nucleic acid molecule is present extrachromosomally or at a chromosomal position different from its natural chromosomal position. An isolated nucleic acid encoding the antigen-binding molecule refers to one or more nucleic acid molecules encoding antibody heavy and light chains (or fragments thereof), including such nucleic acid molecule(s) in a single vector or separate vectors and such nucleic acid molecule(s) present at one or more locations in a host cell. Unless otherwise stated, a particular nucleic acid sequence also implicitly encompasses conservatively modified variants thereof (e.g., degenerate codon substitutions) and complementary sequences, as well as the sequence explicitly indicated. Specifically, as detailed below, degenerate codon substitutions may be obtained by generating sequences in which the third position of one or more selected (or all) codons is substituted with degenerate bases and/or deoxyinosine residues.

The terms "polypeptide" and "protein" are used interchangeably herein and refer to a polymer of amino acid residues. The terms apply to amino acid polymers in which one or more amino acid residues are corresponding artificial chemical mimics of naturally occurring amino acids, as well as to naturally occurring amino acid polymers and non-naturally occurring amino acid polymers. Unless otherwise stated, a particular polypeptide sequence also implicitly encompasses conservatively modified variants thereof.

The term "sequence identity" refers to the degree (percentage) to which the amino acids/nucleic acids of two sequences are identical at equivalent positions when the two sequences are optimally aligned. In the alignment process, gaps may be allowed to be introduced, if necessary, to achieve the maximum percent sequence identity, but any conservative substitution is not considered a part that constitutes sequence identity. To determine percent sequence identity, alignments can be accomplished by techniques known to those skilled in the art, for example, using publicly available computer software, such as BLAST, BLAST-2, ALIGN, ALIGN-2, or Megalign (DNASTAR) software. Those skilled in the art can determine parameters suitable for measuring alignment, including any algorithms required to achieve maximum alignment of the full length of the aligned sequences.

The term "fusion" or "linkage" means that components (e.g., antigen-binding moieties and Fc domains) are covalently linked directly or via a linker.

The term "vector" means a polynucleotide molecule capable of transporting another polynucleotide linked thereto. One type of vector is a "plasmid", which refers to a circular double-stranded DNA loop into which additional DNA segments can be ligated. Another type of vector is a viral vector, such as an adeno-associated viral vector (AAV or AAV2), wherein additional DNA segments can be ligated into the viral genome. Certain vectors are capable of autonomous replication in a host cell into which they are introduced (e.g., bacterial vectors having a bacterial origin of replication and episomal mammalian vectors). Other vectors (e.g., non-episomal mammalian vectors) can be integrated into the genome of a host cell upon introduction into the host cell, and thereby are replicated along with the host genome. The term "expression vector" or "expression construct" refers to a vector that is applied to transform a host cell and comprises a nucleic acid sequence that directs and/or controls (along with the host cell) the expression of one or more heterologous coding regions operably linked thereto. Expression constructs may include, but are not limited to, sequences that affect or control transcription and translation and affect RNA splicing of a coding region operably linked thereto in the presence of an intron.

The terms "host cell", "host cell line", and "host cell culture" are used interchangeably and refer to cells into which exogenous nucleic acids have been introduced, including progenies of such cells. Host cells include "transformants" and "transformed cells", which include primary transformed cells and progeny derived therefrom, regardless of the number of passages. Progeny may not be completely identical to parent cells in terms of nucleic acid content and may contain mutations. As used herein, the term includes mutant progenies that have the same function or biological activity as the cells screened or selected from the primary transformed cells. Host cells include prokaryotic and eukaryotic host cells, wherein eukaryotic host cells include, but are not limited to, mammalian cells, insect cell lines, plant cells, and fungal cells. Mammalian host cells include human, mouse, rat, canine, monkey, porcine, goat, bovine, equine, and hamster cells, including but not limited to, Chinese hamster ovary (CHO) cells, NSO, SP2 cells, HeLa cells, baby hamster kidney (BHK) cells, monkey kidney cells (COS), human hepatocellular carcinoma cells (e.g., Hep G2), A549 cells, 3T3 cells, and HEK-293 cells. Fungal cells include yeast and filamentous fungal cells, including, for example, *Pichiapastoris, Pichia finlandica, Pichia trehalophila, Pichia koclamae, Pichia membranaefaciens, Pichia minuta (Ogataea minuta, Pichia lindneri), Pichiaopuntiae, Pichia thermotolerans, Pichia salictaria, Pichia guercuum, Pichia pijperi, Pichia stiptis, Pichia methanolica,* Pichia, *Saccharomycescerevisiae,* Saccharomyces, *Hansenula polymorpha,* Kluyveromyces, *Kluyveromyces lactis, Candida albicans, Aspergillus nidulans, Aspergillus niger, Aspergillus oryzae, Trichoderma reesei, Chrysosporium lucknowense, Fusarium* sp., *Fusarium gramineum, Fusarium venenatum, Physcomitrella patens,* and *Neurospora crassa.* Pichia, any Saccharomyces, *Hansenula polymorpha,* any Kluyveromyces, *Candida albicans,* any Aspergillus, *Trichoderma reesei, Chrysosporium lucknowense,* any Fusarium, *Yarrowia lipolytica,* and *Neurospora crassa.* The host cell of the present patent does not include objects not authorized by the Patent Laws.

As used in the present application, the expressions "cell", "cell line", and "cell culture" are used interchangeably, and all such designations include their progenies. Thus, the words "transformant" and "transformed cell" include the primary subject cell and cultures derived therefrom, regardless of the passage number. It is also understood that not all progeny have precisely identical DNA contents due to deliberate or inadvertent mutations. Variant progeny that have the identical function or biological activity as the original transformed cell from which they were selected are included.

"Optional" or "optionally" means that the event or circumstance subsequently described may, but does not necessarily, occur, and this description includes instances where the event or circumstance occurs or does not occur.

The term "pharmaceutical composition" refers to a mixture comprising one or more of the antigen-binding molecules or antibodies described herein and other chemical components; the other components are, for example, physiological/pharmaceutically acceptable carriers and excipients.

The term "pharmaceutically acceptable carrier" refers to an ingredient in a pharmaceutical formulation that is different from the active ingredient and is not toxic to the subject. Pharmaceutically acceptable carriers include, but are not limited to, buffers, excipients, stabilizers, or preservatives.

The term "subject" or "individual" includes humans and non-human animals. Non-human animals include all vertebrates (e.g., mammals and non-mammals) such as non-human primates (e.g., cynomolgus monkeys), sheep, dogs, cows, chickens, amphibians, and reptiles. Unless clearly indicated, the terms "patient" and "subject" are used interchangeably herein. As used herein, the term "cynomolgus monkey (cyno)" or "cynomolgus" refers to Macaca fascicularis. In certain embodiments, the individual or subject is a human.

"Administrating" or "giving", when applied to animals, humans, experimental subjects, cells, tissues, organs, or biological fluids, refers to contact of an exogenous drug, a therapeutic agent, a diagnostic agent, or a composition with the animals, humans, subjects, cells, tissues, organs, or biological fluids.

The term "sample" refers to a collection of similar fluids, cells or tissues isolated from a subject, as well as fluids, cells or tissues present within a subject. Exemplary samples are biological fluids (such as blood; serum; serosal fluids; plasma; lymph; urine; saliva; cystic fluids; tears; excretions; sputum; mucosal secretions of secretory tissue and organs; vaginal secretions; ascites; fluids in the pleura, pericardium, peritoneum, abdominal cavity, and other body cavities; fluids collected from bronchial lavage; synovial fluids; liquid solutions in contact with a subject or biological source, e.g., cell and organ culture media (including cell or organ conditioned culture media); lavage fluids; and the like), tissue biopsy samples, fine needle punctures, surgically excised tissues, organ cultures, or cell cultures.

"Treatment" or "treat" (and grammatical variations thereof) refers to clinical intervention intended to be applied to the treated individual, which may be performed either for prophylactic purposes or during the course of clinical pathology. Desirable effects of the treatment include, but are not limited to, preventing the occurrence or recurrence of a disease, alleviating symptoms, alleviating/reducing any direct or indirect pathological consequences of the disease, preventing metastasis, decreasing the rate of disease progression, ameliorating or alleviating the disease state, and regressing or improving prognosis. In some embodiments, the molecule of the present disclosure is used to delay the development of or slow the progression of disease.

The terms "recurrence", "relapse", or "relapsed" refers to the return of a cancer or disease after clinical assessment of the disappearance of disease. A diagnosis of distant metastasis or local recurrence can be considered a relapse.

The term "refractory" or "resistant" refers to a cancer or disease that has not responded to treatment.

"Effective amount" is generally an amount sufficient to reduce the severity and/or frequency of symptoms, eliminate symptoms and/or underlying causes, prevent the appearance of symptoms and/or their underlying causes, and/or ameliorate or alleviate damage (e.g., lung disease) caused by or associated with a disease state. In some examples, the effective amount is a therapeutically effective amount or a prophylactically effective amount. "Therapeutically effective amount" is an amount sufficient to treat a disease state or symptom, particularly a state or symptom associated with the disease state, or to otherwise prevent, hinder, delay, or reverse the progression of the disease state or any other undesirable symptoms associated with the disease in any way. "Prophylactically effective amount" is an amount that, when administered to a subject, will have a predetermined prophylactic effect, e.g., preventing or delaying the onset (or recurrence) of the disease state, or reducing the likelihood of the onset (or recurrence) of the disease state or associated symptoms. A complete therapeutic or prophylactic effect does not necessarily occur after administration of one dose and may occur after administration of a series of doses. Thus, a therapeutically or prophylactically effective amount may be administered in one or more doses. "Therapeutically effective amount" and "prophylactically effective amount" may vary depending on a variety of factors such as the disease state, age, sex, and weight of the individual, and the ability of a therapeutic agent or combination of therapeutic agents to elicit a desired response in the individual. Exemplary indicators of an effective therapeutic agent or combination of therapeutic agents include, for example, improved health of a patient.

The term "immune checkpoint" refers to the group of molecules on the cell surface of CD4 T cells and CD8 T cells. These molecules can effectively act as "brakes" that down-regulate or inhibit anti-tumor immune responses. Immune checkpoint molecules include, but are not limited to, programmed death receptor 1 (PD-1), cytotoxic T lymphocyte antigen 4 (CTLA-4), B7H1, B7H4, OX-40, CD137, CD40, and LAG-3, which directly inhibit immune cells.

Immune checkpoint inhibitors for use in the present disclosure are substances that inhibit the function of immune checkpoint molecules. The immune checkpoint inhibitor is not particularly limited as long as the inhibitor is a substance that can inhibit the function (signal) of the immune checkpoint molecule. Inhibitors that may be used as immune checkpoint inhibitors in the methods of the present disclosure include, but are not limited to, inhibitors of PD-1, PD-L2, CTLA-4, TIM-3, LAG-3, VISTA, BTLA, TIGIT, LAIR1, CD160, 2B4, CEACAM (e.g., CEACAM-1, CEACAM-3, and/or CEACAM-5), and/or TGFR$\beta$. Inhibition of the inhibitory molecules can be performed by inhibition at the DNA, RNA or protein level. In embodiments, an inhibitory nucleic acid (e.g., dsRNA, siRNA, or shRNA) can be used to inhibit the expression of an inhibitory molecule. In other embodiments, the inhibitor of an inhibitory signal is a polypeptide that binds to an inhibitory molecule, e.g., a soluble ligand or an antibody. Examples of immune checkpoint inhibitors used in the present disclosure may include, but are not particularly limited to, anti-PD-1 antibodies, anti-PD-L1 antibodies, and anti-CTLA-4 antibodies, and anti-PD-1 antibodies and anti-PD-L1 antibodies may be preferred.

## Antigen-Binding Molecules of the Present Disclosure

The present disclosure provides an antigen-binding molecule having a number of advantageous properties, such as good *in vitro* killing activity, therapeutic activity, safety, pharmacokinetic properties, and druggability (e.g., yield, purity, and stability).

## Exemplary Antigen-Binding Molecules

The antigen-binding molecule of the present disclosure includes antigen-binding molecules (e.g., bispecific antibodies) that specifically bind to GUCY2C and CD3 and anti-GUCY2C antibodies. Particularly, the antigen-binding molecule of the present disclosure has any one of the following properties:

a. High affinity for GUCY2C. In some embodiments, the GUCY2C monoclonal antibody binds to human GUCY2C with a KD value of less than 100 nM (e.g., less than 80 nM, less than 50 nM, less than 10 nM, less than 8 nM, less than 1 nM, or less than 0.5 nM) as measured by Biacore.

b. Good specific binding to GUCY2C on the surface of cells. In some embodiments, the binding activity of the GUCY2C monoclonal antibody is better than 1608. In some embodiments, the GUCY2C-CD3 bispecific antibody of the present disclosure has good specific binding activity for cells expressing different levels of GUCY2C, and the binding activity is better than that of 1608-BsAb; the GUCY2C-CD3 bispecific antibody of the present disclosure has good specific binding activity for cells expressing Jurkat, and the binding activity is better than that of 1608-BsAb.

c. No non-specific binding to non-target cells (e.g., T cells). In some embodiments, the GUCY2C monoclonal antibody does not non-specifically bind to Jurkat cells that do not express GUCY2C; 1608 significantly binds to Jurkat cells. In some embodiments, the GUCY2C monoclonal antibody of the present disclosure does not non-specifically bind to PBMCs and T cells that do not express GUCY2C, and the control antibody 1608 does significantly non-specifically bind to PBMCs and T cells. The target specificity of the GUCY2C monoclonal antibody is better than that of 1608.

d. The antigen epitope bound by the GUCY2C monoclonal antibody is significantly different from that bound by 1608, and thus the GUCY2C monoclonal antibody can not compete with 1608 for binding to the GUCY2C antigen.

e. Good binding effect with human GUCY2C protein. In some embodiments, the GUCY2C-CD3 bispecific antibody binds to human GUCY2C with a KD value of less than 1 nM (e.g., less than 0.5 nM, less than 0.2 nM, or less than 0.1 nM), and the EC$_{50}$ value measured by ELISA.

f. The GUCY2C-CD3 bispecific antibody exhibits good cytotoxic activity for target cells with different expression levels.

g. The GUCY2C-CD3 bispecific antibody can effectively induce the release of IFN-γ related to cytotoxic activity, and the release level of cytokine IL-6 related to inflammation is relatively low.

h. Stronger *in vivo* therapeutic activity. In some embodiments, the GUCY2C-CD3 bispecific antibody, or the GUCY2C-CD3 bispecific antibody in combination with an EGFR-CD28 bispecific antibody, has good *in vivo* therapeutic activity. When the GUCY2C-CD3 bispecific antibody is used in combination with the EGFR-CD28 bispecific antibody, the respective drug effect of the two can be effectively improved.

The present disclosure provides an antigen-binding molecule comprising at least one antigen-binding moiety that specifically binds to GUCY2C and at least one antigen-binding moiety that specifically binds to CD3, wherein the antigen-binding moiety that specifically binds to GUCY2C comprises a heavy chain variable region GUCY2C-VH and a light chain variable region GUCY2C-VL, and the antigen-binding moiety that specifically binds to CD3 comprises a heavy chain variable region CD3-VH and a light chain variable region CD3-VL.

Specifically, the examples herein disclose the following antibody series: P3-C7, 81, and 146. Antibody P3-C7 is described below as an example of the antibodies herein.

Provided is an antigen-binding molecule that specifically binds to GUCY2C and CD3 or an anti-GUCY2C antibody, wherein:

the GUCY2C-VH comprises: a GUCY2C-HCDR1 comprising the amino acid sequence of SEQ ID NO: 19, a GUCY2C-HCDR2 comprising the amino acid sequence of SEQ ID NO: 20, and a GUCY2C-HCDR3 comprising the amino acid sequence of SEQ ID NO: 21; and the GUCY2C-VL comprises: a GUCY2C-LCDR1 comprising the amino acid sequence of SEQ ID NO: 47, 22, 43, 44, 45, or 46, a GUCY2C-LCDR2 comprising the amino acid sequence of SEQ ID NO: 23, and a GUCY2C-LCDR3 comprising the amino acid sequence of SEQ ID NO: 24.

Provided is an antigen-binding molecule that specifically binds to GUCY2C and CD3 or an anti-GUCY2C antibody, wherein:

the GUCY2C-VH comprises: a GUCY2C-HCDR1 having the amino acid sequence set forth in SEQ ID NO: 19, a GUCY2C-HCDR2 having the amino acid sequence set forth in SEQ ID NO: 20, and a GUCY2C-HCDR3 having the amino acid sequence set forth in SEQ ID NO: 21; and the GUCY2C-VL comprises: a GUCY2C-LCDR1 having the amino acid sequence set forth in SEQ ID NO: 47, a GUCY2C-LCDR2 having the amino acid sequence set forth in SEQ ID NO: 23, and a GUCY2C-LCDR3 having the amino acid sequence set forth in SEQ ID NO: 24.

In some embodiments, provided is the antigen-binding molecule or the anti-GUCY2C antibody according to the above, wherein the GUCY2C-VH and/or the GUCY2C-VL are murine or humanized. In some embodiments, the GUCY2C-VH and/or the GUCY2C-VL are humanized.

In some embodiments, the humanized GUCY2C-VH comprises an FR1, an FR2, and an FR3 derived from humanized IGHV1-46*01 and an FR4 derived from IGHJ6*01, and it is unsubstituted or has one or more amino acid substitutions selected from the group consisting of 1E, 39L, 43K, 69L, 71V, and 73K; and/or the humanized GUCY2C-VL comprises an FR1, an FR2, and an FR3 derived from IGKV2-40*01 or IGKV1-39*01 and an FR4 derived from IGKJ4*01, and it is unsubstituted or has one or more amino acid substitutions selected from the group consisting of 2V, 3V, 68R, and 74K. In some embodiments, the amino acid positions in the above variable regions are defined according to the Kabat numbering scheme.

In some embodiments, provided is the antigen-binding molecule or the anti-GUCY2C antibody according to any one of the above, wherein the GUCY2C-VH comprises the amino acid sequence of SEQ ID NO: 56, 25, or 57, and the GUCY2C-VL comprises the amino acid sequence of SEQ ID NO: 54, 55, 26, 48, 49, 50, 51, 52, or 53.

In some embodiments, provided is the antigen-binding molecule or the anti-GUCY2C antibody according to any one of the above, wherein:

the GUCY2C-VH comprises the amino acid sequence of SEQ ID NO: 56, and the GUCY2C-VL comprises the amino acid sequence of SEQ ID NO: 54, 55, 48, 49, 50, 51, or 52, or
the GUCY2C-VH comprises the amino acid sequence of SEQ ID NO: 25, and the GUCY2C-VL comprises the amino acid sequence of SEQ ID NO: 26.

In some embodiments, provided is the antigen-binding molecule or the anti-GUCY2C antibody according to any one of the above, wherein:

the amino acid sequence of the GUCY2C-VH is set forth in SEQ ID NO: 56, and the amino acid sequence of the GUCY2C-VL is set forth in SEQ ID NO: 54, or
the amino acid sequence of the GUCY2C-VH is set forth in SEQ ID NO: 56, and the amino acid sequence of the GUCY2C-VL is set forth in SEQ ID NO: 55.

In some embodiments, provided is the antigen-binding molecule according to the above, wherein the CD3-VH comprises: a CD3-HCDR1 comprising the amino acid sequence of SEQ ID NO: 71, a CD3-HCDR2 comprising the amino acid sequence of SEQ ID NO: 72, and a CD3-HCDR3 comprising the amino acid sequence of SEQ ID NO: 73 or 88; and the CD3-VL comprises: a CD3-LCDR1 comprising the amino acid sequence of SEQ ID NO: 74, a CD3-LCDR2 comprising the amino acid sequence of SEQ ID NO: 75, and a CD3-LCDR3 comprising the amino acid sequence of SEQ ID NO: 76.

In some embodiments, provided is the antigen-binding molecule according to any one of the above, wherein the CD3-VH and/or the CD3-VL are murine or humanized.

In some embodiments, the CD3-VH and/or the CD3-VL are humanized.

In some embodiments, the CD3-VH comprises the amino acid sequence of SEQ ID NO: 77 or 89, and the CD3-VL comprises the amino acid sequence of SEQ ID NO: 78.

In some embodiments, the amino acid sequence of the CD3-VH is set forth in SEQ ID NO: 77, and the amino acid sequence of the CD3-VL is set forth in SEQ ID NO: 78.

In some embodiments, the above variable regions and CDRs are defined according to the Kabat numbering scheme.

The antibody series 81 and 146 disclosed according to the examples herein have a technical solution range similar to that of the antibody series P3-C7 described above.

**Structure of Antigen-Binding Molecule**

The bispecific antigen-binding molecule of the present disclosure is not limited to a particular molecular structure, as long as it has the desired antigen binding function.

For example, the bispecific antigen-binding molecule herein may be bivalent (1 + 1), trivalent (2 + 1), or tetravalent (2 + 2). The antigen-binding moieties in the antigen-binding molecule may be any antibody fragments having antigen binding activity, and they are fused via a peptide linker. The peptide linkers of the present disclosure (e.g., linkers 1 through 11) can be any suitable peptide chains, as long as the antigen-binding molecules are capable of exhibiting the desired antigen binding activity. For example, the peptide linkers may be flexible peptides comprising 1-50 or 3-20 amino acid residues. In some embodiments, the peptide linkers each independently have the structure of $L_1$-(GGGGS)n-$L_2$, wherein $L_1$ is a bond, A, G (SEQ ID NO: 82), GS, GGG (SEQ ID NO: 96), GGS (SEQ ID NO: 245), GGGS (SEQ ID NO: 246), or GGGG (SEQ ID NO: 247), n is 0, 1, 2, 3, 4, 5, 6, 7, 8, 9, or 10, $L_2$ is a bond, G (SEQ ID NO: 82), GG, GGG (SEQ ID NO: 96), or GGGG (SEQ ID NO: 247), and the peptide linkers are not bonds. In some embodiments, the peptide linker is 3-15 amino acid residues in length. In some embodiments, the peptide linkers each independently have a structure of (GGGGS)n, wherein n is 1, 2, or 3. In some embodiments, the amino acid sequence of the linker 1 is set forth in SEQ ID NO: 81, the amino acid sequence of the linker 2 is set forth in SEQ ID NO: 82, the amino acid sequence of the linker 3 is set forth in SEQ ID NO: 83, the amino acid sequence of the linker 4 is set forth in SEQ ID NO: 90, the amino acid sequence of the linker 5 is set forth in SEQ ID NO: 95, the amino acid sequence of the linker 6 is set forth in SEQ ID NO: 96, and the amino acid sequence of the linker 7 is set forth in SEQ ID NO: 97.

Illustratively, the antigen-binding molecule of the present disclosure comprises one first chain having a structure represented by formula (a) and one second chain having a structure represented by formula (b):

(a) [GUCY2C-VL]-[linker 1]-[CD3-VH]-[linker 2]-[Fc1], and
(b) [CD3-VL]-[linker 3]-[GUCY2C-VH]-[linker 2]-[Fc2],

wherein the linker 1, the linker 2, and the linker 3 are identical or different peptide linkers; or the linker 1, the linker 2, and the linker 3 are absent;
the structures represented by formulas (a) and (b) are arranged from N-terminus to C-terminus.

Illustratively, the antigen-binding molecule of the present disclosure comprises one first chain having a structure represented by formula (a) and one second chain having a structure represented by formula (b):

(a) [GUCY2C-VL]-[linker 1]-[CD3-VH]-[linker 2]-[Fc1], and
(b) [CD3-VL]-[linker 3]-[GUCY2C-VH]-[linker 2]-[Fc2],

wherein:

the GUCY2C-VH comprises the amino acid sequence of SEQ ID NO: 56, and the GUCY2C-VL comprises the amino acid sequence of SEQ ID NO: 54; and the CD3-VH comprises the amino acid sequence of SEQ ID NO: 77, and the CD3-VL comprises the amino acid sequence of SEQ ID NO: 78; or
the GUCY2C-VH comprises the amino acid sequence of SEQ ID NO: 56, and the GUCY2C-VL comprises the amino acid sequence of SEQ ID NO: 55; and the CD3-VH comprises the amino acid sequence of SEQ ID NO: 77, and the CD3-VL comprises the amino acid sequence of SEQ ID NO: 78;
the linker 1, the linker 2, and the linker 3 are identical or different peptide linkers; or the linker 1, the linker 2, and the linker 3 are absent;
the amino acid sequence of the Fc1 is set forth in SEQ ID NO: 79, and the amino acid sequence of the Fc2 is set forth in SEQ ID NO: 80;
the structures represented by formulas (a) and (b) are arranged from N-terminus to C-terminus.

Illustratively, the antigen-binding molecule of the present disclosure comprises one first chain having a structure represented by formula (a) and one second chain having a structure represented by formula (b):

(a) [GUCY2C-VL]-[GGGSGGGG]-[CD3-VH]-[G]-[Fc1], and
(b) [CD3-VL]-[GGGGSGGGG]-[GUCY2C-VH]-[G]-[Fc2],

wherein: the structures represented by formulas (a) and (b) are arranged from N-terminus to C-terminus.
Illustratively, the antigen-binding molecule of the present disclosure comprises one first chain having a structure represented by formula (a) and one second chain having a structure represented by formula (b):

(a) [GUCY2C-VL]-[GGGSGGGG]-[CD3-VH]-[G]-[Fc1], and
(b) [CD3-VL]-[GGGGSGGGG]-[GUCY2C-VH]-[G]-[Fc2],

wherein:

the GUCY2C-VH comprises: a GUCY2C-HCDR1 comprising the amino acid sequence of SEQ ID NO: 19, a GUCY2C-HCDR2 comprising the amino acid sequence of SEQ ID NO: 20, and a GUCY2C-HCDR3 comprising the amino acid sequence of SEQ ID NO: 21; and the GUCY2C-VL comprises: a GUCY2C-LCDR1 comprising the amino acid sequence of SEQ ID NO: 47, a GUCY2C-LCDR2 comprising the amino acid sequence of SEQ ID NO: 23, and a GUCY2C-LCDR3 comprising the amino acid sequence of SEQ ID NO: 24;
The CD3-VH comprises: a CD3-HCDR1 set forth in SEQ ID NO: 71, a CD3-HCDR2 set forth in SEQ ID NO: 72, and a CD3-HCDR3 set forth in SEQ ID NO: 73; and the CD3-VL comprises: a CD3-LCDR1 set forth in SEQ ID NO: 74, a CD3-LCDR2 set forth in SEQ ID NO: 75, and a CD3-LCDR3 set forth in SEQ ID NO: 76;
the amino acid sequence of the Fc1 is set forth in SEQ ID NO: 79, and the amino acid sequence of the Fc2 is set forth in SEQ ID NO: 80;
the structures represented by formulas (a) and (b) are arranged from N-terminus to C-terminus.

An exemplary antigen-binding molecule comprises:

one first chain comprising the amino acid sequence of SEQ ID NO: 99 and one second chain comprising the amino acid sequence of SEQ ID NO: 100; or
one first chain comprising the amino acid sequence of SEQ ID NO: 101 and one second chain comprising the amino acid sequence of SEQ ID NO: 100.

Illustratively, the antigen-binding molecule comprises one first chain having a structure represented by formula (c), one second chain having a structure represented by formula (d), one third chain having a structure represented by formula (e), and one fourth chain having a structure represented by formula (f):

(c) [CD3-VH]-[CH1]-[Fc1],
(d) [CD3-VL]-[CL],
(e) [GUCY2C-VH]-[linker 4]-[Obscurin chain]-[Fc2], and
(f) [GUCY2C-VL]-[linker 4]-[Titin chain],
wherein the linker 4 is a peptide linker; or the linker 4 is absent;
the structures represented by formulas (c), (d), (e), and (f) are arranged from the N-terminus to the C-terminus.

Illustratively, the antigen-binding molecule comprises one first chain having a structure represented by formula (c), one second chain having a structure represented by formula (d), one third chain having a structure represented by formula (e), and one fourth chain having a structure represented by formula (f):

(c) [CD3-VH]-[CH1]-[Fc1],

(d) [CD3-VL]-[CL],
(e) [GUCY2C-VH]-[linker 4]-[Obscurin chain]-[Fc2], and
(f) [GUCY2C-VL]-[linker 4]-[Titin chain],

wherein:

the GUCY2C-VH comprises the amino acid sequence of SEQ ID NO: 15, and the GUCY2C-VL comprises the amino acid sequence of SEQ ID NO: 16; and the CD3-VH comprises the amino acid sequence of SEQ ID NO: 77, and the CD3-VL comprises the amino acid sequence of SEQ ID NO: 78;
the linker 4 is a peptide linker; or linker 4 is absent;
the amino acid sequence of the Obscurin chain is set forth in SEQ ID NO: 236, and the amino acid sequence of the Titin chain is set forth in SEQ ID NO: 198;
the amino acid sequence of the Fc1 is set forth in SEQ ID NO: 85, and the amino acid sequence of the Fc2 is set forth in SEQ ID NO: 86;
the amino acid sequence of the CH1 is set forth in SEQ ID NO: 84, and the amino acid sequence of the CL is set forth in SEQ ID NO: 87;
the structures represented by formulas (c), (d), (e), and (f) are arranged from the N-terminus to the C-terminus.

Illustratively, the antigen-binding molecule comprises one first chain having a structure represented by formula (c), one second chain having a structure represented by formula (d), one third chain having a structure represented by formula (e), and one fourth chain having a structure represented by formula (f):

(c) [CD3-VH]-[CH1]-[Fc1],
(d) [CD3-VL]-[CL],
(e) [GUCY2C-VH]-[GGGGS]-[Obscurin chain]-[Fc2], and
(f) [GUCY2C-VL]-[GGGGS]-[Titin chain],

wherein: the structures represented by formulas (c), (d), (e), and (f) are arranged from the N-terminus to the C-terminus.
Illustratively, the antigen-binding molecule comprises one first chain having a structure represented by formula (c), one second chain having a structure represented by formula (d), one third chain having a structure represented by formula (e), and one fourth chain having a structure represented by formula (f):

(c) [CD3-VH]-[CH1]-[Fc1],
(d) [CD3-VL]-[CL],
(e) [GUCY2C-VH]-[GGGGS]-[Obscurin chain]-[Fc2], and
(f) [GUCY2C-VL]-[GGGGS]-[Titin chain],

wherein:

The GUCY2C-VH comprises: a GUCY2C-HCDR1 set forth in SEQ ID NO 4, a GUCY2C-HCDR2 set forth in SEQ ID NO 5, and a GUCY2C-HCDR3 set forth in SEQ ID NO: 27; and the GUCY2C-VL comprises: a GUCY2C-LCDR1 set forth in SEQ ID NO 7, a GUCY2C-LCDR2 set forth in SEQ ID NO 8, and a GUCY2C-LCDR3 set forth in SEQ ID NO: 9;
The CD3-VH comprises: a CD3-HCDR1 set forth in SEQ ID NO: 71, a CD3-HCDR2 set forth in SEQ ID NO: 72, and a CD3-HCDR3 set forth in SEQ ID NO: 73; and the CD3-VL comprises: a CD3-LCDR1 set forth in SEQ ID NO: 74, a CD3-LCDR2 set forth in SEQ ID NO: 75, and a CD3-LCDR3 set forth in SEQ ID NO: 76;
the amino acid sequence of the Obscurin chain is set forth in SEQ ID NO: 236, and the amino acid sequence of the Titin chain is set forth in SEQ ID NO: 198;
the amino acid sequence of the Fc1 is set forth in SEQ ID NO: 85, and the amino acid sequence of the Fc2 is set forth in SEQ ID NO: 86;
the amino acid sequence of the CH1 is set forth in SEQ ID NO: 84, and the amino acid sequence of the CL is set forth in SEQ ID NO: 87;
the structures represented by formulas (c), (d), (e), and (f) are arranged from the N-terminus to the C-terminus.

An exemplary antigen-binding molecule comprises:
one first chain comprising the amino acid sequence of SEQ ID NO: 91, one second chain comprising the amino acid sequence of SEQ ID NO: 92, one third chain comprising the amino acid sequence of SEQ ID NO: 110, and one fourth chain comprising the amino acid sequence of SEQ ID NO: 113.

**Variants of Antigen-Binding Molecules**

In certain embodiments, amino acid sequence variants of the antigen-binding molecules provided herein are encompassed. For example, it may be desirable to improve the binding affinity and/or other biological properties of the antibody. Amino acid sequence variants of an antibody can be prepared by introducing appropriate modifications into the nucleotide sequence encoding the antibody, or by peptide synthesis. Such modifications include, for example, deletions from, and/or insertions into and/or substitutions of residues within the amino acid sequence of the antigen-binding molecule. Any combination of deletion, insertion, and substitution can be made to obtain the final construct, provided that the final construct possesses the desired characteristics, e.g., antigen binding.

**Variants with Substitutions, Insertions, and Deletions**

In certain embodiments, antigen-binding molecule variants having one or more amino acid substitutions are provided. Substitutions may be made in the CDRs and FRs. Conservative substitutions are shown in Table 2 under the heading of "Preferred substitution". More substantial changes are provided in Table 2 under the heading of "exemplary substitution", and as further described below with reference to amino acid side chain classes. Amino acid substitutions can be introduced into an antibody of interest and the products are screened for a desired activity, e.g., retained/improved antigen binding, reduced immunogenicity, or improved ADCC or CDC.

Table 2. Amino acid substitutions

| Original residue | Exemplary substitution | Preferred substitution |
|---|---|---|
| Ala(A) | Val; Leu; Ile | Val |
| Arg(R) | Lys; Gln; Asn | Lys |
| Asn(N) | Gln; His; Asp,Lys; Arg | Gln |
| Asp(D) | Glu; Asn | Glu |
| Cys(C) | Ser; Ala | Ser |
| Gln(Q) | Asn; Glu | Asn |
| Glu(E) | Asp; Gln | Asp |
| Gly(G) | Ala | Ala |
| His(H) | Asn; Gln; Lys; Arg | Arg |
| Ile(I) | Leu; Val; Met; Ala; Phe; norleucine | Leu |
| Leu(L) | Norleucine; Ile; Val; Met; Ala; Phe | Ile |
| Lys(K) | Arg; Gln; Asn | Arg |
| Met(M) | Leu; Phe; Ile | Leu |
| Phe(F) | Trp; Leu; Val; Ile; Ala; Tyr | Tyr |
| Pro(P) | Ala | Ala |
| Ser(S) | Thr | Thr |
| Thr(T) | Ser | Ser |
| Trp(W) | Tyr; Phe | Tyr |
| Tyr(Y) | Trp; Phe; Thr; Ser | Phe |
| Val(V) | Ile; Leu; Met; Phe; Ala; norleucine | Leu |

According to common side-chain properties, amino acids can be grouped as follows:

(1) hydrophobic: norleucine, Met, Ala, Val, Leu, Ile;
(2) neutral, hydrophilic: Cys, Ser, Thr, Asn, Gln;
(3) acidic: Asp, Glu;
(4) basic: His, Lys, Arg;
(5) residues affecting chain orientation: Gly, Pro;

(6) aromatic: Trp, Tyr, Phe.

A non-conservative substitution refers to using a member of one class to replace a member of another class.

One type of substitutional variant involves substituting one or more CDR residues of a parent antibody (e.g., a humanized or human antibody). Generally, the resulting variant selected for further study will have changes (e.g., improvements) in certain biological properties (e.g., increased affinity or reduced immunogenicity) relative to the parent antibody, and/or will have substantially retained certain biological properties of the parent antibody. An exemplary substitutional variant is an affinity-matured antibody, which can be conveniently produced, for example, using phage display-based affinity maturation techniques such as those described herein. Briefly, one or more CDR residues are mutated and the variant antibodies are displayed on phages and screened for a particular biological activity (e.g. binding affinity). Changes (e.g., substitutions) can be made in CDRs, for example, to improve antibody affinity. Such changes can be made in CDR "hotspots", i.e., residues encoded by codons that undergo mutation at high frequency during the somatic maturation process, and/or antigen-contacting residues, and meanwhile, the resulting variant VH or VL is tested for binding affinity. In some embodiments of affinity maturation, diversity is introduced into the variable genes selected for maturation by any one of a variety of methods (e.g., error-prone PCR, chain shuffling, or oligonucleotide-directed mutagenesis). A secondary library is then created. The library is then screened to identify any antibody variants with the desired affinity. Another method for introducing diversity involves CDR-directed methods in which several CDR residues (e.g., 4-6 residues at a time) are randomized. CDR residues involved in antigen binding can be specifically identified, for example, using alanine scanning mutagenesis or modeling.

In certain embodiments, substitutions, insertions or deletions may occur within one or more CDRs, as long as such changes do not substantially reduce the ability of the antibody to bind to the antigen. For example, conservative changes (e.g., conservative substitutions as provided herein) that do not substantially reduce binding affinity may be made in CDRs. In certain embodiments of the variant VH and VL sequences provided above, each CDR is unaltered or contains no more than 1, 2, or 3 amino acid substitutions.

A useful method for identifying residues or regions of an antibody that can be used as mutagenesis targets is called "alanine scanning mutagenesis". In this method, a residue or group of residues (e.g., charged residues such as Arg, Asp, His, Lys, and Glu) are identified and replaced with a neutral or negatively charged amino acid (e.g., Ala or polyalanine) to determine whether the interaction of the antibody with the antigen is affected. Further substitutions may be introduced at amino acid positions that show functional sensitivity to the initial substitutions. In addition, a crystal structure of an antigen-antibody complex may be studied to identify contact points between the antibody and antigen. These contact residues and neighboring residues may be targeted or eliminated as substitution candidates. Variants may be screened to determine whether they contain the desired properties.

Amino acid sequence insertions include: the fusion of 1 residue or polypeptides of 100 or more residues in length to the amino- and/or carboxy-terminus, and intrasequence insertions of single or multiple amino acid residues. Examples of terminal insertions include an antibody with an N-terminal methionyl residue. Other insertional variants of the antibody molecule include the fusion where an enzyme (or a polypeptide that increases the serum half-life of an antibody) is fused to the N- or C-terminus of the antibody.

**Engineering of Fab**

In one aspect, in the antigen-binding molecule of the present disclosure, one of the antigen-binding moiety that specifically binds to GUCY2C and the antigen-binding moiety that specifically binds to CD3 is a replaced Fab, and the replaced Fab comprises a heavy chain variable region, a light chain variable region, a Titin chain, and an Obscurin chain. In the replaced Fab, the original CH1 and CL of the Fab are substituted with the Titin chain and the Obscurin chain. Illustratively, the sequences of the Titin chain and the Obscurin chain are shown in Tables 3-1 and 3-2.

Table 3-1. Amino acid sequences of Titin chains

| No. | SEQ ID NO | Amino acid sequence |
|---|---|---|
| T.0 | 182 | GIPPKIEALPSDISIDEGKVLTVACAFTGEPTPEVTWSCGGRKIHSQEQ GRFHIENTDDLTTLIIMDVQKQDGGLYTLSLGNEFGSDSATVNIHIRSI |
| T.1 | 183 | GIPPKIECLPSDISIDEGKVLTVASAFTGEPTPEVTWSTGGRKIHSQEQG RFHIENTDDLTTLIIMDVQKQDGGLYTLSLGNEFGSDSATVNIHIRSI |
| T.2 | 184 | GIPPKIEALPSDISIDEGKCLTVASAFTGEPTPEVTWSTGGRKIHSQEQG RFHIENTDDLTTLIIMDVQKQDGGLYTLSLGNEFGSDSATVNIHIRSI |

(continued)

| No. | SEQ ID NO | Amino acid sequence |
|---|---|---|
| T.3 | 185 | GIPPKIEALPSDISIDEGKVLTVASCFTGEPTPEVTWSTGGRKIHSQEQG RFHIENTDDLTTLIIMDVQKQDGGLYTLSLGNEFGSDSATVNIHIRSI |
| T.4 | 186 | GIPPKIEALPSDISIDEGKVLCVASAFTGEPTPEVTWSTGGRKIHSQEQ GRFHIENTDDLTTLIIMDVQKQDGGLYTLSLGNEFGSDSATVNIHIRSI |
| T.5 | 187 | KAGIRGIPPKIECLPSDISIDEGKVLTVASAFTGEPTPEVTWSTGGRKIH SQEQGRFHIENTDDLTTLIIMDVQKQDGGLYTLSLGNEFGSDSATVNI HIRSI |
| T.6 | 188 | KAGIRGIPPKIEALPSDISIDEGKVLTVACAFTGEPTPEVTWSCGGRKIH SQEQGRFHIENTDDLTTLIIMDVQKQDGGLYTLSLGNEFGSDSATVNI HIRSI |
| T.7 | 189 | GIPPKIECLPSDISIDEGKVLTVASAFTGEPTPEVTWSTGGRKIHSQEQG RFHIENTDDLTTLIISDVQKQDGGLYSLSLGNEFGSDSATVNIHIRSI |
| T.8 | 190 | GIPPKIECLPSDISIDEGKVLTVASAFTGEPTPEVTWSTGGRKIHSQEQG RFHIENTDDLTTLIIKDVQRQDGGLYTLTLRNEFGSDSATVNIHIRSI |
| T.9 | 191 | GIWPKIECLPIDLSIDEGKVLMVASAFTGEPTPEVTWSTGGRKIHSQEQ GRFHIENTDDLTTLIIMDVQKQDGGLYTLSLGMEFGSDSATVNIHIRSI |
| T.10 | 192 | GIPPKIECLPIDISIDEGKVLTVASAFTGEPTPEVTWSTGGRKIHSQEQG RFHIENTDDLTTLIIKDVQKQDGGLYTLTLRNEFGSDSATVNIHIRSI |
| T.11 | 193 | GIPPKIECLPSDISIDEGKVLTVASAFTGEPTPEVTWSTSGKKISSEEGG RFHIESTEDLTTLIIMDVQKQDGGVYTLSLGNDLGSDSATVNIHIRSI |
| T.12 | 194 | GIPPKIECLPSDISIDEGKVLTVASAFTGEPTPEVTWSTGGRKIHSEEGG RFHIESTEDLTTLIIMDVQKQDGGVYTLSLGNEFGSDSATVNIHIRSI |
| T.13 | 195 | GIPPKIECLPSDISIDEGKVLTVASAFTGEPTPEVTWSTGGRKIHSQEQG RFHIESTEDLTTLIIMDVQKQDGGVYTLSLGNEFGSDSATVNIHIRSI |
| T.14 | 196 | GIPPKIECLPSDISIDEGKVLTVASAFTGEPTPEVTWSTGGRKIHSQEQG RFHIESTEDLTTLIISDVQKQDGGLYSLSLGNEFGSDSATVNIHIRSI |
| T.15 | 197 | KAGIRGIPPKIECLPSDISIDEGKVLTVASAFTGEPTPEVTWSTGGRKIH SQEQGRFHIESTEDLTTLIISDVQKQDGGLYSLSLGNEFGSDSATVNIHI RSI |
| T.16 | 198 | GIPPKIECLPIDISIDEGKVLTVASAFTGEPTPEVTWSTGGRKIHSQEQG RFHIENTDDSTTLTIKDVQKQDGGLYTLTLRNEFGSDSATVNIHIRSI |
| T.17 | 199 | GIPPKIECLPIDISIDEGKCLTVASAFTGEPTPEVTWSTGGRKIHSQEQG RFHIENTDDSTTLTIKDVQKQDGGLYTLTLRNEFGSDSATVNIHIRSI |
| T.18 | 200 | GIPPKIECLPIDISIDEGKVLTVASCFTGEPTPEVTWSTGGRKIHSQEQG RFHIENTDDSTTLTIKDVQKQDGGLYTLTLRNEFGSDSATVNIHIRSI |

Table 3-2. Amino acid sequences of Obscurin chains

| No. | SEQ ID NO | Amino acid sequence |
|---|---|---|
| O.0 | 201 | SGAPRFLTRPKAFVVSVGKDATLSCQIVGNPTPQVSWEKDQQPVAAG ARFRLAQDGDLYRLTILDLALGDSGQYVCRARNAIGEAFAAVGLQVD AEA |

(continued)

| No. | SEQ ID NO | Amino acid sequence |
|---|---|---|
| OL.0 | 202 | QGSPPCFLRFPRPVRVVSGAEAELKCVVLGEPPPVVVWEKGGQQLA ASERLSFPADGAEHGLLLTAALPTDAGVYVCRARNAAGEAYAAAAV TVLEPP |
| O.1 | 203 | SGAPRFLTRPKAFVVSVGKDATLSCQIVGNPTPQVSWEKDQQPVAAG ARFRLAQDGDLYRLTILDLALGDSGQYVCRARNAIGEAFACVGLQVD AEA |
| O.2 | 204 | SGCPRFLTRPKAFVVSVGKDATLSCQIVGNPTPQVSWEKDQQPVAAG ARFRLAQDGDLYRLTILDLALGDSGQYVCRARNAIGEAFAAVGLQVD AEA |
| O.3 | 205 | SGAPRFLTCPKAFVVSVGKDATLSCQIVGNPTPQVSWEKDQQPVAAG ARFRLAQDGDLYRLTILDLALGDSGQYVCRARNAIGEAFAAVGLQVD AEA |
| O.4 | 206 | SGAPRFLTRPKAFVVSVGKDATLSSQIVGNPTPQVSWEKDQQPVAAG ARFRLAQDGDLYRLTILDLALGDSGQYVSRARNAIGEAFACVGLQVD AEA |
| O.5 | 207 | SGCPRFLTRPKAFVVSVGKDATLSSQIVGNPTPQVSWEKDQQPVAAG ARFRLAQDGDLYRLTILDLALGDSGQYVSRARNAIGEAFAAVGLQVD AEA |
| O.6 | 208 | SGAPRFLTCPKAFVVSVGKDATLSSQIVGNPTPQVSWEKDQQPVAAG ARFRLAQDGDLYRLTILDLALGDSGQYVSRARNAIGEAFAAVGLQVD AEA |
| O.7 | 209 | SGAPRFKTRPKAFVVSVGKDATLSSQIVGNPTPQVSWEKDQQPVAAG ARFRLAQDGDLYRLKILDLALGDSGQYVSRARNAIGEAFACVGLQV DAEA |
| O.8 | 210 | SGAPRFKTRPKAFVVSVGKDATLSSQIVGNPTPQVSWEKDQQPVAAG ARFRLAQDGDLYRLHILDLALGDSGQYVSRARNAIGEAFACVGLQV DAEA |
| O.9 | 211 | SGAPRFLTRPLAFVVSVGKDATLSSQIVGNPTPQVSWEKDQQPVAAG ARFRLAQDGDLYRLKILDLALGDSGQYVSRARNAIGEAFACVGLQV DAEA |
| O.10 | 212 | SGAPRFLTRPLAFVVSVGKDATLSSQIVGNPTPQVSWEKDQQPVAAG ARFRLAQDGDLYRLHILDLALGDSGQYVSRARNAIGEAFACVGLQV DAEA |
| O.11 | 213 | DQPQFSGAPRFLTRPKAFVVSVGKDATLSSQIVGNPTPQVSWEKDQQ PVAAGARFRLAQDGDLYRLTILDLALGDSGQYVSRARNAIGEAFACV GLQVDAEA |
| O.12 | 214 | DQPQFSGAPRFKTRPKAFVVSVGKDATLSSQIVGNPTPQVSWEKDQQ PVAAGARFRLAQDGDLYRLKILDLALGDSGQYVSRARNAIGEAFACV GLQVDAEA |
| O.13 | 215 | DQPQFSGAPRFKTRPKAFVVSVGKDATLSSQIVGNPTPQVSWEKDQQ PVAAGARFRLAQDGDLYRLHILDLALGDSGQYVSRARNAIGEAFACV GLQVDAEA |
| O.14 | 216 | DQPQFSGAPRFRTRPKAFVVSVGKDATLSSQIVGNPTPQVSWEKDQQ PVAAGARFRLAQDGDLYRLKILDLALGDSGQYVSRARNAIGEAFACV GLQVDAEA |

(continued)

| No. | SEQ ID NO | Amino acid sequence |
|---|---|---|
| O.15 | 217 | DQPQFSGAPRFRTRPKAFVVSVGKDATLSSQIVGNPTPQVSWEKDQQ PVAAGARFRLAQDGDLYRLHILDLALGDSGQYVSRARNAIGEAFACV GLQVDAEA |
| O.16 | 218 | DQPQFSGAPRFLTRPKAFVVSVGKDATLSCQIVGNPTPQVSWEKDQQ PVAAGARFRLAQDGDLYRLTILDLALGDSGQYVCRARNAIGEAFAAV GLQVDAEA |
| OL.1 | 219 | QGSPPEFLRFPRPVRVVSGAEAELKCVVLGEPPPVVVWEKGGQQLAA SERLSFPADGAEHGLLLTAALPTDAGCYVCRARNAAGEAYAAAAVTV LEPP |
| OL.2 | 220 | QGSPPEFLRFPRPVRVVSGAEAELKCVVLGEPPPVVVWEKGGQQLAA SERLSFPADGAEHGLLLTAALPTDAGVYVCRARNAACEAYAAAAVTV LEPP |
| OL.3 | 221 | QGSPPEFLRFPRPVRVVSGAEAELKCVVLGEPPPVVVWEKGGQQLAA SERLSFPADGAEHGLLLTAALPTDAGVYVCRARNAAGECYAAAAVT VLEPP |
| OL.4 | 222 | QGSPPEFLRFPRPVRVVSGAEAELKSVVLGEPPPVVVWEKGGQQLAA SERLSFPADGAEHGLLLTAALPTDAGCYVSRARNAAGEAYAAAAVTV LEPP |
| OL.5 | 223 | QGSPPEFLRFPRPVRVVSGAEAELKSVVLGEPPPVVVWEKGGQQLAA SERLSFPADGAEHGLLLTAALPTDAGVYVSRARNAACEAYAAAAVTV LEPP |
| OL.6 | 224 | QGSPPEFLRFPRPVRVVSGAEAELKSVVLGEPPPVVVWEKGGQQLAA SERLSFPADGAEHGLLLTAALPTDAGVYVSRARNAAGECYAAAAVTV LEPP |
| O.17 | 225 | SGAPRFLTRPKSYTVSVGKDASLSSQIVGNPTPQVSWEKDQQPVAAG ARFRLAQDGDLYRLKILDLALGDSGQYVSRARNAIGEAFACVGLQV DAEA |
| O.18 | 226 | SEAPRFLTRPLAFVVSEGKDATLSSQIVGDPPPEVTWEKDQQPITAGA RFRLAQDGDVYRLEILDLQLSDSGQYVSRARNAIGEAFACVGLQVDA EG |
| O.19 | 227 | SGAPRFLTRPLAFVVSVGKLAMLSSQIVGNPTPQVSWEKDQQPVAAG ARFRLLQDGDLYRLKILDLALGDSGQYVSRARNAIGEAFACVGLQVD AEA |
| O.20 | 228 | SGAPRFLTRPLAFVVSVGKDATLSSQIVGNPTPQVSWEKDKQPVTAG ARFRLAQDGDLYRLKILDLQLSDSGQYVSRARNAIGEAFACLGLQVD AEA |
| O.21 | 229 | SGAPRFLTRPLAFVVSVGKDATLSSQIVGNPTPQVSWEKDQLPVTAG ARFRLAQDGDLYRLKILDLTLSDSGQYVSRARNAIGEAFACVGLEVG AEA |
| O.22 | 230 | SGAPRFLTRPLSYVVSVGKDASLSSQIVGNPTPQVSWEKDQLPVTAG ARFRLAQDGDLYRLKILDLQLSDSGQYVSRARNAIGEAFACVGLEVG AEA |
| O.23 | 231 | DQPQFSGAPRFLTRPLSYVVSVGKDASLSSQIVGNPTPQVSWEKDQLP VTAGARFRLAQDGDLYRLKILDLQLSDSGQYVSRARNAIGEAFACVG LEVGAEA |

(continued)

| No. | SEQ ID NO | Amino acid sequence |
|---|---|---|
| O.24 | 232 | SGAPRFLTRPKAFVVSVGKDATLSSQIVGNPFPQVSWEKDKQPVTAG VRFRLAQDGDLYRLKILDLQLSDSGQYVSRARNAIGEAFACLGLQVD AEA |
| O.25 | 233 | SGAPRFLTRPKASVVSVGKDATLSSQIVGNPFPQVSWEKDKQPVTAG VRFRLAQDGDLYRLKILDLQLSDSGQYVSRARNAHGEAFACLGLQV DAEA |
| O.26 | 234 | SGCPRFLTRPKASVVSVGKDATLSSQIVGNPFPQVSWEKDKQPVTAG VRFRLAQDGDLYRLKILDLQLSDSGQYVSRARNAHGEAFACLGLQV DAEA |
| O.27 | 235 | SGAPRFLTCPKASVVSVGKDATLSSQIVGNPFPQVSWEKDKQPVTAG VRFRLAQDGDLYRLKILDLQLSDSGQYVSRARNAHGEAFACLGLQV DAEA |
| O.28 | 236 | SGAPRFLTRPKASVVSVGKDATLSCQIVGNPFPQVSWEKDKQPVTAG VRFRLAQDGDLYRLKILDLQLSDSGQYVCRARNAHGEAFACLGLQV DAEA |
| O.29 | 237 | SGCPRFLTRPKASVVSVGKDATLSCQIVGNPFPQVSWEKDKQPVTAG VRFRLAQDGDLYRLKILDLQLSDSGQYVCRARNAHGEAFACLGLQV DAEA |
| O.30 | 238 | SGAPRFLTCPKASVVSVGKDATLSCQIVGNPFPQVSWEKDKQPVTAG VRFRLAQDGDLYRLKILDLQLSDSGQYVCRARNAHGEAFACLGLQV DAEA |
| O.31 | 239 | SGAPRFLTRPKASVVSVGKDATLSSQIVGNPFPQVSWEKDKQPVTAG VRFRLAQDGDLYRLKILDCQLSDSGQYVSRARNAHGEAFACLGLQC DAEA |
| O.32 | 240 | SGCPRFLTRPKASVVSVGKDATLSSQIVGNPFPQVSWEKDKQPVTAG VRFRLAQDGDLYRLKILDCQLSDSGQYVSRARNAHGEAFACLGLQC DAEA |
| O.33 | 241 | SGAPRFLTCPKASVVSVGKDATLSSQIVGNPFPQVSWEKDKQPVTAG VRFRLAQDGDLYRLKILDCQLSDSGQYVSRARNAHGEAFACLGLQC DAEA |

**Engineering of Fc region**

In one aspect, the Fc region of the antigen-binding molecule of the present disclosure comprises one or more amino acid substitutions that reduce its binding to an Fc receptor, e.g., its binding to the Fcγ receptor, and reduce or eliminate effector functions. A natural IgG Fc region, specifically an IgG$_1$ Fc region or an IgG$_4$ Fc region, may cause the antigen-binding molecule of the present disclosure to target cells expressing an Fc receptor, rather than cells expressing an antigen. The engineered Fc region of the present disclosure exhibits reduced binding affinity for an Fc receptor and/or reduced effector functions. In some embodiments, the engineered Fc region shows no less than 50%, 80%, 90%, or 95% reduction in binding affinity for an Fc receptor as compared to the native Fc region. In some embodiments, the Fc receptor is an Fcγ receptor. In some embodiments, the Fc receptor is a human Fcγ receptor, e.g., FcγRI, FcγRIIa, FcγRIIB, or FcγRIIIa. In some embodiments, the engineered Fc region also has reduced binding affinity for a complement (e.g., C1q) as compared to the native Fc region. In some embodiments, the engineered Fc region has no reduced binding affinity for a neonatal Fc receptor (FcRn) as compared to the native Fc region. In some examples, the engineered Fc region has reduced effector functions, which may include, but are not limited to, one or more of the following: reduced complement-dependent cytotoxicity (CDC), reduced antibody-dependent cell-mediated cytotoxicity (ADCC), reduced antibody-dependent cellular phagocytosis (ADCP), reduced cytokine secretion, reduced immune complex-mediated antigen uptake by antigen presenting cells, reduced binding to NK cells, reduced binding to macrophages, reduced binding to monocytes, reduced binding to polymorphonuclear cells, reduced direct signaling-induced apoptosis, reduced dendritic cell maturation, and reduced T cell priming. For the $IgG_1$ Fc region, amino acid residue substitutions at positions such as positions 238, 265, 269, 270, 297, 327, and 329 can reduce effector functions. In some embodiments, the Fc region is a human $IgG_1$ Fc region, and the amino acid residues at positions 234 and 235 are A, as numbered according to the EU index. For the $IgG_4$ Fc region, amino acid residue substitutions at positions such as position 228 can reduce effector functions.

The antigen-binding molecule may also comprise disulfide bond engineering, such as 354C in the first subunit and 349C in the second subunit. To increase the serum half-life of the antigen-binding molecule, 252Y, 254T, and 256E mutations may be introduced.

When the antigen-binding molecule comprises different binding moieties fused to the two subunits of the Fc region, it may lead to undesired homodimerization. To improve yield and purity, it is thus advantageous to introduce modifications that promote heterodimerization in the Fc region of the antigen-binding molecule of the present disclosure. In some embodiments, the Fc region of the present disclosure comprises modifications according to the knob-into-hole (KIH) technique, which involves introducing a knob structure at the interface of the first subunit and a hole structure at the interface of the second subunit. As such, the knob structure can be positioned in the hole structure, thereby promoting the formation of heterodimers and inhibiting the production of homodimers. The knob structure is constructed by substituting a small amino acid side chain at the interface of the first subunit with a larger side chain (e.g., tyrosine or tryptophan). The hole structure is created at the interface of the second subunit by substituting a large amino acid side chain with a smaller amino acid side chain (e.g., alanine or threonine). The knob structure and the hole structure are prepared by altering the nucleic acid encoding the polypeptide, with optional amino acid substitutions shown in Table 4:

Table 4. Combinations of KIH mutations

| First subunit | T366Y | T366W | T394W | F405W | T366W | T366Y F405A | T366W F405W | F405W Y407A |
|---|---|---|---|---|---|---|---|---|
| Second subunit | Y407T | Y407A | F405A | T394S | T366S L368A Y407V | T394W Y407T | T394W Y407A | T366W T394S |

In addition to the knob-into-hole technique, other techniques for modifying the CH3 domain of the heavy chain to achieve heterodimerization are also known in the art, e.g., WO1996027011A1, WO1998050431A2, EP1870459A1, WO2007110205A2, WO2009089004A1, WO2010129304A2, WO2011143545A1, WO2012058768A1, WO2013157954A1, and WO2013096291A2.

The C-terminus of the Fc region may be an intact C-terminus ending with amino acid residues PGK or a truncated C-terminus, e.g., a truncated C-terminus in which one or two C-terminal amino acid residues are removed. In a preferred aspect, the C-terminus of the heavy chain is a shortened C-terminus ending with PG. Thus, in some embodiments, a composition of intact antibodies may comprise antibody populations with all K447 residues and/or G446 + K447 residues removed. In some embodiments, a composition of intact antibodies may comprise antibody populations without a K447 residue and/or G446 + K447 residues removed. In some embodiments, a composition of intact antibodies comprises antibody populations having a mixture of antibodies with and without a K447 residue and/or G446 + K447 residues.

**Recombination Method**

The antigen-binding molecule may be produced using recombination methods. For these methods, one or more isolated nucleic acids encoding the antigen-binding molecule are provided.

In the case of a native antibody, a native antibody fragment or a bispecific antibody with homodimeric heavy chains, two nucleic acids are required, one for the light chain or a fragment thereof and the other for the heavy chain or a fragment thereof. Such nucleic acids encode an amino acid sequence comprising the VL of the antibody and/or an amino acid sequence comprising the VH of the antibody (e.g., the light and/or heavy chain of the antibody). These nucleic acids can be on the same expression vector or different expression vectors.

In the case of a bispecific antibody with heterodimeric heavy chains, four nucleic acids, for example, are required, one for a first light chain, one for a first heavy chain comprising a first heteromonomeric Fc-region polypeptide, one for a second light chain, and one for a second heavy chain comprising a second heteromonomeric Fc-region polypeptide. The four nucleic acids can be contained in one or more nucleic acid molecules or expression vectors. Generally, these nucleic acids are located on two or three expression vectors; that is, one vector can comprise more than one of these nucleic acids.

In one embodiment, the present disclosure provides an isolated nucleic acid encoding the aforementioned antibody. Such nucleic acids may independently encode any one of the polypeptide chains described above. In another aspect, the present disclosure provides one or more vectors (e.g., expression vectors) comprising such nucleic acids. In another aspect, the present disclosure provides a host cell comprising such nucleic acids. In one embodiment, provided is a method for preparing an antigen-binding molecule, wherein the method comprises culturing a host cell comprising a nucleic acid encoding the antibody, as provided above, under conditions suitable for antibody expression, and optionally collecting the antibody from the host cell (or host cell culture medium).

For recombinant production of the antigen-binding molecule, the nucleic acid encoding the protein is isolated and inserted into one or more vectors for further cloning and/or expression in a host cell. Such nucleic acids can be readily isolated and sequenced using conventional procedures (e.g., by using oligonucleotide probes that are capable of binding specifically to genes encoding the heavy and light chains of an antibody), or produced by recombinant methods or obtained by chemical synthesis.

Suitable host cells for cloning or expressing a vector encoding the antibody include prokaryotic or eukaryotic cells described herein. For example, the antibody can be produced in bacteria, particularly when the antibody does not require glycosylation and Fc effector functions. After expression, the antibody can be isolated from the bacterial cell paste in a soluble fraction and can be further purified.

In addition to prokaryotes, eukaryotic microorganisms such as filamentous fungi or yeast are suitable cloning or expression hosts for antibody-encoding vectors, including fungal and yeast strains, whose glycosylation pathways have been "humanized", such that an antibody with a partially or fully human glycosylation pattern is produced. Suitable host cells for expression of (glycosylated) antibodies may also be derived from multicellular organisms (invertebrates and vertebrates); examples of invertebrate cells include plant and insect cells. A number of baculovirus strains have been identified, which can be used in combination with insect cells, particularly for transfection of *Spodoptera frugiperda* cells; plant cell cultures may also be used as hosts, see, e.g., US5959177, US 6040498, US6420548, US 7125978 and US6417429; vertebrate cells can also be used as hosts, e.g., mammalian cell lines adapted for growth in a suspension. Other examples of suitable mammalian host cell lines include an SV40-transformed monkey kidney CVIline (COS-7); a human embryonic kidney line (293 or 293T cells); baby hamster kidney cells (BHK); mouse sertoli cells (TM4 cells); monkey kidney cells (CV1); African green monkey kidney cells (VERO-76); human cervical cancer cells (HELA); canine kidney cells (MDCK); buffalo rat liver cells (BRL3A); human lung cells (W138); human liver cells (Hep G2); mouse mammary tumor cells (MMT 060562); TRI cells; MRC 5 cells; and FS4 cells. Other suitable mammalian host cell lines include Chinese hamster ovary (CHO) cells, including DHFR-CHO cells; and myeloma cell lines such as Y0, NS0, and Sp2/0. For reviews of certain mammalian host cell lines suitable for the production of the antibody, see, e.g., Yazaki, P. and Wu, A.M., Methods in Molecular Biology, Vol. 248, Lo, B.K.C. (eds.), Humana Press, Totowa, NJ (2004), pp. 255-268.

**Immunoconjugate**

The present disclosure also provides an immunoconjugate comprising an antigen-binding molecule conjugated to one or more cytotoxic agents, such as a chemotherapeutic agent or drug, a growth inhibitor, a toxin (e.g., a protein toxin, an enzymatically active toxin, or a fragment thereof, of bacterial, fungal, plant or animal origin), or a radioisotope.

**Diagnostic and Therapeutic Compositions**

In certain embodiments, the antigen-binding molecule provided in the present disclosure can be used to detect the presence of GUCY2C and/or CD3 in a biological sample. As used herein, the term "detection" encompasses quantitative or qualitative detection. In certain embodiments, the biological sample includes a cell or tissue, such as tumor tissue.

In one embodiment, provided is an antigen-binding molecule for use in a diagnostic or detection method. In yet another aspect, provided is a method for detecting the presence of GUCY2C and/or CD3 in a biological sample. In certain embodiments, the method comprises contacting the biological sample with an antigen-binding molecule under suitable conditions and detecting whether a complex is formed between the detection agent and the antigen. Such methods may be *in vitro* or *in vivo* methods. In one embodiment, an antigen-binding molecule is used to select a subject suitable for treatment; for example, GUCY2C and/or CD3 are biomarkers for selecting a patient.

Exemplary disorders that can be diagnosed using the antigen-binding molecule of the present disclosure are for example, proliferative diseases or tumors (e.g., cancer).

In certain embodiments, provided is a labeled antigen-binding molecule. Labels include, but are not limited to, labels or moieties that are detected directly (such as fluorescent, chromophoric, electron-dense, chemiluminescent, and radioactive labels), and moieties that are detected indirectly (e.g., moieties that are detected indirectly through an enzymatic reaction or molecular interaction, such as enzymes or ligands).

In an additional aspect, provided is a pharmaceutical composition comprising the antigen-binding molecule, for example, for use in any of the following treatment methods. In one aspect, the pharmaceutical composition comprises any of the antigen-binding molecules provided herein and a pharmaceutically acceptable carrier. In another aspect, the pharmaceutical composition comprises any of the antigen-binding molecules provided herein and at least one additional therapeutic agent.

The pharmaceutical composition of the antigen-binding molecule described in the present disclosure is prepared by: mixing such an antigen-binding molecule having desired purity with one or more optional pharmaceutically acceptable carriers. The pharmaceutical composition is in the form of a lyophilized composition or an aqueous solution. Formulations for *in vivo* administration are generally sterile. Sterility can be readily achieved, for example, by filtration through a sterile filter membrane.

**Treatment Method and Route of Administration**

Any of the antigen-binding molecules provided herein can be used in the treatment method.

In yet another aspect, the present disclosure provides use of the antigen-binding molecule in the manufacture or preparation of a medicament. In one embodiment, the medicament is used for treating a proliferative disease or tumor. In addition, the medicament is present in an amount effective for the diseases described above. In some embodiments, the effective amount is a unit daily dose or a unit weekly dose. In one such embodiment, the use further comprises administering to a subject an effective amount of at least one additional therapeutic agent (e.g., one, two, three, four, five, or six additional therapeutic agents). The "subject" according to any of the above embodiments may be a human.

In yet another aspect, provided is a pharmaceutical composition comprising the antigen-binding molecule, e.g., for any of the above pharmaceutical uses or treatment methods. In another embodiment, the pharmaceutical composition also comprises at least one additional therapeutic agent.

The antigen-binding molecule of the present disclosure may be used alone or in combination with other agents for the treatment. For example, the antigen-binding molecule of the present disclosure may be co-administered with at least one additional therapeutic agent. In some embodiments, the additional therapeutic agent is a bispecific antibody that specifically binds to EGFR and CD28.

The antigen-binding molecule of the present disclosure (and any additional therapeutic agents) may be administered by any suitable means, including parenteral, intrapulmonary, and intranasal administration, and if required for local treatment, intralesional administration. Parenteral infusion includes intramuscular, intravenous, intra-arterial, intraperitoneal, or subcutaneous administration. Administration may be performed by any suitable route, e.g., by injection, such as intravenous or subcutaneous injection, depending in part on whether the administration is short-term or long-term. Various administration schedules are contemplated herein, including but not limited to, single administration or multiple administrations at multiple time points, bolus administration, and pulse infusion.

The antigen-binding molecule of the present disclosure will be formulated, administered, and applied in a manner consistent with Good Medical Practice. Factors considered in this context include the specific disorder being treated, the specific mammal being treated, the clinical state of the individual patient, the cause of the disorder, the site of delivery of the agent, the method of administration, the timing of administration, and other factors known to medical practitioners. The antigen-binding molecule needs not be, but is optionally, formulated along with one or more agents currently used for preventing or treating the disorder. The effective amount of such additional agents depends on the amount of the antigen-binding molecule present in the pharmaceutical composition, the type of the disorder or treatment, and other factors discussed above. These are generally used in the same dose and with routes of administration as described herein, or in about 1 to 99% of the dose described herein, or in any dose and by any route that is empirically/clinically determined to be appropriate.

For the prevention or treatment of a disease, the appropriate dose of the antigen-binding molecule of the present disclosure (when used alone or in combination with one or more additional therapeutic agents) will depend on the type of disease to be treated, the type of therapeutic molecule, the severity and course of the disease, the purpose of the administration (prophylactic or therapeutic), previous treatment, clinical history and response to the therapeutic molecule of the patient, and the discretion of the attending physician. The therapeutic molecule is suitably administered to a patient in one or a series of treatments. Depending on the type and severity of the disease, about 1 $\mu$g/kg to 15 mg/kg of the antigen-binding molecule can be an initial candidate dose for administration to the patient, whether, for example, by one or more separate administrations, or by continuous infusion. One typical daily dose may range from about 1 $\mu$g/kg to 100 mg/kg or more, depending on the factors mentioned above. Accordingly, taking 50 kg body weight as an example, an exemplary unit daily dose is 50 $\mu$g-5 g.

**Article of Manufacture**

In another aspect of the present disclosure, provided is an article of manufacture, which comprises materials useful for the treatment, prevention, and/or diagnosis of the disorders described above. The article of manufacture comprises a container and a label or package insert on or associated with the container. Suitable containers include, for example, bottles, vials, syringes, IV solution bags, and the like. The containers may be formed from a variety of materials such as glass or plastic. The container contains a composition effective in the treatment, prevention, and/or diagnosis of a disease, either alone or in combination with another composition, and may have a sterile access port (e.g., the container may be an intravenous solution bag or vial with a stopper pierceable by a hypodermic injection needle). At least one active agent in the composition is the antigen-binding molecule of the present disclosure. The label or package insert indicates that the composition is used to treat the selected condition. Further, the article of manufacture may comprise: (a) a first container containing a composition that comprises the antigen-binding molecule of the present disclosure; and (b) a second container containing a composition that comprises other cytotoxic agents or additional therapeutic agents. The article of manufacture in this embodiment of the present disclosure may further comprise a package insert indicating that the composition may be used to treat a particular condition. Alternatively or additionally, the article of manufacture may further comprise a second (or third) container containing a pharmaceutically acceptable buffer. From a commercial and user standpoint, it may further contain other materials as required, including other buffers, diluents, filters, needles, and syringes.

**Examples and Test Examples**

The present disclosure is further described below with reference to the following examples and test examples, which, however, do not limit the present disclosure. The experimental methods in the examples and test examples of the present disclosure in which specific conditions are not specified are generally performed under conventional conditions such as Antibodies: A Laboratory Manual and Molecular Cloning: A Laboratory Manual by Cold Spring Harbor Laboratory, or under conditions recommended by the manufacturers of the starting materials or commercial products. Reagents without specific origins indicated are commercially available conventional reagents.

**Example 1. Antigen-Binding Molecules Comprising Titin Chain/Obscurin Chain**

The Titin chain/Obscurin chain of the present disclosure may be derived from any suitable polypeptide, including polypeptides derived from WO2021139758A1 (incorporated herein by reference in its entirety) and CN202110527339.7 and the patents which refer to it as a priority document (incorporated herein by reference in their entireties). Bispecific antibodies were constructed, where the CL was the kappa light chain constant region in WO2021139758A1, the amino acid sequences of the Titin and Obscurin chains are shown in Tables 3-1 and 3-2, and the linker sequences include GGGGS (SEQ ID NO: 90), ASTKG (SEQ ID NO: 243), or RTVAS (SEQ ID NO: 244). The amino acid sequences of the Fc1, Fc2, and CH1 in this example are shown below.

> Example 1-Fc1 (knob, SEQ ID NO: 85)

DKTHTCPPCPAPEAAGGPSVFLFPPKPKDTLMISRTPEVTCVVVDVSHEDPEV
KFNWYVDGVEVHNAKTKPREEQYNSTYRVVSVLTVLHQDWLNGKEYKCKV
SNKALPAPIEKTISKAKGQPREPQVYTLPPCREEMTKNQVSLWCLVKGFYPSDI
AVEWESNGQPENNYKTTPPVLDSDGSFFLYSKLTVDKSRWQQGNVFSCSVMH
EALHNHYTQKSLSLSPGK

> Example 1-Fc2 (hole, SEQ ID NO: 86)

DKTHTCPPCPAPEAAGGPSVFLFPPKPKDTLMISRTPEVTCVVVDVSHEDPEV
KFNWYVDGVEVHNAKTKPREEQYNSTYRVVSVLTVLHQDWLNGKEYKCKV
SNKALPAPIEKTISKAKGQPREPQVCTLPPSREEMTKNQVSLSCAVKGFYPSDI
AVEWESNGQPENNYKTTPPVLDSDGSFFLVSKLTVDKSRWQQGNVFSCSVMH
EALHNHYTQKSLSLSPGK

> Example 1-CH1 (SEQ ID NO: 84)

ASTKGPSVFPLAPSSKSTSGGTAALGCLVKDYFPEPVTVSWNSGALTSGVHTF
PAVLQSSGLYSLSSVVTVPSSSLGTQTYICNVNHKPSNTKVDKKVEPKSC

**1.1. DI bispecific antibodies**

DI bispecific antibodies against hNGF and hRANKL, DI-2 to DI-20, which comprise a first heavy chain, a second heavy chain, a first light chain, and a second light chain as described below, were constructed with reference to Example 5 in WO2021139758A1:

the first heavy chain: when viewed from N-terminus to C-terminus, [VH1-I]-[linker 1]-[Obscurin chain]-[Fc2];
the first light chain: when viewed from N-terminus to C-terminus, [VL1-I]-[linker 2]-[Titin chain];
the second heavy chain: when viewed from N-terminus to C-terminus, [VH2-D]-[CH1]-[Fc1]; and
the second light chain: when viewed from N-terminus to C-terminus, [VL2-D]-[CL];

wherein the VH1-I and VL1-I were the heavy chain variable region and the light chain variable region of I0 in WO2021139758A1, respectively, and the VH2-D and VL2-D were the heavy chain variable region and the light chain variable region of D0 in WO2021139758A1, respectively. The structures of the Obscurin chain, the Titin chain, the linker 1, and the linker 2 in the DI bispecific antibodies of this example are shown in the following table.

Table 5. Obscurin chain/Titin chain and linkers in DI bispecific antibodies

| No. | First heavy chain | | First light chain | |
|---|---|---|---|---|
| | Obscurin chain | Linker 1 | Titin chain | Linker 2 |
| DI-2 | O.20 | GGGGS | T.10 | GGGGS |
| DI-3 | O.25 | GGGGS | T.16 | GGGGS |
| DI-4 | O.26 | GGGGS | T.17 | GGGGS |
| DI-5 | O.27 | GGGGS | T.18 | GGGGS |
| DI-6 | O.28 | GGGGS | T.16 | GGGGS |
| DI-7 | O.29 | GGGGS | T.17 | GGGGS |
| DI-8 | O.30 | GGGGS | T.18 | GGGGS |
| DI-9 | O.31 | GGGGS | T.16 | GGGGS |
| DI-10 | O.32 | GGGGS | T.17 | GGGGS |
| DI-11 | O.33 | GGGGS | T.18 | GGGGS |
| DI-12 | O.25 | ASTKG | T.16 | RTVAS |
| DI-13 | O.26 | ASTKG | T.17 | RTVAS |
| DI-14 | O.27 | ASTKG | T.18 | RTVAS |
| DI-15 | O.28 | ASTKG | T.16 | RTVAS |
| DI-16 | O.29 | ASTKG | T.17 | RTVAS |
| DI-17 | O.30 | ASTKG | T.18 | RTVAS |
| DI-18 | O.31 | ASTKG | T.16 | RTVAS |
| DI-19 | O.32 | ASTKG | T.17 | RTVAS |
| DI-20 | O.33 | ASTKG | T.18 | RTVAS |
| Note: The numbering of the Titin and Obscurin chains in the table is shown in Tables 3-1 and 3-2. | | | | |

The binding activity of the bispecific antibodies DI-2 to DI-20 for their antigens was assayed by the method of Test Example 4 of WO2021139758A1. The thermal stability of the antibodies was studied. Study method: The antibodies were diluted with PBS to a concentration of 5 mg/mL, and their thermal stability was measured using a high throughput differential scanning fluorimeter (UNCHAINED; model: Unit). The experimental results show that there was no significant change in the antigen binding activity of the engineered bispecific antibodies; additionally, there were significant increases in the Tm1 (°C) and Tonset (°C) of DI-4 to DI-8, DI-10 to DI-16, and DI-20 as compared to DI-2, suggesting that the thermal stability of the bispecific antibodies is better.

Table 6. Binding activity of DI bispecific antibodies

| No. | RANKL | NGF | No. | RANKL | NGF |
|---|---|---|---|---|---|
| | EC$_{50}$ (nM) | | | EC$_{50}$ (nM) | |
| DI-2 | 0.3832 | 6.633 | DI-12 | 0.4125 | 6.5156 |
| DI-3 | 0.3613 | 5.7730 | DI-13 | 0.4440 | 5.5420 |
| DI-4 | 0.3959 | 6.2930 | DI-14 | 0.4182 | 3.2610 |
| DI-5 | 0.3290 | 6.1890 | DI-15 | 0.2206 | 5.2800 |
| DI-6 | 0.2509 | 5.6720 | DI-16 | 0.1474 | 5.5140 |
| DI-7 | 0.2557 | 6.6430 | DI-17 | 0.2329 | 6.7270 |
| DI-8 | 0.3643 | 7.6250 | DI-18 | 0.2662 | 5.9080 |
| DI-9 | 0.2944 | 8.4950 | DI-19 | 0.1843 | 5.9280 |
| DI-10 | 0.3460 | 7.1660 | DI-20 | 0.3184 | 6.4250 |
| DI-11 | 0.3721 | 10.9600 | | | |

Table 7. Thermal stability of DI bispecific antibodies

| No. | Tml (°C) | Tonset (°C) | No. | Tml (°C) | Tonset (°C) |
|---|---|---|---|---|---|
| DI-2 | 55.6 | 48.3 | DI-11 | 57.35 | - |
| DI-4 | 60.1 | 52.493 | DI-12 | 59.9 | 51.726 |
| DI-5 | 61 | 51.967 | DI-13 | 61 | 50.988 |
| DI-6 | 60.8 | 53.012 | DI-14 | 61.2 | 52.191 |
| DI-7 | 60.34 | 52.003 | DI-15 | 60.41 | 50.558 |
| DI-8 | 60.61 | 50.425 | DI-16 | 61.5 | 50.691 |
| DI-10 | 60.2 | 52.766 | DI-20 | 60.7 | 51.859 |

Solutions of the DI bispecific antibodies were prepared using a buffer containing 10 mM acetic acid at pH 5.5 and 9% sucrose, and the solutions were incubated in a 40 °C incubator for four weeks. Following the incubation, the antibody solutions were concentrated to the concentration at the start of the incubation, and the solutions were observed for precipitate formation. The experimental results show that there was a precipitate formed in the solution of the DI-2 bispecific antibody group. DI-3 to DI-7 are more stable than DI-2.

Table 8. Precipitate formation in the solutions of the DI bispecific antibodies

| No. | Initial concentration | Concentration after solution concentration at week 4 | Precipitate formation in solution |
|---|---|---|---|
| DI-2 | 20 mg/mL | 20 mg/mL | Precipitation occurred |
| DI-3 | 20 mg/mL | 20 mg/mL | No precipitation |
| DI-4 | 60 mg/mL | 60 mg/mL | No precipitation |
| DI-5 | 25 mg/mL | 25 mg/mL | No precipitation |
| DI-6 | 60 mg/mL | 60 mg/mL | No precipitation |
| DI-7 | 16 mg/mL | 16 mg/mL | No precipitation |

## 1.2. PL bispecific antibodies

PL bispecific antibodies against hPDL1 and hCTLA4, PL-1 to PL-19, which comprise a first heavy chain, a second heavy chain, a first light chain, and a second light chain as described below, were constructed:

the first heavy chain: when viewed from N-terminus to C-terminus, [VH1-P]-[linker 1]-[Obscurin chain]-[Fc1];

the first light chain: when viewed from N-terminus to C-terminus, [VL1-P]-[linker 2]-[Titin chain];
the second heavy chain: when viewed from N-terminus to C-terminus, [VH2-L]-[CH1]-[Fc2]; and
the second light chain: when viewed from N-terminus to C-terminus, [VL2-L]-[CL];

wherein the VH1-P and VL1-P were the heavy chain variable region and the light chain variable region of the h1831K antibody in WO2020177733A1, respectively. The amino acid sequences of the VH2-L and VL2-L are shown below.

> VH2-L (SEQ ID NO: 180)

QVQLVESGGGVVQPGRSLRLSCAASGFTFSSYTMHWVRQAPGKGLEWVTFIS
YDGNNKYYADSVKGRFTISRDNSKNTLYLQMNSLRAEDTAIYYCARTGWLGP
FDYWGQGTLVTVSS

> VL2-L (SEQ ID NO: 181)

EIVLTQSPGTLSLSPGERATLSCRASQSVGSSYLAWYQQKPGQAPRLLIYGAFS
RATGIPDRFSGSGSGTDFTLTISRLEPEDFAVYYCQQYGSSPWTFGQGTKVEIK

The structures of the Obscurin chain, the Titin chain, the linker 1, and the linker 2 in the PL bispecific antibodies of this example are shown in the following table.

Table 9. Obscurin chain/Titin chain and linkers in PL bispecific antibodies

| No. | First heavy chain | | First light chain | |
|---|---|---|---|---|
| | Obscurin chain | Linker 1 | Titin chain | Linker 2 |
| PL-1 | O.20 | GGGGS | T.10 | GGGGS |
| PL-2 | O.25 | GGGGS | T.16 | GGGGS |
| PL-3 | O.26 | GGGGS | T.17 | GGGGS |
| PL-4 | O.27 | GGGGS | T.18 | GGGGS |
| PL-5 | O.28 | GGGGS | T.16 | GGGGS |
| PL-6 | O.29 | GGGGS | T.17 | GGGGS |
| PL-7 | O.30 | GGGGS | T.18 | GGGGS |
| PL-8 | O.31 | GGGGS | T.16 | GGGGS |
| PL-9 | O.32 | GGGGS | T.17 | GGGGS |
| PL-10 | O.33 | GGGGS | T.18 | GGGGS |
| PL-11 | O.25 | ASTKG | T.16 | RTVAS |
| PL-12 | O.26 | ASTKG | T.17 | RTVAS |
| PL-13 | O.27 | ASTKG | T.18 | RTVAS |
| PL-14 | O.28 | ASTKG | T.16 | RTVAS |
| PL-15 | O.29 | ASTKG | T.17 | RTVAS |
| PL-16 | O.30 | ASTKG | T.18 | RTVAS |
| PL-17 | O.31 | ASTKG | T.16 | RTVAS |
| PL-18 | O.32 | ASTKG | T.17 | RTVAS |
| PL-19 | O.33 | ASTKG | T.18 | RTVAS |
| Note: The numbering of the Titin and Obscurin chains in the table is shown in Tables 3-1 and 3-2. | | | | |

The binding activity of PL bispecific antibodies was tested by the ELISA assay with reference to Test Example 4 of WO2021139758A1, wherein hPDL1 and hCTLA4 antigens were purchased from Sino biology. The thermal stability of the antibodies was studied. Method: The antibodies were diluted with PBS to a concentration of 1.4-3 mg/mL, and their thermal stability was measured using a high throughput differential scanning fluorimeter (UNCHAINED; model: Unit). The experimental results show that the PL bispecific antibodies retained good antigen binding activity; additionally, there were significant increases in the Tm1 (°C), Tagg 266 (°C), and Tonset (°C) of PL-2 to PL-19 as compared to PL-1, suggesting that the thermal stability of the bispecific antibodies is better.

Table 10. Binding activity of PL bispecific antibodies

| No. | hPDL1 | hCTLA4 | No. | hPDL1 | hCTLA4 |
|-----|-------|--------|-----|-------|--------|
|     | $EC_{50}$ (nM) | | | $EC_{50}$ (nM) | |
| PL-1 | 1.6 | 16.5 | PL-11 | 0.6 | 5.6 |
| PL-2 | 0.5 | 8.0 | PL-12 | 0.5 | 8.0 |
| PL-3 | 0.6 | 7.9 | PL-13 | 0.5 | 4.4 |
| PL-4 | 0.7 | 6.5 | PL-14 | 1.3 | 6.0 |
| PL-5 | 0.7 | 6.7 | PL-15 | 1.4 | 3.2 |
| PL-6 | 0.8 | 5.3 | PL-16 | 1.7 | 34.9 |
| PL-7 | 0.5 | 7.3 | PL-17 | 1.4 | 6.2 |
| PL-8 | 1.4 | 14.5 | PL-18 | 1.6 | 6.7 |
| PL-9 | 0.6 | 5.5 | PL-19 | 1.6 | 6.5 |
| PL-10 | 0.6 | 6.9 |  |  |  |

Table 11. Thermal stability of PL bispecific antibodies

| No. | Tml (°C) | Tagg 266 (°C) | Tonset (°C) | No. | Tml (°C) | Tagg 266 (°C) | Tonset (°C) |
|-----|----------|---------------|-------------|-----|----------|---------------|-------------|
| PL-1 | 61.64 | 64.82 | 52.566 | PL-11 | 65.88 | - | 57.976 |
| PL-2 | 66.20 | 66.55 | 58.317 | PL-12 | 65.54 | 67.94 | - |
| PL-3 | 64.07 | 68.28 | 57.661 | PL-13 | 71.85 | 68.17 | 58.581 |
| PL-4 | 70.71 | 67.29 | 56.246 | PL-14 | 74.18 | 69.42 | 58.589 |
| PL-5 | 74.56 | 68.11 | 58.407 | PL-15 | 70.96 | 69.91 | 58.622 |
| PL-6 | 70.43 | 70.12 | 61.069 | PL-16 | 63.48 | 68.98 | 58.702 |
| PL-7 | 68.46 | 67.41 | 63.031 | PL-17 | 70.15 | 69.86 | 56.193 |
| PL-8 | 65.00 | - | - | PL-18 | - | 69.43 | - |
| PL-9 | 69.24 | 67.83 | 58.597 | PL-19 | - | 69.52 | 57.766 |
| PL-10 | 69.63 | 68.12 | 56.788 |  |  |  |  |

## 1.3. HJ bispecific antibodies

HJ bispecific antibodies against hIL5 and hTSLP, HJ-3 to HJ11, which comprise a first heavy chain, a second heavy chain, a first light chain, and a second light chain as described below, were constructed:

the first heavy chain: when viewed from N-terminus to C-terminus, [VH1-H]-[linker 1]-[Titin chain]-[Fc1];
the first light chain: when viewed from N-terminus to C-terminus, [VL1-H]-[linker 2]-[Obscurin chain];
the second heavy chain: when viewed from N-terminus to C-terminus, [VH2-J]-[CH1]-[Fc2]; and
the second light chain: when viewed from N-terminus to C-terminus, [VL2-J]-[CL];

wherein the VH1-H and VL1-H were the heavy chain variable region and the light chain variable region of H0 in WO2021139758A1, respectively, and the VH2-J and VL2-J were the heavy chain variable region and the light chain variable region of J1 in WO2021139758A1, respectively. The structures of the Obscurin chain, the Titin chain, the linker 1, and the linker 2 in the HJ bispecific antibodies of this example are shown in the following table.

Table 12. Obscurin chain/Titin chain and linkers in HJ bispecific antibodies

| No. | First heavy chain | | First light chain | |
|---|---|---|---|---|
| | Titin chain | Linker 1 | Obscurin chain | Linker 2 |
| HJ-3 | T.10 | GGGGS | O.20 | GGGGS |
| HJ-5 | T.16 | GGGGS | O.25 | GGGGS |
| HJ-6 | T.16 | GGGGS | O.27 | GGGGS |
| HJ-7 | T.16 | GGGGS | O.28 | GGGGS |
| HJ-8 | T.16 | ASTKG | O.25 | RTVAS |
| HJ-9 | T.16 | ASTKG | O.27 | RTVAS |
| HJ-10 | T.16 | ASTKG | O.28 | RTVAS |
| HJ-11 | T.16 | ASTKG | O.29 | RTVAS |

The antigen binding activity of HJ bispecific antibodies was assayed with reference to the method in Test Example 4 of WO2021139758A1. The thermal stability of the antibodies was studied. Method: Diluted solutions of the HJ bispecific antibodies were prepared with a buffer containing 10 mM acetic acid at pH 5.5 and 9% sucrose and then concentrated by ultrafiltration to obtain HJ bispecific antibody solutions with different concentrations (the concentrations of the HJ bispecific antibodies are shown in Table 13-2). Subsequently, the concentrated solutions were incubated in a 40 °C incubator. At day 0 (i.e., before the 40 °C incubation, D0), day 7 (the 7th day of the 40 °C incubation, D7), day 14 (the 14th day of the 40 °C incubation, D14), day 21 (the 21st day of the 40 °C incubation, D21), and day 28 (the 28th day of the 40 °C incubation, D28), samples were taken and their SEC purity was tested. Immediately after 28 days of incubation at 40 °C, samples were taken and their CE-SDS purity was tested. The experimental results show that there was no significant change in the antigen binding activity of the HJ bispecific antibodies constructed in the present disclosure; additionally, the thermal stability of the HJ-5 to HJ-11 bispecific antibodies is better than that of HJ-3.

Table 13-1. Binding activity of HJ bispecific antibodies

| No. | hIL5 | hTSLP | No. | hIL5 | hTSLP |
|---|---|---|---|---|---|
| | $EC_{50}$ (nM) | | | $EC_{50}$ (nM) | |
| HJ-3 | 17.38 | 3.234 | HJ-8 | 18.74 | 3.332 |
| HJ-5 | 15.96 | 2.639 | HJ-9 | 10.86 | 3.184 |
| HJ-6 | 37.05 | 2.884 | HJ-10 | 20.81 | 3.173 |
| HJ-7 | 17.69 | 2.182 | HJ-11 | 9.464 | 3.005 |

Table 13-2. Accelerated stability test results for HJ bispecific antibodies

| No. | D0 concentration (mg/mL) | D0 SEC purity (%) | D28 SEC purity (%) | D28△ SEC purity (%) | D28 nonreduced CE-SDS purity (%) |
|---|---|---|---|---|---|
| HJ-3 | 50 | 98 | 84 | 14 | 67 |
| HJ-5 | 60.8 | 98.09 | 93.86 | 4.23 | 82.93 |
| HJ-6 | 54 | 98.14 | 89.68 | 8.46 | 80.76 |
| HJ-7 | 56.8 | 97.7 | 92.72 | 4.98 | 85.47 |
| HJ-8 | 62.6 | 93.39 | 84.28 | 9.11 | 73.03 |
| HJ-9 | 53.5 | 90.01 | 85.93 | 4.08 | 80.32 |
| HJ-10 | 69.8 | 90.9 | 88.31 | 2.59 | 80.4 |
| HJ-11 | 50.4 | 92.55 | 90.89 | 1.66 | 82.96 |

**Example 2. Screening and Identification of Anti-Human GUCY2C Murine Antibodies**

**2.1 Construction of Cell strains**

Cell strains CHO-K1/hGUCY2C and HCT116/hGUCY2C were constructed, and the sequences of related proteins are as follows:
Human GUCY2C protein (UniProtKB: P25092):

MKTLLLDLALWSLLFQPGWLSFSSQVSQNCHNGSYEISVLMMGNSAFAEPLK
NLEDAVNEGLEIVRGRLQNAGLNVTVNATFMYSDGLIHNSGDCRSSTCEGLD
LLRKISNAQRMGCVLIGPSCTYSTFQMYLDTELSYPMISAGSFGLSCDYKETLT
RLMSPARKLMYFLVNFWKTNDLPFKTYSWSTSYVYKNGTETEDCFWYLNAL
EASVSYFSHELGFKVVLRQDKEFQDILMDHNRKSNVIIMCGGPEFLYKLKGDR
AVAEDIVIILVDLFNDQYFEDNVTAPDYMKNVLVLTLSPGNSLLNSSFSRNLSPT
KRDFALAYLNGILLFGHMLKIFLENGENITTPKFAHAFRNLTFEGYDGPVTLDD
WGDVDSTMVLLYTSVDTKKYKVLLTYDTHVNKTYPVDMSPTFTWKNSKLP
NDITGRGPQILMIAVFTLTGAVVLLLLVALLMLRKYRKDYELRQKKWSHIPPE
NIFPLETNETNHVSLKIDDDKRRDTIQRLRQCKYDKKRVILKDLKHNDGNFTE
KQKIELNKLLQIDYYNLTKFYGTVKLDTMIFGVIEYCERGSLREVLNDTISYPD
GTFMDWEFKISVLYDIAKGMSYLHSSKTEVHGRLKSTNCVVDSRMVVKITDF
GCNSILPPKKDLWTAPEHLRQANISQKGDVYSYGIIAQEIILRKETFYTLSCRDR
NEKIFRVENSNGMKPFRPDLFLETAEEKELEVYLLVKNCWEEDPEKRPDFKKI
ETTLAKIFGLFHDQKNESYMDTLIRRLQLYSRNLEHLVEERTQLYKAERDRAD
RLNFMLLPRLVVKSLKEKGFVEPELYEEVTIYFSDIVGFTTICKYSTPMEVVDM
LNDIYKSFDHIVDHHDVYKVETIGDAYMVASGLPKRNGNRHAIDIAKMALEIL
SFMGTFELEHLPGLPIWIRIGVHSGPCAAGVVGIKMPRYCLFGDTVNTASRME
STGLPLRIHVSGSTIAILKRTECQFLYEVRGETYLKGRGNETTYWLTGMKDQK
FNLPTPPTVENQQRLQAEFSDMIANSLQKRQAAGIRSQKPRRVASYKKGTLEY
LQLNTTDKESTYF

SEQ ID NO: 1

The full-length gene encoding human GUCY2C was cloned into the mammalian cell expression vector pCDH, and HEK293T cells (ATCC, CRL-11268) were co-transfected with three plasmids, pVSV-G, pCMV-dR8.91 and pCDH-human GUCY2C, to package viruses. After 48 h of transfection, viruses were collected to infect CHO-K1 (ATCC, CCL-61) or HCT116 cells (ATCC, CCL-247). After two weeks of pressure screening, cell subcloning was performed, and cell strains CHO-K1/hGUCY2C and HCT116/hGUCY2C highly expressing GUCY2C were obtained by FACS.

**2.2 Construction of fusion proteins for immunization or screening**

The human GUCY2C extracellular region was selected, and a His or Fc tag was linked at the C terminus to construct a fusion protein for immunization and screening. The sequences of related proteins are as follows:

hGUCY2C ECD-hFc:

SQVSQNCHNGSYEISVLMMGNSAFAEPLKNLEDAVNEGLEIVRGRLQNAGLN
VTVNATFMYSDGLIHNSGDCRSSTCEGLDLLRKISNAQRMGCVLIGPSCTYST
FQMYLDTELSYPMISAGSFGLSCDYKETLTRLMSPARKLMYFLVNFWKTNDL
PFKTYSWSTSYVYKNGTETEDCFWYLNALEASVSYFSHELGFKVVLRQDKEF
QDILMDHNRKSNVIIMCGGPEFLYKLKGDRAVAEDIVIILVDLFNDQYFEDNVT

APDYMKNVLVLTLSPGNSLLNSSFSRNLSPTKRDFALAYLNGILLFGHMLKIFL
ENGENITTPKFAHAFRNLTFEGYDGPVTLDDWGDVDSTMVLLYTSVDTKKYK
VLLTYDTHVNKTYPVDMSPTFTWKNSKLPNDITGRGPQEPKSSDKTHTCPPCP
APELLGGPSVFLFPPKPKDTLMISRTPEVTCVVVDVSHEDPEVKFNWYVDGVE
VHNAKTKPREEQYNSTYRVVSVLTVLHQDWLNGKEYKCKVSNKALPAPIEKT
ISKAKGQPREPQVYTLPPSRDELTKNQVSLTCLVKGFYPSDIAVEWESNGQPEN
NYKTTPPVLDSDGSFFLYSKLTVDKSRWQQGNVFSCSVMHEALHNHYTQKSL
SLSPGK

SEQ ID NO: 2

hGUCY2C ECD-His:

SQVSQNCHNGSYEISVLMMGNSAFAEPLKNLEDAVNEGLEIVRGRLQNAGLN
VTVNATFMYSDGLIHNSGDCRSSTCEGLDLLRKISNAQRMGCVLIGPSCTYST
FQMYLDTELSYPMISAGSFGLSCDYKETLTRLMSPARKLMYFLVNFWKTNDL
PFKTYSWSTSYVYKNGTETEDCFWYLNALEASVSYFSHELGFKVVLRQDKEF
QDILMDHNRKSNVIIMCGGPEFLYKLKGDRAVAEDIVIILVDLFNDQYFEDNVT
APDYMKNVLVLTLSPGNSLLNSSFSRNLSPTKRDFALAYLNGILLFGHMLKIFL
ENGENITTPKFAHAFRNLTFEGYDGPVTLDDWGDVDSTMVLLYTSVDTKKYK
VLLTYDTHVNKTYPVDMSPTFTWKNSKLPNDITGRGPQHHHHHH

SEQ ID NO: 3

## 2.3 Screening and identification of anti-GUCY2C murine antibodies

In the present disclosure, monoclonal antibodies against human GUCY2C were prepared by hybridoma fusion or Single B technology, and the obtained antibodies specifically bind to human GUCY2C with high affinity and can have cross binding with cynomolgus monkey GUCY2C and have good binding activity for human GUCY2C on the cell surface.

hGUCY2C ECD-hFc, hGUCY2C ECD-His, the CHO-K1/hGUCY2C cell, or the HCT116/hGUCY2C cell was used as the immunogen. For immunization with protein, 50 μg of protein was used in the primary immunization and 25 μg of protein was used in each boost immunization; cross-immunizations were performed using the adjuvants TiterMax® Gold Adjuvant (Sigma Cat No. T2684) and Thermo Imject® Alum (Thermo Cat No. 77161). For immunization with cells, the immunization was performed according to $1 \times 10^7$ cells/mouse/immunization. After primary immunization and 4-7 times of boost immunization, mice with high antibody titer in serum were selected for spleen cell fusion or single B cell screening.

After fusion, the hybridoma culture supernatant was assayed based on the growth density of hybridoma cells. The hybridomas with good binding activity for human GUCY2C and antigens on the cell surface were selected by screening. Hybridoma cells were harvested, and the RNA was extracted using NucleoZol (MN) (following the procedures in the kit instructions) and reverse-transcribed (PrimeScript™ Reverse Transcriptase, Takara, cat # 2680A). The cDNA obtained by the reverse transcription was amplified by PCR using mouse Ig-Primer Set (Novagen, TB326 Rev.B 0503) and then sent for sequencing, and the amino acid sequences of the CDRs and variable regions of the antibodies were obtained.

Table 14. CDRs of anti-GUCY2C antibodies resulting from hybridoma fusion screening

| Antibody | Heavy chain CDR | | Light chain CDR | |
|---|---|---|---|---|
| M81 | HCDR1 | DHNMN (SEQ ID NO: 4) | LCDR1 | SASSSVNYMY (SEQ ID NO: 7) |
| | HCDR2 | VMNPKFGTINYNQKFKD (SEQ ID NO: 5) | LCDR2 | RTSNLAS (SEQ ID NO: 8) |
| | HCDR3 | DDYPNYFDY (SEQ ID NO: 6) | LCDR3 | QQYHDYPPT (SEQ ID NO: 9) |
| M146 | HCDR1 | DYNMN (SEQ ID NO: 10) | LCDR1 | SASSSLNYMY (SEQ ID NO: 13) |
| | HCDR2 | LINPKYGTTNYNQKFKG (SEQ ID NO: 11) | LCDR2 | RTSNLAS (SEQ ID NO: 8) |
| | HCDR3 | DDYPAWFAY (SEQ ID NO: 12) | LCDR3 | QQYHSYPPT (SEQ ID NO: 14) |
| M42 | HCDR1 | SYAVH | LCDR1 | RASSFVGSGYLH |
| | | (SEQ ID NO: 250) | | (SEQ ID NO: 253) |
| | HCDR2 | VIWTGGNTRSNAAFMS | LCDR2 | STSKLAS |
| | | (SEQ ID NO: 251) | | (SEQ ID NO: 254) |
| | HCDR3 | NSLLDYAMDY | LCDR3 | QQSSGYPLT |
| | | (SEQ ID NO: 252) | | (SEQ ID NO: 255) |
| M61 | HCDR1 | DYYMS | LCDR1 | SASSSISSNFLH |
| | | (SEQ ID NO: 256) | | (SEQ ID NO: 259) |
| | HCDR2 | DINPNNGGTSDNQKFKG | LCDR2 | RTSNLAS |
| | | (SEQ ID NO: 257) | | (SEQ ID NO: 8) |
| | HCDR3 | GRGMVARYFDI | LCDR3 | QQGSGLPLT |
| | | (SEQ ID NO: 258) | | (SEQ ID NO: 260) |
| M64 | HCDR1 | DYGMH | LCDR1 | SASSSVSYMY |
| | | (SEQ ID NO: 261) | | (SEQ ID NO: 264) |
| | HCDR2 | YISSGSSTIYYADTVKG | LCDR2 | DTSKLAS |
| | | (SEQ ID NO: 262) | | (SEQ ID NO: 265) |
| | HCDR3 | TRVYFYGSYWYFDV | LCDR3 | QQWSSDPPYT |
| | | (SEQ ID NO: 263) | | (SEQ ID NO: 266) |
| M93 | HCDR1 | RYWMH | LCDR1 | KASQDVSSAVA |
| | | (SEQ ID NO: 267) | | (SEQ ID NO: 270) |
| | HCDR2 | MIDPSDSYTKYNQKFMG | LCDR2 | SASYRST |
| | | (SEQ ID NO: 268) | | (SEQ ID NO: 271) |
| | HCDR3 | SIKVAGWYFDV | LCDR3 | QQHFSTTLT |
| | | (SEQ ID NO: 269) | | (SEQ ID NO: 272) |

>M81 VH:

EFQLQQSGPELVKPGASVKISCKASGYSFT<u>DHNMN</u>WVKQSNGKSLEWIG<u>VM
NPKFGTINYNQKFKD</u>KATLTVDQSSSTAYMQLNSLTSEDSAVYYCVR<u>DDYPN
YFDY</u>WGQGTTLTVSS

SEQ ID NO: 15

>M81 VL:

QIVLTQSPAIMSASPGEKVTISCSASSSVNYMYWYQQKPGSSPKSWIYRTSNLA
SGVSARFSGSGSGTSYSLTISSMEAEDAATYYCQQYHDYPPTFGGGTKLEIK

SEQ ID NO: 16

>M146 VH:

EFQLQQSGPELVKPGASVKISCKASGYSFTDYNMNWVKQSNGKSLEWIGLIN
PKYGTTNYNQKFKGKATLTVDQSSSTAYMQLNSLTSEDSAVYYCAIDDYPAW
FAYWGQGTLVTVSA

SEQ ID NO: 17

>M146 VL:

QIVLTQSPAIMSASPGEKVTISCSASSSLNYMYWYQQKPGSSPKSWIYRTSNLA
SGVPARFSGSGSGTSYSLTISSMEAEDAATYYCQQYHSYPPTFGAGTKLELK

SEQ ID NO: 18

>M42VH:

QIQLKQSGPGLVQPSQSLSITCTVSGFSLTSYAVHWVRQSPGKGLEWLGVIWT
GGNTRSNAAFMSRLTITKDNSKSQVFFKMNSLQADDTAIYFCAKNSLLDYAM
DYWGQGTSVTVSS

SEQ ID NO: 273

>M42VL:

ENVLTQSPVIMSASPGEKVTMTCRASSFVGSGYLHWYQQKSGASPKLWIYST
SKLASGVPARFSGSGSGTSFFLTISRVEIEDAAAYYCQQSSGYPLTFGGGSKLEI
K

SEQ ID NO: 274

>M61VH:

EVQLQQSGPELVRPGASVKISCTASGYTITDYYMSWVKQSHGKSPEWIGDINP
NNGGTSDNQKFKGKATLTVDKSSSTAYMEYRSLTSEDSAVYYCARGRGMVAR
YFDIWGTGTTVTVSS

SEQ ID NO: 275

>M61VL:

EILLTQSPTTMAASPGEKITITCSASSSISSNFLHWYQEKPGFSPKLLIYRTSNLA
SGVPARFSGSGSGTSYSLTIGSMEAEDVATYYCQQGSGLPLTFGAGTKLELK

SEQ ID NO: 276

>M64VH:

EVQLVESGGGLVKPGGSLKLSCAASGFTFS<u>DYGMH</u>WVRQAPEKGLEWVA<u>YIS SGSSTIYYADTVKG</u>RFTISRDNAKNTLFLQMTSLRSEDTAMYYCAR<u>TRVYFYG SYWYFDV</u>WGTGTTVTVSS

SEQ ID NO: 277

>M64VL:

QIVLTQSPAIMSASPGEKVTMTC<u>SASSSVSYMY</u>WYQQKSGTSPKRWIY<u>DTSKL AS</u>GVPARFSGSGSGTSYSLTISSLEAEDAATYYC<u>QQWSSDPPYT</u>FGGGTKLEIK

SEQ ID NO: 278

>M93VH:

QVQLQQPGAELVRPGTSVKLSCKASGYTFT<u>RYWMH</u>GVKRRPGQGLEWIG<u>MI DPSDSYTKYNQKFMG</u>KAILTVDTSSSTAYMQFSSLTSEDSAVYYCAR<u>SIKVAG WYFDV</u>WGTGTTVTVSS

SEQ ID NO: 279

>M93VL:

DIVMTQSHKFMSTSVGDRVSITC<u>KASQDVSSAVA</u>WYQQKPGQSPKLLIY<u>SASY RST</u>GVPDRFTGSGSGTDFTFTISSVQAEDLAIYYC<u>QQHFSTTLT</u>FGAGTKLELK

SEQ ID NO: 280

Note: The single underlined parts are CDRs according to the Kabat scheme, and the rest are FRs.

Screening of Single B cells was mainly performed by using forward and reverse screening, 10*genomic, and bioinformatics techniques to obtain a series of antibody variable region sequences. The CDR classification was performed on the obtained sequences, and murine variable region sequences were selected to be connected to the human antibody constant region sequences to express chimeric antibodies, which were allowed to bind to hGUCY2C ECD-His and cynoGUCY2C ECD-His (ACRO# GUC-C52H6); such methods as surface plasmon resonance and cell binding were used for screening to select antibody sequences specifically binding to hGUCY2C ECD and cynoGUCY2C ECD. The sequences of the heavy chain variable regions and light chain variable regions of the antibodies resulting from screening were as follows:

Table 15. CDRs of anti-GUCY2C antibodies resulting from Single B screening

| Antibody | Heavy chain CDR | | | Light chain CDR | |
|---|---|---|---|---|---|
| P3-C7 | HCDR1 | DHYIH | LCDR1 | RSSQSLVHSNGNTYLH | |
| | | (SEQ ID NO: 19) | | (SEQ ID NO: 22) | |
| | HCDR2 | VINPYNDGATYNQKFKG | LCDR2 | KVSNRFS | |
| | | (SEQ ID NO: 20) | | (SEQ ID NO: 23) | |
| | HCDR3 | PPRAYGNYAGY | LCDR3 | SQSTHVPYT | |
| | | (SEQ ID NO: 21) | | (SEQ ID NO: 24) | |

(continued)

| Antibody | Heavy chain CDR | | | Light chain CDR | | |
|---|---|---|---|---|---|---|
| P1-D2 | HCDR1 | DHNMN | | LCDR1 | SANSSVNYIY | |
| | | (SEQ ID NO: 281) | | | (SEQ ID NO: 284) | |
| | HCDR2 | VINPKYGTISNNQKFKG | | LCDR2 | RTSNLAS | |
| | | (SEQ ID NO: 282) | | | (SEQ ID NO: 8) | |
| | HCDR3 | DDYPNYFDY | | LCDR3 | QQYHSFPPT | |
| | | (SEQ ID NO: 283) | | | (SEQ ID NO: 285) | |
| P1-D3 | HCDR1 | TYGMS | | LCDR1 | SASSSVNYMH | |
| | | (SEQ ID NO: 286) | | | (SEQ ID NO: 289) | |
| | HCDR2 | WINTYSGVPTYADDFKG | | LCDR2 | DTSKLAS | |
| | | (SEQ ID NO: 287) | | | (SEQ ID NO: 265) | |
| | HCDR3 | RGYSNLYYYALDY | | LCDR3 | QQWSSNPST | |
| | | (SEQ ID NO: 288) | | | (SEQ ID NO: 290) | |
| P1-C6 | HCDR1 | SYGIT | | LCDR1 | RASESVSLIGTNLIH | |
| | | (SEQ ID NO: 291) | | | (SEQ ID NO: 294) | |
| | HCDR2 | EIYPRSGNTYYNEKFKG | | LCDR2 | HASNLEA | |
| | | (SEQ ID NO: 292) | | | (SEQ ID NO: 295) | |
| | HCDR3 | AITTVVGDWYFDV | | LCDR3 | LQSRKIRT | |
| | | (SEQ ID NO: 293) | | | (SEQ ID NO: 296) | |
| P1-D9 | HCDR1 | DYGMH | | LCDR1 | SASSSVSYMY | |
| | | (SEQ ID NO: 261) | | | (SEQ ID NO: 264) | |
| | HCDR2 | HISSGSSTIYYADTVKG | | LCDR2 | LTSNLAS | |
| | | (SEQ ID NO: 297) | | | (SEQ ID NO: 299) | |
| | HCDR3 | SRNYYGSRIFDY | | LCDR3 | QQWSSNPPT | |
| | | (SEQ ID NO: 298) | | | (SEQ ID NO: 300) | |
| P1-F10 | HCDR1 | DYGMH | | LCDR1 | SASSSVNYMY | |
| | | (SEQ ID NO: 261) | | | (SEQ ID NO: 303) | |
| | HCDR2 | HISSGSSTIYYADTVKG | | LCDR2 | LTSNLAS | |
| | | (SEQ ID NO: 301) | | | (SEQ ID NO: 299) | |
| | HCDR3 | SRNYYGSRVFDY | | LCDR3 | QQWNSNPPT | |
| | | (SEQ ID NO: 302) | | | (SEQ ID NO: 304) | |
| P2-C8 | HCDR1 | DYYMH | | LCDR1 | RSSQSLVHSNGNTYLH | |
| | | (SEQ ID NO: 305) | | | (SEQ ID NO: 22) | |
| | HCDR2 | LINPYNGVTSYNQKFKG | | LCDR2 | KVSNRFS | |
| | | (SEQ ID NO: 306) | | | (SEQ ID NO: 23) | |
| | HCDR3 | PPRAYGNYAGY | | LCDR3 | SQSTHVPYT | |
| | | (SEQ ID NO: 21) | | | (SEQ ID NO: 24) | |

(continued)

| Antibody | Heavy chain CDR | | | Light chain CDR | | |
|---|---|---|---|---|---|---|
| P2-B9 | HCDR1 | SYWMH | | LCDR1 | KASKKVTIFGSISALH | |
| | | (SEQ ID NO: 128) | | | (SEQ ID NO: 309) | |
| | HCDR2 | MIHPKSDSVNNNEKFKN | | LCDR2 | NGAKLES | |
| | | (SEQ ID NO: 307) | | | (SEQ ID NO: 310) | |
| | HCDR3 | GGDSGYSMDY | | LCDR3 | LQNKEVPYT | |
| | | (SEQ ID NO: 308) | | | (SEQ ID NO: 311) | |
| P3-D2 | HCDR1 | DYFMH | | LCDR1 | RSSQSLVHSNGNTYLH | |
| | | (SEQ ID NO: 312) | | | (SEQ ID NO: 22) | |
| | HCDR2 | LINPYSGGTTYNQKFKG | | LCDR2 | KVSNRFS | |
| | | (SEQ ID NO: 313) | | | (SEQ ID NO: 23) | |
| | HCDR3 | PPRAYGDYAGY | | LCDR3 | SQSTHVPLT | |
| | | (SEQ ID NO: 314) | | | (SEQ ID NO: 315) | |
| P3-B4 | HCDR1 | TYGMS | | LCDR1 | SASSSVSYML | |
| | | (SEQ ID NO: 286) | | | (SEQ ID NO: 317) | |
| | HCDR2 | WINTYSGVPTYADDFKG | | LCDR2 | DTSKLAS | |
| | | (SEQ ID NO: 287) | | | (SEQ ID NO: 265) | |
| | HCDR3 | RAGSSLYYYAMDY | | LCDR3 | QQWSSNPPT | |
| | | (SEQ ID NO: 316) | | | (SEQ ID NO: 300) | |
| P3-E4 | HCDR1 | NYWMH | | LCDR1 | RASESVSIHGTHLMH | |
| | | (SEQ ID NO: 318) | | | (SEQ ID NO: 321) | |
| | HCDR2 | EIDPSDGYTDYNQKFKG | | LCDR2 | AASNLES | |
| | | (SEQ ID NO: 319) | | | (SEQ ID NO: 322) | |
| | HCDR3 | SLRGPITFGPFPY | | LCDR3 | QHSIEDPRT | |
| | | (SEQ ID NO: 320) | | | (SEQ ID NO: 323) | |
| P3-F6 | HCDR1 | DYYMN | | LCDR1 | RSSQSLVHSNGNTYLH | |
| | | (SEQ ID NO: 324) | | | (SEQ ID NO: 22) | |
| | HCDR2 | DINPNNGGTSYNQKFKG | | LCDR2 | KVSNRFS | |
| | | (SEQ ID NO: 325) | | | (SEQ ID NO: 23) | |
| | HCDR3 | KTWLPYASVY | | LCDR3 | SQSTHVPPYT | |
| | | (SEQ ID NO: 326) | | | (SEQ ID NO: 327) | |
| P3-D9 | HCDR1 | DYGMH | | LCDR1 | SASSSVSYMY | |
| | | (SEQ ID NO: 261) | | | (SEQ ID NO: 264) | |
| | HCDR2 | HISSGSSTIYYGDTVKG | | LCDR2 | LTSSLAS | |
| | | (SEQ ID NO: 328) | | | (SEQ ID NO: 329) | |
| | HCDR3 | SRNYYGSRVFDY | | LCDR3 | QQWSSNPPT | |
| | | (SEQ ID NO: 302) | | | (SEQ ID NO: 300) | |

>P3-C7 VH:

EVQLQQSGPVLLKPGASVKMSCKASGYTFT<u>DHYIH</u>WVKLSHGKSLEWIG<u>VIN
PYNDGATYNQKFKG</u>KATLTVDKSSSTAYMELNSLTSEDSAVYYCAR<u>PPRAYG
NYAGY</u>WGQGTTLTVSS

SEQ ID NO: 25

>P3-C7 VL:

DVVMTQTPLSLPVSLGDQASISC<u>RSSQSLVHSNGNTYLH</u>WYLQKPGQSPKLLI
Y<u>KVSNRFS</u>GVPDRFIGSGSRTDFTLKISRVEAEDLGVYFC<u>SQSTHVPYT</u>FGGGT
RLEIK

SEQ ID NO: 26

>P1-D2 VH

EFQLQQSGPELVKPGASVKISCKASGYSFT<u>DHNMN</u>WVKQSNGKSLEWIG<u>VIN
PKYGTISNNQKFKG</u>KATLTVDQSSSTAYMQLNSLTAEDSAVYYCAR<u>DDYPNYF
DY</u>WGQGTTLTVSS

SEQ ID NO: 330

>P1-D2 VL

QIVLTQSPAIMSASPGEKVTISC<u>SANSSVNYIY</u>WYQQKPGSSPKPWIY<u>RTSNLA
S</u>GVPARFSGSGSGTSYSLTISSMEAEDAATYYC<u>QQYHSFPPT</u>FGGGTKLEIK

SEQ ID NO: 331

>P1-D3 VH

QIQLIQSGPELKKPGETVKISCKASGYTFT<u>TYGMS</u>WVKQAPGKGLKWMG<u>WIN
TYSGVPTYADDFKG</u>RFAFSLETSANTAYLQINNLKNEDTATYFCAR<u>RGYSNLY
YYALDY</u>WGQGASVTVSS

SEQ ID NO: 332

>P1-D3 VL

QIVLTQSPAIMSASPGEKVTMTC<u>SASSSVNYMH</u>WYQQKSGTSPKRWIY<u>DTSK
LAS</u>GVPTRFSGSGSGTSYSLTISSMEAEDAATYFC<u>QQWSSNPST</u>FGGGTKLEIK

SEQ ID NO: 333

>P1-C6 VH

QVQLQQSGAELARPGASVKLSCKASGYTFT<u>SYGIT</u>WVKRRTGQGLGWIG<u>EIY
PRSGNTYYNEKFKG</u>KATLTADKSSSTAYMELRSLTSEDSAVYFCAR<u>AITTVVG
DWYFDV</u>WGTGTTVTVSS

SEQ ID NO: 334

>P1-C6 VL

DIVLTQSPASLAMSLGKRATISC<u>RASESVSLIGTNLIH</u>WYQQKPGQPPKLLIH<u>H
ASNLEA</u>GVPARFSGSGSRTDFTLTIDPVEEDDVAIYYC<u>LQSRKIRT</u>FGGGTKLEI
K

SEQ ID NO: 335

>P1-D9 VH

EVQLVESGGGLVKPGGSLKLSCAASGFSFS<u>DYGMH</u>WVRQAPEKGLEWIA<u>HIS
SGSSTIYYADTVKG</u>RFTISRDNAKNTLFLQMTSLRSEDTAMYYCAR<u>SRNYYGS
RIFDY</u>WGQGTTLTVSS

SEQ ID NO: 336

>P1-D9 VL

QIVLTQSPARMSASPGEKVTMTC<u>SASSSVSYMY</u>WYQQKPRSSPKPWIY<u>LTSNL
AS</u>GVPARFSGSGSGTSYSLTISSMEAEDAATYYC<u>QQWSSNPPT</u>FGSGTKLEIK

SEQ ID NO: 337

>P1-F10 VH

EVQLVESGGGLVKPGGSLKLSCAASGFAFS<u>DYGMH</u>WVRQAPEKGLEWVA<u>HIS
SGSSTIYYADTVKG</u>RFTISRDNVKNTLFLQMTSLRSEDTAMYYCAR<u>SRNYYGS
RVFDY</u>WGQGTPLTVSS

SEQ ID NO: 338

>P1-F10 VL

QIVLTQSPALMSVSPGEKVTMTCSASSSVNYMYWYQQKPRSSPKPWIYLTSNL
ASGVPARFSGSGSGTSYSLTISSMEAEDAATYYCQQWNSNPPTFGSGTKLEIK

SEQ ID NO: 339

>P2-C8 VH

EVQLQQSGPVLVKPGASVKMSCKVSGYTFTDYYMHWVKQSHGKSLEWIGLI NPYNGVTSYNQKFKGKATLTVDKSSSTAYMELNSLTSEDSAVYYCARPPRAY GNYAGYWGQGTTLTVSS

SEQ ID NO: 340

>P2-C8 VL

DVVMTQTPLSLPVSLGDQASISCRSSQSLVHSNGNTYLHWYLQKPGQSPKLLI YKVSNRFSGVPDRFSGSGSGTDFTLKISRVEAEDLGVYFCSQSTHVPYTFGGG TKLEIK

SEQ ID NO: 341

>P2-B9 VH

QVQLQQPGAELVKPGASVKLSCKASGYTFSSYWMHWVKQRPGQGLEWIGMI HPKSDSVNNNEKFKNKATLTVDKSSSTVYLQLSSLTSEDSAVYSCARGGDSGY SMDYWGQGTSVTVSS

SEQ ID NO: 342

>P2-B9 VL

DIVLTQSPASLAVSLGQKATISCKASKKVTIFGSISALHWYQQKPGQPPKLIYN GAKLESGVSARFSDSGSQNRSPFGNQLSFTLTIDPVEADDAATYYCLQNKEVP YTFGGGTKLEIK

SEQ ID NO: 343

>P3-D2 VH

EVQLQQSGPVLVKPGASVKMSCKASGYTFTDYFMHWVKQSHGKSLEWIGLI NPYSGGTTYNQKFKGKATLTVDKSSSTAYMELNSLTSEDSAVYYCARPPRAYG DYAGYWGQGTTLTVSS

SEQ ID NO: 344

>P3-D2 VL

DVVMTQTPLSLPVSLGDQASISCRSSQSLVHSNGNTYLHWYLQKPGQSPKLLI YKVSNRFSGVPDRFSGSGSGTDFTLKISRVEAEDLGVYFCSQSTHVPLTFGTGT KLEIK

SEQ ID NO: 345

>P3-B4 VH

QIQLVQSGPELKKPGEIVKISCKASGYTFT<u>TYGMS</u>WVKQAPGKGLKWMG<u>WIN TYSGVPTYADDFKG</u>RFAFSLETSASTAYLQINNLKNEDTATYFCAR<u>RAGSSLYY YAMDY</u>WGQGTSVTVSS

SEQ ID NO: 346

>P3-B4 VL

QIVLTQSPAIMSASPGEKVTMTC<u>SASSSVSYML</u>WYQQKSGTSPKRWIY<u>DTSKL AS</u>GVPARFSGSGSGTSYSLTISSMEAEDAATYYC<u>QQWSSNPPT</u>FGGGTKLEIK

SEQ ID NO: 347

>P3-E4 VH

QVQLQQPGAELVMPGAAVKLSCKASGYTF<u>INYWMH</u>WLKQRPGQGLEWIG<u>EI DPSDGYTDYNQKFKG</u>KATLTVDKSSSTAYMQLSSLTSEDSAVYYCAI<u>SLRGPIT FGPFPY</u>WGQGTLVTVSS

SEQ ID NO: 348

>P3-E4 VL

DIVLTQSPASLAVSLGQRATISC<u>RASESVSIHGTHLMH</u>WYQQKPGQPPKLLIY<u>A ASNLES</u>GVPARFSGSGSETDFTLNIHPVAEEDAATYFC<u>QHSIEDPRT</u>FGGGTKL EIK

SEQ ID NO: 349

>P3-F6 VH

EVQLQQSGPELVKPGASVKISCKASGYTFT<u>DYYMN</u>WVKQSHGKSLEWIG<u>DIN PNNGGTSYNQKFKG</u>KATLTVDKSSSTAYMELRSLTSEDSAVYYCAR<u>KTWLPY ASVY</u>WGQGTSVTVSS

SEQ ID NO: 350

>P3-F6 VL

DVVMTQTPLSLPVSLGDQASISC<u>RSSQSLVHSNGNTYLH</u>WYLQKPGQSPKLLI Y<u>KVSNRFS</u>GVPDRFSGSGSGTDFTLKISRVEAEDLGVYFC<u>SQSTHVPPYT</u>FGG GTKLEIK

SEQ ID NO: 351

>P3-D9 VH

EVQLVESGGGGLVKPGGSLKLSCAASGFAFS<u>DYGMH</u>WVRQAPEKGLEWVA<u>HIS SGSSTIYYGDTVKG</u>RFTISRDNAKNTLFLQMTSLRSEDTAMYYCTR<u>SRNYYGS RVFDY</u>WGQGTTLTVSS

SEQ ID NO: 352

>P3-D9 VL

QIVLTQSPALMSVSPGEKVTMTC<u>SASSSVSYMY</u>WYQQKPRSSPKPWIY<u>LTSSL AS</u>GVPARFSGSGSGTSYSLTINNVEAEDAATYYC<u>QQWSSNPPT</u>FGSGTKVEIK

SEQ ID NO: 353

Note: The single underlined parts are CDRs according to the Kabat scheme, and the rest are FRs.

The variable region sequences of the murine anti-GUCY2C antibodies were combined with the constant regions set forth in SEQ ID NO: 58 and SEQ ID NO: 59 to obtain chimeric antibodies. Illustratively, M81CHI or P3-C7 CHI represents a chimeric antibody comprising M81 or P3-C7 murine heavy chain variable region and light chain variable region and the constant regions, and the specific sequences are shown below. This applies similarly to the remaining chimeric antibodies M146 CHI, M42 CHI, M61 CHI, M64 CHI, M93 CHI, P1-D2 CHI, P1-D3 CHI, P1-C6 CHI, P1-D9 CHI, P1-F10 CHI, P2-C8 CHI, P2-B9 CHI, P3-D2 CHI, P3-B4 CHI, P3-E4 CHI, P3-F6 CHI, and P3-D9 CHI; their full-length sequences may be unambiguously determined and therefore are not all listed for the sake of brevity.

Illustratively, the full-length sequences of the anti-GUCY2C chimeric antibodies are as follows:

M81CHI heavy chain:

EFQLQQSGPELVKPGASVKISCKASGYSFT<u>DHNMN</u>WVKQSNGKSLEWIG<u>VM NPKFGTINYNQKFKD</u>KATLTVDQSSSTAYMQLNSLTSEDSAVYYCVR<u>DDYPN YFDY</u>WGQGTTLTVSS*ASTKGPSVFPLAPSSKSTSGGTAALGCLVKDYFPEPVTVS WNSGALTSGVHTFPAVLQSSGLYSLSSVVTVPSSSLGTQTYICNVNHKPSNTKVDKK VEPKSCDKTHTCPPCPAPELLGGPSVFLFPPKPKDTLMISRTPEVTCVVVDVSHED PEVKFNWYVDGVEVHNAKTKPREEQYNSTYRVVSVLTVLHQDWLNGKEYKCKVS NKALPAPIEKTISKAKGQPREPQVYTLPPSRDELTKNQVSLTCLVKGFYPSDIAVEW ESNGQPENNYKTTPPVLDSDGSFFLYSKLTVDKSRWQQGNVFSCSVMHEALHNHY TQKSLSLSPGK*

SEQ ID NO: 354

M81CHI light chain:

QIVLTQSPAIMSASPGEKVTISC<u>SASSSVNYMY</u>WYQQKPGSSPKSWIY<u>RTSNLA S</u>GVSARFSGSGSGTSYSLTISSMEAEDAATYYC<u>QQYHDYPPT</u>FGGGTKLEIK*RT VAAPSVFIFPPSDEQLKSGTASVVCLLNNFYPREAKVQWKVDNALQSGNSQESVTE QDSKDSTYSLSSTLTLSKADYEKHKVYACEVTHQGLSSPVTKSFNRGEC*

SEQ ID NO: 355

P3-C7 CHI heavy chain:

EVQLQQSGPVLLKPGASVKMSCKASGYTFT<u>DHYIH</u>WVKLSHGKSLEWIG<u>VIN PYNDGATYNQKFKG</u>KATLTVDKSSSTAYMELNSLTSEDSAVYYCARP<u>PRAYG NYAGY</u>WGQGTTLTVSS*ASTKGPSVFPLAPSSKSTSGGTAALGCLVKDYFPEPVTV SWNSGALTSGVHTFPAVLQSSGLYSLSSVVTVPSSSLGTQTYICNVNHKPSNTKVDKK*

*VEPKSCDKTHTCPPCPAPELLGGPSVFLFPPKPKDTLMISRTPEVTCVVVDVSHED PEVKFNWYVDGVEVHNAKTKPREEQYNSTYRVVSVLTVLHQDWLNGKEYKCKVS NKALPAPIEKTISKAKGQPREPQVYTLPPSRDELTKNQVSLTCLVKGFYPSDIAVEW ESNGQPENNYKTTPPVLDSDGSFFLYSKLTVDKSRWQQGNVFSCSVMHEALHNHY TQKSLSLSPGK*

SEQ ID NO: 356

P3-C7 CHI light chain:

DVVMTQTPLSLPVSLGDQASISC<u>RSSQSLVHSNGNTYLH</u>WYLQKPGQSPKLLI Y<u>KVSNRFS</u>GVPDRFIGSGSRTDFTLKISRVEAEDLGVYFC<u>SQSTHVPYT</u>FGGGT RLEIK*RTVAAPSVFIFPPSDEQLKSGTASVVCLLNNFYPREAKVQWKVDNALQSGN SQESVTEQDSKDSTYSLSSTLTLSKADYEKHKVYACEVTHQGLSSPVTKSFNRGEC*

SEQ ID NO: 357

**Example 3: Humanization of Anti-GUCY2C Monoclonal Antibodies**

Humanization of the murine antibodies was performed according to the method described in many publications in the art. In brief, based on the typical structures of the VH/VL CDRs of the murine antibodies obtained, a human germline database was searched for homologous sequences of the light chain variable regions (VLs) and the heavy chain variable regions (VHs), the sequences were ranked in a descending manner according to the FR homology, and the germline with the highest FR homology was selected as the template; the CDRs of the murine antibodies were grafted onto a human template, and certain amino acids of the variable regions were mutated; the constant regions of the murine antibodies were replaced with a human constant region to obtain the final humanized antibodies.

**3-1. Humanization of antibody M81**

For the humanized antibodies of the antibody M81, IGKV3-11*01, IGKV6-21*02 or IGKV1-9*01, and IGKJ4*01 were selected as the light chain framework region templates, and IGHV1-3*01 and IGHJ6*01 were selected as the heavy chain framework region templates. The CDR regions of the murine antibody were grafted onto the selected humanization templates, and certain amino acids in the variable regions were substituted to obtain the light chain variable regions and heavy chain variable regions of the humanized antibody; specific framework replacements and amino acid substitutions are as follows:

Table 16. Framework replacements and amino acid substitutions for humanization of antibody M81

| VL | | | VH | | |
|---|---|---|---|---|---|
| L1 | Graft(IGKV3-11*01) + R45K, L46S, L47W, I58V, F71Y | | H1 | Graft(IGHV1-3*01) + Q1E,R38K,R66K,V67A + D96N | |
| L2 | Graft(IGKV6-21*02) + K16G, L46S, L47W, K49Y, N76S | | H2 | Graft(IGHV1-3*01) + Q1E,R38K,R66K,V67A | |
| L3 | Graft(IGKV1-9*01) + V15P, K42S, A43S, L46S, L47W, F71Y | | | | |

(continued)

| | VL | VH | |
|---|---|---|---|
| L4 | Graft(IGKV1-9*01) + V15P, K42S, A43S, L46S, L47W, F71Y+L54D | | |
| L5 | Graft(IGKV1-9*01) + V15P, K42S, A43S, L46S, L47W, F71Y+ S56Q | | |
| L6 | Graft(IGKV1-9*01) + V15P, K42S, A43S, L46S, L47W, F71Y+S52Q | | |
| FR4 | IGKJ4*01 | FR4 | IGHJ6*01 |
| Note: the numbering of amino acids is according to the Kabat numbering scheme. | | | |

The CDRs of the humanized antibodies of M81 are as follows:

Table 17. CDRs of humanized antibodies of M81

| Variable region | CDR | Sequence | SEQ ID NO |
|---|---|---|---|
| h81 H1 | HCDR3 | DNYPNYFDY | 27 |
| h81 L4 | LCDR2 | RTSNDAS | 28 |
| h81 L5 | LCDR2 | RTSNLAQ | 29 |
| h81 L6 | LCDR2 | RTQNLAS | 30 |

The variable region sequences of the humanized antibodies of M81 are as follows:

> h81 L1:

EIVLTQSPATLSLSPGERATLSCSASSSVNYMYWYQQKPGQAPKSWIYRTSNL
ASGVPARFSGSGSGTDYTLTISSLEPEDFAVYYCQQYHDYPPTFGGGTKVEIK

SEQ ID NO: 31

> h81 L2:

EIVLTQSPDFQSVTPGEKVTITCSASSSVNYMYWYQQKPDQSPKSWIYRTSNL
ASGVPSRFSGSGSGTDFTLTISSLEAEDAATYYCQQYHDYPPTFGGGTKVEIK

SEQ ID NO: 32

> h81 L3:

DIQLTQSPSFLSASPGDRVTITCSASSSVNYMYWYQQKPGSSPKSWIYRTSNLA
SGVPSRFSGSGSGTEYTLTISSLQPEDFATYYCQQYHDYPPTFGGGTKVEIK

SEQ ID NO: 33

> h81 L4:

DIQLTQSPSFLSASPGDRVTITCSASSSVNYMYWYQQKPGSSPKSWIYRTSND

ASGVPSRFSGSGSGTE<u>Y</u>TLTISSLQPEDFATYYC<u>QQYHDYPPT</u>FGGGTKVEIK

SEQ ID NO: 34

> h81 L5:

DIQLTQSPSFLSAS<u>P</u>GDRVTITC<u>SASSSVNYMY</u>WYQQKPG<u>SS</u>PK<u>SW</u>IY<u>RTSNLA</u>
<u>Q</u>GVPSRFSGSGSGTE<u>Y</u>TLTISSLQPEDFATYYC<u>QQYHDYPPT</u>FGGGTKVEIK

SEQ ID NO: 35

> h81 L6:

DIQLTQSPSFLSAS<u>P</u>GDRVTITC<u>SASSSVNYMY</u>WYQQKPG<u>SS</u>PK<u>SW</u>IY<u>RTQNL</u>
<u>A</u>SGVPSRFSGSGSGTE<u>Y</u>TLTISSLQPEDFATYYC<u>QQYHDYPPT</u>FGGGTKVEIK

SEQ ID NO: 36

> h81 H1:

<u>E</u>VQLVQSGAEVKKPGASVKVSCKASGYTFT<u>DHNMN</u>WV<u>K</u>QAPGQRLEWMG
<u>VMNPKFGTINYNQKFKD</u><u>KA</u>TITRDTSASTAYMELSSLRSEDTAVYYCARD<u>NY</u>
<u>PNYFDY</u>WGQGTTVTVSS

SEQ ID NO: 37

> h81 H2:

<u>E</u>VQLVQSGAEVKKPGASVKVSCKASGYTFT<u>DHNMN</u>WV<u>K</u>QAPGQRLEWMG
<u>VMNPKFGTINYNQKFKD</u><u>KA</u>TITRDTSASTAYMELSSLRSEDTAVYYCAR<u>DDY</u>
<u>PNYFDY</u>WGQGTTVTVSS

SEQ ID NO: 38

Note: the single underlined parts are CDRs, the dotted underlined bold parts are the amino acid substitution sites, and the rest are FRs.

**3-2. Humanization of antibody M146**

For the humanized antibodies of the antibody M146, IGKV3-11*01 and IGKJ4*01 were selected as the light chain framework region templates, and IGHV1-3*01 and IGHJ6*01 were selected as the heavy chain framework region templates. The CDR regions of the murine antibody were grafted onto the selected humanization templates, and certain amino acids in the variable regions were substituted to obtain the light chain variable regions and heavy chain variable regions of the humanized antibody; specific framework replacements and amino acid substitutions are as follows:

Table 18. Framework replacements and amino acid substitutions for humanization of antibody M146

| VL | | VH | |
|---|---|---|---|
| L1 | Graft(IGKV3-11*01) + Q42S,A43S,L46P,L47-W,I58V,F71Y+N 53Q | H1 | Graft(IGHV1-3*01) + Q1E,V2F,T28-S,I69L,R71V,T73Q,A75S +D96N |
| FR4 | IGKJ4*01 | FR4 | IGHJ6*01 |
| Note: the numbering of amino acids is according to the Kabat numbering scheme. | | | |

The CDRs of the humanized antibodies of M146 were as follows:

Table 19. CDRs of humanized antibodies of M146

| Variable region | CDR | Sequence | SEQ ID NO |
|---|---|---|---|
| h146 L1 | LCDR2 | RTSQLAS | 39 |
| h146 H1 | HCDR3 | DNYPAWFAY | 40 |

The variable region sequences of the humanized antibodies of M146 are as follows:

>h146 L1:

EIVLTQSPATLSLSPGERATLSCSASSSLNYMYWYQQKPGSSPRPWIYRTSQLA
SGVPARFSGSGSGTDYTLTISSLEPEDFAVYYCQQYHSYPPTFGGGTKVEIK

SEQ ID NO: 41

> h146 H1:

EFQLVQSGAEVKKPGASVKVSCKASGYSFTDYNMNWVRQAPGQRLEWMGL
INPKYGTTNYNQKFKGRVTLTVDQSSSTAYMELSSLRSEDTAVYYCARDNYP
AWFAYWGQGTTVTVSS

SEQ ID NO: 42

Note: the single underlined parts are CDRs, the dotted underlined bold parts are the amino acid substitution sites, and the rest are FRs.

### 3-3. Humanization of antibody P3-C7

For the humanized antibodies of the antibody P3-C7, IGKV2-40*01 or IGKV1-39*01 and IGKJ4*01 were selected as the light chain framework region templates, and IGHV1-46*01 and IGHJ6*01 were selected as the heavy chain framework region templates. The CDR regions of the murine antibody were grafted onto the selected humanization templates, and certain amino acids in the variable regions were substituted to obtain the light chain variable regions and heavy chain variable regions of the humanized antibody; specific framework replacements and amino acid substitutions are as follows:

Table 20. Framework replacements and amino acid substitutions for humanization of antibody P3-C7

| VL | | VH | |
|---|---|---|---|
| L1 | Graft(IGKV2-40*01) | H1 | Graft(IGHV1-46*01) + Q1E, R71V, T73K |
| L2 | Graft(IGKV2-40*01) + I2V, G68R | H2 | Graft(IGHV1-46*01) + Q1E, Q39L, Q43K, M69L, R71V, T73K |
| L3 | Graft(IGKV2-40*01) + I2V, G68R + N28Q | | |
| L4 | Graft(IGKV2-40*01) + I2V, G68R + N28S | | |
| L5 | Graft(IGKV2-40*01) + I2V, G68R + N28T | | |
| L6 | Graft(IGKV2-40*01) + I2V, G68R + G29V | | |
| L7 | Graft(IGKV2-40*01) + I2V, G68R + G29Q | | |
| L8 | Graft(IGKV1-39*01) + I2V, Q3V, G68R, T74K + G29Q | | |

(continued)

| VL | | VH | |
|---|---|---|---|
| FR4 | IGKJ4*01 | FR4 | IGHJ6*01 |

Note: the numbering of amino acids is according to the Kabat numbering scheme.

The CDRs of the humanized antibodies of P3-C7 were as follows:

Table 21. CDRs of humanized antibodies of P3-C7

| Variable region | CDR | Sequence | SEQ ID NO |
|---|---|---|---|
| hP3-C7 L3 | LCDR1 | RSSQSLVHS**Q**GNTYLH | 43 |
| hP3-C7 L4 | LCDR1 | RSSQSLVHS**S**GNTYLH | 44 |
| hP3-C7 L5 | LCDR1 | RSSQSLVHS**T**GNTYLH | 45 |
| hP3-C7 L6 | LCDR1 | RSSQSLVHSN**V**NTYLH | 46 |
| hP3-C7 L7/ hP3-C7 L8 | LCDR1 | RSSQSLVHSN**Q**NTYLH | 47 |

The variable region sequences of the humanized antibodies of P3-C7 are as follows:

> hP3-C7 L1:

DIVMTQTPLSLPVTPGEPASISC<u>RSSQSLVHSNGNTYLH</u>WYLQKPGQSPQLLIY<u>KVSNRFS</u>GVPDRFSGSGSGTDFTLKISRVEAEDVGVYYC<u>SQSTHVPYT</u>FGGGTKVEIK

SEQ ID NO: 48

> hP3-C7 L2:

D**V**VMTQTPLSLPVTPGEPASISC<u>RSSQSLVHSNGNTYLH</u>WYLQKPGQSPQLLIY<u>KVSNRFS</u>GVPDRFSGSGSGS**R**TDFTLKISRVEAEDVGVYYC<u>SQSTHVPYT</u>FGGGTKVEIK

SEQ ID NO: 49

> hP3-C7 L3:

D**V**VMTQTPLSLPVTPGEPASISC<u>RSSQSLVHS**Q**GNTYLH</u>WYLQKPGQSPQLLIY<u>KVSNRFS</u>GVPDRFSGSGSGS**R**TDFTLKISRVEAEDVGVYYC<u>SQSTHVPYT</u>FGGGTKVEIK

SEQ ID NO: 50

> hP3-C7 L4:

DVVMTQTPLSLPVTPGEPASISCRSSQSLVHSSGNTYLHWYLQKPGQSPQLLIY
KVSNRFSGVPDRFSGSGSRTDFTLKISRVEAEDVGVYYCSQSTHVPYTFGGGT
KVEIK

SEQ ID NO: 51

> hP3-C7 L5:

DVVMTQTPLSLPVTPGEPASISCRSSQSLVHSTGNTYLHWYLQKPGQSPQLLIY
KVSNRFSGVPDRFSGSGSRTDFTLKISRVEAEDVGVYYCSQSTHVPYTFGGGT
KVEIK

SEQ ID NO: 52

> hP3-C7 L6:

DVVMTQTPLSLPVTPGEPASISCRSSQSLVHSNVNTYLHWYLQKPGQSPQLLI
YKVSNRFSGVPDRFSGSGSRTDFTLKISRVEAEDVGVYYCSQSTHVPYTFGGG
TKVEIK

SEQ ID NO: 53

> hP3-C7 L7:

DVVMTQTPLSLPVTPGEPASISCRSSQSLVHSNQNTYLHWYLQKPGQSPQLLI
YKVSNRFSGVPDRFSGSGSRTDFTLKISRVEAEDVGVYYCSQSTHVPYTFGGG
TKVEIK

SEQ ID NO: 54

> hP3-C7 L8:

DVVMTQSPSSLSASVGDRVTITCRSSQSLVHSNQNTYLHWYQQKPGKAPKLLI
YKVSNRFSGVPSRFSGSGSRTDFTLKISSLQPEDFATYYCSQSTHVPYTFGGGT
KVEIK

SEQ ID NO: 55

> hP3-C7 H1:

EVQLVQSGAEVKKPGASVKVSCKASGYTFTDHYIHWVRQAPGQGLEWMGVI
NPYNDGATYNQKFKGRVTMTVDKSTSTVYMELSSLRSEDTAVYYCARPPRAY
GNYAGYWGQGTTVTVSS

SEQ ID NO: 56

> hP3-C7 H2:

EVQLVQSGAEVKKPGASVKVSCKASGYTFTDHYIHWVRLAPGKGLEWMGVI

<u>NPYNDGATYNQKFKGRVT</u><u>L</u>T<u>V</u>D<u>K</u>STSTVYMELSSLRSEDTAVYYCAR<u>PPRAY GNYAGY</u>WGQGTTVTVSS

SEQ ID NO: 57

Note: the single underlined parts are CDRs, the dotted underlined parts are the amino acid substitution sites, and the rest are FRs.

The heavy chain variable region and the light chain variable region of the anti-GUCY2C humanized antibody were recombined with the heavy chain constant region hIgG1:CH1-Fc and the light chain constant region CL, respectively, to obtain the full-length humanized antibody.

> hIgG1: CH1-Fc:

ASTKGPSVFPLAPSSKSTSGGTAALGCLVKDYFPEPVTVSWNSGALTSGVHTF
PAVLQSSGLYSLSSVVTVPSSSLGTQTYICNVNHKPSNTKVDKKVEPKSCDKT
HTCPPCPAPELLGGPSVFLFPPKPKDTLMISRTPEVTCVVVDVSHEDPEVKFNW
YVDGVEVHNAKTKPREEQYNSTYRVVSVLTVLHQDWLNGKEYKCKVSNKA
LPAPIEKTISKAKGQPREPQVYTLPPSRDELTKNQVSLTCLVKGFYPSDIAVEWE
SNGQPENNYKTTPPVLDSDGSFFLYSKLTVDKSRWQQGNVFSCSVMHEALHN
HYTQKSLSLSPGK

SEQ ID NO: 58

> CL:

RTVAAPSVFIFPPSDEQLKSGTASVVCLLNNFYPREAKVQWKVDNALQSGNSQ
ESVTEQDSKDSTYSLSSTLTLSKADYEKHKVYACEVTHQGLSSPVTKSFNRGE
C

SEQ ID NO: 59

Anti-GUCY2C humanized antibodies obtained by the present disclosure were as follows:

Table 22-1. Humanized antibodies of the series 81

| Variable region | h81 L1 | h81 L2 | h81 L3 | h81 L4 | h81 L5 | h81 L6 |
|---|---|---|---|---|---|---|
| h81 H1 | 81 L1H1 | 81 L2H1 | 81 L3H1 | 81 L4H1 | 81 L5H1 | 81 L6H1 |
| h81 H2 | 81 L1H2 | 81 L2H2 | 81 L3H2 | 81 L4H2 | 81 L5H2 | 81 L6H2 |

Table 22-2. Humanized antibodies of the series 146

| Variable region | h146 L1 |
|---|---|
| h146 H1 | 146 L1H1 |

Table 22-3. Humanized antibodies of the series P3-C7

| Variable region | hP3-C7 L1 | hP3-C7 L2 | hP3-C7 L3 | hP3-C7 L4 | hP3-C7 L5 | hP3-C7 L6 | hP3-C7 L7 | hP3-C7 L8 |
|---|---|---|---|---|---|---|---|---|
| hP3-C7 H1 | P3-C7 L1H1 | P3-C7 L2H1 | P3-C7 L3H1 | P3-C7 L4H1 | P3-C7 L5H1 | P3-C7 L6H1 | P3-C7 L7H1 | P3-C7 L8H1 |

(continued)

| Variable region | hP3-C7 L1 | hP3-C7 L2 | hP3-C7 L3 | hP3-C7 L4 | hP3-C7 L5 | hP3-C7 L6 | hP3-C7 L7 | hP3-C7 L8 |
|---|---|---|---|---|---|---|---|---|
| hP3-C7 H2 | P3-C7 L1H2 | P3-C7 L2H2 | P3-C7 L3H2 | P3-C7 L4H2 | P3-C7 L5H2 | P3-C7 L6H2 | P3-C7 L7H2 | P3-C7 L8H2 |

Illustratively, the full-length sequences of the anti-GUCY2C humanized antibodies are as follows:

81 L3H1 heavy chain:

EVQLVQSGAEVKKPGASVKVSCKASGYTFTDHNMNWVKQAPGQRLEWMGVMNPKFGTINYNQKFKDKATITRDTSASTAYMELSSLRSEDTAVYYCARDNYPNYFDYWGQGTTVTVSS*ASTKGPSVFPLAPSSKSTSGGTAALGCLVKDYFPEPVTVSWNSGALTSGVHTFPAVLQSSGLYSLSSVVTVPSSSLGTQTYICNVNHKPSNTKVDKKVEPKSCDKTHTCPPCPAPELLGGPSVFLFPPKPKDTLMISRTPEVTCVVVDVSHEDPEVKFNWYVDGVEVHNAKTKPREEQYNSTYRVVSVLTVLHQDWLNGKEYKCKVSNKALPAPIEKTISKAKGQPREPQVYTLPPSRDELTKNQVSLTCLVKGFYPSDIAVEWESNGQPENNYKTTPPVLDSDGSFFLYSKLTVDKSRWQQGNVFSCSVMHEALHNHYTQKSLSLSPGK*

SEQ ID NO: 60

81 L3H1 light chain:

DIQLTQSPSFLSASPGDRVTITCSASSSVNYMYWYQQKPGSSPKSWIYRTSNLASGVPSRFSGSGSGTEYTLTISSLQPEDFATYYCQQYHDYPPTFGGGTKVEIK*RTVAAPSVFIFPPSDEQLKSGTASVVCLLNNFYPREAKVQWKVDNALQSGNSQESVTEQDSKDSTYSLSSTLTLSKADYEKHKVYACEVTHQGLSSPVTKSFNRGEC*

SEQ ID NO: 61

146 L1H1 heavy chain:

EFQLVQSGAEVKKPGASVKVSCKASGYSFTDYNMNWVRQAPGQRLEWMGLINPKYGTTNYNQKFKGRVTLTVDQSSSTAYMELSSLRSEDTAVYYCARDNYPAWFAYWGQGTTVTVSS*ASTKGPSVFPLAPSSKSTSGGTAALGCLVKDYFPEPVTVSWNSGALTSGVHTFPAVLQSSGLYSLSSVVTVPSSSLGTQTYICNVNHKPSNTKVDKKVEPKSCDKTHTCPPCPAPELLGGPSVFLFPPKPKDTLMISRTPEVTCVVVDVSHEDPEVKFNWYVDGVEVHNAKTKPREEQYNSTYRVVSVLTVLHQDWLNGKEYKCKVSNKALPAPIEKTISKAKGQPREPQVYTLPPSRDELTKNQVSLTCLVKGFYPSDIAVEWESNGQPENNYKTTPPVLDSDGSFFLYSKLTVDKSRWQQGNVFSCSVMHEALHNHYTQKSLSLSPGK*

SEQ ID NO: 62

146 L1H1 light chain:

EIVLTQSPATLSLSPGERATLSC<u>SASSSLNYMY</u>WYQQKPG<u>SS</u>PR<u>PW</u>IYR<u>TSQ</u>LA<u>SGV</u>PARFSGSGSGTD<u>Y</u>TLTISSLEPEDFAVYYC<u>QQYHSYPPT</u>FGGGTKVEIK*RTVAAPSVFIFPPSDEQLKSGTASVVCLLNNFYPREAKVQWKVDNALQSGNSQESVTEQDSKDSTYSLSSTLTLSKADYEKHKVYACEVTHQGLSSPVTKSFNRGEC*

SEQ ID NO: 63

P3-C7 L7H1 heavy chain:

EVQLVQSGAEVKKPGASVKVSCKASGYTFT<u>DHYIH</u>WVRQAPGQGLEWMG<u>VINPYNDGATYNQKFKG</u>RVTMT<u>V</u>D<u>K</u>STSTVYMELSSLRSEDTAVYYCAR<u>PPRAYGNYAGY</u>WGQGTTVTVSS*ASTKGPSVFPLAPSSKSTSGGTAALGCLVKDYFPEPVTVSWNSGALTSGVHTFPAVLQSSGLYSLSSVVTVPSSSLGTQTYICNVNHKPSNTKVDKKVEPKSCDKTHTCPPCPAPELLGGPSVFLFPPKPKDTLMISRTPEVTCVVVDVSHEDPEVKFNWYVDGVEVHNAKTKPREEQYNSTYRVVSVLTVLHQDWLNGKEYKCKVSNKALPAPIEKTISKAKGQPREPQVYTLPPSRDELTKNQVSLTCLVKGFYPSDIAVEWESNGQPENNYKTTPPVLDSDGSFFLYSKLTVDKSRWQQGNVFSCSVMHEALHNHYTQKSLSLSPGK*

SEQ ID NO: 64

P3-C7 L7H1 light chain:

D<u>V</u>VMTQTPLSLPVTPGEPASISC<u>RSSQSLVHSNQNTYLH</u>WYLQKPGQSPQLLIY<u>KVSNRFS</u>GVPDRFSGSGS<u>R</u>TDFTLKISRVEAEDVGVYYC<u>SQSTHVPYT</u>FGGGTKVEIK*RTVAAPSVFIFPPSDEQLKSGTASVVCLLNNFYPREAKVQWKVDNALQSGNSQESVTEQDSKDSTYSLSSTLTLSKADYEKHKVYACEVTHQGLSSPVTKSFNRGEC*

SEQ ID NO: 65

P3-C7 L8H1 heavy chain (same as P3-C7 L7H1 heavy chain, SEQ ID NO: 64)
P3-C7 L8H1 light chain:

D<u>VV</u>MTQSPSSLSASVGDRVTITC<u>RSSQSLVHSNQNTYLH</u>WYQQKPGKAPKLLIY<u>KVSNRFS</u>GVPSRFSGSGS<u>R</u>TDFTL<u>K</u>ISSLQPEDFATYYC<u>SQSTHVPYT</u>FGGGTKVEIK*RTVAAPSVFIFPPSDEQLKSGTASVVCLLNNFYPREAKVQWKVDNALQSGNSQESVTEQDSKDSTYSLSSTLTLSKADYEKHKVYACEVTHQGLSSPVTKSFNRGEC*

SEQ ID NO: 66

Note: the single underlined parts are CDRs, the dotted underlined bold parts are the amino acid substitution sites, and the italicized parts are constant regions.

The control antibodies of the anti-GUCY2C antibodies used in the present disclosure are as follows:
1608 (variable region sequences were constructed with reference to GUCY2c end of GUCY2c-1608 bispecific antibody in patent No. WO2019224716A2, and constant region sequences are set forth in SEQ ID NO: 58 and SEQ ID NO: 59), the full-length sequence of which is as follows:

> 1608 heavy chain:

EVQLVESGGGLVQPGGSLRLSCAASGFTFS*SYWMH*WVRQAPGKGLEWIG*EIK PSNELTNVHEKFKD*RFTISVDKAKNSAYLQMNSLRAEDTAVYYCTR*TITTTEG YWFFD*VWGQGTLVTVSS*ASTKGPSVFPLAPSSKSTSGGTAALGCLVKDYFPEPVT VSWNSGALTSGVHTFPAVLQSSGLYSLSSVVTVPSSSLGTQTYICNVNHKPSNTKVDK KVEPKSCDKTHTCPPCPAPELLGGPSVFLFPPKPKDTLMISRTPEVTCVVVDVSHE DPEVKFNWYVDGVEVHNAKTKPREEQYNSTYRVVSVLTVLHQDWLNGKEYKCKV SNKALPAPIEKTISKAKGQPREPQVYTLPPSRDELTKNQVSLTCLVKGFYPSDIAVE WESNGQPENNYKTTPPVLDSDGSFFLYSKLTVDKSRWQQGNVFSCSVMHEALHN HYTQKSLSLSPGK*

SEQ ID NO: 67

> 1608 light chain:

DIQLTQSPSSLSASVGDRVTITC*RASESVDYYGSSLLQ*WYQQKPGKAPKLLIY*A ASKLAS*GVPSRFSGSGSGTDFTLTISSLQPEDFATYYC*QQTRKAYT*FGQGTKLE IK*RTVAAPSVFIFPPSDEQLKSGTASVVCLLNNFYPREAKVQWKVDNALQSGNSQES VTEQDSKDSTYSLSSTLTLSKADYEKHKVYACEVTHQGLSSPVTKSFNRGEC*

SEQ ID NO: 68

Antibody 5F9 (variable region sequences were prepared with reference to patent No. WO2014134311A1, and constant region sequences are set forth in SEQ ID NO: 58 and SEQ ID NO: 59), the specific sequences of which are as follows:

> 5F9 heavy chain:

QVQLQQWGAGLLKPSETLSLTCAVFGGSFS*GYYWS*WIRQPPGKGLEWIG*EIN HRGNTNDNPSLKS*RVTISVDTSKNQFALKLSSVTAADTAVYYCAR*ERGYTYG NFDH*WGQGTLVTVSS*ASTKGPSVFPLAPSSKSTSGGTAALGCLVKDYFPEPVTVS WNSGALTSGVHTFPAVLQSSGLYSLSSVVTVPSSSLGTQTYICNVNHKPSNTKVDKK VEPKSCDKTHTCPPCPAPELLGGPSVFLFPPKPKDTLMISRTPEVTCVVVDVSHED PEVKFNWYVDGVEVHNAKTKPREEQYNSTYRVVSVLTVLHQDWLNGKEYKCKVS NKALPAPIEKTISKAKGQPREPQVYTLPPSRDELTKNQVSLTCLVKGFYPSDIAVEW ESNGQPENNYKTTPPVLDSDGSFFLYSKLTVDKSRWQQGNVFSCSVMHEALHNHY TQKSLSLSPGK*

SEQ ID NO: 69

> 5F9 light chain:

EIVMTQSPATLSVSPGERATLSC<u>RASQSVSRNLA</u>WYQQKPGQAPRLLIY<u>GAST</u>
<u>RAT</u>GIPARFSGSGSGTEFTLTIGSLQSEDFAVYYC<u>QQYKTWPRT</u>FGQGTNVEIK
*RTVAAPSVFIFPPSDEQLKSGTASVVCLLNNFYPREAKVQWKVDNALQSGNSQESV*
*TEQDSKDSTYSLSSTLTLSKADYEKHKVYACEVTHQGLSSPVTKSFNRGEC*

SEQ ID NO: 70

**Example 4. Preparation of Anti-GUCY2C-CD3 Bispecific Antibodies**

The GUCY2C arm of the GUCY2C-CD3 bispecific antibody of the present disclosure is the anti-GUCY2C antibody in the present disclosure, and the CD3 arm may be derived from any suitable antibody. Specifically, the CD3 arm used in the present disclosure is S107E or N103QN106Q.

Table 23. CDRs of S107E

| Antibody | Heavy chain | | Light chain | |
|---|---|---|---|---|
| S107E | HCDR1 | KYAMN (SEQ ID NO: 71) | LCDR1 | GSSTGAVTSGNYPN (SEQ ID NO: 74) |
| | HCDR2 | RIRSKYNNYATYYADSVKD (SEQ ID NO: 72) | LCDR2 | GTKFLAP (SEQ ID NO: 75) |
| | HCDR3 | HGNFGNEYISYWAY (SEQ ID NO: 73) | LCDR3 | VLWYSNRWV (SEQ ID NO: 76) |

The variable region sequences of S107E are as follows:

> S107E-VH:

EVQLVESGGGLVQPGGSLKLSCAASGFTFN<u>KYAMN</u>WVRQAPGKGLEWVA<u>RI</u>
<u>RSKYNNYATYYADSVKD</u>RFTISRDDSKNTAYLQMNNLKTEDTAVYYCVR<u>HGN</u>
<u>FGNEYISYWAY</u>WGQGTLVTVSS

SEQ ID NO: 77

> S107E-VL:

QTVVTQEPSLTVSPGGTVTLTC<u>GSSTGAVTSGNYPN</u>WVQQKPGQAPRGLIG<u>GT</u>
<u>KFLAP</u>GTPARFSGSLLGGKAALTLSGVQPEDEAEYYC<u>VLWYSNRWV</u>FGGGTK
LTVL

SEQ ID NO: 78

Table 24. CDRs of N103QN106Q

| Antibody | Heavy chain | | Light chain | |
|---|---|---|---|---|
| N103Q | HCDR1 | KYAMN (SEQ ID NO: 71) | LCDR1 | GSSTGAVTSGNYPN (SEQ ID NO: 74) |
| N106Q | HCDR2 | RIRSKYNNYATYYADSVKD (SEQ ID NO: 72) | LCDR2 | GTKFLAP (SEQ ID NO: 75) |
| | HCDR3 | HGQFGQSYISYWAY (SEQ ID NO: 88) | LCDR3 | VLWYSNRWV (SEQ ID NO: 76) |

The variable region sequences of N103QN106Q are as follows:

> N103QN106Q-VH:

EVQLVESGGGLVQPGGSLKLSCAASGFTFNKYAMNWVRQAPGKGLEWVARI
RSKYNNYATYYADSVKDRFTISRDDSKNTAYLQMNNLKTEDTAVYYCVRHGQ
FGQSYISYWAYWGQGTLVTVSS

SEQ ID NO: 89

> N103QN106Q-VL (same as S107E-VL, SEQ ID NO: 78)

The GUCY2C-CD3 bispecific antibody in the present disclosure has the following molecular structures of Format 1 to Format 5:
Format 1 is an asymmetrically structured molecule; the complete molecule comprises two chains, wherein the two chains are different and are specifically as follows:

Chain 1: [GUCY2C-VL]-[linker 1]-[CD3-VH]-[linker 2]-[IgG1Fc(Knob-DI)]
Chain 2: [CD3-VL]-[linker 3]-[GUCY2C-VH]-[linker 2]-[IgG1Fc(Hole-DI)];

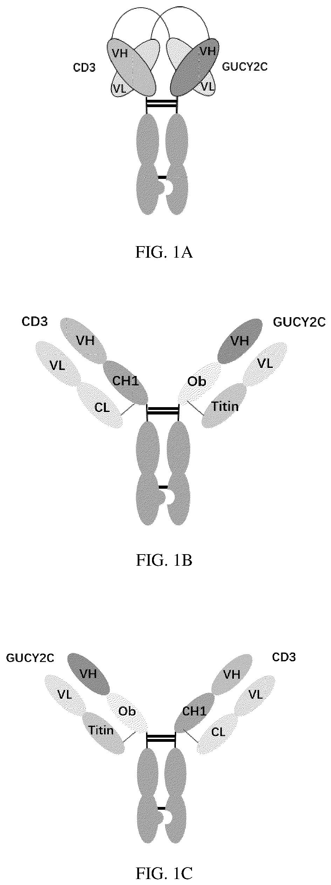

its schematic diagram is shown in FIG. 1A.
The related sequences are as follows:

> IgG1Fc (Knob-DI, L234A/L235A/G237A/Y349C/T366W):

CPPCPAPEAAGAPSVFLFPPKPKDTLMISRTPEVTCVVVDVSHEDPEVKFNWY
VDGVEVHNAKTKPREEQYNSTYRVVSVLTVLHQDWLNGKEYKCKVSNKAL
PAPIEKTISKAKGQPREPQVCTLPPSREEMTKNQVSLWCLVKGFYPSDIAVEW
ESNGQPENNYKTTPPVLDSDGSFFLYSKLTVDKSRWQQGNVFSCSVMHEALH
NHYTQKSLSLSPG

SEQ ID NO: 79

> IgG1Fc (Hole-DI, L234A/L235A/G237A/S354C/T366S/L368A/Y407V):

CPPCPAPEAAGAPSVFLFPPKPKDTLMISRTPEVTCVVVDVSHEDPEVKFNWY
VDGVEVHNAKTKPREEQYNSTYRVVSVLTVLHQDWLNGKEYKCKVSNKAL
PAPIEKTISKAKGQPREPQVYTLPPCREEMTKNQVSLSCAVKGFYPSDIAVEWE
SNGQPENNYKTTPPVLDSDGSFFLVSKLTVDKSRWQQGNVFSCSVMHEALHN
HYTQKSLSLSPG

SEQ ID NO: 80

> Linker 1:
GGGSGGGG
SEQ ID NO: 81
> Linker 2:
G
SEQ ID NO: 82
> Linker 3:
GGGGSGGGG

SEQ ID NO: 83

Format 2 is an asymmetrically structured molecule; the complete molecule comprises four chains, wherein the four chains are different and are specifically as follows:

Chain 1: [CD3-VH]-[IgG1(CH1)]-[IgG1Fc(Knob)];
Chain 2: [CD3-VL]-[CL(Lambda)];
Chain 3: [GUCY2C-VH]-[linker 4]-[Obscurin chain]-[IgG1Fc(Hole)];
Chain 4: [GUCY2C-VL]-[linker 4]-[Titin chain];

its schematic diagram is shown in FIG. 1B (where Ob stands for Obscurin).

The related sequences are as follows:

> IgG1(CH1):

ASTKGPSVFPLAPSSKSTSGGTAALGCLVKDYFPEPVTVSWNSGALTSGVHTF
PAVLQSSGLYSLSSVVTVPSSSLGTQTYICNVNHKPSNTKVDKKVEPKSC

SEQ ID NO: 84

> IgG1Fc (Knob, L234A/L235A/S354C/T366W):

DKTHTCPPCPAPEAAGGPSVFLFPPKPKDTLMISRTPEVTCVVVDVSHEDPEV
KFNWYVDGVEVHNAKTKPREEQYNSTYRVVSVLTVLHQDWLNGKEYKCKV
SNKALPAPIEKTISKAKGQPREPQVYTLPPCREEMTKNQVSLWCLVKGFYPSD
IAVEWESNGQPENNYKTTPPVLDSDGSFFLYSKLTVDKSRWQQGNVFSCSVM
HEALHNHYTQKSLSLSPGK

SEQ ID NO: 85

> IgG1Fc (Hole, L234A/L235A/Y349C/T366S/L368A/Y407V):

DKTHTCPPCPAPEAAGGPSVFLFPPKPKDTLMISRTPEVTCVVVDVSHEDPEV
KFNWYVDGVEVHNAKTKPREEQYNSTYRVVSVLTVLHQDWLNGKEYKCKV
SNKALPAPIEKTISKAKGQPREPQVCTLPPSREEMTKNQVSLSCAVKGFYPSDI
AVEWESNGQPENNYKTTPPVLDSDGSFFLVSKLTVDKSRWQQGNVFSCSVMH
EALHNHYTQKSLSLSPGK

SEQ ID NO: 86

> CL(Lambda):

GQPKANPTVTLFPPSSEELQANKATLVCLISDFYPGAVTVAWKADGSPVKAGV
ETTKPSKQSNNKYAASSYLSLTPEQWKSHRSYSCQVTHEGSTVEKTVAPTEC

SEQ ID NO: 87

> Titin(T.16):

GIPPKIECLPIDISIDEGKVLTVASAFTGEPTPEVTWSTGGRKIHSQEQGRFHIEN
TDDSTTLTIKDVQKQDGGLYTLTLRNEFGSDSATVNIHIRSI

SEQ ID NO: 198

> Obscurin(O.28):

SGAPRFLTRPKASVVSVGKDATLSCQIVGNPFPQVSWEKDKQPVTAGVRFRLA
QDGDLYRLKILDLQLSDSGQYVCRARNAHGEAFACLGLQVDAEA

SEQ ID NO: 236

> Linker 4:
GGGGS
SEQ ID NO: 90

Format 3 is an asymmetrically structured molecule; the complete molecule comprises four chains, wherein the four chains are different and are specifically as follows:

Chain 1: [GUCY2C-VH]-[linker 4]-[Obscurin chain]-[IgG1Fc(Knob)];
Chain 2: [GUCY2C-VL]-[linker 4]-[Titin chain];
Chain 3: [CD3-VH]-[IgG1(CH1)]-[IgG1Fc(Hole)];
Chain 4: [CD3-VL]-[CL(Lambda)];

its schematic diagram is shown in FIG. 1C (where Ob stands for Obscurin).
The related sequences are as follows:

> IgG1(CH1) (SEQ ID NO: 84)
> IgG1Fc(Knob) (SEQ ID NO: 85)
> IgG1Fc(Hole) (SEQ ID NO: 86)
> CL(Lambda) (SEQ ID NO: 87)
> Titin(T.16) (SEQ ID NO: 198)
> Obscurin(O.28) (SEQ ID NO: 236)
> Linker 4 (SEQ ID NO: 90)
> chain 3 (Format 3-3, CD3-VH-IgG1(CH1)-IgG1Fc(Hole)):

EVQLVESGGGLVQPGGSLKLSCAASGFTFNKYAMNWVRQAPGKGLEWVARI
RSKYNNYATYYADSVKDRFTISRDDSKNTAYLQMNNLKTEDTAVYYCVRHGN
FGNEYISYWAYWGQGTLVTVSSASTKGPSVFPLAPSSKSTSGGTAALGCLVKD
YFPEPVTVSWNSGALTSGVHTFPAVLQSSGLYSLSSVVTVPSSSLGTQTYICNV
NHKPSNTKVDKKVEPKSCDKTHTCPPCPAPEAAGGPSVFLFPPKPKDTLMISR
TPEVTCVVVDVSHEDPEVKFNWYVDGVEVHNAKTKPREEQYNSTYRVVSVL

TVLHQDWLNGKEYKCKVSNKALPAPIEKTISKAKGQPREPQVCTLPPSREEMT
KNQVSLSCAVKGFYPSDIAVEWESNGQPENNYKTTPPVLDSDGSFFLVSKLTV
DKSRWQQGNVFSCSVMHEALHNHYTQKSLSLSPGK

SEQ ID NO: 93

> chain 4 (Format 3-4, same as Format 2-2, SEQ ID NO: 92)

Format 4 is a symmetrically structured molecule; the complete molecule comprises four chains, wherein the four chains consist of two identical chains 1 and two identical chains 2 and are specifically as follows:

Chain 1: [GUCY2C-VH]-[IgG1(CH1)]-[CD3-VH]-[linker 5]-[CD3-VL]-[linker 6]-[IgG1Fc];
Chain 2: [GUCY2C-VL]-[CL];

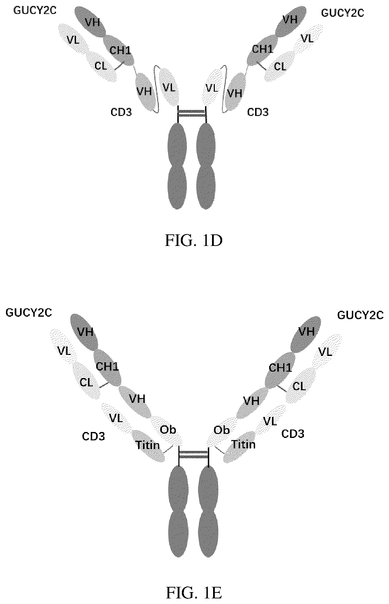

its schematic diagram is shown in FIG. 1D.

The related sequences are as follows:

> IgG1(CH1) (SEQ ID NO: 84)
> CL (SEQ ID NO: 59)
> IgG1Fc (L234A/L235A):

DKTHTCPPCPAPE**AA**GGPSVFLFPPKPKDTLMISRTPEVTCVVVDVSHEDPEV

KFNWYVDGVEVHNAKTKPREEQYNSTYRVVSVLTVLHQDWLNGKEYKCKV

SNKALPAPIEKTISKAKGQPREPQVYTLPPSREEMTKNQVSLTCLVKGFYPSDI

AVEWESNGQPENNYKTTPPVLDSDGSFFLYSKLTVDKSRWQQGNVFSCSVMH

EALHNHYTQKSLSLSPGK

SEQ ID NO: 94

> Linker 5:
GGGGSGGGGSGGGGS
SEQ ID NO: 95
> Linker 6
GGG
SEQ ID NO: 96

Format 5 is a symmetrically structured molecule; the complete molecule comprises six chains, wherein the six chains consist of two identical chains 1, two identical chains 2, and two identical chains 3, and are specifically as follows:

Chain 1: [GUCY2C-VH]-[IgG1(CH1)]-[linker 7]-[CD3-VH]-[linker 4]-[Obscurin chain]-[IgG1Fc];
Chain 2: [GUCY2C-VL]-[CL];
Chain 3: [CD3-VL]-[linker 4]-[Titin chain];

its schematic diagram is shown in FIG. 1E (where Ob stands for Obscurin).

The related sequences are as follows:

> IgG1(CH1) (SEQ ID NO: 84)
> IgG1Fc (SEQ ID NO: 86)
> CL (SEQ ID NO: 59)
> Titin(T.16) (SEQ ID NO: 198)
> Obscurin(O.28) (SEQ ID NO: 236)
> Linker 4 (SEQ ID NO: 90)
> Linker 7:
GGGGSGGGGS
SEQ ID NO: 97
> Chain 3 (Format 5-3, CD3-VL-Linker 4-Titin)

QTVVTQEPSLTVSPGGTVTLTCGSSTGAVTSGNYPNWVQQKPGQAPRGLIGGT
KFLAPGTPARFSGSLLGGKAALTLSGVQPEDEAEYYCVLWYSNRWVFGGGTK
LTVLGGGGSGIPPKIECLPIDISIDEGKVLTVASAFTGEPTPEVTWSTGGRKIHSQ
EQGRFHIENTDD**S**TTL**T**IKDVQKQDGGLYTLTLRNEFGSDSATVNIHIRSI

SEQ ID NO: 98

Antigen-binding molecules were constructed according to Table 25, and the variable region of S107E or N103QN106Q was used as the CD3 arm of all bispecific antibody molecules. For the numbering of the anti-GUCY2C antibodies, the variable region is shown in the name of the antibody, the number followed indicates the Format used, the note (N103QN106Q) behind the Format indicates that the CD3 arm used by the bispecific antibody is N103QN106Q, and the absence of note behind the Format indicates that the CD3 arm used by the bispecific antibody is S107E. For example, P3-C7 L7H1-1 represents that the molecule uses the variable region of antibody P3-C7 L7H1 as the GUCY2C binding domain, Format 1 as the molecular structure, and S107E as the CD3 arm. This applies similarly to other bispecific antibodies.

Table 25. GUCY2C-CD3 bispecific antibodies of the present disclosure

| No. | Chain 1 | Chain 2 | Chain 3 | Chain 4 |
|---|---|---|---|---|
| P3-C7 L7H1-1 | P3-C7 L7H1-1-1 | P3-C7 L7H1-1-2 | | |
| P3-C7 L8H1-1 | P3-C7 L8H1-1-1 | P3-C7 L8H1-1-2 | | |
| P3-C7 L7H1-2 | Format 2-1 | Format 2-2 | P3-C7 L7H1-2-3 | P3-C7 L7H1-2-4 |
| P3-C7 L7H1-3 | P3-C7 L7H1-3-1 | P3-C7 L7H1-3-2 | Format 3-3 | Format 3-4 |
| P3-C7 L7H1-4 | P3-C7 L7H1-4-1 | P3-C7 L7H1-4-2 | | |
| P3-C7 L8H1-4 | P3-C7 L8H1-4-1 | P3-C7 L8H1-4-2 | | |
| P3-C7 L7H1-5 | P3-C7 L7H1-5-1 | P3-C7 L7H1-5-2 | P3-C7 L7H1-5-3 | |
| P3-C7 L8H1-5 | P3-C7 L8H1-5-1 | P3-C7 L8H1-5-2 | P3-C7 L8H1-5-3 | |
| M81-1 | M81-1-1 | M81-1-2 | | |
| M81-2 | Format 2-1 | Format 2-2 | M81-2-3 | M81-2-4 |
| 81 L1H1-2 | Format 2-1 | Format 2-2 | 81 L1H1-2-3 | 81 L1H1-2-4 |
| 81 L2H1-2 | Format 2-1 | Format 2-2 | 81 L2H1-2-3 | 81 L2H1-2-4 |
| 81 L3H1-2 | Format 2-1 | Format 2-2 | 81 L3H1-2-3 | 81 L3H1-2-4 |
| 81 L3H1-2 (N103QN106Q) | Format 2-1 (N103QN106Q) | Format 2-2 | 81 L3H1-2-3 | 81 L3H1-2-4 |
| 81 L4H1-2 | Format 2-1 | Format 2-2 | 81 L4H1-2-3 | 81 L4H1-2-4 |
| 81 L5H1-2 | Format 2-1 | Format 2-2 | 81 L5H1-2-3 | 81 L5H1-2-4 |
| 81 L6H1-2 | Format 2-1 | Format 2-2 | 81 L6H1-2-3 | 81 L6H1-2-4 |
| 81 L1H1-3 | 81 L1H1-3-1 | 81 L1H1-3-2 | Format 3-3 | Format 3-4 |
| 81 L2H1-3 | 81 L2H1-3-1 | 81 L2H1-3-2 | Format 3-3 | Format 3-4 |
| 81 L3H1-3 | 81 L3H1-3-1 | 81 L3H1-3-2 | Format 3-3 | Format 3-4 |
| 81 L4H1-3 | 81 L4H1-3-1 | 81 L4H1-3-2 | Format 3-3 | Format 3-4 |
| 81 L5H1-3 | 81 L5H1-3-1 | 81 L5H1-3-2 | Format 3-3 | Format 3-4 |
| 81 L6H1-3 | 81 L6H1-3-1 | 81 L6H1-3-2 | Format 3-3 | Format 3-4 |
| M146-1 | M146-1-1 | M146-1-2 | | |
| M146-2 | Format 2-1 | Format 2-2 | M146-2-3 | M146-2-4 |
| 146 L1H1-2 | Format 2-1 | Format 2-2 | 146 L1H1-2-3 | 146 L1H1-2-4 |

(continued)

| No. | Chain 1 | Chain 2 | Chain 3 | Chain 4 |
|---|---|---|---|---|
| 146 L1H1-3 | 146 L1H1-3-1 | 146 L1H1-3-2 | Format 3-3 | Format 3-4 |

Illustratively, the specific amino acid sequences of bispecific antibodies constructed with Format 1 in the present disclosure are as follows:

**1. Full-length sequence of P3-C7 L7H1-1**

> Chain 1 (P3-C7 L7H1-1-1):

DVVMTQTPLSLPVTPGEPASISCRSSQSLVHSNQNTYLHWYLQKPGQSPQLLI
YKVSNRFSGVPDRFSGSGSRTDFTLKISRVEAEDVGVYYCSQSTHVPYTFGGG
TKVEIKGGGSGGGGEVQLVESGGGLVQPGGSLKLSCAASGFTFNKYAMNWV
RQAPGKGLEWVARIRSKYNNYATYYADSVKDRFTISRDDSKNTAYLQMNNLK
TEDTAVYYCVRHGNFGNEYISYWAYWGQGTLVTVSSGCPPCPAPEAAGAPSV
FLFPPKPKDTLMISRTPEVTCVVVDVSHEDPEVKFNWYVDGVEVHNAKTKPR
EEQYNSTYRVVSVLTVLHQDWLNGKEYKCKVSNKALPAPIEKTISKAKGQPR

EPQVCTLPPSREEMTKNQVSLWCLVKGFYPSDIAVEWESNGQPENNYKTTPPV
LDSDGSFFLYSKLTVDKSRWQQGNVFSCSVMHEALHNHYTQKSLSLSPG

SEQ ID NO: 99

> Chain 2 (P3-C7 L7H1-1-2):

QTVVTQEPSLTVSPGGTVTLTCGSSTGAVTSGNYPNWVQQKPGQAPRGLIGGT
KFLAPGTPARFSGSLLGGKAALTLSGVQPEDEAEYYCVLWYSNRWVFGGGTK
LTVLGGGGSGGGGEVQLVQSGAEVKKPGASVKVSCKASGYTFTDHYIHWVR
QAPGQGLEWMGVINPYNDGATYNQKFKGRVTMTVDKSTSTVYMELSSLRSE
DTAVYYCARPPRAYGNYAGYWGQGTTVTVSSGCPPCPAPEAAGAPSVFLFPP
KPKDTLMISRTPEVTCVVVDVSHEDPEVKFNWYVDGVEVHNAKTKPREEQY
NSTYRVVSVLTVLHQDWLNGKEYKCKVSNKALPAPIEKTISKAKGQPREPQV
YTLPPCREEMTKNQVSLSCAVKGFYPSDIAVEWESNGQPENNYKTTPPVLDSD
GSFFLVSKLTVDKSRWQQGNVFSCSVMHEALHNHYTQKSLSLSPG

SEQ ID NO: 100

**2. Full-length sequence of P3-C7 L8H1-1**

> Chain 1 (P3-C7 L8H1-1-1):

DVVMTQSPSSLSASVGDRVTITCRSSQSLVHSNQNTYLHWYQQKPGKAPKLLI
YKVSNRFSGVPSRFSGSGSRTDFTLKISSLQPEDFATYYCSQSTHVPYTFGGGT
KVEIKGGGSGGGGEVQLVESGGGLVQPGGSLKLSCAASGFTFNKYAMNWVR
QAPGKGLEWVARIRSKYNNYATYYADSVKDRFTISRDDSKNTAYLQMNNLKT
EDTAVYYCVRHGNFGNEYISYWAYWGQGTLVTVSSGCPPCPAPEAAGAPSVF
LFPPKPKDTLMISRTPEVTCVVVDVSHEDPEVKFNWYVDGVEVHNAKTKPRE
EQYNSTYRVVSVLTVLHQDWLNGKEYKCKVSNKALPAPIEKTISKAKGQPRE
PQVCTLPPSREEMTKNQVSLWCLVKGFYPSDIAVEWESNGQPENNYKTTPPVL
DSDGSFFLYSKLTVDKSRWQQGNVFSCSVMHEALHNHYTQKSLSLSPG

SEQ ID NO: 101

> Chain 2 (P3-C7 L8H1-1-2, same as P3-C7 L7H1-1-2, SEQ ID NO: 100)

**3. Full-length sequence of M81-1**

> Chain 1 (M81-1-1):

<u>QIVLTQSPAIMSASPGEKVTISCSASSSVNYMYWYQQKPGSSPKSWIYRTSNLA
SGVSARFSGSGSGTSYSLTISSMEAEDAATYYCQQYHDYPPTFGGGTKLEIKG</u>
GGSGGGGEVQLVESGGGLVQPGGSLKLSCAASGFTFNKYAMNWVRQAPGKG
LEWVARIRSKYNNYATYYADSVKDRFTISRDDSKNTAYLQMNNLKTEDTAVY
YCVRHGNFGNEYISYWAYWGQGTLVTVSSGCPPCPAPEAAGAPSVFLFPPKPK
DTLMISRTPEVTCVVVDVSHEDPEVKFNWYVDGVEVHNAKTKPREEQYNST

YRVVSVLTVLHQDWLNGKEYKCKVSNKALPAPIEKTISKAKGQPREPQVCTL
PPSREEMTKNQVSLWCLVKGFYPSDIAVEWESNGQPENNYKTTPPVLDSDGSF
FLYSKLTVDKSRWQQGNVFSCSVMHEALHNHYTQKSLSLSPG

SEQ ID NO: 102

> Chain 2 (M81-1-2):

QTVVTQEPSLTVSPGGTVTLTCGSSTGAVTSGNYPNWVQQKPGQAPRGLIGGT
KFLAPGTPARFSGSLLGGKAALTLSGVQPEDEAEYYCVLWYSNRWVFGGGTK
LTVLGGGGSGGGGEFQLQQSGPELVKPGASVKISCKASGYSFTDHNMNWVK
QSNGKSLEWIGVMNPKFGTINYNQKFKDKATLTVDQSSSTAYMQLNSLTSEDS
AVYYCVRDDYPNYFDYWGQGTTLTVSSGCPPCPAPEAAGAPSVFLFPPKPKD
TLMISRTPEVTCVVVDVSHEDPEVKFNWYVDGVEVHNAKTKPREEQYNSTY
RVVSVLTVLHQDWLNGKEYKCKVSNKALPAPIEKTISKAKGQPREPQVYTLPP
CREEMTKNQVSLSCAVKGFYPSDIAVEWESNGQPENNYKTTPPVLDSDGSFFL
VSKLTVDKSRWQQGNVFSCSVMHEALHNHYTQKSLSLSPG

SEQ ID NO: 103

**4. Full-Length sequence of M146-1**

> Chain 1 (M146-1-1):

QIVLTQSPAIMSASPGEKVTISCSASSSLNYMYWYQQKPGSSPKSWIYRTSNLA
SGVPARFSGSGSGTSYSLTISSMEAEDAATYYCQQYHSYPPTFGAGTKLELKG
GGSGGGGEVQLVESGGGLVQPGGSLKLSCAASGFTFNKYAMNWVRQAPGKG
LEWVARIRSKYNNYATYYADSVKDRFTISRDDSKNTAYLQMNNLKTEDTAVY
YCVRHGNFGNEYISYWAYWGQGTLVTVSSGCPPCPAPEAAGAPSVFLFPPKPK
DTLMISRTPEVTCVVVDVSHEDPEVKFNWYVDGVEVHNAKTKPREEQYNST
YRVVSVLTVLHQDWLNGKEYKCKVSNKALPAPIEKTISKAKGQPREPQVCTL
PPSREEMTKNQVSLWCLVKGFYPSDIAVEWESNGQPENNYKTTPPVLDSDGSF
FLYSKLTVDKSRWQQGNVFSCSVMHEALHNHYTQKSLSLSPG

SEQ ID NO: 104

> Chain 2 (M146-1-2):

QTVVTQEPSLTVSPGGTVTLTCGSSTGAVTSGNYPNWVQQKPGQAPRGLIGGT
KFLAPGTPARFSGSLLGGKAALTLSGVQPEDEAEYYCVLWYSNRWVFGGGTK
LTVLGGGGSGGGGEFQLQQSGPELVKPGASVKISCKASGYSFTDYNMNWVK
QSNGKSLEWIGLINPKYGTTNYNQKFKGKATLTVDQSSSTAYMQLNSLTSEDS
AVYYCAIDDYPAWFAYWGQGTLVTVSAGCPPCPAPEAAGAPSVFLFPPKPKDT
LMISRTPEVTCVVVDVSHEDPEVKFNWYVDGVEVHNAKTKPREEQYNSTYR
VVSVLTVLHQDWLNGKEYKCKVSNKALPAPIEKTISKAKGQPREPQVYTLPP

CREEMTKNQVSLSCAVKGFYPSDIAVEWESNGQPENNYKTTPPVLDSDGSFFL
VSKLTVDKSRWQQGNVFSCSVMHEALHNHYTQKSLSLSPG

SEQ ID NO: 105

Illustratively, the specific amino acid sequences of bispecific antibodies constructed with Format 2 in the present disclosure are as follows:

**1. Full-length sequence of P3-C7 L7H1-2**

> Chain 1 (Format 2-1):

EVQLVESGGGLVQPGGSLKLSCAASGFTFNKYAMNWVRQAPGKGLEWVARI
RSKYNNYATYYADSVKDRFTISRDDSKNTAYLQMNNLKTEDTAVYYCVRHGN
FGNEYISYWAYWGQGTLVTVSSASTKGPSVFPLAPSSKSTSGGTAALGCLVKD
YFPEPVTVSWNSGALTSGVHTFPAVLQSSGLYSLSSVVTVPSSSLGTQTYICNV
NHKPSNTKVDKKVEPKSCDKTHTCPPCPAPEAAGGPSVFLFPPKPKDTLMISR
TPEVTCVVVDVSHEDPEVKFNWYVDGVEVHNAKTKPREEQYNSTYRVVSVL
TVLHQDWLNGKEYKCKVSNKALPAPIEKTISKAKGQPREPQVYTLPPCREEM
TKNQVSLWCLVKGFYPSDIAVEWESNGQPENNYKTTPPVLDSDGSFFLYSKLT
VDKSRWQQGNVFSCSVMHEALHNHYTQKSLSLSPGK

SEQ ID NO: 91

> Chain 2 (Format 2-2):

QTVVTQEPSLTVSPGGTVTLTCGSSTGAVTSGNYPNWVQQKPGQAPRGLIGGT
KFLAPGTPARFSGSLLGGKAALTLSGVQPEDEAEYYCVLWYSNRWVFGGGTK
LTVLGQPKANPTVTLFPPSSEELQANKATLVCLISDFYPGAVTVAWKADGSPV
KAGVETTKPSKQSNNKYAASSYLSLTPEQWKSHRSYSCQVTHEGSTVEKTVA
PTEC

SEQ ID NO: 92

> Chain 3 (P3-C7 L7H1-2-3):

EVQLVQSGAEVKKPGASVKVSCKASGYTFT<u>DHYIH</u>WVRQAPGQGLEWMG<u>VI</u>
<u>NPYNDGATYNQKFKG</u>RVTMT<u>V</u>D<u>K</u>STSTVYMELSSLRSEDTAVYYCAR<u>PPRAY</u>
<u>GNYAGY</u>WGQGTTVTVSS<u>GGGGS</u><u>SGAPRFLTRPKASVVSVGKDATLSCQIVGN</u>
<u>PFPQVSWEKDKQPVTAGVRFRLAQDGDLYRLKILDLQLSDSGQYVCRARNA</u>
<u>HGEAFACLGLQVDAEA</u>*DKTHTCPPCPAPE*<u>*AA*</u>*GGPSVFLFPPKPKDTLMISRTPE*
*VTCVVVDVSHEDPEVKFNWYVDGVEVHNAKTKPREEQYNSTYRVVSVLTVLHQD*
*WLNGKEYKCKVSNKALPAPIEKTISKAKGQPREPQV*<u>*C*</u>*TLPPSREEMTKNQVSLS*<u>*C*</u>*A*
*VKGFYPSDIAVEWESNGQPENNYKTTPPVLDSDGSFFL*<u>*V*</u>*SKLTVDKSRWQQGNVF*
*SCSVMHEALHNHYTQKSLSLSPGK*

## SEQ ID NO: 106

> Chain 4 (P3-C7 L7H1-2-4):

D<u>V</u>VMTQTPLSLPVTPGEPASISC<u>RSSQSLVHSNQ</u>NTYLHWYLQKPGQSPQLLI
Y<u>KVSNRFS</u>GVPDRFSGSGS**R**TDFTLKISRVEAEDVGVYYC<u>SQSTHVPYT</u>FGGG
TKVEIKGGGGS<u>GIPPKIECLPIDISIDEGKVLTVASAFTGEPTPEVTWSTGGRKIH</u>
<u>SQEQGRFHIENTDDSTTLTIKDVQKQDGGLYTLTLRNEFGSDSATVNIHIRSI</u>

SEQ ID NO: 107

## 2. Full-length sequence of M81-2

> Chain 1 (Format 2-1, SEQ ID NO: 91)
> Chain 2 (Format 2-2, SEQ ID NO: 92)
> Chain 3 (M81-2-3):

EFQLQQSGPELVKPGASVKISCKASGYSFT<u>DHNMN</u>WVKQSNGKSLEWIG<u>VM</u>
<u>NPKFGTINYNQKFKD</u>KATLTVDQSSSTAYMQLNSLTSEDSAVYYCVR<u>DDYPN</u>
<u>YFD</u>YWGQGTTLTVSS<u>GGGGS</u><u>SGAPRFLTRPKASVVSVGKDATLSCQIVGNPFP</u>
<u>QVSWEKDKQPVTAGVRFRLAQDGDLYRLKILDLQLSDSGQYVCRARNAHGE</u>
<u>AFACLGLQVDAEA</u>*DKTHTCPPCPAPE*<u>*AA*</u>*GGPSVFLFPPKPKDTLMISRTPEVTCV*
*VVDVSHEDPEVKFNWYVDGVEVHNAKTKPREEQYNSTYRVVSVLTVLHQDWLNG*
*KEYKCKVSNKALPAPIEKTISKAKGQPREPQV*<u>*C*</u>*TLPPSREEMTKNQVSLS*<u>*C*</u>*A*VKGF*
*YPSDIAVEWESNGQPENNYKTTPPVLDSDGSFFL*<u>*V*</u>*SKLTVDKSRWQQGNVFSCSV*
*MHEALHNHYTQKSLSLSPGK*

SEQ ID NO: 108

> Chain 4 (M81-2-4):

QIVLTQSPAIMSASPGEKVTISCSASSSVNYMYWYQQKPGSSPKSWIYRTSNLA
SGVSARFSGSGSGTSYSLTISSMEAEDAATYYCQQYHDYPPTFGGGTKLEIKG
GGGSGIPPKIECLPIDISIDEGKVLTVASAFTGEPTPEVTWSTGGRKIHSQEQGRF
HIENTDDSTTLTIKDVQKQDGGLYTLTLRNEFGSDSATVNIHIRSI

SEQ ID NO: 109

### 3. Full-length sequence of 81 L1H1-2

> Chain 1 (Format 2-1, SEQ ID NO: 91)
> Chain 2 (Format 2-2, SEQ ID NO: 92)
> Chain 3 (81 L1H1-2-3):

EVQLVQSGAEVKKPGASVKVSCKASGYTFTDHNMNWVKQAPGQRLEWMG
VMNPKFGTINYNQKFKDKATITRDTSASTAYMELSSLRSEDTAVYYCARDNY
PNYFDYWGQGTTVTVSSGGGGSSGAPRFLTRPKASVVSVGKDATLSCQIVGN
PFPQVSWEKDKQPVTAGVRFRLAQDGDLYRLKILDLQLSDSGQYVCRARNA

HGEAFACLGLQVDAEA*DKTHTCPPCPAPEAAGGPSVFLFPPKPKDTLMISRTPE*
*VTCVVVDVSHEDPEVKFNWYVDGVEVHNAKTKPREEQYNSTYRVVSVLTVLHQD*
*WLNGKEYKCKVSNKALPAPIEKTISKAKGQPREPQVCTLPPSREEMTKNQVSLSCA*
*VKGFYPSDIAVEWESNGQPENNYKTTPPVLDSDGSFFLVSKLTVDKSRWQQGNVF*
*SCSVMHEALHNHYTQKSLSLSPGK*

SEQ ID NO: 110

> Chain 4 (81 L1H1-2-4):

EIVLTQSPATLSLSPGERATLSCSASSSVNYMYWYQQKPGQAPKSWIYRTSNL
ASGVPARFSGSGSGTDYTLTISSLEPEDFAVYYCQQYHDYPPTFGGGTKVEIKG
GGGSGIPPKIECLPIDISIDEGKVLTVASAFTGEPTPEVTWSTGGRKIHSQEQGRF
HIENTDDSTTLTIKDVQKQDGGLYTLTLRNEFGSDSATVNIHIRSI

SEQ ID NO: 111

### 4. Full-length sequence of 81 L2H1-2

> Chain 1 (Format 2-1, SEQ ID NO: 91)
> Chain 2 (Format 2-2, SEQ ID NO: 92)
> Chain 3 (81 L2H1-2-3, same as 81 L1H1-2-3, SEQ ID NO: 110)
> Chain 4 (81 L2H1-2-4):

EIVLTQSPDFQSVTPGEKVTITCSASSSVNYMYWYQQKPDQSPKSWIYRTSNL
ASGVPSRFSGSGSGTDFTLTISSLEAEDAATYYCQQYHDYPPTFGGGTKVEIKG
GGGSGIPPKIECLPIDISIDEGKVLTVASAFTGEPTPEVTWSTGGRKIHSQEQGRF
HIENTDDSTTLTIKDVQKQDGGLYTLTLRNEFGSDSATVNIHIRSI

SEQ ID NO: 112

**5. Full-length sequence of 81 L3H1-2**

> Chain 1 (Format 2-1, SEQ ID NO: 91)
> Chain 2 (Format 2-2, SEQ ID NO: 92)
> Chain 3 (81 L3H1-2-3, same as 81 L1H1-2-3, SEQ ID NO: 110)
> Chain 4 (81 L3H1-2-4):

DIQLTQSPSFLSASPGDRVTITCSASSSVNYMYWYQQKPGSSPKSWIYRTSNLA
SGVPSRFSGSGSGTEYTLTISSLQPEDFATYYCQQYHDYPPTFGGGTKVEIKGG
GGSGIPPKIECLPIDISIDEGKVLTVASAFTGEPTPEVTWSTGGRKIHSQEQGRFH
IENTDDSTTLTIKDVQKQDGGLYTLTLRNEFGSDSATVNIHIRSI

SEQ ID NO: 113

**6. Full-length sequence of 81 L3H1-2 (N103QN106Q)**

> Chain 1 (Format 2-1 (N103QN106Q))

EVQLVESGGGLVQPGGSLKLSCAASGFTFNKYAMNWVRQAPGKGLEWVARI

RSKYNNYATYYADSVKDRFTISRDDSKNTAYLQMNNLKTEDTAVYYCVRHGQ

FGQSYISYWAYWGQGTLVTVSSASTKGPSVFPLAPSSKSTSGGTAALGCLVKD

YFPEPVTVSWNSGALTSGVHTFPAVLQSSGLYSLSSVVTVPSSSLGTQTYICNV

NHKPSNTKVDKKVEPKSCDKTHTCPPCPAPEAAGGPSVFLFPPKPKDTLMISR

TPEVTCVVVDVSHEDPEVKFNWYVDGVEVHNAKTKPREEQYNSTYRVVSVL

TVLHQDWLNGKEYKCKVSNKALPAPIEKTISKAKGQPREPQVYTLPPCREEM

TKNQVSLWCLVKGFYPSDIAVEWESNGQPENNYKTTPPVLDSDGSFFLYSKLT

VDKSRWQQGNVFSCSVMHEALHNHYTQKSLSLSPGK

SEQ ID NO: 242

> Chain 2 (Format 2-2, SEQ ID NO: 92)
> Chain 3 (81 L3H1-2-3, same as 81 L1H1-2-3, SEQ ID NO: 110)
> Chain 4 (81 L3H1-2-4, SEQ ID NO: 113):

**6. Full-length sequence of 81 L4H1-2**

> Chain 1 (Format 2-1, SEQ ID NO: 91)
> Chain 2 (Format 2-2, SEQ ID NO: 92)
> Chain 3 (81 L4H1-2-3, same as 81 L1H1-2-3, SEQ ID NO: 110)
> Chain 4 (81 L4H1-2-4):

DIQLTQSPSFLSAS**P**GDRVTITC<u>SASSSVNYMY</u>WYQQKPG<u>SS</u>PK<u>SW</u>IYRT<u>SN**D**</u>
<u>A</u>SGVPSRFSGSGSGTE<u>Y</u>TLTISSLQPEDFATYYC<u>QQYHDYPPT</u>FGGGTKVEIKG
GGG<u>S</u>GIPPKIECLPIDIS<u>IDEGKVLTVASAFTGEPTPEVTWSTGGRKIHSQEQGRF
HIENTDDSTTLTIKDVQKQDGGLYTLTLRNEFGSDSATVNIHIRSI

SEQ ID NO: 114

**7. Full-length sequence of 81 L5H1-2**

> Chain 1 (Format 2-1, SEQ ID NO: 91)
> Chain 2 (Format 2-2, SEQ ID NO: 92)
> Chain 3 (81 L5H1-2-3, same as 81 L1H1-2-3, SEQ ID NO: 110)
> Chain 4 (81 L5H1-2-4):

DIQLTQSPSFLSAS**P**GDRVTITC<u>SASSSVNYMY</u>WYQQKPG<u>SS</u>PK<u>SW</u>IYRT<u>SNLA</u>
**Q**GVPSRFSGSGSGTE<u>Y</u>TLTISSLQPEDFATYYC<u>QQYHDYPPT</u>FGGGTKVEIKGG
GG<u>S</u>GIPPKIECLPIDIS<u>IDEGKVLTVASAFTGEPTPEVTWSTGGRKIHSQEQGRFH
IENTDDSTTLTIKDVQKQDGGLYTLTLRNEFGSDSATVNIHIRSI

SEQ ID NO: 115

**8. Full-length sequence of 81 L6H1-2**

> Chain 1 (Format 2-1, SEQ ID NO: 91)
> Chain 2 (Format 2-2, SEQ ID NO: 92)
> Chain 3 (81 L6H1-2-3, same as 81 L1H1-2-3, SEQ ID NO: 110)
> Chain 4 (81 L6H1-2-4)

DIQLTQSPSFLSAS**P**GDRVTITC<u>SASSSVNYMY</u>WYQQKPG<u>SS</u>PK<u>SW</u>IYRT**Q**NL
<u>A</u>SGVPSRFSGSGSGTE<u>Y</u>TLTISSLQPEDFATYYC<u>QQYHDYPPT</u>FGGGTKVEIKG
GGG<u>S</u>GIPPKIECLPIDIS<u>IDEGKVLTVASAFTGEPTPEVTWSTGGRKIHSQEQGRF
HIENTDDSTTLTIKDVQKQDGGLYTLTLRNEFGSDSATVNIHIRSI

SEQ ID NO: 116

**9. Full-Length sequence of M146-2**

91

> Chain 1 (Format 2-1, SEQ ID NO: 91)
> Chain 2 (Format 2-2, SEQ ID NO: 92)
> Chain 3 (M146-2-3):

EFQLQQSGPELVKPGASVKISCKASGYSFTDYNMNWVKQSNGKSLEWIGLIN
PKYGTTNYNQKFKGKATLTVDQSSSTAYMQLNSLTSEDSAVYYCAIDDYPAW
FAYWGQGTLVTVSAGGGGS<u>SGAPRFLTRPKASVVSVGKDATLSCQIVGNPFPQ</u>
<u>VSWEKDKQPVTAGVRFRLAQDGDLYRLKILDLQLSDSGQYVCRARNAHGEA</u>
<u>FACLGLQVDAEA</u>*DKTHTCPPCPAPEAAGGPSVFLFPPKPKDTLMISRTPEVTCVV*
*VDVSHEDPEVKFNWYVDGVEVHNAKTKPREEQYNSTYRVVSVLTVLHQDWLNGK*
*EYKCKVSNKALPAPIEKTISKAKGQPREPQVCTLPPSREEMTKNQVSLSCAVKGFY*
*PSDIAVEWESNGQPENNYKTTPPVLDSDGSFFLVSKLTVDKSRWQQGNVFSCSVM*
*HEALHNHYTQKSLSLSPGK*

SEQ ID NO: 117

> Chain 4 (M146-2-4):

QIVLTQSPAIMSASPGEKVTISC<u>SASSSLNYMY</u>WYQQKPGSSPKSWIY<u>RTSNLA</u>
<u>S</u>GVPARFSGSGSGTSYSLTISSMEAEDAATYYC<u>QQYHSYPPT</u>FGAGTKLELKG
GGGS<u>GIPPKIECLPIDISIDEGKVLTVASAFTGEPTPEVTWSTGGRKIHSQEQGRF</u>
<u>HIENTDDSTTLTIKDVQKQDGGLYTLTLRNEFGSDSATVNIHIRSI</u>

SEQ ID NO: 118

**10. Full-length sequence of 146 L1H1-2**

> Chain 1 (Format 2-1, SEQ ID NO: 91)
> Chain 2 (Format 2-2, SEQ ID NO: 92)
> Chain 3 (146 L1H1-2-3):

E<u>FQLVQSGAEVKKPGASVKVSCKASGYSFTDYNMN</u>WVRQAPGQRLEWMG<u>L</u>
<u>INPKYGTTNYNQKFKGRVTLTVDQSS</u>STAYMELSSLRSEDTAVYYCAR<u>DNYP</u>
<u>AWFAY</u>WGQGTTVTVSSGGGGS<u>SGAPRFLTRPKASVVSVGKDATLSCQIVGNP</u>
<u>FPQVSWEKDKQPVTAGVRFRLAQDGDLYRLKILDLQLSDSGQYVCRARNAH</u>
<u>GEAFACLGLQVDAEA</u>*DKTHTCPPCPAPEAAGGPSVFLFPPKPKDTLMISRTPEVT*
*CVVVDVSHEDPEVKFNWYVDGVEVHNAKTKPREEQYNSTYRVVSVLTVLHQDWL*
*NGKEYKCKVSNKALPAPIEKTISKAKGQPREPQVCTLPPSREEMTKNQVSLSCAVK*
*GFYPSDIAVEWESNGQPENNYKTTPPVLDSDGSFFLVSKLTVDKSRWQQGNVFSC*
*SVMHEALHNHYTQKSLSLSPGK*

SEQ ID NO: 119

> Chain 4 (146 L1H1-2-4):

EIVLTQSPATLSLSPGERATLSC<u>SASSSLNYMY</u>WYQQKPG<u>SS</u>PR<u>PW</u>IY<u>RTSQLA</u>
<u>SGV</u>PARFSGSGSGTD<u>Y</u>TLTISSLEPEDFAVYYC<u>QQYHSYPPT</u>FGGGTKVEIK<u>GG</u>
<u>GGSGIPPKIECLPIDISIDEGKVLTVASAFTGEPTPEVTWSTGGRKIHSQEQGRFH</u>
<u>IENTDDSTTLTIKDVQKQDGGLYTLTLRNEFGSDSATVNIHIRSI</u>

SEQ ID NO: 120

Illustratively, the specific amino acid sequences of bispecific antibodies constructed with Format 3 in the present disclosure are as follows:

**1. Full-length sequence of P3-C7 L7H1-3**

> Chain 1 (P3-C7 L7H1-3-1):

EVQLVQSGAEVKKPGASVKVSCKASGYTFTDHYIHWVRQAPGQGLEWMGVI
NPYNDGATYNQKFKGRVTMTVDKSTSTVYMELSSLRSEDTAVYYCARPPRAY
GNYAGYWGQGTTVTVSSGGGGSSGAPRFLTRPKASVVSVGKDATLSCQIVGN
PFPQVSWEKDKQPVTAGVRFRLAQDGDLYRLKILDLQLSDSGQYVCRARNA
HGEAFACLGLQVDAEADKTHTCPPCPAPEAAGGPSVFLFPPKPKDTLMISRTP
EVTCVVVDVSHEDPEVKFNWYVDGVEVHNAKTKPREEQYNSTYRVVSVLTV
LHQDWLNGKEYKCKVSNKALPAPIEKTISKAKGQPREPQVYTLPPCREEMTK
NQVSLWCLVKGFYPSDIAVEWESNGQPENNYKTTPPVLDSDGSFFLYSKLTVD
KSRWQQGNVFSCSVMHEALHNHYTQKSLSLSPGK

SEQ ID NO: 121

> Chain 2 (P3-C7 L7H1-3-2, same as P3-C7 L7H1-2-4, SEQ ID NO: 107)
> Chain 3 (Format 3-3, SEQ ID NO: 93)
> Chain 4 (Format 3-4, same as Format 2-2; SEQ ID NO: 92)

**2. Full-length sequence of 81 L1H1-3**

> Chain 1 (81 L1H1-3-1):

EVQLVQSGAEVKKPGASVKVSCKASGYTFT<u>DHNMN</u>WV**K**QAPGQRLEWMG

<u>VMNPKFGTINYNQKFKD</u>**KA**TITRDTSASTAYMELSSLRSEDTAVYYCARD<u>N</u>Y

<u>PNYFD</u>YWGQGTTVTVSSGGGGSSGAPRFLTRPKASVVSVGKDATLSCQIVGN

PFPQVSWEKDKQPVTAGVRFRLAQDGDLYRLKILDLQLSDSGQYVCRARNA

HGEAFACLGLQVDAEADKTHTCPPCPAPEAAGGPSVFLFPPKPKDTLMISRTP

EVTCVVVDVSHEDPEVKFNWYVDGVEVHNAKTKPREEQYNSTYRVVSVLTV

LHQDWLNGKEYKCKVSNKALPAPIEKTISKAKGQPREPQVYTLPPCREEMTK

NQVSLWCLVKGFYPSDIAVEWESNGQPENNYKTTPPVLDSDGSFFLYSKLTVD

KSRWQQGNVFSCSVMHEALHNHYTQKSLSLSPGK

SEQ ID NO: 122

> Chain 2 (81 L1H1-3-2, same as 81 L1H1-2-4, SEQ ID NO: 111)
> Chain 3 (Format 3-3, SEQ ID NO: 93)
> Chain 4 (Format 3-4, same as Format 2-2; SEQ ID NO: 92)

**3. Full-length sequence of 81 L2H1-3**

> Chain 1 (81 L2H1-3-1, same as 81 L1H1-3-1, SEQ ID NO: 122)
> Chain 2 (81 L2H1-3-2, same as 81 L2H1-2-4, SEQ ID NO: 112)
> Chain 3 (Format 3-3, SEQ ID NO: 93)
> Chain 4 (Format 3-4, same as Format 2-2; SEQ ID NO: 92)

**4. Full-length sequence of 81 L3H1-3**

> Chain 1 (81 L3H1-3-1, same as 81 L1H1-3-1, SEQ ID NO: 122)
> Chain 2 (81 L3H1-3-2, same as 81 L3H1-2-4, SEQ ID NO: 113)
> Chain 3 (Format 3-3, SEQ ID NO: 93)
> Chain 4 (Format 3-4, same as Format 2-2; SEQ ID NO: 92)

**5. Full-length sequence of 81 L4H1-3**

> Chain 1 (81 L4H1-3-1, same as 81 L1H1-3-1, SEQ ID NO: 122)
> Chain 2 (81 L4H1-3-2, same as 81 L4H1-2-4, SEQ ID NO: 114)
> Chain 3 (Format 3-3, SEQ ID NO: 93)
> Chain 4 (Format 3-4, same as Format 2-2; SEQ ID NO: 92)

**6. Full-length sequence of 81 L5H1-3**

> Chain 1 (81 L5H1-3-1, same as 81 L1H1-3-1, SEQ ID NO: 122)
> Chain 2 (81 L5H1-3-2, same as 81 L5H1-2-4, SEQ ID NO: 115)
> Chain 3 (Format 3-3, SEQ ID NO: 93)
> Chain 4 (Format 3-4, same as Format 2-2; SEQ ID NO: 92)

**7. Full-length sequence of 81 L6H1-3**

> Chain 1 (81 L6H1-3-1, same as 81 L1H1-3-1, SEQ ID NO: 122)
> Chain 2 (81 L6H1-3-2, same as 81 L6H1-2-4, SEQ ID NO: 116)
> Chain 3 (Format 3-3, SEQ ID NO: 93)
> Chain 4 (Format 3-4, same as Format 2-2; SEQ ID NO: 92)

**8. Full-length sequence of 146 L1H1-3**

> Chain 1 (146 L1H1-3-1):

EFQLVQSGAEVKKPGASVKVSCKASGYSFTDYNMNWVRQAPGQRLEWMGL

INPKYGTTNYNQKFKGRVTLTVDQSSSTAYMELSSLRSEDTAVYYCARDNYP

AWFAYWGQGTTVTVSSGGGGSSGAPRFLTRPKASVVSVGKDATLSCQIVGNP

FPQVSWEKDKQPVTAGVRFRLAQDGDLYRLKILDLQLSDSGQYVCRARNAH

GEAFACLGLQVDAEADKTHTCPPCPAPEAAGGPSVFLFPPKPKDTLMISRTPE

VTCVVVDVSHEDPEVKFNWYVDGVEVHNAKTKPREEQYNSTYRVVSVLTVL

HQDWLNGKEYKCKVSNKALPAPIEKTISKAKGQPREPQVYTLPPCREEMTKN

QVSLWCLVKGFYPSDIAVEWESNGQPENNYKTTPPVLDSDGSFFLYSKLTVDK

SRWQQGNVFSCSVMHEALHNHYTQKSLSLSPGK

SEQ ID NO: 123

> Chain 2 (146 L1H1-3-2, same as 146 L1H1-2-4, SEQ ID NO: 120)
> Chain 3 (Format 3-3, SEQ ID NO: 93)
> Chain 4 (Format 3-4, same as Format 2-2; SEQ ID NO: 92)

Illustratively, the specific amino acid sequences of bispecific antibodies constructed with Format 4 in the present disclosure are as follows:

**1. Full-length sequence of P3-C7 L7H1-4**

> Chain 1 (P3-C7 L7H1-4-1):

EVQLVQSGAEVKKPGASVKVSCKASGYTFTDHYIHWVRQAPGQGLEWMGVI
NPYNDGATYNQKFKGRVTMTVDKSTSTVYMELSSLRSEDTAVYYCARPPRAY
GNYAGYWGQGTTVTVSS*ASTKGPSVFPLAPSSKSTSGGTAALGCLVKDYFPEPVT
VSWNSGALTSGVHTFPAVLQSSGLYSLSSVVTVPSSSLGTQTYICNVNHKPSNTKVDK
KVEPKSC*EVQLVESGGGLVQPGGSLKLSCAASGFTFNKYAMNWVRQAPGKGL
EWVARIRSKYNNYATYYADSVKDRFTISRDDSKNTAYLQMNNLKTEDTAVYY
CVRHGNFGNEYISYWAYWGQGTLVTVSSGGGGSGGGGSGGGGSQTVVTQEP
SLTVSPGGTVTLTCGSSTGAVTSGNYPNWVQQKPGQAPRGLIGGTKFLAPGTP
ARFSGSLLGGKAALTLSGVQPEDEAEYYCVLWYSNRWVFGGGTKLTVLGGG
*DKTHTCPPCPAPEAAGGPSVFLFPPKPKDTLMISRTPEVTCVVVDVSHEDPEVKF
NWYVDGVEVHNAKTKPREEQYNSTYRVVSVLTVLHQDWLNGKEYKCKVSNKALPA*

*PIEKTISKAKGQPREPQVYTLPPSREEMTKNQVSLTCLVKGFYPSDIAVEWESNGQP
ENNYKTTPPVLDSDGSFFLYSKLTVDKSRWQQGNVFSCSVMHEALHNHYTQKSLS
LSPGK*

SEQ ID NO: 124

> Chain 2 (P3-C7 L7H1-4-2, same as P3-C7 L7H1 light chain, SEQ ID NO: 65)

**2. Full-length sequence of P3-C7 L8H1-4**

> Chain 1 (P3-C7 L8H1-4-1, same as P3-C7 L7H1-4-1, SEQ ID NO: 124)
> Chain 2 (P3-C7 L8H1-4-2, same as P3-C7 L8H1 light chain, SEQ ID NO: 66)

Illustratively, the specific amino acid sequences of bispecific antibodies constructed with Format 5 in the present disclosure are as follows:

**1. Full-length sequence of P3-C7 L7H1-5**

> Chain 1 (P3-C7 L7H1-5-1):

EVQLVQSGAEVKKPGASVKVSCKASGYTFT<u>DHYIH</u>WVRQAPGQGLEWMG<u>VI
NPYNDGATYNQKFKG</u>RVTMTVDKSTSTVYMELSSLRSEDTAVYYCAR<u>PPRAY
GNYAGY</u>WGQGTTVTVSS*ASTKGPSVFPLAPSSKSTSGGTAALGCLVKDYFPEPVT
VSWNSGALTSGVHTFPAVLQSSGLYSLSSVVTVPSSSLGTQTYICNVNHKPSNTKVDK
KVEPKSCGGGGSGGGGS*EVQLVESGGGLVQPGGSLKLSCAASGFTF<u>NKYAMN</u>
WVRQAPGKGLEWVA<u>RIRSKYNNYATYY</u>ADSVKDRFTISRDDSKNTAYLQMN
NLKTEDTAVYYCVR<u>HGNFGNEYISYWAY</u>WGQGTLVTVSSGGGGS<u>SGAPRFLT
RPKASVVSVGKDATLSCQIVGNPFPQVSWEKDKQPVTAGVRFRLAQDGDLYR
LKILDLQLSDSGQYVCRARNAHGEAFACLGLQVDAEA</u>*DKTHTCPPCPAPE*A̲A̲
*GGPSVFLFPPKPKDTLMISRTPEVTCVVVDVSHEDPEVKFNWYVDGVEVHNAKTK
PREEQYNSTYRVVSVLTVLHQDWLNGKEYKCKVSNKALPAPIEKTISKAKGQPREP
QVYTLPPSREEMTKNQVSLTCLVKGFYPSDIAVEWESNGQPENNYKTTPPVLDSDG
SFFLYSKLTVDKSRWQQGNVFSCSVMHEALHNHYTQKSLSLSPGK*

SEQ ID NO: 125

> Chain 2 (P3-C7 L7H1-5-2, same as P3-C7 L7H1 light chain, SEQ ID NO: 65)
> Chain 3 (Format 5-3, SEQ ID NO: 98)

**2. Full-length sequence of P3-C7 L8H1-5**

> Chain 1 (P3-C7 L8H1-5-1, same as P3-C7 L7H1-5-1, SEQ ID NO: 125)
> Chain 2 (P3-C7 L8H1-5-2, same as P3-C7 L8H1 light chain, SEQ ID NO: 66)
> Chain 3 (Format 5-3, SEQ ID NO: 98)

In addition, the GUCY2C-CD3 bispecific antibody GUCY2c-1608 (named 1608-BsAb in the present disclosure), which is prepared with reference to patent No. WO2019224716A2, is also used in the present disclosure; it has a DIABODY structure, and the complete molecule comprises two chains, which are specifically as follows:

> Chain 1 (GUCY2c-1608-Knob sequence):

DIQLTQSPSSLSASVGDRVTITC<u>RASESVDYYGSSLLQ</u>WYQQKPGKAPKLLIY<u>A
ASKLAS</u>GVPSRFSGSGSGTDFTLTISSLQPEDFATYYC<u>QQTRKAYT</u>FGQGTKLE
IKGGGSGGGGEVQLVESGGGLVQPGGSLRLSCAASGFTFS<u>DYYMT</u>WVRQAP
GKGLEWVA<u>FIRNQARGYTSDHNPSVKG</u>RFTISRDNAKNSLYLQMNSLRAEDT
AVYYCAR<u>DRPSYYVLDY</u>WGQGTTVTVSS*GCPPCPAPE*A̲A̲*GAPSVFLFPPKPKD
TLMISRTPEVTCVVVDVSHEDPEVKFNWYVDGVEVHNAKTKPREEQYNSTYRVVSV
LTVLHQDWLNGKEYKCKVSNKALPAPIEKTISKAKGQPREPQV*C̲*TLPPSREEMTKN
QVSL*W̲*CLVKGFYPSDIAVEWESNGQPENNYKTTPPVLDSDGSFFLYSKLTVDKSRW
QQGNVFSCSVMHEALHNHYTQKSLSLSPG*

SEQ ID NO: 126

> Chain 2 (GUCY2c-1608-Hole sequence):

DIQMTQSPSSLSASVGDRVTITCTSSQSLFNVRSQKNYLAWYQQKPGKAPKLL
IYWASTRESGVPSRFSGSGSGTDFTLTISSLQPEDFATYYCKQSYDLFTFGGGTK
VEIKGGGGSGGGGEVQLVESGGGLVQPGGSLRLSCAASGFTFSSYWMHWVR
QAPGKGLEWIGEIKPSNELTNVHEKFKDRFTISVDKAKNSAYLQMNSLRAEDT
AVYYCTRTITTTEGYWFFDVWGQGTLVTVSSGCPPCPAPEAAGAPSVFLFPPKP
*KDTLMISRTPEVTCVVVDVSHEDPEVKFNWYVDGVEVHNAKTKPREEQYNSTYRV*
*VSVLTVLHQDWLNGKEYKCKVSNKALPAPIEKTISKAKGQPREPQVYTLPPCREEM*
*TKNQVSLSCAVKGFYPSDIAVEWESNGQPENNYKTTPPVLDSDGSFFLVSKLTVDK*
*SRWQQGNVFSCSVMHEALHNHYTQKSLSLSPG*

SEQ ID NO: 127

Table 26. CDR sequences of 1608-BsAb

| | | |
|---|---|---|
| GUCY2C | HCDR1 | SYWMH (SEQ ID NO: 128) |
| | HCDR2 | EIKPSNELTNVHEKFKD (SEQ ID NO: 129) |
| | HCDR3 | TITTTEGYWFFDV (SEQ ID NO: 130) |
| | LCDR1 | RASESVDYYGSSLLQ (SEQ ID NO: 131) |
| | LCDR2 | AASKLAS (SEQ ID NO: 132) |
| | LCDR3 | QQTRKAYT (SEQ ID NO: 133) |
| CD3 | HCDR1 | DYYMT (SEQ ID NO: 134) |
| | HCDR2 | FIRNQARGYTSDHNPSVKG (SEQ ID NO: 135) |
| | HCDR3 | DRPSYYVLDY (SEQ ID NO: 136) |
| | LCDR1 | TSSQSLFNVRSQKNYLA (SEQ ID NO: 137) |
| | LCDR2 | WASTRES (SEQ ID NO: 138) |
| | LCDR3 | KQSYDLFT (SEQ ID NO: 139) |

In addition, the EGFR-CD28 bispecific antibody 97H2L3OT-ZAL, which is prepared with reference to patent No. CN202210371538.8, is also used in the present disclosure, and its sequence is specifically as follows:

Table 27. CDRs of EGFR-CD28 bispecific antibody 97H2L3OT-ZAL

| | | Heavy chain | | Light chain |
|---|---|---|---|---|
| EGFR | HCDR1 | TYGMH (SEQ ID NO: 140) | LCDR1 | RASQDISSALV (SEQ ID NO: 143) |
| | HCDR2 | VIWDDGSYKYYGDSVKG (SEQ ID NO: 141) | LCDR2 | DASSLES (SEQ ID NO: 144) |
| | HCDR3 | DGITMVRGVMKDYFDY (SEQ ID NO: 142) | LCDR3 | QQFNSYPLT (SEQ ID NO: 145) |
| CD28 | HCDR1 | SYWIT (SEQ ID NO: 146) | LCDR1 | RASQDISNYLN (SEQ ID NO: 149) |
| | HCDR2 | DIYPGSGRTNYNEMFKN (SEQ ID NO: 147) | LCDR2 | YTSRLHS (SEQ ID NO: 150) |
| | HCDR3 | RHYGTGYESFDV (SEQ ID NO: 148) | LCDR3 | QQGNTLPWT (SEQ ID NO: 151) |

EGFR-VH:

QVQLVESGGGVVQPGRSLRLSCAASGFTFSTYGMHWVRQAPGKGLEWVAVI
WDDGSYKYYGDSVKGRFTISRDNSKNTLYLQMNSLRAEDTAVYYCARDGIT
MVRGVMKDYFDYWGQGTLVTVSS

SEQ ID NO: 152

EGFR-VL:

DIQLTQSPSSLSASVGDRVTITCRASQDISSALVWYQQKPGKAPKLLIYDASSL
ESGVPSRFSGSESGTDFTLTISSLQPEDFATYYCQQFNSYPLTFGGGTKVEIK

SEQ ID NO: 153

CD28-VH:

EVQLVQSGAEVKKPGASVKVSCKASGYTLTSYWITWVRQAPGQGLEWMGDI
YPGSGRTNYNEMFKNRVTMTVDTSTSTAYMELSSLRSEDTAVYYCARRHYGT
GYESFDVWGQGTTVTVSS

SEQ ID NO: 154

CD28-VL:

DIQMTQSPSSLSASVGDRVTITCRASQDISNYLNWYQQKPDGTIKLLIYYTSRL
HSGVPSRFSGSGSGTDYTFTISSLQPEDIATYYCQQGNTLPWTFGGGTKVEIK

SEQ ID NO: 155

Full-length sequence of EGFR-CD28 bispecific antibody 97H2L3OT-ZAL:

> Chain 1:

EVQLVQSGAEVKKPGASVKVSCKASGYTLTSYWITWVRQAPGQGLEWMGDI
YPGSGRTNYNEMFKNRVTMTVDTSTSTAYMELSSLRSEDTAVYYCARRHYGT
GYESFDVWGQGTTVTVSSGGGGSGIPPKIECLPIDISIDEGKVLTVASAFTGEPT
PEVTWSTGGRKIHSQEQGRFHIENTDDSTTLTIKDVQKQDGGLYTLTLRNEFG
SDSATVNIHIRSIDKTHTCPPCPAPEAAGGPSVFLFPPKPKDTLMISRTPEVTCV
VVDVSHEDPEVKFNWYVDGVEVHNAKTKPREEQYNSTYRVVSVLTVLHQD
WLNGKEYKCKVSNKALPAPIEKTISKAKGQPREPQVYTLPPCRDELTKNQVSL
WCLVKGFYPSDIAVEWESNGQPENNYKTTPPVLDSDGSFFLYSKLTVDKSRW
QQGNVFSCSVMHEALHNHYTQKSLSLSPGK

SEQ ID NO: 156

> Chain 2:

DIQMTQSPSSLSASVGDRVTITCRASQDISNYLNWYQQKPDGTIKLLIYYTSRL
HSGVPSRFSGSGSGTDYTFTISSLQPEDIATYYCQQGNTLPWTFGGGTKVEIKG
GGGSSGAPRFLTRPKASVVSVGKDATLSCQIVGNPFPQVSWEKDKQPVTAGV
RFRLAQDGDLYRLKILDLQLSDSGQYVCRARNAHGEAFACLGLQVDAEA

SEQ ID NO: 157

> Chain 3:

QVQLVESGGGVVQPGRSLRLSCAASGFTFSTYGMHWVRQAPGKGLEWVAVI
WDDGSYKYYGDSVKGRFTISRDNSKNTLYLQMNSLRAEDTAVYYCARDGIT
MVRGVMKDYFDYWGQGTLVTVSSASTKGPSVFPLAPSSKSTSGGTAALGCLV
KDYFPEPVTVSWNSGALTSGVHTFPAVLQSSGLYSLSSVVTVPSSSLGTQTYIC
NVNHKPSNTKVDKKVEPKSCDKTHTCPPCPAPEAAGGPSVFLFPPKPKDTLMI
SRTPEVTCVVVDVSHEDPEVKFNWYVDGVEVHNAKTKPREEQYNSTYRVVS
VLTVLHQDWLNGKEYKCKVSNKALPAPIEKTISKAKGQPREPQVCTLPPSRDE
LTKNQVSLSCAVKGFYPSDIAVEWESNGQPENNYKTTPPVLDSDGSFFLVSKLT
VDKSRWQQGNVFSCSVMHEALHNHYTQKSLSLSPGK

SEQ ID NO: 158

> Chain 4:

DIQLTQSPSSLSASVGDRVTITCRASQDISSALVWYQQKPGKAPKLLIYDASSL
ESGVPSRFSGSESGTDFTLTISSLQPEDFATYYCQQFNSYPLTFGGGTKVEIKRT

VAAPSVFIFPPSDEQLKSGTASVVCLLNNFYPREAKVQWKVDNALQSGNSQES
VTEQDSKDSTYSLSSTLTLSKADYEKHKVYACEVTHQGLSSPVTKSFNRGEC

SEQ ID NO: 159

In addition, the EGFR-CD28 bispecific antibodies 129H4L1OT-ZAL and 94H6L12-ZALOT, both of which were prepared with reference to patent No. CN202210371538.8, are used in the present disclosure, and their sequences are specifically as follows:

Table 28. CDRs of EGFR-CD28 bispecific antibody 129H4L1OT-ZAL

| | | Heavy chain | | Light chain | |
|---|---|---|---|---|---|
| EGFR | HCDR1 | TYGMH (SEQ ID NO: 140) | LCDR1 | RASQDISSALV (SEQ ID NO: 143) | |
| | HCDR2 | VIWDDGSYKYYGDSVKG (SEQ ID NO: 141) | LCDR2 | DASSLES (SEQ ID NO: 144) | |
| | HCDR3 | DGITMVRGVMKDYFDY (SEQ ID NO: 142) | LCDR3 | QQFNSYPLT (SEQ ID NO: 145) | |
| CD28 | HCDR1 | SHWIQ (SEQ ID NO: 160) | LCDR1 | RASQDISNYLN (SEQ ID NO: 149) | |
| | HCDR2 | EIFPGSGSTHYNEKFKG (SEQ ID NO: 161) | LCDR2 | YTSRLHS (SEQ ID NO: 150) | |
| | HCDR3 | LHYGTSYDAMDY (SEQ ID NO: 162) | LCDR3 | QQGYTLPRT (SEQ ID NO: 163) | |

EGFR-VH (same as EGFR-VH of 129H4L1OT-ZAL, SEQ ID NO: 152)
EGFR-VL (same as EGFR-VL of 129H4L1OT-ZAL, SEQ ID NO: 153)

CD28-VH:

EVQLVQSGAEVKKPGASVKVSCKAPGYTFTSHWIQWVRQAPGQGLEWMGEI
FPGSGSTHYNEKFKGRVTMTVDTSTSTAYMELSSLRSEDTAVYYCARLHYGTS
YDAMDYWGQGTTVTVSS

SEQ ID NO: 164

CD28-VL:

DIQMTQSPSSLSASVGDRVTITCRASQDISNYLNWYQQKPGKAVKLLIYYTSR
LHSGVPSRFSGSGSGTDFTFTISSLQPEDIATYYCQQGYTLPRTFGGGTKVEIK

SEQ ID NO: 165

Full-length sequence of EGFR-CD28 bispecific antibody 129H4L1OT-ZAL:
> Chain 1 (SEQ ID NO: 166):

EVQLVQSGAEVKKPGASVKVSCKAPGYTFTSHWIQWVRQAPGQGLEWMGEI
FPGSGSTHYNEKFKGRVTMTVDTSTSTAYMELSSLRSEDTAVYYCARLHYGTS
YDAMDYWGQGTTVTVSSGGGGSGIPPKIECLPIDISIDEGKVLTVASAFTGEPT

PEVTWSTGGRKIHSQEQGRFHIENTDDSTTLTIKDVQKQDGGLYTLTLRNEFG
SDSATVNIHIRSIDKTHTCPPCPAPEAAGGPSVFLFPPKPKDTLMISRTPEVTCV
VVDVSHEDPEVKFNWYVDGVEVHNAKTKPREEQYNSTYRVVSVLTVLHQD
WLNGKEYKCKVSNKALPAPIEKTISKAKGQPREPQVYTLPPCRDELTKNQVSL
WCLVKGFYPSDIAVEWESNGQPENNYKTTPPVLDSDGSFFLYSKLTVDKSRW
QQGNVFSCSVMHEALHNHYTQKSLSLSPGK

> Chain 2 (SEQ ID NO: 167):

DIQMTQSPSSLSASVGDRVTITCRASQDISNYLNWYQQKPGKAVKLLIYYTSR
LHSGVPSRFSGSGSGTDFTFTISSLQPEDIATYYCQQGYTLPRTFGGGTKVEIKG
GGGSSGAPRFLTRPKASVVSVGKDATLSCQIVGNPFPQVSWEKDKQPVTAGV
RFRLAQDGDLYRLKILDLQLSDSGQYVCRARNAHGEAFACLGLQVDAEA

> Chain 3 (same as chain 3 of 97H2L3OT-ZAL, SEQ ID NO: 158)
> Chain 4 (same as chain 4 of 97H2L3OT-ZAL, SEQ ID NO: 159)

Table 29. CDRs of EGFR-CD28 bispecific antibody 94H6L12-ZALOT

| | | Heavy chain | | Light chain | |
|---|---|---|---|---|---|
| EGFR | HCDR1 | TYGMH (SEQ ID NO: 140) | LCDR1 | RASQDISSALV (SEQ ID NO: 143) | |
| | HCDR2 | VIWDDGSYKYYGDSVKG (SEQ ID NO: 141) | LCDR2 | DASSLES (SEQ ID NO: 144) | |
| | HCDR3 | DGITMVRGVMKDYFDY (SEQ ID NO: 142) | LCDR3 | QQFNSYPLT (SEQ ID NO: 145) | |

(continued)

| | | Heavy chain | | | Light chain | |
|---|---|---|---|---|---|---|
| CD28 | HCDR1 | GYYMN (SEQ ID NO: 168) | | LCDR1 | LASQTIGTWLA (SEQ ID NO: 171) | |
| | HCDR2 | EINPSTGDTTYNQKFKA (SEQ ID NO: 169) | | LCDR2 | AATTGAD (SEQ ID NO: 172) | |
| | HCDR3 | PYYDLTPFLY (SEQ ID NO: 170) | | LCDR3 | QQLYSTPYT (SEQ ID NO: 173) | |

EGFR-VH (same as EGFR-VH of 129H4L1OT-ZAL, SEQ ID NO: 152)
EGFR-VL (same as EGFR-VL of 129H4L1OT-ZAL, SEQ ID NO: 153)
CD28-VH:

EVQLVQSGAEVKKPGASVKVSCKASGYSFTGYYMNWVRQAPGQRLEWMGE
INPSTGDTTYNQKFKAKVTLTVDKSASTAYMELSSLRSEDTAVYYCASPYYDL
TPFLYWGQGTLVTVSS

SEQ ID NO: 174

CD28-VL:

DIQMTQSPSSVSASVGDRVTITCLASQTIGTWLAWYQQKPGKSPKLLIYAATT
GADGVPSRFSGSGSGTKFTLTISSLQPEDFATYYCQQLYSTPYTFGGGTKVEIK
SEQ ID NO: 175

Full-length sequence of EGFR-CD28 bispecific antibody 94H6L12-ZALOT:

> Chain 1:

EVQLVQSGAEVKKPGASVKVSCKASGYSFTGYYMNWVRQAPGQRLEWMGE
INPSTGDTTYNQKFKAKVTLTVDKSASTAYMELSSLRSEDTAVYYCASPYYDL
TPFLYWGQGTLVTVSSASTKGPSVFPLAPSSKSTSGGTAALGCLVKDYFPEPVT
VSWNSGALTSGVHTFPAVLQSSGLYSLSSVVTVPSSSLGTQTYICNVNHKPSNT
KVDKKVEPKSCDKTHTCPPCPAPEAAGGPSVFLFPPKPKDTLMISRTPEVTCV
VVDVSHEDPEVKFNWYVDGVEVHNAKTKPREEQYNSTYRVVSVLTVLHQD
WLNGKEYKCKVSNKALPAPIEKTISKAKGQPREPQVYTLPPCRDELTKNQVSL
WCLVKGFYPSDIAVEWESNGQPENNYKTTPPVLDSDGSFFLYSKLTVDKSRW
QQGNVFSCSVMHEALHNHYTQKSLSLSPGK
SEQ ID NO: 176

> Chain 2:

DIQMTQSPSSVSASVGDRVTITCLASQTIGTWLAWYQQKPGKSPKLLIYAATT
GADGVPSRFSGSGSGTKFTLTISSLQPEDFATYYCQQLYSTPYTFGGGTKVEIK
RTVAAPSVFIFPPSDEQLKSGTASVVCLLNNFYPREAKVQWKVDNALQSGNSQ
ESVTEQDSKDSTYSLSSTLTLSKADYEKHKVYACEVTHQGLSSPVTKSFNRGE
C

SEQ ID NO: 177

> Chain 3:

QVQLVESGGGVVQPGRSLRLSCAASGFTFSTYGMHWVRQAPGKGLEWVAVI
WDDGSYKYYGDSVKGRFTISRDNSKNTLYLQMNSLRAEDTAVYYCARDGIT
MVRGVMKDYFDYWGQGTLVTVSSGGGGSSGAPRFLTRPKASVVSVGKDATL
SCQIVGNPFPQVSWEKDKQPVTAGVRFRLAQDGDLYRLKILDLQLSDSGQYV
CRARNAHGEAFACLGLQVDAEADKTHTCPPCPAPEAAGGPSVFLFPPKPKDT
LMISRTPEVTCVVVDVSHEDPEVKFNWYVDGVEVHNAKTKPREEQYNSTYR
VVSVLTVLHQDWLNGKEYKCKVSNKALPAPIEKTISKAKGQPREPQVCTLPPS
RDELTKNQVSLSCAVKGFYPSDIAVEWESNGQPENNYKTTPPVLDSDGSFFLV
SKLTVDKSRWQQGNVFSCSVMHEALHNHYTQKSLSLSPGK
SEQ ID NO: 178

> Chain 4:

DIQLTQSPSSLSASVGDRVTITCRASQDISSALVWYQQKPGKAPKLLIYDASSL
ESGVPSRFSGSESGTDFTLTISSLQPEDFATYYCQQFNSYPLTFGGGTKVEIKGG
GGSGIPPKIECLPIDISIDEGKVLTVASAFTGEPTPEVTWSTGGRKIHSQEQGRFH
IENTDDSTTLTIKDVQKQDGGLYTLTLRNEFGSDSATVNIHIRSI
SEQ ID NO: 179

> IgG1Fc-Knob (L234A/L235A/S354C/T366W)

DKTHTCPPCPAPEAAGGPSVFLFPPKPKDTLMISRTPEVTCVVVDVSHEDPEV
KFNWYVDGVEVHNAKTKPREEQYNSTYRVVSVLTVLHQDWLNGKEYKCKV
SNKALPAPIEKTISKAKGQPREPQVYTLPPCRDELTKNQVSLWCLVKGFYPSDI
AVEWESNGQPENNYKTTPPVLDSDGSFFLYSKLTVDKSRWQQGNVFSCSVMH
EALHNHYTQKSLSLSPGK
SEQ ID NO: 248

> IgG1Fc-Hole (L234A/L235A/Y349C/T366S/L368A/Y407V)

DKTHTCPPCPAPEAAGGPSVFLFPPKPKDTLMISRTPEVTCVVVDVSHEDPEV
KFNWYVDGVEVHNAKTKPREEQYNSTYRVVSVLTVLHQDWLNGKEYKCKV
SNKALPAPIEKTISKAKGQPREPQVCTLPPSRDELTKNQVSLSCAVKGFYPSDIA
VEWESNGQPENNYKTTPPVLDSDGSFFLVSKLTVDKSRWQQGNVFSCSVMHE
ALHNHYTQKSLSLSPGK

SEQ ID NO: 249

> Linker 8 (SEQ ID NO: 90)

**Test Examples**

**Test Example 1. Affinity of GUCY2C Monoclonal Antibodies of the Present Disclosure for Antigen**

To test the affinity of the GUCY2C monoclonal antibodies of the present disclosure for GUCY2C, in this test example, the instruments Biacore 8K and Biacore T200 were used to detect the ability of the antibodies to bind to hGUCY2C-ECD-His (SEQ ID NO: 3). The specific method is as follows: The antibody was affinity-captured using a Protein A biosensor chip conjugated with an anti-human antibody, then a solution containing GUCY2C Protein flowed on the surface of the chip, and reaction signals were detected in real time with an instrument to obtain association and dissociation curves. After dissociation was completed for each cycle, the biosensor chip was washed with 10 mM glycine-hydrochloric acid (pH 1.5) for regeneration. Data fitting was performed using a 1:1 model. The affinity data for the binding of GUCY2C monoclonal antibodies of the present disclosure to the corresponding antigen are shown in the tables below.

Table 30-1. Binding of monoclonal antibodies to human GUCY2C

| Antibody | ka (1/Ms) | kd (1/s) | KD (nM) |
|---|---|---|---|
| 81 L3H1 | 1.81E+05 | 5.19E-05 | 0.29 |
| 146 L1H1 | 1.27E+05 | 1.17E-04 | 0.92 |

Table 30-2. Binding of monoclonal antibodies to human GUCY2C

| Antibody | ka (1/Ms) | kd (1/s) | KD (nM) |
|---|---|---|---|
| 81 L2H2 | 2.74E+05 | 1.27E-04 | 0.46 |
| 81 L3H2 | 2.51E+05 | 1.03E-04 | 0.41 |
| 81 L4H2 | 2.12E+05 | 2.07E-04 | 0.98 |
| 81 L6H2 | 2.41E+05 | 1.14E-04 | 0.47 |
| M81 CHI | 2. 16E+05 | 5.90E-05 | 0.27 |
| M146 CHI | 2.03E+05 | 9.22E-05 | 0.46 |

Table 30-3. Binding of monoclonal antibodies to human GUCY2C

| Antibody | ka (1/Ms) | kd (1/s) | KD (nM) |
|---|---|---|---|
| P3-C7 CHI | 2.26E+04 | 1.49E-04 | 6.61 |
| P3-C7 L1H1 | 2.37E+04 | 1.56E-04 | 6.55 |
| P3-C7 L2H1 | 3.10E+04 | 1.66E-04 | 5.35 |
| P3-C7 L3H1 | 2.84E+04 | 1.35E-03 | 47.50 |
| P3-C7 L4H1 | 2.85E+04 | 2.09E-03 | 73.40 |
| P3-C7 L5H1 | 2.69E+04 | 7.19E-04 | 26.70 |
| P3-C7 L7H1 | 2.39E+04 | 1.86E-04 | 7.77 |

Table 30-4. Binding of monoclonal antibodies to human GUCY2C

| Antibody | ka (1/Ms) | kd (1/s) | KD (nM) |
|----------|-----------|----------|---------|
| M42 CHI | 3.73E+04 | 3.35E-04 | 8.99 |
| M61 CHI | 5.08E+04 | 2.28E-04 | 4.49 |
| M64 CHI | 1.71E+04 | 3.48E-04 | 20.40 |
| M93 CHI | 2.12E+04 | 3.60E-04 | 17.00 |

Table 30-5. Binding of monoclonal antibodies to human GUCY2C

| Antibody | ka (1/Ms) | kd (1/s) | KD (nM) |
|----------|-----------|----------|---------|
| P1-D2 CHI | 1.95E+05 | 1.80E-04 | 0.92 |
| P1-D3 CHI | 4.60E+04 | 2.33E-04 | 5.06 |
| P1-C6 CHI | 7.07E+04 | 1.81E-04 | 2.57 |
| P1-D9 CHI | 2.03E+04 | 3.99E-04 | 19.60 |
| P1-F10 CHI | 3.23E+04 | 2.79E-04 | 8.64 |
| P2-C8 CHI | 3.49E+04 | 1.78E-04 | 5.12 |
| P2-B9 CHI | 5.81E+04 | 1.75E-04 | 3.02 |
| P3-D2 CHI | 3.86E+04 | 1.73E-04 | 4.50 |
| P3-B4 CHI | 2.58E+04 | 2.14E-04 | 8.28 |
| P3-E4 CHI | 1.77E+04 | 2.28E-04 | 12.90 |
| P3-F6 CHI | 6.61E+03 | 2.45E-04 | 37.00 |
| P3-D9 CHI | 1.90E+04 | 5.04E-04 | 26.50 |

The results show that the GUCY2C monoclonal antibodies of the present disclosure exhibit good binding affinity for human GUCY2C.

**Test Example 2. Binding Activity of Anti-GUCY2C Monoclonal Antibodies for Cell Surface Antigen**

In this test example, the binding activity of GUCY2C monoclonal antibodies for human GUCY2C on the surface of stably transfected cell strain HCT116/hGUCY2C and intestinal cancer cells HT-55 and T84 that overexpress human GUCY2C was detected by flow cytometry. The specific procedures are as follows:

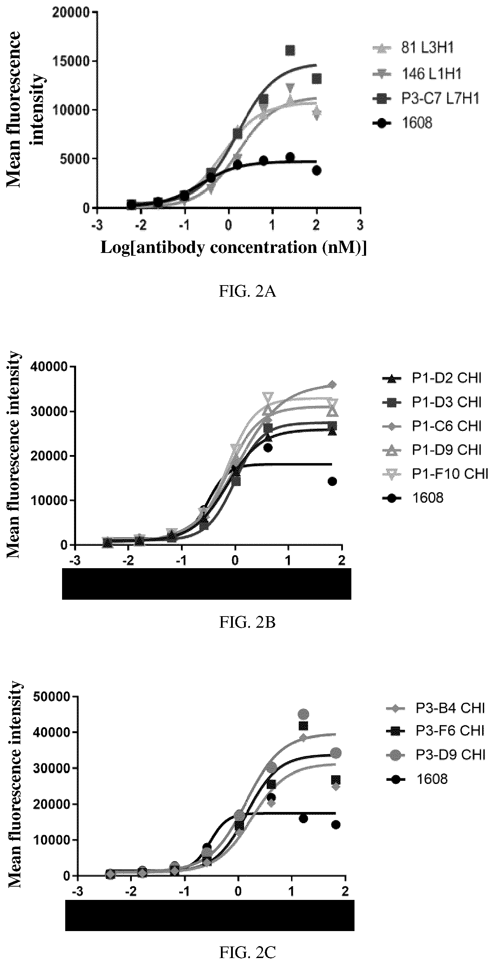

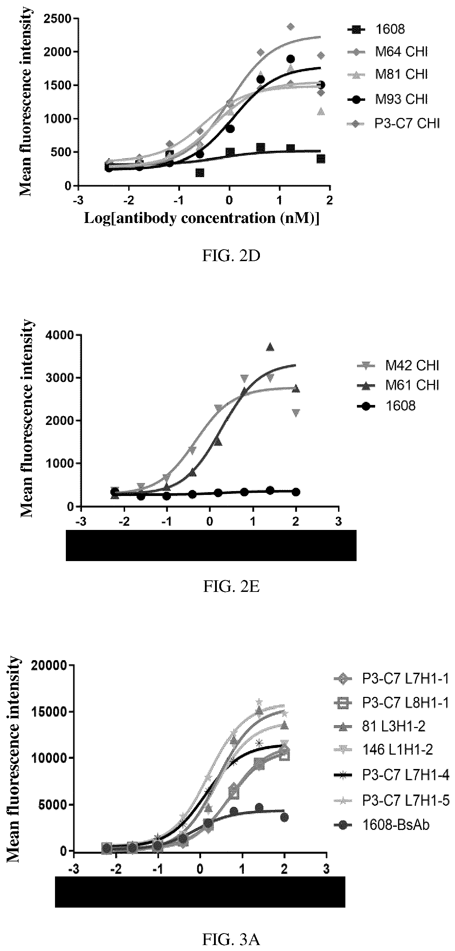

1E5 cells resuspended in 1% BSA were added to the reaction wells, and the supernatant was removed after centrifugation. Diluted antibody of the corresponding concentration was added, and the mixture was incubated at room temperature for 30 min and then washed once with 1% BSA. Then, PE-labeled F(ab')$_2$-goat anti-human IgG Fc secondary antibody (Cat. # H10104, Invitrogen) was added, and the mixture was resuspended and incubated at room temperature for 30 min in the dark. After the plate was washed twice with 1% BSA, fluorescence signals were read using a flow cytometer. Exemplary FACS results are shown in FIGs. 2A-2E.

The result shows that the GUCY2C monoclonal antibodies of the present disclosure exhibit good specific binding for GUCY2C on the cell surface, and the binding activity is better than that of 1608.

**Test Example 3. Non-Specific Binding of GUCY2C Humanized Antibody of the Present Disclosure**

In this test example, the non-specific binding of the GUCY2C humanized antibody for non-target cells (e.g., T cells) is detected. The specific procedures are as follows:

Experiment 1: The flow cytometry was used; 1E5 Jurkat cells (ATCC# TIB-152) resuspended in 1% BSA were added to each well, and the supernatant was removed after centrifugation. Diluted antibody of the corresponding concentration was added, and the mixture was incubated at room temperature for 30 min and then washed once with 1% BSA. Then, PE-labeled F(ab')$_2$-goat anti-human IgG Fc secondary antibody (Cat. # H10104, Invitrogen) was added, and the mixture was resuspended and incubated at room temperature for 30 min in the dark. After the plate was washed twice with 1% BSA, fluorescence signals were read using a flow cytometer. The relative mean fluorescence intensity value was obtained by subtracting the background fluorescence intensity of the cells without incubated antibody from the mean fluorescence intensity of antibody binding. The results are shown in Table 31 below.

Table 31. Binding of GUCY2C humanized monoclonal antibodies to Jurkat

| Antibody | Relative mean fluorescence intensity value | |
|---|---|---|
| | 100 nM | 25 nM |
| P3-C7 L7H1 | 27 | No binding |
| 1608 | 1714 | 970 |
| 1608-BsAb | 1352 | 613 |

The results show that the GUCY2C humanized monoclonal antibody of the present disclosure does not non-specifically bind to Jurkat cells that do not express GUCY2C; 1608 significantly binds to Jurkat cells.

Experiment 2: The flow cytometry was used to detect whether the GUCY2C humanized monoclonal antibody of the present disclosure non-specifically bound to human PBMCs and CD3+ T cells in PBMCs; 3E5 human peripheral blood cells resuspended 1% BSA were added to each well, and the supernatant was removed after centrifugation. Diluted antibody of the corresponding concentration was added, and the mixture was incubated at room temperature for 30 min and then washed once with 1% BSA. Then, a secondary antibody working solution of PE-Cy7 anti-human CD3 (BD#557851) and APC anti-human IgG Fc (BioLegend#409306) was added, and the mixture was resuspended and incubated at room temperature for 30 min in the dark. After the plate was washed twice with 1% BSA, fluorescence signals were read using a flow cytometer. The relative mean fluorescence intensity value was obtained by subtracting the background fluorescence intensity of the cells without incubated antibody from the mean fluorescence intensity of antibody binding. The results are shown in Table 32 below.

Table 32. Binding of GUCY2C humanized monoclonal antibodies to human PBMC

| Antibody | Relative mean fluorescence intensity value | | | |
|---|---|---|---|---|
| | PBMC | | CD3+ T cells | |
| P3-C7 L7H1 | 1 | No binding | 3 | No binding |
| 1608 | 560 | 541 | 503 | 499 |

The results show that the GUCY2C humanized monoclonal antibody of the present disclosure does not non-specifically bind to PBMCs and T cells that do not express GUCY2C, and the control antibody 1608 does significantly non-specifically bind to PBMCs and T cells. The target specificity of the GUCY2C humanized monoclonal antibody is better than that of 1608.

**Test Example 4. Epitope Analysis of GUCY2C Monoclonal Antibodies of the Present Disclosure**

To test the epitopes involved in the binding of the GUCY2C monoclonal antibodies of the present disclosure to an antigen, the ELISA assay was used to detect differences in the antibody epitopes to which the antibodies bound. The specific procedures are as follows:

The plate was coated with the monoclonal antibody tested (100 ng) and incubated overnight at 4 °C. After plate washing, the antibody was blocked with 1% BSA. Then, 50 ng of hGUCY2C ECD-His antigen was added, and the mixture was incubated at 37 °C for 1 h. The plate was washed, and then 100 ng of biotinylated antibody was added. The resulting mixture was incubated at 37 °C for 1 h, and finally the signals were detected by using HRP-streptavidin. Relative competition coefficient = (signal value of antibody self-competition/signal value of other antibody competition) × 100.

Two antibodies with identical epitopes have a competition coefficient of 100 in competitive binding, and the larger the difference between the binding epitopes, the smaller the competition coefficient between antibodies. Specific results are shown in Table 33 below:

Table 33. Competitive binding of GUCY2C monoclonal antibodies to antigen

| Competitive binding / Coating | 1608-biotinylated | P3-C7 L7H1-biotinylated | 81 L3H1-biotinylated | 146 L1H1-biotinylated |
|---|---|---|---|---|
| | 100ng | 100ng | 100ng | 100ng |
| 1608 | 100 | 3 | 6 | 12 |
| P3-C7 L7H1 | 10 | 100 | 6 | 13 |
| 81 L3H1 | 11 | 3 | 100 | 81 |
| 146 L1H1 | 12 | 4 | 99 | 100 |

The results show that the antigen epitopes bound by the GUCY2C monoclonal antibodies of the present disclosure are significantly different from that bound by 1608, and thus the GUCY2C monoclonal antibodies can not compete with 1608 for binding to the GUCY2C antigen.

**Test Example 5. Protein Binding Activity of GUCY2C-CD3 Bispecific Antibodies for Antigen**

In this test example, the binding activity of the GUCY2C-CD3 bispecific antibodies for human GUCY2C was detected by ELISA. The specific procedures are as follows:
The plate was coated by adding 100 ng of streptavidin (ACRO#STN-N5116) to the reaction wells and incubated overnight at 4 °C. After the blocking with 1% BSA (PBS), 100 ng of hGUCY2C ECD-His was added, and the mixture was incubated at 37 °C for 1 h. After plate washing, the gradiently diluted antibody was added, and the mixture was incubated at 37 °C for 1 h. Then, Peroxidase AffiniPure goat anti-human IgG (H+L) (Cat. #109-035--003, Jackson Immuno Research) was added, and the resulting mixture was incubated at 37 °C for 1 h. After plate washing, a substrate solution was added, and a stop solution was then added after sufficient color development to stop the reaction. The signal values at 450 nM were read using a microplate reader. The binding activity of the antibodies is shown in Table 34 below.

Table 34. Binding activity of GUCY2C-CD3 bispecific antibodies for human <u>GUCY2C protein</u> (absorbance values)

| Antibody | Top | $EC_{50}$ (nM) |
|---|---|---|
| P3-C7 L7H1-1 | 2.402 | 0.37 |
| 81 L3H1-2 | 2.334 | 0.12 |
| 146 L1H1-2 | 2.353 | 0.06 |
| 1608-BsAb | 2.153 | 1.23 |

The results show that the GUCY2C-CD3 bispecific antibodies of the present disclosure exhibit good binding activity for human GUCY2C protein.

**Test Example 6. Binding Activity of GUCY2C-CD3 Bispecific Antibodies for Cell Surface Antigen**

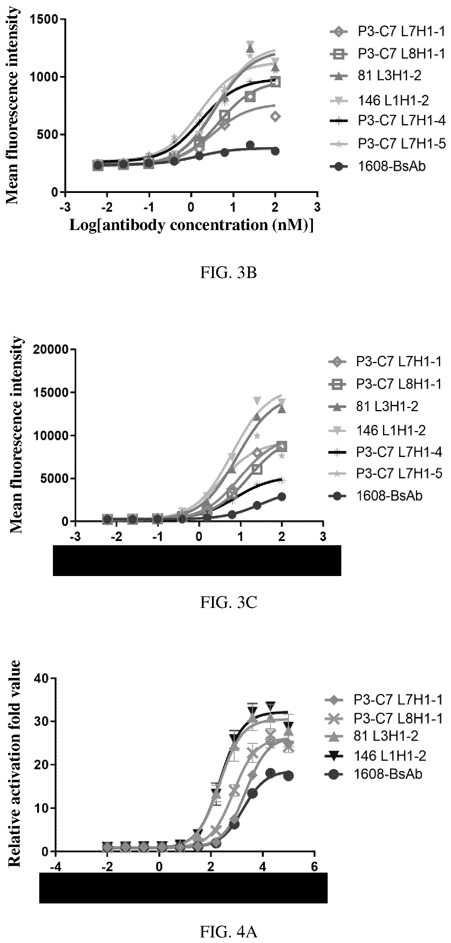

In this test example, the binding activity of GUCY2C-CD3 bispecific antibodies for the stably transfected cell strain HCT116/hGUCY2C and the intestinal cancer cells HT55 (Nanjing Cobioer, #CBP60012) overexpressing human GUCY2C and the Jurkat cells (ATCC#TIB-152) expressing CD3 was detected by flow cytometry. The expression levels of GUCY2C in HCT116/hGUCY2C cells and intestinal cancer cells HT55 were high expression (+++) and moderate expression (++), respectively. The specific procedures are as follows:
Experiment 1: 1E5 cells resuspended in 1% BSA were added to the reaction wells, and the supernatant was removed after centrifugation. Diluted antibody of the corresponding concentration was added, and the mixture was incubated at room temperature for 30 min and then washed twice with 1% BSA. Then, 1:400 diluted goat anti-human IgG($\gamma$) R-PE conjugate F(ab') secondary antibody (life technologies #H10104) was added, and the resulting mixture was resuspended and incubated at room temperature for 30 min in the dark. After the plate was washed twice with 1% BSA, fluorescence signals were read using a flow cytometer. Exemplary FACS results are shown in FIGs. 3A-3B.
The results show that the GUCY2C-CD3 bispecific antibodies of the present disclosure exhibit good specific binding activity for all the cells expressing GUCY2C at different levels, and the binding activity is better than that of 1608-BsAb.

Experiment 2: the same method as in experiment 1 was used. The binding activity of the antibodies for Jurkat cells is shown in FIG. 3C.

The results show that the GUCY2C-CD3 bispecific antibodies of the present disclosure exhibit good specific binding activity for Jurkat cells expressing CD3, and the binding activity is better than that of 1608-BsAb.

## Test Example 7. Activation of T Cells by GUCY2C-CD3 Bispecific Antibodies

To test the activation of T cells by the GUCY2C-CD3 bispecific antibodies of the present disclosure under the combined action of target cells HCT116/GUCY2C and LS1034 cells, Jurkat-Lucia™ NFAT (InvivoGen, jktl-nfat) was used for cell detection in this test example. The method is as follows:

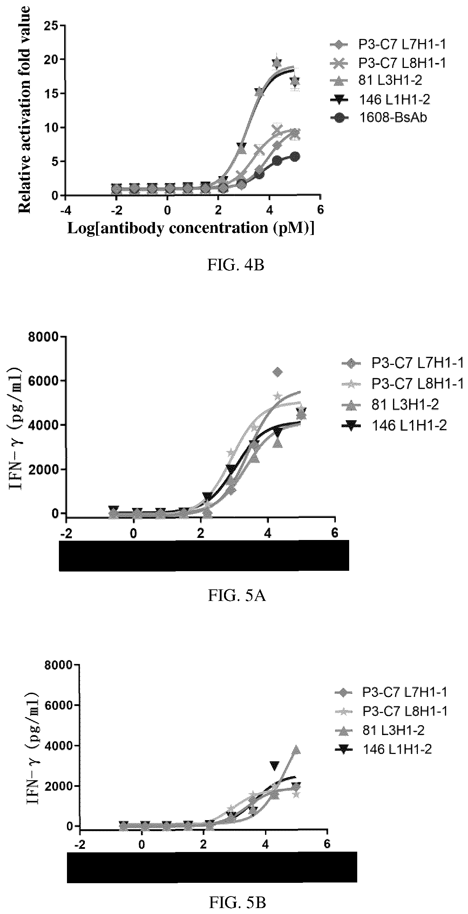

Jurkat Lucia™ NFAT cells and target cells were subcultured in complete medium and corresponding media, respectively. Jurkat-NFAT lum cells were collected by centrifugation, resuspended in the 1640+10% FBS medium and counted, and the number of cells was adjusted to 1E6 cells/mL; the target cells HCT116/GUCY2C and LS1034 were resuspended and counted, and then adjusted to 2E5 cells/mL and 4E5 cells/mL, respectively. The Jurkat Lucia™ NFAT and target cells described above were mixed in equal volume, and the mixture was added at 100 μL/well; the E:T ratios of the HCT116/GUCY2C and LS1034 cells were ensured to be 5:1 and 2.5:1, respectively. The antibody was diluted with 1640+10% FBS medium, and the diluted antibody was added at 20 μL/well. The mixture was left to stand at 37 °C for 5 h. The QUANTI-Luc™ Gold reagent was dissolved in 25 mL of ddH$_2$O as required by the instructions. After 50 μL of QUANTI-Luc™ Gold was added, the resulting mixture was incubated at room temperature for 5 min. The luminescence was detected by Vendor, and the activation of Jurkat Lucia™ NFAT cells by antibodies at different concentrations was calculated. A graph was fitted by using the relative fold value obtained by dividing the Lumin value after antibody action by the background Lumin value. The relative fold values and the EC$_{50}$ results are shown in FIGs. 4A-4B.

The results show that the GUCY2C-CD3 bispecific antibodies of the present disclosure exhibit good activation effect on T cells under the combined action with target cells, and the activation effect is better than that of 1608-BsAb.

## Test Example 8. Cytotoxic Activity of GUCY2C-CD3 Bispecific Antibodies of the Present Disclosure

In this test example, the killing activity of the GUCY2C-CD3 bispecific antibodies of the present disclosure as T cell engager molecules for tumor cells was investigated. The specific procedures are as follows:

The target-specific cytotoxic activity of the bispecific antibodies of the present disclosure was detected by using stably transfected LucG cell strains constructed with the intestinal cancer cell strains LS1034, HT-55, and LS174T (ATCC#CL-188) expressing GUCY2C as high-expression, moderate-expression, and low-expression target cells, respectively. Cryopreserved PBMCs (purchased from Milestone) were thawed, resuspended in 1640+10% FBS complete medium, and then incubated in a T75 culture flask for 4 h (density: 2E6 cells/mL). The PBMCs were collected, centrifuged, resuspended in 1640+10% FBS, and counted, and the number of cells was adjusted to 1.5E6 cells/mL. Target cells were collected, centrifuged at 1000 rpm for 3 min, resuspended, and counted, and the cell concentration was adjusted to 1.5E5 cells/mL. The PBMC suspension and the target cell suspension described above were mixed in equal volume, and the mixture was added at 100 μL/well; the E:T ratio was ensured to be 10:1. A PBMC ONLY group was set up separately; the PBMC suspension described above and the 1640+10% FBS complete medium were mixed in equal volume. The antibody was 5-fold diluted in 1640+10% FBS medium from an initial concentration of 600 nM (6× final concentration) to obtain 9 concentration points, and the diluted antibody was added at 20 μL/well. The treated cells were incubated in an incubator at 37 °C and 5% CO$_2$ for 48 h before the luminescence detection. Dose-response graphs were plotted based on the log concentrations and signal values of the antibody using Graphpad Prism 8.0 software and fitted to obtain the maximum inhibition (Imax) and IC$_{50}$ of the antibody-mediated killing. The maximum inhibition of the antibody was calculated by setting the inhibition to 0% for the wells containing only the target cells and PBMC but no antibody. The results are shown in Tables 35-1, 35-2, and 35-3 below.

Table 35-1. Cytotoxic activity of GUCY2C-CD3 bispecific antibodies for target cells

| Antibody | LS1034(+++) | | HT-55(++) | | LS174T(+) | |
|---|---|---|---|---|---|---|
| | Imax (%) | IC$_{50}$ (nM) | Imax (%) | IC$_{50}$ (nM) | Imax (%) | IC$_{50}$ (nM) |
| P3-C7 L7H1-1 | 87 | 0.75 | 39 | 0.85 | 65 | 0.75 |
| P3-C7 L8H1-1 | 86 | 0.17 | 41 | 0.60 | 62 | 0.33 |
| 81 L3H1-2 | 92 | 0.31 | 47 | 0.39 | 67 | 0.58 |

(continued)

| Antibody | LS1034(+++) | | HT-55(++) | | LS174T(+) | |
|---|---|---|---|---|---|---|
| | Imax (%) | IC$_{50}$ (nM) | Imax (%) | IC$_{50}$ (nM) | Imax (%) | IC$_{50}$ (nM) |
| 146 L1H1-2 | 87 | 0.17 | 46 | 0.56 | 69 | 0.49 |
| Note: + represents the expression level of GUCY2C, where the more the +, the higher the expression level of GUCY2C. | | | | | | |

Table 35-2. Cytotoxic activity of GUCY2C-CD3 bispecific antibodies for target cells

| Antibody | LS174T | |
|---|---|---|
| | Max (%) | IC50 (nM) |
| P3-C7 L7H1-3 | 92 | 0.49 |

Table 35-3. Cytotoxic activity of GUCY2C-CD3 bispecific antibodies for target cells

| Antibody | LS174T | |
|---|---|---|
| | Max (%) | IC$_{50}$ (nM) |
| 81 L3H1-2 (N103QN106Q) | 82 | 0.16 |

The results show that the GUCY2C-CD3 bispecific antibodies of the present disclosure exhibit good cytotoxic activity for target cells with different expression levels.

**Test Example 9. Effect of GUCY2C-CD3 Bispecific Antibodies of the Present Disclosure on Release of Cytokines**

In this test example, the changes in the secretion of cytokines IFN-γ and IL-6 in PBMCs under the combined action of the GUCY2C-CD3 bispecific antibody and target cells (LS1034 cells and LS174T cells in Test Example 8) were detected.

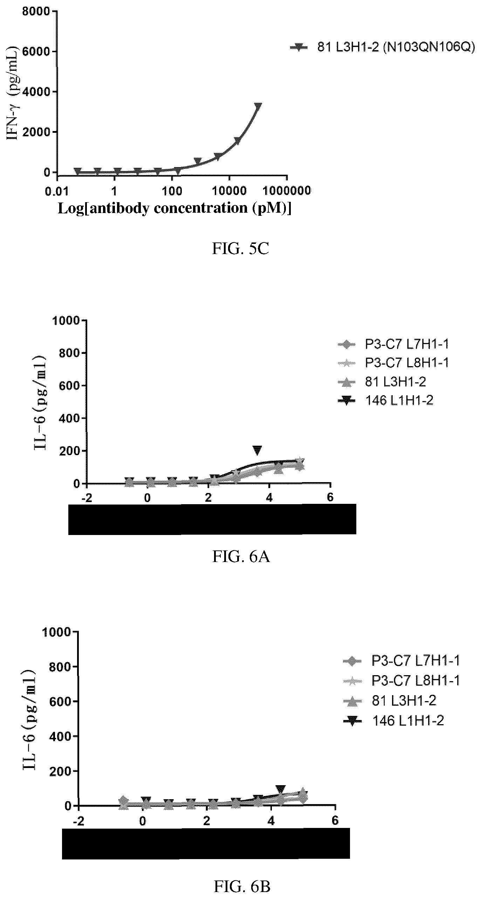

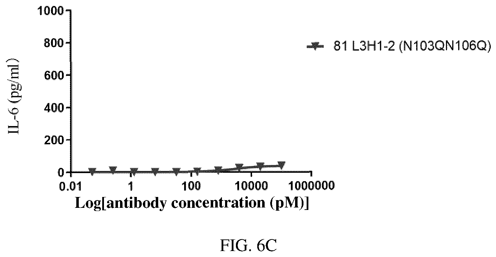

The supernatant collected in the cell killing experiment (Test Example 8) and cryopreserved in a freezer at -20 °C was thawed at room temperature and shaken for mixing well, and the sample was 20-fold diluted with the 1640+4% FBS medium for later use. Powders of IL-6 and IFN-γ standards were dissolved in sterile water and the standards were diluted to the desired concentration using the 1640+4% FBS medium. The kits for the cytokines to be detected, including a human IL6 ELISA kit (NeoBioscience, EHC007.96) and a human IFN-γ kit (CISBIO, 62HIFNGPEG), were taken out and equilibrated to normal temperature, and the detection was carried out according to the operations described in the instructions. The results are shown in FIGs. 5A-5C and FIGs. 6A-6C.

The results show that the GUCY2C-CD3 bispecific antibodies of the present disclosure are able to efficiently induce the release of IFN-γ associated with cytotoxic activity, and the release level of the cytokine IL-6 associated with inflammation is relatively low.

**Biological Evaluation of *In Vivo* Activity**

**Test Example 10. Efficacy of GUCY2C-CD3 Bispecific Antibodies of the Present Disclosure in LS1034 Subcutaneous Xenograft Tumor Model**

In the present disclosure, the *in vivo* efficacy of the GUCY2C-CD3 bispecific antibodies was evaluated using a human colon cancer cell LS1034 mouse subcutaneous xenograft tumor model.

Female NOG mice were purchased from Beijing Vital River (Shanghai branch). The mice were 6-8 weeks old when purchased. The human PBMCs used in this test example were purchased from Milestone.

LS1034 cells were subcutaneously inoculated into the right flank of the NOG mice at $1 \times 10^6$ cells/mouse/200 μL (containing 50% matrigel), and human PBMCs in good state after thawing were injected into the abdominal cavity of the NOG mice at $5 \times 10^6$ cells/mouse/100 μL the next day after inoculation. When the tumor volume of the tumor-bearing mice reached about 150 mm$^3$, the mice with overlarge volume and too small body weight were excluded, and the remaining mice were randomly divided into groups of 8. The day of grouping was defined as the day 0 of the experiment. From day 0, equimolar amounts of antibodies were each administered intraperitoneally twice weekly for a total of two to three weeks.

The tumor volume and body weight of animals were monitored twice a week, and data were recorded. The tumor-bearing animals were euthanized as the experimental endpoint when the tumor volume of the experimental animals exceeded 1000 mm$^3$ or when most tumors showed rupture or when the animals showed 20% of body weight loss.

The tumor volume (V) was calculated as follows: $V = 1/2 \times a \times b^2$, where a and b represent length and width, respectively.

$$\text{Relative tumor proliferation rate } T/C(\%) = (T - T_0)/(C - C_0) \times 100\%,$$

where T and C are the tumor volume at the end of the experiment in the treatment group and control group, respectively; $T_0$ and $C_0$ are the tumor volume at the beginning of the experiment.

$$\text{Tumor growth inhibition rate TGI } (\%) = 1 - T/C \ (\%).$$

Table 36-1. Anti-tumor activity of GUCY2C-CD3 bispecific antibodies

| Group | Day 0 | Day 21 | TGI (%) |
|---|---|---|---|
| | Mean±SEM (mm$^3$) | Mean±SEM (mm$^3$) | |
| Vehicle control | 140.5±9.7 | 1327.6±103.5 | - |
| P3-C7 L7H1-1 (0.3 mpk) | 140.5±11.8 | 123.2±15.7*** | > 100% |
| P3-C7 L7H1-1 (0.06 mpk) | 138.3±10.8 | 363.5±52*** | 81.03% |
| P3-C7 L7H1-4 (0.12 mpk) | 138.4±11.7 | 160.1±25.5*** | 98.17% |
| P3-C7 L7H1-5 (0.12 mpk) | 139.1±9.8 | 321.6±157.3*** | 84.63% |
| Note: *p < 0.05, **p < 0.01, ***p < 0.001 vs vehicle control, T-test. | | | |

Table 36-2. Anti-tumor activity of GUCY2C-CD3 bispecific antibodies

| Group | Day 0 | Day 21 | TGI (%) |
|---|---|---|---|
| | Mean±SEM (mm$^3$) | Mean±SEM (mm$^3$) | |
| Vehicle control | 159±15.3 | 921.7±80.4 | - |
| P3-C7 L8H1-1 (0.2 mpk) | 158.7±15.9 | 77.4±15.9*** | > 100% |
| P3-C7 L8H1-1 (0.05 mpk) | 158.4±15.0 | 422.4±96.3** | 65.39% |
| 81 L3H1-2 (0.3 mpk) | 158.4±13.7 | 94.5±8.26*** | > 100% |
| 81 L3H1-2 (0.075 mpk) | 160.8±15.2 | 135.2±10.4*** | > 100% |
| Note: *p < 0.05, **p < 0.01, ***p < 0.001 vs vehicle control, T-test. | | | |

The results show that the GUCY2C-CD3 bispecific antibodies of the present disclosure exhibit significant tumor inhibition effect on this model.

**Test Example 11. Efficacy of Bispecific Antibodies of the Present Disclosure in LS174T Subcutaneous Xenograft Tumor Model**

In the present disclosure, the *in vivo* efficacy of the GUCY2C-CD3 bispecific antibodies was evaluated using a human colon cancer cell LS 174T mouse subcutaneous xenograft tumor model.

Female NOG mice were purchased from Beijing Vital River (Shanghai branch). The mice were 6-8 weeks old when purchased. The human PBMCs used in this test example were purchased from Milestone.

A mixture of LS174T cells ($1 \times 10^6$ cells/mouse/100 μL) and thawed PBMCs after cryopreservation (E:T =1:1) was inoculated subcutaneously into the right flank of NOG mice. When the tumor volume of tumor-bearing mice reached about 150 mm$^3$, mice with too large or too small tumor volume and with too small body weight were excluded, and the remaining mice were randomly divided into groups of 8. The day of grouping was defined as day 0 of the experiment, and each antibody was intraperitoneally injected on days 0, 3, 7, and 10 for a total of 4 times. The tumor volume and body weight of animals were monitored twice a week, and data were recorded. The tumor-bearing animals were euthanized as the experimental endpoint when the tumor volume of the experimental animals exceeded 1000 mm$^3$ or when most tumors showed rupture or when the animals showed 20% of body weight loss.

Table 37-1. Anti-tumor activity of GUCY2C-CD3 bispecific antibodies

| Group | Day 0 | Day 14 |
| --- | --- | --- |
| | Mean±SEM (mm$^3$) | Mean±SEM (mm$^3$) |
| Vehicle control | 193.4±21.9 | - |
| P3-C7 L7H1-1 (0.5 mpk) | 193.7±20.9 | 481.5±76.5 |
| 1608-BsAb (0.5 mpk) | 194.4±23.5 | 809.9±227 |
| Note: *p < 0.05, **p < 0.01, ***p < 0.001 vs vehicle control, T-test. | | |

Table 37-2. Anti-tumor activity of GUCY2C-CD3 bispecific antibodies

| Group | Day 0 | Day13 |
| --- | --- | --- |
| | Mean±SEM (mm$^3$) | Mean ± SEM (mm$^3$) |
| Vehicle control | 181.0±13.8 | 1506.3±237.3 |
| 81 L3H1-2 (0.75 mpk) | 180.2±15.7 | 377.2±70.5*** |
| 81 L3H1-2 (0.375 mpk) | 181.1±14.7 | 346.2±63.8*** |
| 81 L3H1-2 (0.188 mpk) | 181.1±14.9 | 415.1±92.5*** |
| Note: *p < 0.05, **p < 0.01, ***p < 0.001 vs vehicle control, T-test. | | |

The results show that the GUCY2C-CD3 bispecific antibodies of the present disclosure exhibit significant tumor inhibition effect on this model. During the experiment, the tumor weight was basically consistent with the trend of tumor volume.

**Test Example 12. Anti-Tumor Activity of Combined Use of GUCY2C-CD3 Bispecific Antibody and EGFR-CD28 Bispecific Antibody**

In the present disclosure, the anti-tumor activity of the combined used of the GUCY2C-CD3 bispecific antibody and the EGFR-CD28 bispecific antibody (97H2L3OT-ZAL, prepared with reference to patent No. CN202210371538.8) was studied using the LS174T subcutaneous tumor model.

Female NOG mice were purchased from Beijing Vital River (Shanghai branch). The mice were 6-8 weeks old when purchased. The human PBMCs used in this test example were purchased from Milestone.

A mixture of LS174T cells ($1 \times 10^6$ cells/mouse/100 µL) and thawed PBMCs after cryopreservation (E:T =1:1) was inoculated subcutaneously into the right flank of NOG mice. When the tumor volume of tumor-bearing mice reached about 150 mm$^3$, mice with too large or too small tumor volume and with too small body weight were excluded, and the remaining mice were randomly divided into groups of 8. The day of grouping was defined as day 0 of the experiment, and each antibody was intraperitoneally injected on days 0, 3, 7, and 11 for a total of 4 times. For the combined use group, the drugs were administered simultaneously after being formulated separately. The tumor volume and body weight of animals were monitored twice a week, and data were recorded. The tumor-bearing animals were euthanized as the experimental endpoint when the tumor volume of the experimental animals exceeded 1000 mm$^3$ or when most tumors showed rupture or when the animals showed 20% of body weight loss. The results are shown in Table 38 below.

Table 38. Anti-tumor activity of combined use of GUCY2C-CD3 bispecific antibody and EGFR-CD28 bispecific antibody

| Group | Day 0 | Day13 | TGI (%) |
| --- | --- | --- | --- |
| | Mean±SEM (mm$^3$) | Mean±SEM (mm$^3$) | |
| Vehicle control | 181.0±13.8 | 1506.3±237.3 | - |
| P3-C7 L7H1-1 (0.25 mpk) | 181.4±15.7 | 520.5±91.3** | 74.41% |

(continued)

| Group | Day 0 | Day13 | TGI (%) |
| --- | --- | --- | --- |
| | Mean±SEM (mm³) | Mean±SEM (mm³) | |
| 81 L3H1-2 (0.188 mpk) | 181.1±14.9 | 415.1±92.5*** | 82.34% |
| 97H2L3OT-ZAL (2.5 mpk) | 180.8±13.7 | 742.3±104.9* | 57.63% |
| P3-C7 L7H1-1 (0.25 mpk) + 97H2L3OT-ZAL (2.5 mpk) | 180.5±12.3 | 241.3±95.2*** | 95.41% |
| 81 L3H1-2 (0.188 mpk) + 97H2L3OT-ZAL (2.5 mpk) | 181.0±12.2 | 195.5±34.4*** | 98.91 % |
| Note: *p < 0.05, **p < 0.01, ***p < 0.001 vs vehicle control, T-test. | | | |

The results show that the combined use of the GUCY2C-CD3 bispecific antibody and the EGFR-CD28 bispecific antibody of the present disclosure can effectively improve the respective drug effect of the two antibodies.

Although the foregoing invention has been described in detail by way of drawings and examples for purposes of clarity of understanding, the description and examples should not be construed as limiting the scope of the present disclosure. The disclosures of all the patents and scientific literature cited herein are clearly incorporated by reference in their entireties.

## Claims

1. An antigen-binding molecule that specifically binds to GUCY2C and CD3, comprising at least one antigen-binding moiety that specifically binds to GUCY2C and at least one antigen-binding moiety that specifically binds to CD3, wherein the antigen-binding moiety that specifically binds to GUCY2C comprises a heavy chain variable region GUCY2C-VH and a light chain variable region GUCY2C-VL, and the antigen-binding moiety that specifically binds to CD3 comprises a heavy chain variable region CD3-VH and a light chain variable region CD3-VL; wherein:

   i) the GUCY2C-VH comprises: a GUCY2C-HCDR1 comprising the amino acid sequence of SEQ ID NO: 19, a GUCY2C-HCDR2 comprising the amino acid sequence of SEQ ID NO: 20, and a GUCY2C-HCDR3 comprising the amino acid sequence of SEQ ID NO: 21; and the GUCY2C-VL comprises: a GUCY2C-LCDR1 comprising the amino acid sequence of SEQ ID NO: 47, 22, 43, 44, 45, or 46, a GUCY2C-LCDR2 comprising the amino acid sequence of SEQ ID NO: 23, and a GUCY2C-LCDR3 comprising the amino acid sequence of SEQ ID NO: 24; or

   ii) the GUCY2C-VH comprises: a GUCY2C-HCDR1 comprising the amino acid sequence of SEQ ID NO: 4, a GUCY2C-HCDR2 comprising the amino acid sequence of SEQ ID NO: 5, and a GUCY2C-HCDR3 comprising the amino acid sequence of SEQ ID NO: 27 or 6; and the GUCY2C-VL comprises: a GUCY2C-LCDR1 comprising the amino acid sequence of SEQ ID NO: 7, a GUCY2C-LCDR2 comprising the amino acid sequence of SEQ ID NO: 8, 28, 29, or 30, and a GUCY2C-LCDR3 comprising the amino acid sequence of SEQ ID NO: 9; or

   iii) the GUCY2C-VH comprises: a GUCY2C-HCDR1 comprising the amino acid sequence of SEQ ID NO: 10, a GUCY2C-HCDR2 comprising the amino acid sequence of SEQ ID NO: 11, and a GUCY2C-HCDR3 comprising the amino acid sequence of SEQ ID NO: 40 or 12; and the GUCY2C-VL comprises: a GUCY2C-LCDR1 comprising the amino acid sequence of SEQ ID NO: 13, a GUCY2C-LCDR2 comprising the amino acid sequence of SEQ ID NO: 39 or 8, and a GUCY2C-LCDR3 comprising the amino acid sequence of SEQ ID NO: 14; or

   iv-1) the GUCY2C-VH comprises: a GUCY2C-HCDR1 comprising the amino acid sequence of SEQ ID NO: 250, a GUCY2C-HCDR2 comprising the amino acid sequence of SEQ ID NO: 251, and a GUCY2C-HCDR3 comprising the amino acid sequence of SEQ ID NO: 252; and the GUCY2C-VL comprises: a GUCY2C-LCDR1 comprising the amino acid sequence of SEQ ID NO: 253, a GUCY2C-LCDR2 comprising the amino acid sequence of SEQ ID NO: 254, and a GUCY2C-LCDR3 comprising the amino acid sequence of SEQ ID NO: 255; or

   iv-2) the GUCY2C-VH comprises: a GUCY2C-HCDR1 comprising the amino acid sequence of SEQ ID NO: 256, a GUCY2C-HCDR2 comprising the amino acid sequence of SEQ ID NO: 257, and a GUCY2C-HCDR3 comprising the amino acid sequence of SEQ ID NO: 258; and the GUCY2C-VL comprises: a GUCY2C-LCDR1 comprising the amino acid sequence of SEQ ID NO: 259, a GUCY2C-LCDR2 comprising the amino acid sequence of SEQ ID NO: 8, and a GUCY2C-LCDR3 comprising the amino acid sequence of SEQ ID NO: 260; or

   iv-3) the GUCY2C-VH comprises: a GUCY2C-HCDR1 comprising the amino acid sequence of SEQ ID NO: 261, a GUCY2C-HCDR2 comprising the amino acid sequence of SEQ ID NO: 262, and a GUCY2C-HCDR3 comprising the amino acid sequence of SEQ ID NO: 263; and the GUCY2C-VL comprises: a GUCY2C-LCDR1 comprising the amino acid sequence of SEQ ID NO: 264, a GUCY2C-LCDR2 comprising the amino acid sequence of SEQ ID NO: 265, and a GUCY2C-LCDR3 comprising the amino acid sequence of SEQ ID NO: 266; or

iv-4) the GUCY2C-VH comprises: a GUCY2C-HCDR1 comprising the amino acid sequence of SEQ ID NO: 267, a GUCY2C-HCDR2 comprising the amino acid sequence of SEQ ID NO: 268, and a GUCY2C-HCDR3 comprising the amino acid sequence of SEQ ID NO: 269; and the GUCY2C-VL comprises: a GUCY2C-LCDR1 comprising the amino acid sequence of SEQ ID NO: 270, a GUCY2C-LCDR2 comprising the amino acid sequence of SEQ ID NO: 271, and a GUCY2C-LCDR3 comprising the amino acid sequence of SEQ ID NO: 272; or

iv-5) the GUCY2C-VH comprises: a GUCY2C-HCDR1 comprising the amino acid sequence of SEQ ID NO: 281, a GUCY2C-HCDR2 comprising the amino acid sequence of SEQ ID NO: 282, and a GUCY2C-HCDR3 comprising the amino acid sequence of SEQ ID NO: 283; and the GUCY2C-VL comprises: a GUCY2C-LCDR1 comprising the amino acid sequence of SEQ ID NO: 284, a GUCY2C-LCDR2 comprising the amino acid sequence of SEQ ID NO: 8, and a GUCY2C-LCDR3 comprising the amino acid sequence of SEQ ID NO: 285; or

iv-6) the GUCY2C-VH comprises: a GUCY2C-HCDR1 comprising the amino acid sequence of SEQ ID NO: 286, a GUCY2C-HCDR2 comprising the amino acid sequence of SEQ ID NO: 287, and a GUCY2C-HCDR3 comprising the amino acid sequence of SEQ ID NO: 288; and the GUCY2C-VL comprises: a GUCY2C-LCDR1 comprising the amino acid sequence of SEQ ID NO: 289, a GUCY2C-LCDR2 comprising the amino acid sequence of SEQ ID NO: 265, and a GUCY2C-LCDR3 comprising the amino acid sequence of SEQ ID NO: 290; or

iv-7) the GUCY2C-VH comprises: a GUCY2C-HCDR1 comprising the amino acid sequence of SEQ ID NO: 291, a GUCY2C-HCDR2 comprising the amino acid sequence of SEQ ID NO: 292, and a GUCY2C-HCDR3 comprising the amino acid sequence of SEQ ID NO: 293; and the GUCY2C-VL comprises: a GUCY2C-LCDR1 comprising the amino acid sequence of SEQ ID NO: 294, a GUCY2C-LCDR2 comprising the amino acid sequence of SEQ ID NO: 295, and a GUCY2C-LCDR3 comprising the amino acid sequence of SEQ ID NO: 296; or

iv-8) the GUCY2C-VH comprises: a GUCY2C-HCDR1 comprising the amino acid sequence of SEQ ID NO: 261, a GUCY2C-HCDR2 comprising the amino acid sequence of SEQ ID NO: 297, and a GUCY2C-HCDR3 comprising the amino acid sequence of SEQ ID NO: 298; and the GUCY2C-VL comprises: a GUCY2C-LCDR1 comprising the amino acid sequence of SEQ ID NO: 264, a GUCY2C-LCDR2 comprising the amino acid sequence of SEQ ID NO: 299, and a GUCY2C-LCDR3 comprising the amino acid sequence of SEQ ID NO: 300; or

iv-9) the GUCY2C-VH comprises: a GUCY2C-HCDR1 comprising the amino acid sequence of SEQ ID NO: 261, a GUCY2C-HCDR2 comprising the amino acid sequence of SEQ ID NO: 301, and a GUCY2C-HCDR3 comprising the amino acid sequence of SEQ ID NO: 302; and the GUCY2C-VL comprises: a GUCY2C-LCDR1 comprising the amino acid sequence of SEQ ID NO: 303, a GUCY2C-LCDR2 comprising the amino acid sequence of SEQ ID NO: 299, and a GUCY2C-LCDR3 comprising the amino acid sequence of SEQ ID NO: 304; or

iv-10) the GUCY2C-VH comprises: a GUCY2C-HCDR1 comprising the amino acid sequence of SEQ ID NO: 305, a GUCY2C-HCDR2 comprising the amino acid sequence of SEQ ID NO: 306, and a GUCY2C-HCDR3 comprising the amino acid sequence of SEQ ID NO: 21; and the GUCY2C-VL comprises: a GUCY2C-LCDR1 comprising the amino acid sequence of SEQ ID NO: 22, a GUCY2C-LCDR2 comprising the amino acid sequence of SEQ ID NO: 23, and a GUCY2C-LCDR3 comprising the amino acid sequence of SEQ ID NO: 24; or

iv-11) the GUCY2C-VH comprises: a GUCY2C-HCDR1 comprising the amino acid sequence of SEQ ID NO: 128, a GUCY2C-HCDR2 comprising the amino acid sequence of SEQ ID NO: 307, and a GUCY2C-HCDR3 comprising the amino acid sequence of SEQ ID NO: 308; and the GUCY2C-VL comprises: a GUCY2C-LCDR1 comprising the amino acid sequence of SEQ ID NO: 309, a GUCY2C-LCDR2 comprising the amino acid sequence of SEQ ID NO: 310, and a GUCY2C-LCDR3 comprising the amino acid sequence of SEQ ID NO: 311; or

iv-12) the GUCY2C-VH comprises: a GUCY2C-HCDR1 comprising the amino acid sequence of SEQ ID NO: 312, a GUCY2C-HCDR2 comprising the amino acid sequence of SEQ ID NO: 313, and a GUCY2C-HCDR3 comprising the amino acid sequence of SEQ ID NO: 314; and the GUCY2C-VL comprises: a GUCY2C-LCDR1 comprising the amino acid sequence of SEQ ID NO: 22, a GUCY2C-LCDR2 comprising the amino acid sequence of SEQ ID NO: 23, and a GUCY2C-LCDR3 comprising the amino acid sequence of SEQ ID NO: 315; or

iv-13) the GUCY2C-VH comprises: a GUCY2C-HCDR1 comprising the amino acid sequence of SEQ ID NO: 286, a GUCY2C-HCDR2 comprising the amino acid sequence of SEQ ID NO: 287, and a GUCY2C-HCDR3 comprising the amino acid sequence of SEQ ID NO: 316; and the GUCY2C-VL comprises: a GUCY2C-LCDR1 comprising the amino acid sequence of SEQ ID NO: 317, a GUCY2C-LCDR2 comprising the amino acid sequence of SEQ ID NO: 265, and a GUCY2C-LCDR3 comprising the amino acid sequence of SEQ ID NO: 300; or

iv-14) the GUCY2C-VH comprises: a GUCY2C-HCDR1 comprising the amino acid sequence of SEQ ID NO: 318, a GUCY2C-HCDR2 comprising the amino acid sequence of SEQ ID NO: 319, and a GUCY2C-HCDR3 comprising the amino acid sequence of SEQ ID NO: 320; and the GUCY2C-VL comprises: a GUCY2C-LCDR1 comprising the amino acid sequence of SEQ ID NO: 321, a GUCY2C-LCDR2 comprising the amino acid sequence of SEQ ID NO: 322, and a GUCY2C-LCDR3 comprising the amino acid sequence of SEQ ID NO: 323;

or

iv-15) the GUCY2C-VH comprises: a GUCY2C-HCDR1 comprising the amino acid sequence of SEQ ID NO: 324, a GUCY2C-HCDR2 comprising the amino acid sequence of SEQ ID NO: 325, and a GUCY2C-HCDR3 comprising the amino acid sequence of SEQ ID NO: 326; and the GUCY2C-VL comprises: a GUCY2C-LCDR1 comprising the amino acid sequence of SEQ ID NO: 22, a GUCY2C-LCDR2 comprising the amino acid sequence of SEQ ID NO: 23, and a GUCY2C-LCDR3 comprising the amino acid sequence of SEQ ID NO: 327; or
iv-16) the GUCY2C-VH comprises: a GUCY2C-HCDR1 comprising the amino acid sequence of SEQ ID NO: 261, a GUCY2C-HCDR2 comprising the amino acid sequence of SEQ ID NO: 328, and a GUCY2C-HCDR3 comprising the amino acid sequence of SEQ ID NO: 302; and the GUCY2C-VL comprises: a GUCY2C-LCDR1 comprising the amino acid sequence of SEQ ID NO: 264, a GUCY2C-LCDR2 comprising the amino acid sequence of SEQ ID NO: 329, and a GUCY2C-LCDR3 comprising the amino acid sequence of SEQ ID NO: 300;

preferably,

i) the GUCY2C-VH comprises: a GUCY2C-HCDR1 comprising the amino acid sequence of SEQ ID NO: 19, a GUCY2C-HCDR2 comprising the amino acid sequence of SEQ ID NO: 20, and a GUCY2C-HCDR3 comprising the amino acid sequence of SEQ ID NO: 21; and the GUCY2C-VL comprises: a GUCY2C-LCDR1 comprising the amino acid sequence of SEQ ID NO: 47, a GUCY2C-LCDR2 comprising the amino acid sequence of SEQ ID NO: 23, and a GUCY2C-LCDR3 comprising the amino acid sequence of SEQ ID NO: 24; or
ii) the GUCY2C-VH comprises: a GUCY2C-HCDR1 comprising the amino acid sequence of SEQ ID NO: 4, a GUCY2C-HCDR2 comprising the amino acid sequence of SEQ ID NO: 5, and a GUCY2C-HCDR3 comprising the amino acid sequence of SEQ ID NO: 27; and the GUCY2C-VL comprises: a GUCY2C-LCDR1 comprising the amino acid sequence of SEQ ID NO: 7, a GUCY2C-LCDR2 comprising the amino acid sequence of SEQ ID NO: 8, and a GUCY2C-LCDR3 comprising the amino acid sequence of SEQ ID NO: 9; or
iii) the GUCY2C-VH comprises: a GUCY2C-HCDR1 comprising the amino acid sequence of SEQ ID NO: 10, a GUCY2C-HCDR2 comprising the amino acid sequence of SEQ ID NO: 11, and a GUCY2C-HCDR3 comprising the amino acid sequence of SEQ ID NO: 40; and the GUCY2C-VL comprises: a GUCY2C-LCDR1 comprising the amino acid sequence of SEQ ID NO: 13, a GUCY2C-LCDR2 comprising the amino acid sequence of SEQ ID NO: 39, and a GUCY2C-LCDR3 comprising the amino acid sequence of SEQ ID NO: 14.

2. The antigen-binding molecule according to claim 1, wherein:

the CD3-VH comprises: a CD3-HCDR1 comprising the amino acid sequence of SEQ ID NO: 71, a CD3-HCDR2 comprising the amino acid sequence of SEQ ID NO: 72, and a CD3-HCDR3 comprising the amino acid sequence of SEQ ID NO: 73 or 88; and the CD3-VL comprises: a CD3-LCDR1 comprising the amino acid sequence of SEQ ID NO: 74, a CD3-LCDR2 comprising the amino acid sequence of SEQ ID NO: 75, and a CD3-LCDR3 comprising the amino acid sequence of SEQ ID NO: 76;
preferably, the CD3-VH comprises: a CD3-HCDR1 comprising the amino acid sequence of SEQ ID NO: 71, a CD3-HCDR2 comprising the amino acid sequence of SEQ ID NO: 72, and a CD3-HCDR3 comprising the amino acid sequence of SEQ ID NO: 73; and the CD3-VL comprises: a CD3-LCDR1 comprising the amino acid sequence of SEQ ID NO: 74, a CD3-LCDR2 comprising the amino acid sequence of SEQ ID NO: 75, and a CD3-LCDR3 comprising the amino acid sequence of SEQ ID NO: 76.

3. The antigen-binding molecule according to claim 1 or 2, wherein:

i) the GUCY2C-VH comprises the amino acid sequence of SEQ ID NO: 56, 25, or 57, and the GUCY2C-VL comprises the amino acid sequence of SEQ ID NO: 54, 55, 26, 48, 49, 50, 51, 52, or 53; or
ii) the GUCY2C-VH comprises the amino acid sequence of SEQ ID NO: 37, 15, or 38, and the GUCY2C-VL comprises the amino acid sequence of SEQ ID NO: 33, 16, 31, 32, 34, 35, or 36; or
iii) the GUCY2C-VH comprises the amino acid sequence of SEQ ID NO: 42 or 17, and the GUCY2C-VL comprises the amino acid sequence of SEQ ID NO: 41 or 18;

preferably,

i) the GUCY2C-VH comprises the amino acid sequence of SEQ ID NO: 56, and the GUCY2C-VL comprises the amino acid sequence of SEQ ID NO: 54, 55, 48, 49, 50, 51, or 52, or
the GUCY2C-VH comprises the amino acid sequence of SEQ ID NO: 25, and the GUCY2C-VL comprises the amino acid sequence of SEQ ID NO: 26, or

ii) the GUCY2C-VH comprises the amino acid sequence of SEQ ID NO: 37, and the GUCY2C-VL comprises the amino acid sequence of SEQ ID NO: 33, or
the GUCY2C-VH comprises the amino acid sequence of SEQ ID NO: 38, and the GUCY2C-VL comprises the amino acid sequence of SEQ ID NO: 33, 32, 34, or 36, or the GUCY2C-VH comprises the amino acid sequence of SEQ ID NO: 15, and the GUCY2C-VL comprises the amino acid sequence of SEQ ID NO: 16, or
iii) the GUCY2C-VH comprises the amino acid sequence of SEQ ID NO: 42, and the GUCY2C-VL comprises the amino acid sequence of SEQ ID NO: 41, or
the GUCY2C-VH comprises the amino acid sequence of SEQ ID NO: 17, and the GUCY2C-VL comprises the amino acid sequence of SEQ ID NO: 18, or

more preferably:

i) the GUCY2C-VH comprises the amino acid sequence of SEQ ID NO: 56, and the GUCY2C-VL comprises the amino acid sequence of SEQ ID NO: 54, or
the GUCY2C-VH comprises the amino acid sequence of SEQ ID NO: 56, and the GUCY2C-VL comprises the amino acid sequence of SEQ ID NO: 55; or
ii) the GUCY2C-VH comprises the amino acid sequence of SEQ ID NO: 37, and the GUCY2C-VL comprises the amino acid sequence of SEQ ID NO: 33; or
iii) the GUCY2C-VH comprises the amino acid sequence of SEQ ID NO: 42, and the GUCY2C-VL comprises the amino acid sequence of SEQ ID NO: 41.

4. The antigen-binding molecule according to any one of claims 1-3, wherein:

the CD3-VH comprises the amino acid sequence of SEQ ID NO: 77 or 89, and the CD3-VL comprises the amino acid sequence of SEQ ID NO: 78;
preferably,
the CD3-VH comprises the amino acid sequence of SEQ ID NO: 77, and the CD3-VL comprises the amino acid sequence of SEQ ID NO: 78.

5. The antigen-binding molecule according to any one of claims 1-4, wherein the antigen-binding moiety that specifically binds to GUCY2C or the antigen-binding moiety that specifically binds to CD3 comprises a Titin chain and an Obscurin chain capable of forming a dimer;
preferably,

the Titin chain comprises an amino acid sequence selected from the group consisting of SEQ ID NO: 182 to SEQ ID NO: 200, and the Obscurin chain comprises an amino acid sequence selected from the group consisting of SEQ ID NO: 201 to SEQ ID NO: 241;
more preferably,
the Titin chain comprises the amino acid sequence of SEQ ID NO: 198, and the Obscurin chain comprises the amino acid sequence of SEQ ID NO: 236.

6. The antigen-binding molecule according to any one of claims 1-5, comprising an Fc region, wherein the Fc region is preferably an IgG Fc region, and the Fc region is further preferably an IgG1 Fc region;

more preferably, the Fc region comprises one or more amino acid substitutions capable of reducing the binding of the Fc region to an Fcγ receptor;
most preferably, the Fc region is a human IgG1 Fc region, and the amino acids at positions 234 and 235 are A, as numbered according to the EU index.

7. The antigen-binding molecule according to any one of claims 1-6, comprising an Fc region, wherein the Fc region comprises a first subunit Fc1 and a second subunit Fc2 capable of associating with each other, and the Fc1 and the Fc2 each independently have one or more amino acid substitutions that reduce homodimerization of the Fc region;

preferably, the Fc1 has a knob structure according to the knob-into-hole technique, and the Fc2 has a hole structure according to the knob-into-hole technique;
more preferably, for the Fc1, the amino acid at position 366 is W, and for the Fc2, the amino acid at position 366 is S, the amino acid at position 368 is A, and the amino acid at position 407 is V, as numbered according to the EU index;

most preferably, the Fc1 comprises the amino acid sequence of SEQ ID NO: 79 or 85; the Fc2 comprises the amino acid sequence of SEQ ID NO: 80 or 86.

8. The antigen-binding molecule according to any one of claims 1-4, comprising one antigen-binding moiety that specifically binds to GUCY2C and one antigen-binding moiety that specifically binds to CD3, the antigen-binding moieties that specifically bind to GUCY2C and CD3 being both scFvs, wherein preferably,

> the antigen-binding molecule comprises one first chain having a structure represented by formula (a) and one second chain having a structure represented by formula (b):

>> (a) [GUCY2C-VL]-[linker 1]-[CD3-VH]-[linker 2]-[Fc1], and
>> (b) [CD3-VL]-[linker 3]-[GUCY2C-VH]-[linker 2]-[Fc2],

> wherein the linker 1, the linker 2, and the linker 3 are identical or different peptide linkers; or the linker 1, the linker 2, and the linker 3 are absent;
> the structures represented by formulas (a) and (b) are arranged from N-terminus to C-terminus;
> more preferably,

>> i) the GUCY2C-VH comprises the amino acid sequence of SEQ ID NO: 56, and the GUCY2C-VL comprises the amino acid sequence of SEQ ID NO: 54; and the CD3-VH comprises the amino acid sequence of SEQ ID NO: 77, and the CD3-VL comprises the amino acid sequence of SEQ ID NO: 78; or
>> the GUCY2C-VH comprises the amino acid sequence of SEQ ID NO: 56, and the GUCY2C-VL comprises the amino acid sequence of SEQ ID NO: 55; and the CD3-VH comprises the amino acid sequence of SEQ ID NO: 77, and the CD3-VL comprises the amino acid sequence of SEQ ID NO: 78; or
>> ii) the GUCY2C-VH comprises the amino acid sequence of SEQ ID NO: 15, and the GUCY2C-VL comprises the amino acid sequence of SEQ ID NO: 16; and the CD3-VH comprises the amino acid sequence of SEQ ID NO: 77, and the CD3-VL comprises the amino acid sequence of SEQ ID NO: 78; or
>> iii) the GUCY2C-VH comprises the amino acid sequence of SEQ ID NO: 17, and the GUCY2C-VL comprises the amino acid sequence of SEQ ID NO: 18; and the CD3-VH comprises the amino acid sequence of SEQ ID NO: 77, and the CD3-VL comprises the amino acid sequence of SEQ ID NO: 78;

> most preferably,

>> i) the antigen-binding molecule comprises:

>>> one first chain comprising the amino acid sequence of SEQ ID NO: 99 and one second chain comprising the amino acid sequence of SEQ ID NO: 100; or
>>> one first chain comprising the amino acid sequence of SEQ ID NO: 101 and one second chain comprising the amino acid sequence of SEQ ID NO: 100; or

>> ii) the antigen-binding molecule comprises:
>> one first chain comprising the amino acid sequence of SEQ ID NO: 102 and one second chain comprising the amino acid sequence of SEQ ID NO: 103; or
>> iii) the antigen-binding molecule comprises:
>> one first chain comprising the amino acid sequence of SEQ ID NO: 104 and one second chain comprising the amino acid sequence of SEQ ID NO: 105.

9. The antigen-binding molecule according to claim 7, comprising one antigen-binding moiety that specifically binds to CD3 and one antigen-binding moiety that specifically binds to GUCY2C, the antigen-binding moiety that specifically binds to CD3 being a Fab, and the antigen-binding moiety that specifically binds to GUCY2C being a replaced Fab comprising a Titin chain and an Obscurin chain capable of forming a dimer, wherein preferably,

> the antigen-binding molecule comprises one first chain having a structure represented by formula (c), one second chain having a structure represented by formula (d), one third chain having a structure represented by formula (e), and one fourth chain having a structure represented by formula (f):

(c) [CD3-VH]-[CH1]-[Fc1],
(d) [CD3-VL]-[CL],
(e) [GUCY2C-VH]-[linker 4]-[Obscurin chain]-[Fc2], and
(f) [GUCY2C-VL]-[linker 4]-[Titin chain],

wherein the linker 4 is a peptide linker; or the linker 4 is absent;
the structures represented by formulas (c), (d), (e), and (f) are arranged from N-terminus to C-terminus;
more preferably,

i) the CD3-VH comprises the amino acid sequence of SEQ ID NO: 77, and the CD3-VL comprises the amino acid sequence of SEQ ID NO: 78; and the GUCY2C-VH comprises the amino acid sequence of SEQ ID NO: 37, and the GUCY2C-VL comprises the amino acid sequence of SEQ ID NO: 33; or

the CD3-VH comprises the amino acid sequence of SEQ ID NO: 89, and the CD3-VL comprises the amino acid sequence of SEQ ID NO: 78; and the GUCY2C-VH comprises the amino acid sequence of SEQ ID NO: 37, and the GUCY2C-VL comprises the amino acid sequence of SEQ ID NO: 33; or
the CD3-VH comprises the amino acid sequence of SEQ ID NO: 77, and the CD3-VL comprises the amino acid sequence of SEQ ID NO: 78; and the GUCY2C-VH comprises the amino acid sequence of SEQ ID NO: 15, and the GUCY2C-VL comprises the amino acid sequence of SEQ ID NO: 16; or
the CD3-VH comprises the amino acid sequence of SEQ ID NO: 77, and the CD3-VL comprises the amino acid sequence of SEQ ID NO: 78; and the GUCY2C-VH comprises the amino acid sequence of SEQ ID NO: 37, and the GUCY2C-VL comprises the amino acid sequence of SEQ ID NO: 31; or
the CD3-VH comprises the amino acid sequence of SEQ ID NO: 77, and the CD3-VL comprises the amino acid sequence of SEQ ID NO: 78; and the GUCY2C-VH comprises the amino acid sequence of SEQ ID NO: 37, and the GUCY2C-VL comprises the amino acid sequence of SEQ ID NO: 32; or
the CD3-VH comprises the amino acid sequence of SEQ ID NO: 77, and the CD3-VL comprises the amino acid sequence of SEQ ID NO: 78; and the GUCY2C-VH comprises the amino acid sequence of SEQ ID NO: 37, and the GUCY2C-VL comprises the amino acid sequence of SEQ ID NO: 34; or
the CD3-VH comprises the amino acid sequence of SEQ ID NO: 77, and the CD3-VL comprises the amino acid sequence of SEQ ID NO: 78; and the GUCY2C-VH comprises the amino acid sequence of SEQ ID NO: 37, and the GUCY2C-VL comprises the amino acid sequence of SEQ ID NO: 35; or
the CD3-VH comprises the amino acid sequence of SEQ ID NO: 77, and the CD3-VL comprises the amino acid sequence of SEQ ID NO: 78; and the GUCY2C-VH comprises the amino acid sequence of SEQ ID NO: 37, and the GUCY2C-VL comprises the amino acid sequence of SEQ ID NO: 36; or

ii) the CD3-VH comprises the amino acid sequence of SEQ ID NO: 77, and the CD3-VL comprises the amino acid sequence of SEQ ID NO: 78; and the GUCY2C-VH comprises the amino acid sequence of SEQ ID NO: 56, and the GUCY2C-VL comprises the amino acid sequence of SEQ ID NO: 54; or
iii) the CD3-VH comprises the amino acid sequence of SEQ ID NO: 77, and the CD3-VL comprises the amino acid sequence of SEQ ID NO: 78; and the GUCY2C-VH comprises the amino acid sequence of SEQ ID NO: 42, and the GUCY2C-VL comprises the amino acid sequence of SEQ ID NO: 41; or

the CD3-VH comprises the amino acid sequence of SEQ ID NO: 77, and the CD3-VL comprises the amino acid sequence of SEQ ID NO: 78; and the GUCY2C-VH comprises the amino acid sequence of SEQ ID NO: 17, and the GUCY2C-VL comprises the amino acid sequence of SEQ ID NO: 18;
most preferably,
the antigen-binding molecule comprises:

i) one first chain comprising the amino acid sequence of SEQ ID NO: 91, one second chain comprising the amino acid sequence of SEQ ID NO: 92, one third chain comprising the amino acid sequence of SEQ ID NO: 110, and one fourth chain comprising the amino acid sequence of SEQ ID NO: 113; or

one first chain comprising the amino acid sequence of SEQ ID NO: 242, one second chain comprising the amino acid sequence of SEQ ID NO: 92, one third chain comprising the amino acid sequence of SEQ ID NO: 110, and one fourth chain comprising the amino acid sequence of SEQ ID NO: 113; or
one first chain comprising the amino acid sequence of SEQ ID NO: 91, one second chain comprising the amino acid sequence of SEQ ID NO: 92, one third chain comprising the amino acid sequence of SEQ ID NO: 108, and one fourth chain comprising the amino acid sequence of SEQ ID NO: 109; or

one first chain comprising the amino acid sequence of SEQ ID NO: 91, one second chain comprising the amino acid sequence of SEQ ID NO: 92, one third chain comprising the amino acid sequence of SEQ ID NO: 110, and one fourth chain comprising the amino acid sequence of SEQ ID NO: 111; or
one first chain comprising the amino acid sequence of SEQ ID NO: 91, one second chain comprising the amino acid sequence of SEQ ID NO: 92, one third chain comprising the amino acid sequence of SEQ ID NO: 110, and one fourth chain comprising the amino acid sequence of SEQ ID NO: 112; or
one first chain comprising the amino acid sequence of SEQ ID NO: 91, one second chain comprising the amino acid sequence of SEQ ID NO: 92, one third chain comprising the amino acid sequence of SEQ ID NO: 110, and one fourth chain comprising the amino acid sequence of SEQ ID NO: 114; or
one first chain comprising the amino acid sequence of SEQ ID NO: 91, one second chain comprising the amino acid sequence of SEQ ID NO: 92, one third chain comprising the amino acid sequence of SEQ ID NO: 110, and one fourth chain comprising the amino acid sequence of SEQ ID NO: 115; or
one first chain comprising the amino acid sequence of SEQ ID NO: 91, one second chain comprising the amino acid sequence of SEQ ID NO: 92, one third chain comprising the amino acid sequence of SEQ ID NO: 110, and one fourth chain comprising the amino acid sequence of SEQ ID NO: 116; or

ii) one first chain comprising the amino acid sequence of SEQ ID NO: 91, one second chain comprising the amino acid sequence of SEQ ID NO: 92, one third chain comprising the amino acid sequence of SEQ ID NO: 106, and one fourth chain comprising the amino acid sequence of SEQ ID NO: 107; or
iii) one first chain comprising the amino acid sequence of SEQ ID NO: 91, one second chain comprising the amino acid sequence of SEQ ID NO: 92, one third chain comprising the amino acid sequence of SEQ ID NO: 119, and one fourth chain comprising the amino acid sequence of SEQ ID NO: 120; or

one first chain comprising the amino acid sequence of SEQ ID NO: 91, one second chain comprising the amino acid sequence of SEQ ID NO: 92, one third chain comprising the amino acid sequence of SEQ ID NO: 117, and one fourth chain comprising the amino acid sequence of SEQ ID NO: 118.

10. The antigen-binding molecule according to claim 7, comprising one antigen-binding moiety that specifically binds to GUCY2C and one antigen-binding moiety that specifically binds to CD3, the antigen-binding moiety that specifically binds to GUCY2C being a replaced Fab comprising a Titin chain and an Obscurin chain capable of forming a dimer, and the antigen-binding moiety that specifically binds to CD3 being a Fab, wherein preferably,

the antigen-binding molecule comprises one first chain having a structure represented by formula (g), one second chain having a structure represented by formula (f), one third chain having a structure represented by formula (h), and one fourth chain having a structure represented by formula (d):

(g) [GUCY2C-VH]-[linker 4]-[Obscurin chain]-[Fc1],
(f) [GUCY2C-VL]-[linker 4]-[Titin chain],
(h) [CD3-VH]-[CH1]-[Fc2], and
(d) [CD3-VL]-[CL],

wherein the linker 4 is a peptide linker; or the linker 4 is absent;
the structures represented by formulas (g), (f), (h), and (d) are arranged from N-terminus to C-terminus;
more preferably,

i) the CD3-VH comprises the amino acid sequence of SEQ ID NO: 77, and the CD3-VL comprises the amino acid sequence of SEQ ID NO: 78; and the GUCY2C-VH comprises the amino acid sequence of SEQ ID NO: 56, and the GUCY2C-VL comprises the amino acid sequence of SEQ ID NO: 54; or
ii) the CD3-VH comprises the amino acid sequence of SEQ ID NO: 77, and the CD3-VL comprises the amino acid sequence of SEQ ID NO: 78; and the GUCY2C-VH comprises the amino acid sequence of SEQ ID NO: 37, and the GUCY2C-VL comprises the amino acid sequence of SEQ ID NO: 31; or

the CD3-VH comprises the amino acid sequence of SEQ ID NO: 77, and the CD3-VL comprises the amino acid sequence of SEQ ID NO: 78; and the GUCY2C-VH comprises the amino acid sequence of SEQ ID NO: 37, and the GUCY2C-VL comprises the amino acid sequence of SEQ ID NO: 32; or
the CD3-VH comprises the amino acid sequence of SEQ ID NO: 77, and the CD3-VL comprises the amino acid sequence of SEQ ID NO: 78; and the GUCY2C-VH comprises the amino acid sequence of SEQ ID NO: 37, and the GUCY2C-VL comprises the amino acid sequence of SEQ ID NO: 33; or the CD3-VH comprises the amino acid sequence of SEQ ID NO: 77, and the CD3-VL comprises the amino acid sequence of SEQ ID NO: 78; and the GUCY2C-VH comprises the amino acid sequence of SEQ ID NO: 37, and the GUCY2C-VL comprises the amino acid sequence of SEQ ID NO: 34; or the CD3-VH comprises the amino acid sequence of SEQ ID NO: 77, and the CD3-VL comprises the amino acid sequence of SEQ ID NO: 78; and the GUCY2C-VH comprises the amino acid sequence of SEQ ID NO: 37, and the GUCY2C-VL comprises the amino acid sequence of SEQ ID NO: 35; or the CD3-VH comprises the amino acid sequence of SEQ ID NO: 77, and the CD3-VL comprises the amino acid sequence of SEQ ID NO: 78; and the GUCY2C-VH comprises the amino acid sequence of SEQ ID NO: 37, and the GUCY2C-VL comprises the amino acid sequence of SEQ ID NO: 36; or

iii) the CD3-VH comprises the amino acid sequence of SEQ ID NO: 77, and the CD3-VL comprises the amino acid sequence of SEQ ID NO: 78; and the GUCY2C-VH comprises the amino acid sequence of SEQ ID NO: 42, and the GUCY2C-VL comprises the amino acid sequence of SEQ ID NO: 41;

most preferably,
the antigen-binding molecule comprises:

i) one first chain comprising the amino acid sequence of SEQ ID NO: 121, one second chain comprising the amino acid sequence of SEQ ID NO: 107, one third chain comprising the amino acid sequence of SEQ ID NO: 93, and one fourth chain comprising the amino acid sequence of SEQ ID NO: 92; or
ii) one first chain comprising the amino acid sequence of SEQ ID NO: 122, one second chain comprising the amino acid sequence of SEQ ID NO: 111, one third chain comprising the amino acid sequence of SEQ ID NO: 93, and one fourth chain comprising the amino acid sequence of SEQ ID NO: 92; or

one first chain comprising the amino acid sequence of SEQ ID NO: 122, one second chain comprising the amino acid sequence of SEQ ID NO: 112, one third chain comprising the amino acid sequence of SEQ ID NO: 93, and one fourth chain comprising the amino acid sequence of SEQ ID NO: 92; or
one first chain comprising the amino acid sequence of SEQ ID NO: 122, one second chain comprising the amino acid sequence of SEQ ID NO: 113, one third chain comprising the amino acid sequence of SEQ ID NO: 93, and one fourth chain comprising the amino acid sequence of SEQ ID NO: 92; or
one first chain comprising the amino acid sequence of SEQ ID NO: 122, one second chain comprising the amino acid sequence of SEQ ID NO: 114, one third chain comprising the amino acid sequence of SEQ ID NO: 93, and one fourth chain comprising the amino acid sequence of SEQ ID NO: 92; or
one first chain comprising the amino acid sequence of SEQ ID NO: 122, one second chain comprising the amino acid sequence of SEQ ID NO: 115, one third chain comprising the amino acid sequence of SEQ ID NO: 93, and one fourth chain comprising the amino acid sequence of SEQ ID NO: 92; or
one first chain comprising the amino acid sequence of SEQ ID NO: 122, one second chain comprising the amino acid sequence of SEQ ID NO: 116, one third chain comprising the amino acid sequence of SEQ ID NO: 93, and one fourth chain comprising the amino acid sequence of SEQ ID NO: 92; or

iii) one first chain comprising the amino acid sequence of SEQ ID NO: 123, one second chain comprising the amino acid sequence of SEQ ID NO: 120, one third chain comprising the amino acid sequence of SEQ ID NO: 93, and one fourth chain comprising the amino acid sequence of SEQ ID NO: 92.

11. The antigen-binding molecule according to any one of claims 1-4, comprising two antigen-binding moieties that specifically bind to GUCY2C and two antigen-binding moieties that specifically bind to CD3, the antigen-binding moieties that specifically bind to GUCY2C and CD3 being all scFvs, wherein
preferably,

the antigen-binding molecule comprises two first chains having a structure represented by formula (i) and two second chains having a structure represented by formula (j):

(i) [GUCY2C-VH]-[CH1]-[CD3-VH]-[linker 5]-[CD3-VL]-[linker 6]-[one subunit of Fc region], and
(j) [GUCY2C-VL]-[CL],

wherein the linker 5 and the linker 6 are peptide linkers; or the linker 5 and the linker 6 are absent;
the structures represented by formulas (i) and (j) are arranged from N-terminus to C-terminus;

more preferably,

the CD3-VH comprises the amino acid sequence of SEQ ID NO: 77, and the CD3-VL comprises the amino acid sequence of SEQ ID NO: 78; and the GUCY2C-VH comprises the amino acid sequence of SEQ ID NO: 56, and the GUCY2C-VL comprises the amino acid sequence of SEQ ID NO: 54; or

the CD3-VH comprises the amino acid sequence of SEQ ID NO: 77, and the CD3-VL comprises the amino acid sequence of SEQ ID NO: 78; and the GUCY2C-VH comprises the amino acid sequence of SEQ ID NO: 56, and the GUCY2C-VL comprises the amino acid sequence of SEQ ID NO: 55;

most preferably,

the antigen-binding molecule comprises:

two first chains comprising the amino acid sequence of SEQ ID NO: 124 and two second chains comprising the amino acid sequence of SEQ ID NO: 65; or

two first chains comprising the amino acid sequence of SEQ ID NO: 124 and two second chains comprising the amino acid sequence of SEQ ID NO: 66.

12. The antigen-binding molecule according to claim 7, comprising two antigen-binding moieties that specifically bind to GUCY2C and two antigen-binding moieties that specifically bind to CD3, the antigen-binding moieties that specifically bind to GUCY2C being Fabs, and the antigen-binding moieties that specifically bind to CD3 being replaced Fabs comprising a Titin chain and an Obscurin chain capable of forming a dimer; wherein

preferably,

the antigen-binding molecule comprises two first chains having a structure represented by formula (k), two second chains having a structure represented by formula (j), and two third chains having a structure represented by formula (1):

(k) [GUCY2C-VH]-[CH1]-[linker 7]-[CD3-VH]-[linker 4]-[Obscurin chain]-[one subunit of Fc region],
(j) [GUCY2C-VL]-[CL], and
(l) [CD3-VL]-[linker 4]-[Titin chain],

wherein the linker 4 and the linker 7 are peptide linkers; or the linker 4 and the linker 7 are absent;

the structures represented by formulas (k), (j), and (1) are arranged from N-terminus to C-terminus;

more preferably,

the CD3-VH comprises the amino acid sequence of SEQ ID NO: 77, and the CD3-VL comprises the amino acid sequence of SEQ ID NO: 78; and the GUCY2C-VH comprises the amino acid sequence of SEQ ID NO: 56, and the GUCY2C-VL comprises the amino acid sequence of SEQ ID NO: 54; or

the CD3-VH comprises the amino acid sequence of SEQ ID NO: 77, and the CD3-VL comprises the amino acid sequence of SEQ ID NO: 78; and the GUCY2C-VH comprises the amino acid sequence of SEQ ID NO: 56, and the GUCY2C-VL comprises the amino acid sequence of SEQ ID NO: 55;

most preferably,

the antigen-binding molecule comprises:

two first chains comprising the amino acid sequence of SEQ ID NO: 125, two second chains comprising the amino acid sequence of SEQ ID NO: 65, and two third chains comprising the amino acid sequence of SEQ ID NO: 98; or

two first chains comprising the amino acid sequence of SEQ ID NO: 125, two second chains comprising the amino acid sequence of SEQ ID NO: 66, and two third chains comprising the amino acid sequence of SEQ ID NO: 98.

13. An anti-GUCY2C antibody capable of specifically binding to GUCY2C, comprising a heavy chain variable region GUCY2C-VH and a light chain variable region GUCY2C-VL, wherein:

(i) the GUCY2C-VH comprises: a GUCY2C-HCDR1 comprising the amino acid sequence of SEQ ID NO: 19, a GUCY2C-HCDR2 comprising the amino acid sequence of SEQ ID NO: 20, and a GUCY2C-HCDR3 comprising the amino acid sequence of SEQ ID NO: 21; and the GUCY2C-VL comprises: a GUCY2C-LCDR1 comprising the amino acid sequence of SEQ ID NO: 47, 22, 43, 44, 45, or 46, a GUCY2C-LCDR2 comprising the amino acid sequence of SEQ ID NO: 23, and a GUCY2C-LCDR3 comprising the amino acid sequence of SEQ ID NO: 24; or

(ii) the GUCY2C-VH comprises: a GUCY2C-HCDR1 comprising the amino acid sequence of SEQ ID NO: 4, a GUCY2C-HCDR2 comprising the amino acid sequence of SEQ ID NO: 5, and a GUCY2C-HCDR3 comprising the amino acid sequence of SEQ ID NO: 27 or 6; and the GUCY2C-VL comprises: a GUCY2C-LCDR1 comprising the amino acid sequence of SEQ ID NO: 7, a GUCY2C-LCDR2 comprising the amino acid sequence of SEQ ID NO: 8, 28, 29, or 30, and a GUCY2C-LCDR3 comprising the amino acid sequence of SEQ ID NO: 9; or

iii) the GUCY2C-VH comprises: a GUCY2C-HCDR1 comprising the amino acid sequence of SEQ ID NO: 10, a GUCY2C-HCDR2 comprising the amino acid sequence of SEQ ID NO: 11, and a GUCY2C-HCDR3 comprising the amino acid sequence of SEQ ID NO: 40 or 12; and the GUCY2C-VL comprises: a GUCY2C-LCDR1 comprising the amino acid sequence of SEQ ID NO: 13, a GUCY2C-LCDR2 comprising the amino acid sequence of SEQ ID NO: 39 or 8, and a GUCY2C-LCDR3 comprising the amino acid sequence of SEQ ID NO: 14; or

iv-1) the GUCY2C-VH comprises: a GUCY2C-HCDR1 comprising the amino acid sequence of SEQ ID NO: 250, a GUCY2C-HCDR2 comprising the amino acid sequence of SEQ ID NO: 251, and a GUCY2C-HCDR3 comprising the amino acid sequence of SEQ ID NO: 252; and the GUCY2C-VL comprises: a GUCY2C-LCDR1 comprising the amino acid sequence of SEQ ID NO: 253, a GUCY2C-LCDR2 comprising the amino acid sequence of SEQ ID NO: 254, and a GUCY2C-LCDR3 comprising the amino acid sequence of SEQ ID NO: 255; or

iv-2) the GUCY2C-VH comprises: a GUCY2C-HCDR1 comprising the amino acid sequence of SEQ ID NO: 256, a GUCY2C-HCDR2 comprising the amino acid sequence of SEQ ID NO: 257, and a GUCY2C-HCDR3 comprising the amino acid sequence of SEQ ID NO: 258; and the GUCY2C-VL comprises: a GUCY2C-LCDR1 comprising the amino acid sequence of SEQ ID NO: 259, a GUCY2C-LCDR2 comprising the amino acid sequence of SEQ ID NO: 8, and a GUCY2C-LCDR3 comprising the amino acid sequence of SEQ ID NO: 260; or

iv-3) the GUCY2C-VH comprises: a GUCY2C-HCDR1 comprising the amino acid sequence of SEQ ID NO: 261, a GUCY2C-HCDR2 comprising the amino acid sequence of SEQ ID NO: 262, and a GUCY2C-HCDR3 comprising the amino acid sequence of SEQ ID NO: 263; and the GUCY2C-VL comprises: a GUCY2C-LCDR1 comprising the amino acid sequence of SEQ ID NO: 264, a GUCY2C-LCDR2 comprising the amino acid sequence of SEQ ID NO: 265, and a GUCY2C-LCDR3 comprising the amino acid sequence of SEQ ID NO: 266; or

iv-4) the GUCY2C-VH comprises: a GUCY2C-HCDR1 comprising the amino acid sequence of SEQ ID NO: 267, a GUCY2C-HCDR2 comprising the amino acid sequence of SEQ ID NO: 268, and a GUCY2C-HCDR3 comprising the amino acid sequence of SEQ ID NO: 269; and the GUCY2C-VL comprises: a GUCY2C-LCDR1 comprising the amino acid sequence of SEQ ID NO: 270, a GUCY2C-LCDR2 comprising the amino acid sequence of SEQ ID NO: 271, and a GUCY2C-LCDR3 comprising the amino acid sequence of SEQ ID NO: 272; or

iv-5) the GUCY2C-VH comprises: a GUCY2C-HCDR1 comprising the amino acid sequence of SEQ ID NO: 281, a GUCY2C-HCDR2 comprising the amino acid sequence of SEQ ID NO: 282, and a GUCY2C-HCDR3 comprising the amino acid sequence of SEQ ID NO: 283; and the GUCY2C-VL comprises: a GUCY2C-LCDR1 comprising the amino acid sequence of SEQ ID NO: 284, a GUCY2C-LCDR2 comprising the amino acid sequence of SEQ ID NO: 8, and a GUCY2C-LCDR3 comprising the amino acid sequence of SEQ ID NO: 285; or

iv-6) the GUCY2C-VH comprises: a GUCY2C-HCDR1 comprising the amino acid sequence of SEQ ID NO: 286, a GUCY2C-HCDR2 comprising the amino acid sequence of SEQ ID NO: 287, and a GUCY2C-HCDR3 comprising the amino acid sequence of SEQ ID NO: 288; and the GUCY2C-VL comprises: a GUCY2C-LCDR1 comprising the amino acid sequence of SEQ ID NO: 289, a GUCY2C-LCDR2 comprising the amino acid sequence of SEQ ID NO: 265, and a GUCY2C-LCDR3 comprising the amino acid sequence of SEQ ID NO: 290; or

iv-7) the GUCY2C-VH comprises: a GUCY2C-HCDR1 comprising the amino acid sequence of SEQ ID NO: 291, a GUCY2C-HCDR2 comprising the amino acid sequence of SEQ ID NO: 292, and a GUCY2C-HCDR3 comprising the amino acid sequence of SEQ ID NO: 293; and the GUCY2C-VL comprises: a GUCY2C-LCDR1 comprising the amino acid sequence of SEQ ID NO: 294, a GUCY2C-LCDR2 comprising the amino acid sequence of SEQ ID NO: 295, and a GUCY2C-LCDR3 comprising the amino acid sequence of SEQ ID NO: 296; or

iv-8) the GUCY2C-VH comprises: a GUCY2C-HCDR1 comprising the amino acid sequence of SEQ ID NO: 261, a GUCY2C-HCDR2 comprising the amino acid sequence of SEQ ID NO: 297, and a GUCY2C-HCDR3 comprising the amino acid sequence of SEQ ID NO: 298; and the GUCY2C-VL comprises: a GUCY2C-LCDR1 comprising the amino acid sequence of SEQ ID NO: 264, a GUCY2C-LCDR2 comprising the amino acid sequence of SEQ ID NO: 299, and a GUCY2C-LCDR3 comprising the amino acid sequence of SEQ ID NO: 300; or

iv-9) the GUCY2C-VH comprises: a GUCY2C-HCDR1 comprising the amino acid sequence of SEQ ID NO: 261, a GUCY2C-HCDR2 comprising the amino acid sequence of SEQ ID NO: 301, and a GUCY2C-HCDR3 comprising the amino acid sequence of SEQ ID NO: 302; and the GUCY2C-VL comprises: a GUCY2C-LCDR1 comprising the amino acid sequence of SEQ ID NO: 303, a GUCY2C-LCDR2 comprising the amino acid sequence of SEQ ID NO: 299, and a GUCY2C-LCDR3 comprising the amino acid sequence of SEQ ID NO: 304; or

iv-10) the GUCY2C-VH comprises: a GUCY2C-HCDR1 comprising the amino acid sequence of SEQ ID NO: 305, a GUCY2C-HCDR2 comprising the amino acid sequence of SEQ ID NO: 306, and a GUCY2C-HCDR3 comprising the amino acid sequence of SEQ ID NO: 21; and the GUCY2C-VL comprises: a GUCY2C-LCDR1 comprising the amino acid sequence of SEQ ID NO: 22, a GUCY2C-LCDR2 comprising the amino acid sequence of SEQ ID NO: 23, and a GUCY2C-LCDR3 comprising the amino acid sequence of SEQ ID NO: 24; or

iv-11) the GUCY2C-VH comprises: a GUCY2C-HCDR1 comprising the amino acid sequence of SEQ ID NO: 128, a GUCY2C-HCDR2 comprising the amino acid sequence of SEQ ID NO: 307, and a GUCY2C-HCDR3 comprising the amino acid sequence of SEQ ID NO: 308; and the GUCY2C-VL comprises: a GUCY2C-LCDR1 comprising the amino acid sequence of SEQ ID NO: 309, a GUCY2C-LCDR2 comprising the amino acid sequence of SEQ ID NO: 310, and a GUCY2C-LCDR3 comprising the amino acid sequence of SEQ ID NO: 311; or

iv-12) the GUCY2C-VH comprises: a GUCY2C-HCDR1 comprising the amino acid sequence of SEQ ID NO: 312, a GUCY2C-HCDR2 comprising the amino acid sequence of SEQ ID NO: 313, and a GUCY2C-HCDR3 comprising the amino acid sequence of SEQ ID NO: 314; and the GUCY2C-VL comprises: a GUCY2C-LCDR1 comprising the amino acid sequence of SEQ ID NO: 22, a GUCY2C-LCDR2 comprising the amino acid sequence of SEQ ID NO: 23, and a GUCY2C-LCDR3 comprising the amino acid sequence of SEQ ID NO: 315; or

iv-13) the GUCY2C-VH comprises: a GUCY2C-HCDR1 comprising the amino acid sequence of SEQ ID NO: 286, a GUCY2C-HCDR2 comprising the amino acid sequence of SEQ ID NO: 287, and a GUCY2C-HCDR3 comprising the amino acid sequence of SEQ ID NO: 316; and the GUCY2C-VL comprises: a GUCY2C-LCDR1 comprising the amino acid sequence of SEQ ID NO: 317, a GUCY2C-LCDR2 comprising the amino acid sequence of SEQ ID NO: 265, and a GUCY2C-LCDR3 comprising the amino acid sequence of SEQ ID NO: 300; or

iv-14) the GUCY2C-VH comprises: a GUCY2C-HCDR1 comprising the amino acid sequence of SEQ ID NO: 318, a GUCY2C-HCDR2 comprising the amino acid sequence of SEQ ID NO: 319, and a GUCY2C-HCDR3 comprising the amino acid sequence of SEQ ID NO: 320; and the GUCY2C-VL comprises: a GUCY2C-LCDR1 comprising the amino acid sequence of SEQ ID NO: 321, a GUCY2C-LCDR2 comprising the amino acid sequence of SEQ ID NO: 322, and a GUCY2C-LCDR3 comprising the amino acid sequence of SEQ ID NO: 323; or

iv-15) the GUCY2C-VH comprises: a GUCY2C-HCDR1 comprising the amino acid sequence of SEQ ID NO: 324, a GUCY2C-HCDR2 comprising the amino acid sequence of SEQ ID NO: 325, and a GUCY2C-HCDR3 comprising the amino acid sequence of SEQ ID NO: 326; and the GUCY2C-VL comprises: a GUCY2C-LCDR1 comprising the amino acid sequence of SEQ ID NO: 22, a GUCY2C-LCDR2 comprising the amino acid sequence of SEQ ID NO: 23, and a GUCY2C-LCDR3 comprising the amino acid sequence of SEQ ID NO: 327; or

iv-16) the GUCY2C-VH comprises: a GUCY2C-HCDR1 comprising the amino acid sequence of SEQ ID NO: 261, a GUCY2C-HCDR2 comprising the amino acid sequence of SEQ ID NO: 328, and a GUCY2C-HCDR3 comprising the amino acid sequence of SEQ ID NO: 302; and the GUCY2C-VL comprises: a GUCY2C-LCDR1 comprising the amino acid sequence of SEQ ID NO: 264, a GUCY2C-LCDR2 comprising the amino acid sequence of SEQ ID NO: 329, and a GUCY2C-LCDR3 comprising the amino acid sequence of SEQ ID NO: 300;

preferably,

i) the GUCY2C-VH comprises: a GUCY2C-HCDR1 comprising the amino acid sequence of SEQ ID NO: 19, a GUCY2C-HCDR2 comprising the amino acid sequence of SEQ ID NO: 20, and a GUCY2C-HCDR3 comprising the amino acid sequence of SEQ ID NO: 21; and the GUCY2C-VL comprises: a GUCY2C-LCDR1 comprising the amino acid sequence of SEQ ID NO: 47, a GUCY2C-LCDR2 comprising the amino acid sequence of SEQ ID NO: 23, and a GUCY2C-LCDR3 comprising the amino acid sequence of SEQ ID NO: 24; or

ii) the GUCY2C-VH comprises: a GUCY2C-HCDR1 comprising the amino acid sequence of SEQ ID NO: 4, a GUCY2C-HCDR2 comprising the amino acid sequence of SEQ ID NO: 5, and a GUCY2C-HCDR3 comprising the amino acid sequence of SEQ ID NO: 27; and the GUCY2C-VL comprises: a GUCY2C-LCDR1 comprising the amino acid sequence of SEQ ID NO: 7, a GUCY2C-LCDR2 comprising the amino acid sequence of SEQ ID NO: 8, and a GUCY2C-LCDR3 comprising the amino acid sequence of SEQ ID NO: 9; or

iii) the GUCY2C-VH comprises: a GUCY2C-HCDR1 comprising the amino acid sequence of SEQ ID NO: 10, a GUCY2C-HCDR2 comprising the amino acid sequence of SEQ ID NO: 11, and a GUCY2C-HCDR3 comprising the amino acid sequence of SEQ ID NO: 40; and the GUCY2C-VL comprises: a GUCY2C-LCDR1 comprising the amino acid sequence of SEQ ID NO: 13, a GUCY2C-LCDR2 comprising the amino acid sequence of SEQ ID NO: 39, and a GUCY2C-LCDR3 comprising the amino acid sequence of SEQ ID NO: 14.

14. The anti-GUCY2C antibody according to claim 13, wherein:

i) the GUCY2C-VH comprises the amino acid sequence of SEQ ID NO: 56, 25, or 57, and the GUCY2C-VL comprises the amino acid sequence of SEQ ID NO: 54, 55, 26, 48, 49, 50, 51, 52, or 53; or

ii) the GUCY2C-VH comprises the amino acid sequence of SEQ ID NO: 37, 15, or 38, and the GUCY2C-VL comprises the amino acid sequence of SEQ ID NO: 33, 16, 31, 32, 34, 35, or 36; or

iii) the GUCY2C-VH comprises the amino acid sequence of SEQ ID NO: 42 or 17, and the GUCY2C-VL comprises the amino acid sequence of SEQ ID NO: 41 or 18; or

iv-1) the GUCY2C-VH comprises the amino acid sequence of SEQ ID NO: 273, and the GUCY2C-VL comprises the amino acid sequence of SEQ ID NO: 274; or

iv-2) the GUCY2C-VH comprises the amino acid sequence of SEQ ID NO: 275, and the GUCY2C-VL comprises the amino acid sequence of SEQ ID NO: 276; or

iv-3) the GUCY2C-VH comprises the amino acid sequence of SEQ ID NO: 277, and the GUCY2C-VL comprises the amino acid sequence of SEQ ID NO: 278; or

iv-4) the GUCY2C-VH comprises the amino acid sequence of SEQ ID NO: 279, and the GUCY2C-VL comprises the amino acid sequence of SEQ ID NO: 280; or

iv-5) the GUCY2C-VH comprises the amino acid sequence of SEQ ID NO: 330, and the GUCY2C-VL comprises the amino acid sequence of SEQ ID NO: 331; or

iv-6) the GUCY2C-VH comprises the amino acid sequence of SEQ ID NO: 332, and the GUCY2C-VL comprises the amino acid sequence of SEQ ID NO: 333; or

iv-7) the GUCY2C-VH comprises the amino acid sequence of SEQ ID NO: 334, and the GUCY2C-VL comprises the amino acid sequence of SEQ ID NO: 335; or

iv-8) the GUCY2C-VH comprises the amino acid sequence of SEQ ID NO: 336, and the GUCY2C-VL comprises the amino acid sequence of SEQ ID NO: 337; or

iv-9) the GUCY2C-VH comprises the amino acid sequence of SEQ ID NO: 338, and the GUCY2C-VL comprises the amino acid sequence of SEQ ID NO: 339; or

iv-10) the GUCY2C-VH comprises the amino acid sequence of SEQ ID NO: 340, and the GUCY2C-VL comprises the amino acid sequence of SEQ ID NO: 341; or

iv-11) the GUCY2C-VH comprises the amino acid sequence of SEQ ID NO: 342, and the GUCY2C-VL comprises the amino acid sequence of SEQ ID NO: 343; or

iv-12) the GUCY2C-VH comprises the amino acid sequence of SEQ ID NO: 344, and the GUCY2C-VL comprises the amino acid sequence of SEQ ID NO: 345; or

iv-13) the GUCY2C-VH comprises the amino acid sequence of SEQ ID NO: 346, and the GUCY2C-VL comprises the amino acid sequence of SEQ ID NO: 347; or

iv-14) the GUCY2C-VH comprises the amino acid sequence of SEQ ID NO: 348, and the GUCY2C-VL comprises the amino acid sequence of SEQ ID NO: 349; or

iv-15) the GUCY2C-VH comprises the amino acid sequence of SEQ ID NO: 350, and the GUCY2C-VL comprises the amino acid sequence of SEQ ID NO: 351; or

iv-16) the GUCY2C-VH comprises the amino acid sequence of SEQ ID NO: 352, and the GUCY2C-VL comprises the amino acid sequence of SEQ ID NO: 353;

preferably,

i) the GUCY2C-VH comprises the amino acid sequence of SEQ ID NO: 56, and the GUCY2C-VL comprises the amino acid sequence of SEQ ID NO: 54, 55, 48, 49, 50, 51, or 52, or

the GUCY2C-VH comprises the amino acid sequence of SEQ ID NO: 25, and the GUCY2C-VL comprises the amino acid sequence of SEQ ID NO: 26, or

ii) the GUCY2C-VH comprises the amino acid sequence of SEQ ID NO: 37, and the GUCY2C-VL comprises the amino acid sequence of SEQ ID NO: 33, or

the GUCY2C-VH comprises the amino acid sequence of SEQ ID NO: 38, and the GUCY2C-VL comprises the amino acid sequence of SEQ ID NO: 33, 32, 34, or 36, or the GUCY2C-VH comprises the amino acid sequence of SEQ ID NO: 15, and the GUCY2C-VL comprises the amino acid sequence of SEQ ID NO: 16, or

iii) the GUCY2C-VH comprises the amino acid sequence of SEQ ID NO: 42, and the GUCY2C-VL comprises the amino acid sequence of SEQ ID NO: 41, or

the GUCY2C-VH comprises the amino acid sequence of SEQ ID NO: 17, and the GUCY2C-VL comprises the amino acid sequence of SEQ ID NO: 18;

more preferably:

i) the GUCY2C-VH comprises the amino acid sequence of SEQ ID NO: 56, and the GUCY2C-VL comprises the amino acid sequence of SEQ ID NO: 54, or

the GUCY2C-VH comprises the amino acid sequence of SEQ ID NO: 56, and the GUCY2C-VL comprises the amino acid sequence of SEQ ID NO: 55; or

ii) the GUCY2C-VH comprises the amino acid sequence of SEQ ID NO: 37, and the GUCY2C-VL comprises the amino acid sequence of SEQ ID NO: 33; or

iii) the GUCY2C-VH comprises the amino acid sequence of SEQ ID NO: 42, and the GUCY2C-VL comprises the amino acid sequence of SEQ ID NO: 41.

15. The anti-GUCY2C antibody according to claim 13 or 14, wherein the anti-GUCY2C antibody is a murine antibody, a chimeric antibody, or a humanized antibody.

16. The anti-GUCY2C antibody according to any one of claims 13-15, comprising a heavy chain constant region and a light chain constant region, wherein preferably, the heavy chain constant region is a human IgG1 constant region, and the light chain constant region is selected from the group consisting of constant regions of human antibody κ and λ chains; more preferably, the heavy chain constant region comprises the amino acid sequence of SEQ ID NO: 58, and the light chain constant region comprises the amino acid sequence of SEQ ID NO: 59.

17. The anti-GUCY2C antibody according to any one of claims 13-16, comprising a heavy chain and a light chain, wherein:

the heavy chain comprises an amino acid sequence having at least 85% sequence identity to SEQ ID NO: 64, and the light chain comprises an amino acid sequence having at least 85% sequence identity to SEQ ID NO: 65 or 66; or

the heavy chain comprises an amino acid sequence having at least 85% sequence identity to SEQ ID NO: 60, and the light chain comprises an amino acid sequence having at least 85% sequence identity to SEQ ID NO: 61; or the heavy chain comprises an amino acid sequence having at least 85% sequence identity to SEQ ID NO: 62, and the light chain comprises an amino acid sequence having at least 85% sequence identity to SEQ ID NO: 63; preferably, the heavy chain comprises the amino acid sequence of SEQ ID NO: 64, and the light chain comprises the amino acid sequence of SEQ ID NO: 65 or 66; or

the heavy chain comprises the amino acid sequence of SEQ ID NO: 60, and the light chain comprises the amino acid sequence of SEQ ID NO: 61; or

the heavy chain comprises the amino acid sequence of SEQ ID NO: 62, and the light chain comprises the amino acid sequence of SEQ ID NO: 63.

18. The anti-GUCY2C antibody according to any one of claims 13-17, wherein the anti-GUCY2C antibody is an antibody fragment; preferably, the antibody fragment is Fab, Fab', F(ab')2, Fd, Fv, scFv, dsFv, or dAb.

19. A pharmaceutical composition, comprising:
a therapeutically effective amount of the antigen-binding molecule according to any one of claims 1-12 or the anti-GUCY2C antibody according to any one of claims 13-18 and one or more pharmaceutically acceptable carriers, diluents, buffers, or excipients.

20. An isolated nucleic acid encoding the antigen-binding molecule according to any one of claims 1-12 or the anti-GUCY2C antibody according to any one of claims 13-18.

21. A host cell, comprising the isolated nucleic acid according to claim 20.

22. A method for treating a disease, comprising administering to a subject a therapeutically effective amount of the antigen-binding molecule according to any one of claims 1-12 or the anti-GUCY2C antibody according to any one of claims 13-18 or the pharmaceutical composition according to claim 19; wherein preferably, the disease is a tumor; more preferably, the tumor is gastric cancer, gastrointestinal cancer, intestinal cancer, gastric adenocarcinoma, small intestine cancer, colorectal cancer, esophageal cancer, anal cancer, liver cancer, gallbladder cancer, bile duct cancer, pancreatic cancer, prostate cancer, renal cancer, urothelial cancer, breast cancer, bladder cancer, thymus cancer, melanoma, glioma, sarcoma, osteosarcoma, ovarian cancer, thyroid cancer, lung squamous cell cancer, lung adenocarcinoma, cervical squamous cell cancer, endometrial cancer, lung cancer, skin cancer, head and neck cancer, brain cancer, glioblastoma multiforme, gastroesophageal cancer, gastroesophageal adenocarcinoma, adenocarcinomas metastatic to the liver, multiple myeloma, lymphoma, leukemia, or B-cell lymphoma; most preferably, cells of the tumor or vascular endothelial cells close to the tumor express GUCY2C.

23. The method for treating a disease according to claim 22, further comprising using a second therapeutic agent, wherein preferably, the second therapeutic agent comprises an anti-tumor agent, radiation therapy, an antibody-drug conjugate, a bispecific antibody, a bispecific antibody conjugated to an anti-tumor agent, an immune checkpoint inhibitor, or a combination thereof.

24. The method for treating a disease according to claim 23, wherein the second therapeutic agent is a bispecific antibody that specifically binds to CD28 and EGFR, and the bispecific antibody comprises at least one antigen-binding moiety that specifically binds to CD28 and at least one antigen-binding moiety that specifically binds to EGFR, wherein the antigen-binding moiety that specifically binds to CD28 comprises a heavy chain variable region CD28-VH and a light chain variable region CD28-VL, and the antigen-binding moiety that specifically binds to EGFR comprises a heavy chain variable region EGFR-VH and a light chain variable region EGFR-VL; preferably,

i) the CD28-VH comprises: a CD28-HCDR1 comprising the amino acid sequence of SEQ ID NO: 146, a CD28-HCDR2 comprising the amino acid sequence of SEQ ID NO: 147, and a CD28-HCDR3 comprising the amino acid sequence of SEQ ID NO: 148; and the CD28-VL comprises: a CD28-LCDR1 comprising the amino acid sequence of SEQ ID NO: 149, a CD28-LCDR2 comprising the amino acid sequence of SEQ ID NO: 150, and a CD28-LCDR3 comprising the amino acid sequence of SEQ ID NO: 151; or

ii) the CD28-VH comprises: a CD28-HCDR1 comprising the amino acid sequence of SEQ ID NO: 160, a CD28-HCDR2 comprising the amino acid sequence of SEQ ID NO: 161, and a CD28-HCDR3 comprising the amino acid sequence of SEQ ID NO: 162; and the CD28-VL comprises: a CD28-LCDR1 comprising the amino acid sequence of SEQ ID NO: 149, a CD28-LCDR2 comprising the amino acid sequence of SEQ ID NO: 150, and a CD28-LCDR3 comprising the amino acid sequence of SEQ ID NO: 163; or

iii) the CD28-VH comprises: a CD28-HCDR1 comprising the amino acid sequence of SEQ ID NO: 168, a CD28-HCDR2 comprising the amino acid sequence of SEQ ID NO: 169, and a CD28-HCDR3 comprising the amino acid sequence of SEQ ID NO: 170; and the CD28-VL comprises: a CD28-LCDR1 comprising the amino acid sequence of SEQ ID NO: 171, a CD28-LCDR2 comprising the amino acid sequence of SEQ ID NO: 172, and a CD28-LCDR3 comprising the amino acid sequence of SEQ ID NO: 173;

more preferably,

i) the CD28-VH comprises the amino acid sequence of SEQ ID NO: 154, and the CD28-VL comprises the amino acid sequence of SEQ ID NO: 155; or

ii) the CD28-VH comprises the amino acid sequence of SEQ ID NO: 164, and the CD28-VL comprises the amino acid sequence of SEQ ID NO: 165; or

iii) the CD28-VH comprises the amino acid sequence of SEQ ID NO: 174, and the CD28-VL comprises the amino acid sequence of SEQ ID NO: 175.

25. The method for treating a disease according to claim 24, wherein the antigen-binding moiety that specifically binds to EGFR comprises a heavy chain variable region EGFR-VH and a light chain variable region EGFR-VL, wherein:

the EGFR-VH comprises: an EGFR-HCDR1 comprising the amino acid sequence of SEQ ID NO: 140, an EGFR-HCDR2 comprising the amino acid sequence of SEQ ID NO: 141, and an EGFR-HCDR3 comprising the amino acid sequence of SEQ ID NO: 142; and the EGFR-VL comprises: an EGFR-LCDR1 comprising the amino acid sequence of SEQ ID NO: 143, an EGFR-LCDR2 comprising the amino acid sequence of SEQ ID NO: 144, and an EGFR-LCDR3 comprising the amino acid sequence of SEQ ID NO: 145;
more preferably,
the EGFR-VH comprises the amino acid sequence of SEQ ID NO: 152, and the EGFR-VL comprises the amino acid sequence of SEQ ID NO: 153.

26. The method for treating a disease according to claim 25, wherein the bispecific antibody that specifically binds to CD28 and EGFR comprises an Fc region;

preferably, the Fc region comprises a first subunit Fc1 and a second subunit Fc2 capable of associating with each other, the Fc1 has a knob structure according to the knob-into-hole technique, and the Fc2 has a hole structure according to the knob-into-hole technique;
more preferably, for the Fc1, the amino acid at position 366 is W, and for the Fc2, the amino acid at position 366 is S, the amino acid at position 368 is A, and the amino acid at position 407 is V, as numbered according to the EU index;
most preferably, the Fc1 comprises the amino acid sequence of SEQ ID NO: 248, and the Fc2 comprises the amino acid sequence of SEQ ID NO: 249.

27. The method for treating a disease according to claim 26, wherein the bispecific antibody that specifically binds to CD28 and EGFR comprises one antigen-binding moiety that specifically binds to CD28 and one antigen-binding moiety that specifically binds to EGFR, the antigen-binding moiety that specifically binds to CD28 being a replaced Fab comprising a Titin chain and an Obscurin chain capable of forming a dimer, and the antigen-binding moiety that specifically binds to EGFR being a Fab, wherein
preferably,

the bispecific antibody that specifically binds to CD28 and EGFR comprises one first chain having a structure represented by formula (m), a second chain having a structure represented by formula (n), a third chain having a structure represented by formula (o), and a fourth chain having a structure represented by formula (p):

(m) [CD28-VH]-[linker 8]-[Titin chain]-[Fc1],
(n) [CD28-VL]-[linker 8]-[Obscurin chain],
(o) [EGFR-VH]-[CH1]-[Fc2], and
(p) [EGFR-VL]-[CL],

wherein the linker 8 is a peptide linker; or the linker 8 is absent;
the structures represented by formulas (m), (n), (o), and (p) are arranged from N-terminus to C-terminus;
most preferably,
the bispecific antibody that specifically binds to CD28 and EGFR comprises: one first chain comprising the amino acid sequence of SEQ ID NO: 156, one second chain comprising the amino acid sequence of SEQ ID NO: 157, one third chain comprising the amino acid sequence of SEQ ID NO: 158, and one fourth chain comprising the amino acid sequence of SEQ ID NO: 159; or
the bispecific antibody that specifically binds to CD28 and EGFR comprises: one first chain comprising the amino acid sequence of SEQ ID NO: 166, one second chain comprising the amino acid sequence of SEQ ID NO: 167, one third chain comprising the amino acid sequence of SEQ ID NO: 158, and one fourth chain comprising the amino acid sequence of SEQ ID NO: 159.

28. The method for treating a disease according to claim 26, wherein the bispecific antibody that specifically binds to CD28 and EGFR comprises one antigen-binding moiety that specifically binds to CD28 and one antigen-binding moiety that specifically binds to EGFR, the antigen-binding moiety that specifically binds to CD28 being a Fab, and the antigen-binding moiety that specifically binds to EGFR being a replaced Fab comprising a Titin chain and an Obscurin chain capable of forming a dimer, wherein preferably,

the bispecific antibody that specifically binds to CD28 and EGFR comprises one first chain having a structure represented by formula (q), a second chain having a structure represented by formula (r), a third chain having a structure represented by formula (s), and a fourth chain having a structure represented by formula (t):

(q) [CD28-VH]-[CH1]-[Fc1],
(r) [CD28-VL]-[CL],
(s) [EGFR-VH]-[linker 8]-[Obscurin chain]-[Fc2], and
(t) [EGFR-VL]-[linker 8]-[Titin chain],

wherein the linker 8 is a peptide linker; or the linker 8 is absent;
the structures represented by formulas (q), (r), (s), and (t) are arranged from N-terminus to C-terminus;
more preferably,
the bispecific antibody that specifically binds to CD28 and EGFR comprises: one first chain comprising the amino acid sequence of SEQ ID NO: 176, one second chain comprising the amino acid sequence of SEQ ID NO: 177, one third chain comprising the amino acid sequence of SEQ ID NO: 178, and one fourth chain comprising the amino acid sequence of SEQ ID NO: 179.

FIG. 1A

FIG. 1B

FIG. 1C

FIG. 1D

FIG. 1E

FIG. 2A

FIG. 2B

FIG. 2C

FIG. 2D

FIG. 2E

FIG. 3A

FIG. 3B

FIG. 3C

FIG. 4A

FIG. 4B

FIG. 5A

FIG. 5B

FIG. 5C

FIG. 6A

FIG. 6B

FIG. 6C

## INTERNATIONAL SEARCH REPORT

| International application No. |
|---|
| **PCT/CN2023/111149** |

| A. | CLASSIFICATION OF SUBJECT MATTER |
|---|---|

A61P35/00(2006.01)i; C07K16/00(2006.01)i; C07K16/28(2006.01)i; C12N15/13(2006.01)i; C07K16/40(2006.01)i

According to International Patent Classification (IPC) or to both national classification and IPC

| B. | FIELDS SEARCHED |
|---|---|

Minimum documentation searched (classification system followed by classification symbols)

A61P,C07K,C12N

Documentation searched other than minimum documentation to the extent that such documents are included in the fields searched

Electronic data base consulted during the international search (name of data base and, where practicable, search terms used)

CNABS, DWPI, SIPOABS, CNTXT, WOTXT, JPTXT, USTXT, EPTXT, 万方, WANFANG, CNKI, PubMed, GenBank, ISI web of knowledge: 特异性结合, GUCY2C, CD3, 抗原结合分子, 双抗体, 治疗, 肿瘤, 重链可变区, 轻链可变区, 抗原结合模块, Specific Binding, Antigen Binding Molecules, Diabodies, Therapeutics, Tumors, Heavy Chain Variable Regions, Light Chain Variable Regions, Antigen Binding Moieties

| C. | DOCUMENTS CONSIDERED TO BE RELEVANT |
|---|---|

| Category* | Citation of document, with indication, where appropriate, of the relevant passages | Relevant to claim No. |
|---|---|---|
| A | CN 113286634 A (PFIZER INC.) 20 August 2021 (2021-08-20)<br>entire document | 1-28 |
| A | CN 113801238 A (NANJING BIOHENG BIOTECH CO., LTD.) 17 December 2021 (2021-12-17)<br>entire document | 1-28 |
| A | WO 2022127871 A1 (PARASOL BIOTECH LTD.) 23 June 2022 (2022-06-23)<br>entire document | 1-28 |
| A | WO 2010119704 A1 (IMMUNAS PHARMA, INC.) 21 October 2010 (2010-10-21)<br>entire document | 1-28 |
| A | CN 110055224 A (SHENZHEN IN VIVO BIOMEDICINE TECHNOLOGY LIMITED COMPANY) 26 July 2019 (2019-07-26)<br>entire document | 1-28 |

✓ Further documents are listed in the continuation of Box C.　　✓ See patent family annex.

| * | Special categories of cited documents: |
|---|---|
| "A" | document defining the general state of the art which is not considered to be of particular relevance |
| "D" | document cited by the applicant in the international application |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| "T" | later document published after the international filing date or priority date and not in conflict with the application but cited to understand the principle or theory underlying the invention |
|---|---|
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
|---|---|
| **30 August 2023** | **06 September 2023** |

| Name and mailing address of the ISA/CN | Authorized officer |
|---|---|
| **China National Intellectual Property Administration (ISA/CN)**<br>**China No. 6, Xitucheng Road, Jimenqiao, Haidian District, Beijing 100088** | |
| | Telephone No. |

Form PCT/ISA/210 (second sheet) (July 2022)

## INTERNATIONAL SEARCH REPORT

International application No.

**PCT/CN2023/111149**

### C. DOCUMENTS CONSIDERED TO BE RELEVANT

| Category* | Citation of document, with indication, where appropriate, of the relevant passages | Relevant to claim No. |
|---|---|---|
| A | CN 110564693 A (INNOVATIVE CELLULAR THERAPEUTICS CO., LTD.) 13 December 2019 (2019-12-13)<br>entire document | 1-28 |
| A | CN 110257338 A (INNOVATIVE CELLULAR THERAPEUTICS CO., LTD.) 20 September 2019 (2019-09-20)<br>entire document | 1-28 |

Form PCT/ISA/210 (second sheet) (July 2022)

## INTERNATIONAL SEARCH REPORT

| International application No. |
|---|
| **PCT/CN2023/111149** |

**Box No. I**  **Nucleotide and/or amino acid sequence(s) (Continuation of item 1.c of the first sheet)**

1. With regard to any nucleotide and/or amino acid sequence disclosed in the international application, the international search was carried out on the basis of a sequence listing:

   a. ☑ forming part of the international application as filed.

   b. ☐ furnished subsequent to the international filing date for the purposes of international search (Rule 13*ter*.1(a)),

   ☐ accompanied by a statement to the effect that the sequence listing does not go beyond the disclosure in the international application as filed.

2. ☐ With regard to any nucleotide and/or amino acid sequence disclosed in the international application, this report has been established to the extent that a meaningful search could be carried out without a WIPO Standard ST.26 compliant sequence listing.

3. Additional comments:

Form PCT/ISA/210 (continuation of first sheet) (July 2022)

## INTERNATIONAL SEARCH REPORT

International application No.

**PCT/CN2023/111149**

| Box No. II | Observations where certain claims were found unsearchable (Continuation of item 2 of first sheet) |

This international search report has not been established in respect of certain claims under Article 17(2)(a) for the following reasons:

1. ☑ Claims Nos.: **22-28**
   because they relate to subject matter not required to be searched by this Authority, namely:

   Claims 22-28 relate to a method for treatment or diagnosis of a living human or animal body, which falls within the cases set out in PCT Rule 39.1.(IV) for which an international search is not required. In the present report, a search is conducted on the basis of "a use in the preparation of a drug for diseases".

2. ☐ Claims Nos.:
   because they relate to parts of the international application that do not comply with the prescribed requirements to such an extent that no meaningful international search can be carried out, specifically:

3. ☐ Claims Nos.:
   because they are dependent claims and are not drafted in accordance with the second and third sentences of Rule 6.4(a).

Form PCT/ISA/210 (continuation of first sheet) (July 2022)

# EP 4 566 673 A1

| INTERNATIONAL SEARCH REPORT | International application No. |
|---|---|
| Information on patent family members | PCT/CN2023/111149 |

| Patent document cited in search report | | | Publication date (day/month/year) | Patent family member(s) | | | Publication date (day/month/year) |
|---|---|---|---|---|---|---|---|
| CN | 113286634 | A | 20 August 2021 | CO | 2020014345 | A2 | 30 November 2020 |
| | | | | JP | 2021525080 | A | 24 September 2021 |
| | | | | JP | 7057843 | B2 | 20 April 2022 |
| | | | | JP | 2022106751 | A | 20 July 2022 |
| | | | | TW | 202003041 | A | 16 January 2020 |
| | | | | CA | 3100829 | A1 | 28 November 2019 |
| | | | | KR | 20210013165 | A | 03 February 2021 |
| | | | | WO | 2019224716 | A2 | 28 November 2019 |
| | | | | WO | 2019224716 | A3 | 05 March 2020 |
| | | | | WO | 2019224716 | A8 | 01 October 2020 |
| | | | | IL | 278924 | A | 31 January 2021 |
| | | | | EP | 3796983 | A2 | 31 March 2021 |
| | | | | PH | 12020500671 | A1 | 28 June 2021 |
| | | | | SG | 11202010934 | SA | 30 December 2020 |
| | | | | AU | 2019274655 | A1 | 26 November 2020 |
| | | | | AU | 2019274655 | B2 | 09 March 2023 |
| | | | | PE | 20210488 | A1 | 15 March 2021 |
| | | | | TW | 202308693 | A | 01 March 2023 |
| | | | | US | 2020010566 | A1 | 09 January 2020 |
| | | | | US | 11525010 | B2 | 13 December 2022 |
| | | | | AU | 2023202234 | A1 | 11 May 2023 |
| | | | | BR | 112020022595 | A2 | 09 February 2021 |
| | | | | MX | 2020012607 | A | 29 January 2021 |
| CN | 113801238 | A | 17 December 2021 | WO | 2021249462 | A1 | 16 December 2021 |
| | | | | JP | 2023514386 | A | 05 April 2023 |
| | | | | AU | 2021286676 | A1 | 08 September 2022 |
| | | | | US | 2023242661 | A1 | 03 August 2023 |
| | | | | EP | 4112721 | A1 | 04 January 2023 |
| | | | | KR | 20220166837 | A | 19 December 2022 |
| | | | | CA | 3171344 | A1 | 16 December 2021 |
| WO | 2022127871 | A1 | 23 June 2022 | IL | 303610 | A | 01 August 2023 |
| | | | | AU | 2021404641 | A1 | 29 June 2023 |
| | | | | CO | 2023009279 | A2 | 21 July 2023 |
| | | | | CA | 3205007 | A1 | 23 June 2022 |
| WO | 2010119704 | A1 | 21 October 2010 | DK | 2419447 | T3 | 25 September 2017 |
| | | | | US | 2012082667 | A1 | 05 April 2012 |
| | | | | US | 9090679 | B2 | 28 July 2015 |
| | | | | JP | 2012524023 | A | 11 October 2012 |
| | | | | JP | 5812418 | B2 | 11 November 2015 |
| | | | | EP | 2419447 | A1 | 22 February 2012 |
| | | | | EP | 2419447 | A4 | 26 February 2014 |
| | | | | EP | 2419447 | B1 | 23 August 2017 |
| | | | | ES | 2641612 | T3 | 10 November 2017 |
| CN | 110055224 | A | 26 July 2019 | US | 2022185858 | A1 | 16 June 2022 |
| | | | | WO | 2020200325 | A1 | 08 October 2020 |
| | | | | AU | 2020255735 | A1 | 14 October 2021 |
| CN | 110564693 | A | 13 December 2019 | | None | | |
| CN | 110257338 | A | 20 September 2019 | | None | | |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 1996027011 A1
- WO 1998050431 A2
- EP 1870459 A1
- WO 2007110205 A2
- WO 2009089004 A1
- WO 2010129304 A2
- WO 2011143545 A1
- WO 2012058768 A1
- WO 2013157954 A1
- WO 2013096291 A2
- US 5959177 A
- US 6040498 A
- US 6420548 B
- US 7125978 B
- US 6417429 B
- WO 2021139758 A1
- CN 202110527339
- WO 2020177733 A1
- WO 2019224716 A2
- WO 2014134311 A1
- CN 202210371538

### Non-patent literature cited in the description

- **MICHAELA KUHN**. *Physiological Reviews*, 2016, vol. 96 (2), 751-804
- **RUTH BIRBE MD.** *Human Pathology*, 2005, vol. 36 (2), 170-179
- **HADI DANAEE**. *PLoS One*, 2017, vol. 12 (12), e0189953
- **DIVYA MATHUR**. *Clinical Cancer Research*, 2020, vol. 26 (9), 2188-2202
- *J. Biol. Chem*, 1968, vol. 243, 3558
- **ARAN F. LABRIJN et al.** *Nature Reviews Drug Discovery*, 2019, vol. 18, 585-608
- **CHEN S1 et al.** *J Immunol Res.*, 11 February 2019, vol. 2019, 4516041
- **KABAT et al.** Sequences of Proteins of Immunological Interest. National Institutes of Health, 1991
- **MARTIN**. Protein Sequence and Structure Analysis of Antibody Variable Domains[J. *ACR*, 2001
- **LEFRANC, M.P. et al.** *Dev. Comp. Immunol.*, 2003, vol. 27, 55-77
- *Front Immunol.*, 16 October 2018, vol. 9, 2278
- *Meth. Mol. Biol*, 2004, vol. 248, 443-463
- *Prot. Sci.*, 2000, vol. 9, 487-496
- **HARLOW** ; **LANE**. Antibodies. Cold Spring Harbor Press
- **YAZAKI, P** ; **WU, A.M.** Methods in Molecular Biology. Humana Press, 2004, vol. 248, 255-268
- Antibodies: A Laboratory Manual and Molecular Cloning: A Laboratory Manual. Cold Spring Harbor Laboratory